# EUROPEAN PATENT APPLICATION

(11) **EP 4 494 655 A1**
(43) Date of publication of application: **22.01.2025**
(21) Application number: 23770834.2
(22) Date of filing: 15.03.2023
(51) Int. Cl.: A61K 39/395, A61K 45/06, A61P 35/00, A61P 35/02, C07K 16/30, C07K 16/46, C12N 15/13, C12P 21/08, A61K 31/337, A61K 31/47

(54) **COMBINATION OF MULTI-SPECIFIC MOLECULE AND IMMUNE CHECKPOINT INHIBITOR**

(30) Priority: 16.03.2022 JP 2022041253
(71) Applicant: Daiichi Sankyo Company, Limited, Tokyo 103-8426 (JP)
(72) Inventor: ICHIKAWA, Junya, Tokyo 103-8426 (JP); MATSUI YATSU, Ayaka, Tokyo 103-8426 (JP)
(74) Representative: Marks & Clerk LLP
(86) International application number: PCT/JP2023/010071
(87) International publication number: WO 2023/176881

(57) **Abstract**

Provided is a novel combination of medicaments for the treatment of a cancer. The present invention provides a pharmaceutical composition for the treatment and/or prevention of a cancer, comprising amultispecific antibody or antigen-binding fragment thereof [i], the pharmaceutical composition being used in combination with compound [ii] which is an immune checkpoint inhibitor or a chemotherapeutic.

## Description

### Technical Field

The present invention relates to a pharmaceutical composition comprising a multispecific molecule, the pharmaceutical composition being used in combination with an immune checkpoint inhibitor or the like, and a method of treatment or prevention etc.

### Background Art

In the treatment of cancers, a cure or a lifeprolonging effect may be achieved by the surgical removal of a tumor or the killing of cancer cells using an anticancer agent or radiation. However, the treatment of many cancers is attempted using a combination of an anticancer agent and various other drugs because monotherapy is not sufficiently effective. Examples of anticancer agents include chemotherapeutics such as doxorubicin, carboplatin, taxol, and camptothecin, molecular targeted agents such as imatinib, crizotinib, dasatinib and lapatinib, and therapeutic anticancer antibodies such as trastuzumab, bevacizumab, cetuximab, and ramucirumab. The anticancer agents and combination therapy using anticancer agents are determined depending on the type or degree of progression of a cancer and a treatment status.

Immune checkpoint inhibitors, which have become the standard of care treatment in recent years, are drugs that inhibit suppressor molecules of the immune system (immune checkpoints) and activate antitumor immunity (Non-Patent Literatures 1 to 3). For example, anti-PD-1 antibodies nivolumab (Patent Literature 1) and pembrolizumab (Patent Literature 2), anti-PD-L1 antibodies atezolizumab (Patent Literature 3), durvalumab (Patent Literature 4), and avelumab (Patent Literature 5), anti-CTLA-4 antibodies ipilimumab (Patent Literature 6), tremelimumab (Patent Literature 7), spartalizumab (Patent Literature 8), and cemiplimab (Patent Literature 9), and anti-TIGIT antibodies tiragolumab (Patent Literature 10) and vibostolimab (Patent Literature 11) are known immune checkpoint inhibitors. Immune checkpoint inhibitors, in combination with a chemotherapeutic agent, have been found to have a lifeprolonging effect in some cases (Non-Patent Literature 4). Further, some studies have reported a combinatorial effect of a bispecific antibody targeting a cancer antigen and CD3 (Non-Patent Literatures 5 and 6).

However, a drug that has an enhanced anticancer activity when used in combination with a bispecific antibody comprised of an anti-HLA-A2/NY-ESO antibody and an anti-CD3 antibody (Patent Literature 12) is essentially unknown.

### Citation List

### Patent Literature

Patent Literature 1: International Publication No. WO 2006/121168
Patent Literature 2: International Publication No. WO 2008/156712
Patent Literature 3: International Publication No. WO 2010/077634
Patent Literature 4: International Publication No. WO 2011/066389
Patent Literature 5: International Publication No. WO 2013/079174
Patent Literature 6: International Publication No. WO 2001/014424
Patent Literature 7: International Publication No. WO 2000/037504
Patent Literature 8: International Publication No. WO 2015/112900
Patent Literature 9: International Publication No. WO 2015/196051
Patent Literature 10:International Publication No. WO 2017/053748
Patent Literature 11:International Publication No. WO 2016/028656
Patent Literature 12:International Publication No. WO 2021/200857

### Non-Patent Literature

Non-Patent Literature 1: Menon S., et al., Cancers (2016) 8, 106.
Non-Patent Literature 2: Pardoll DM., Nat Rev Cancer (2012) 12, 252-264.
Non-Patent Literature 3: Wolchok JD., Cell (2015) 162, 937.
Non-Patent Literature 4: Motzer, R., et al., New England Journal of Medicine. (2021) 384: 1289-300.
Non-Patent Literature 5: Deegen P., et al., Clincal Cancer Research. (2021) 27: 2928-37.
Non-Patent Literature 6: Tebernero J., et al., Journal of Clinical Oncology. (2017) 35: 15_suppl, 3002-3002.

### Summary of Invention

### Technical Problem

An object of the present invention is to provide, for example, a combination of a multispecific antibody comprised of an anti-HLA-A2/NY-ESO antibody and an anti-CD3 antibody, and another agent, and the antibody or a pharmaceutical composition comprising the antibody, which is to be used in combination with another agent.

Another object of the present invention is to provide a multispecific antibody comprised of an anti-HLA-A2/NY-ESO antibody and an anti-CD3 antibody, for use in the treatment or prevention of a cancer, or a pharmaceutical composition comprising the multispecific antibody, which is to be used in combination with another agent in the treatment or prevention of a cancer.

A further alternative aspect of the present invention is to provide a method for treating or preventing a cancer by administering the multispecific antibody comprised of an anti-HLA-A2/NY-ESO antibody and an anti-CD3 antibody, or the pharmaceutical composition in combination with another agent .

### Solution to Problem

The inventors have conducted studies to attain the above objects and have consequently completed the present invention which is predicated on the finding that a bispecific antibody comprised of an anti-HLA-A2/NY-ESO antibody and an anti-CD3 antibody having anticancer activity produces an excellent antitumor effect when used in combination with various anticancer agents (e.g., chemotherapeutics such as carboplatin, paclitaxel, and Nab paclitaxel, and immune checkpoint inhibitors such as pembrolizumab).

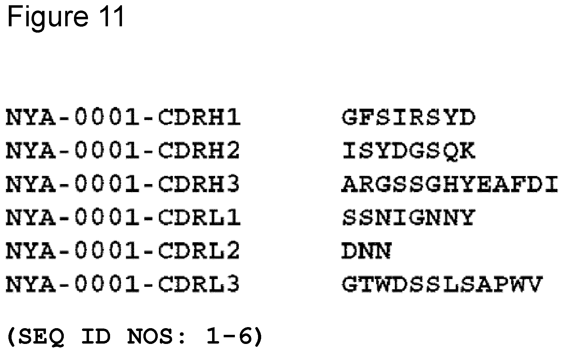

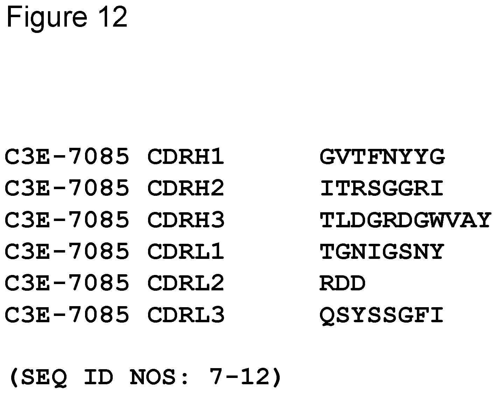

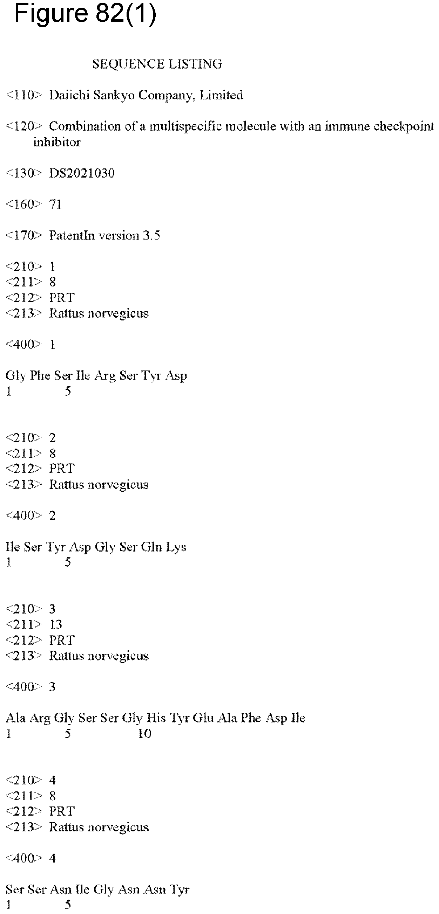

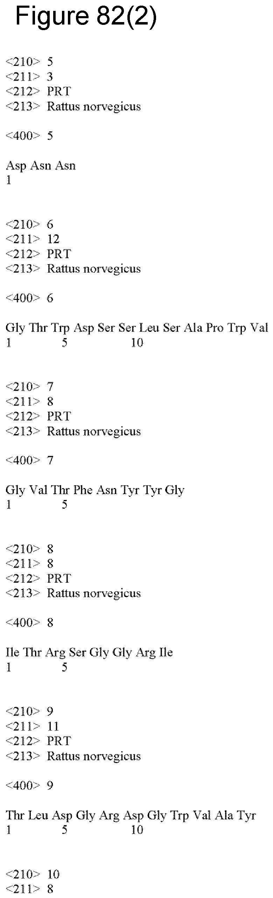

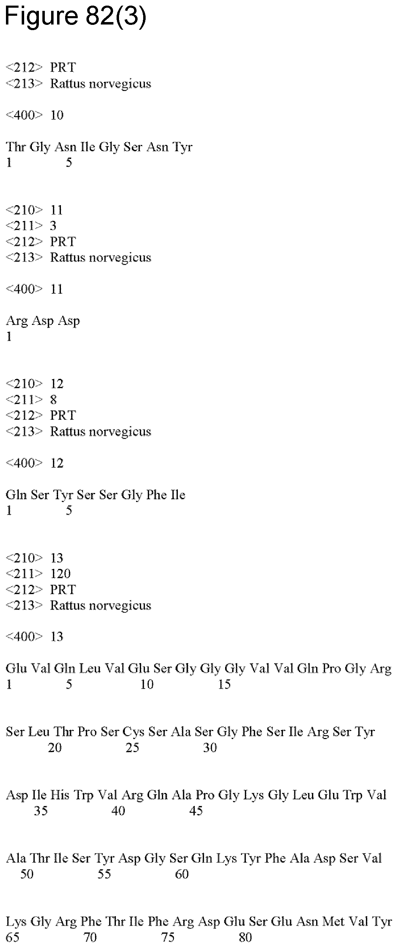

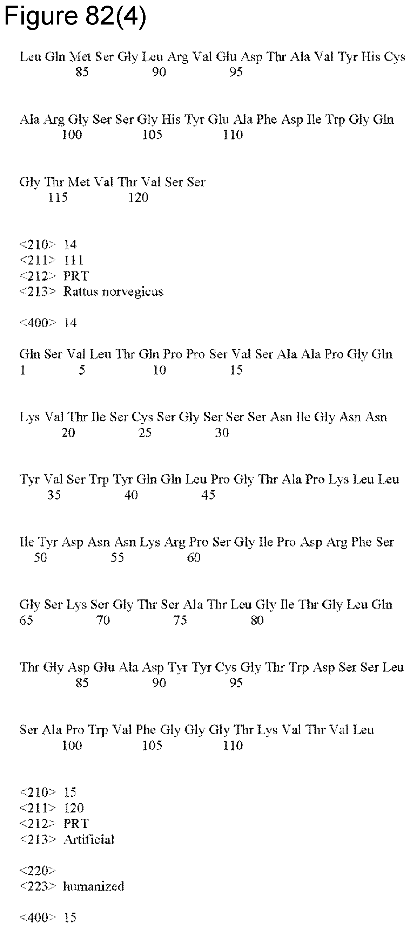

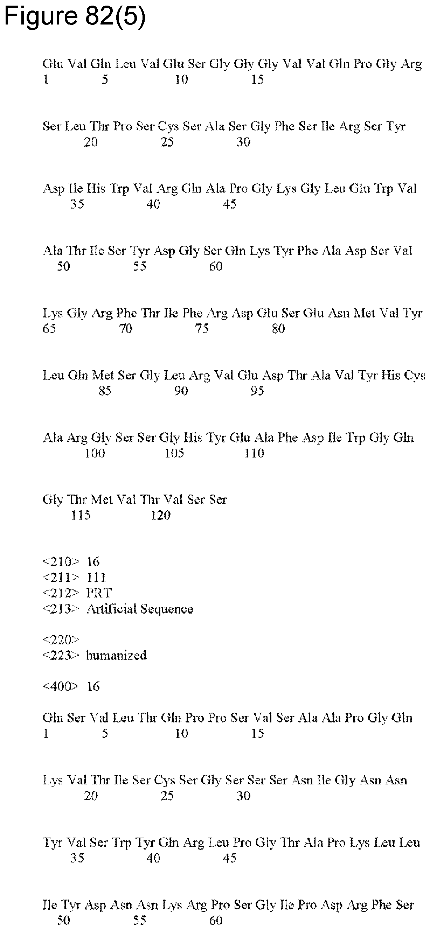

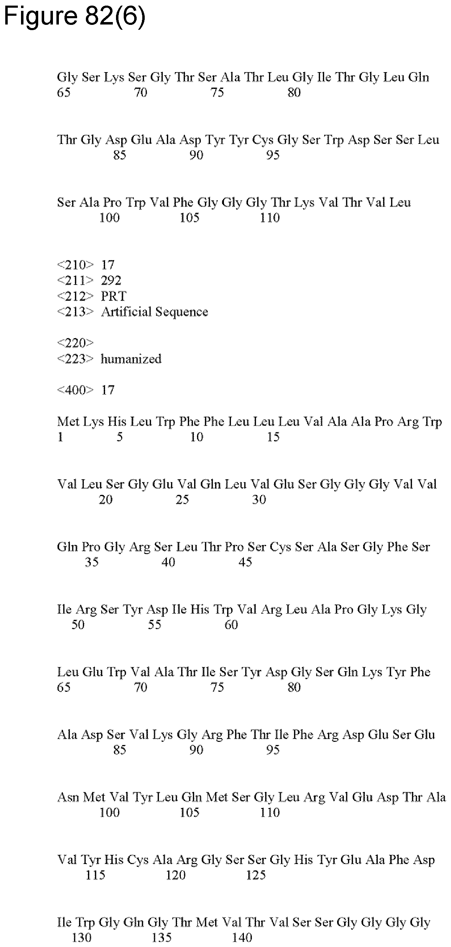

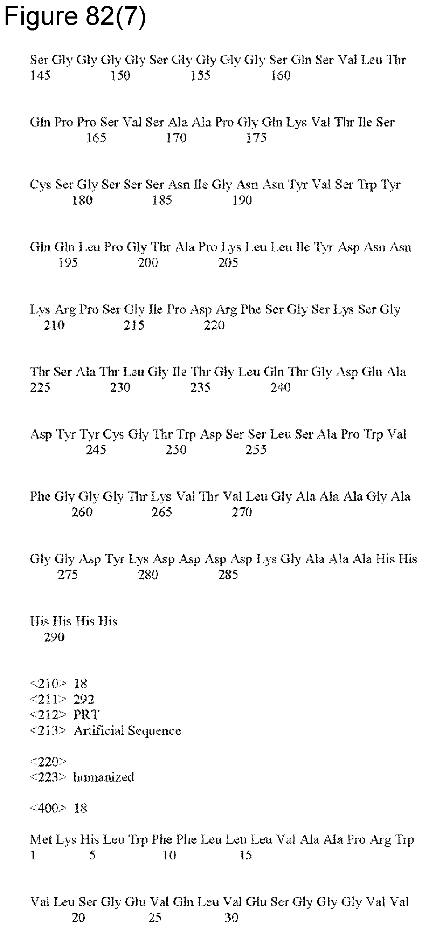

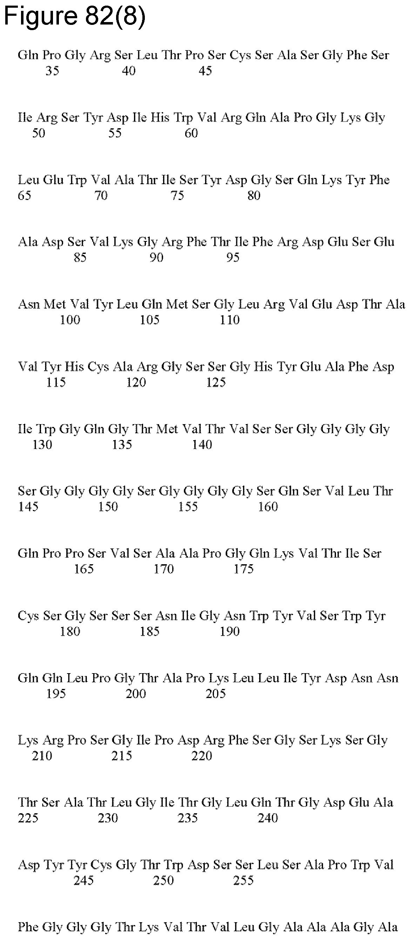

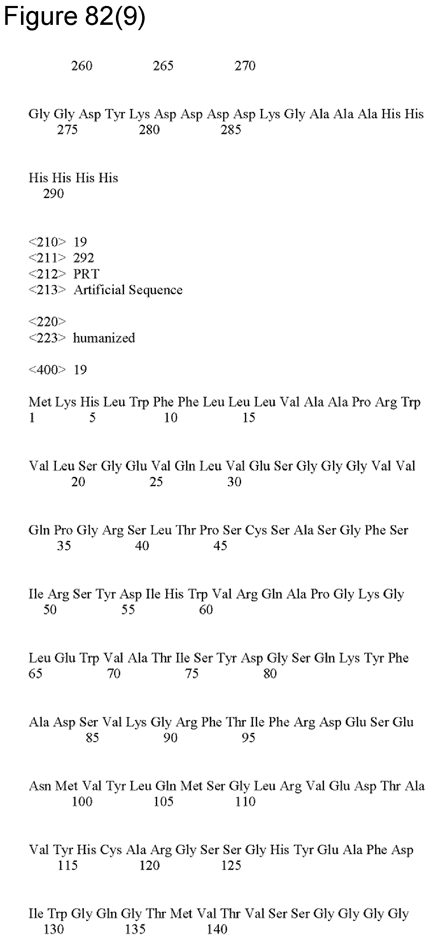

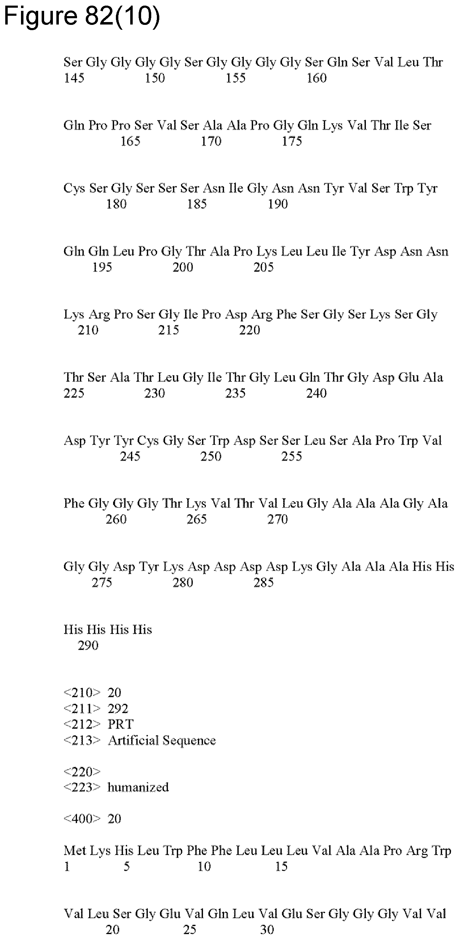

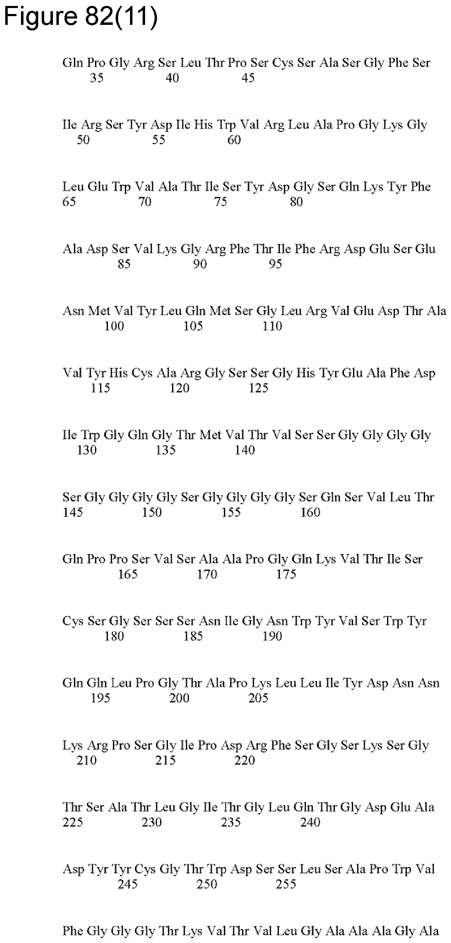

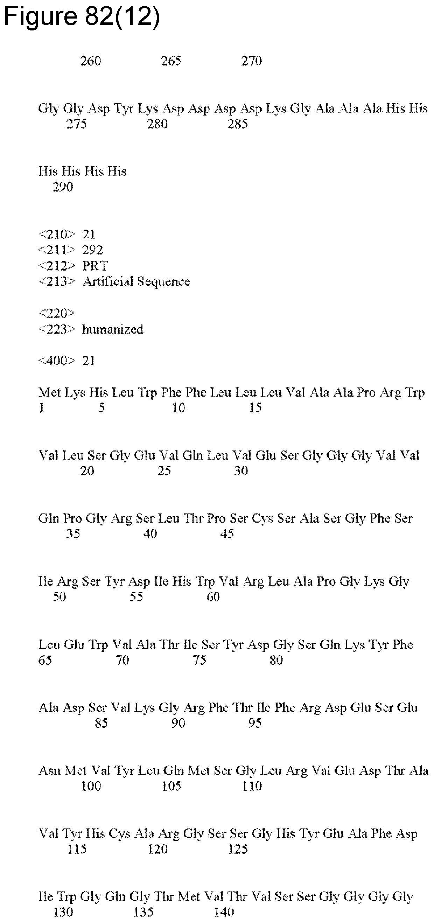

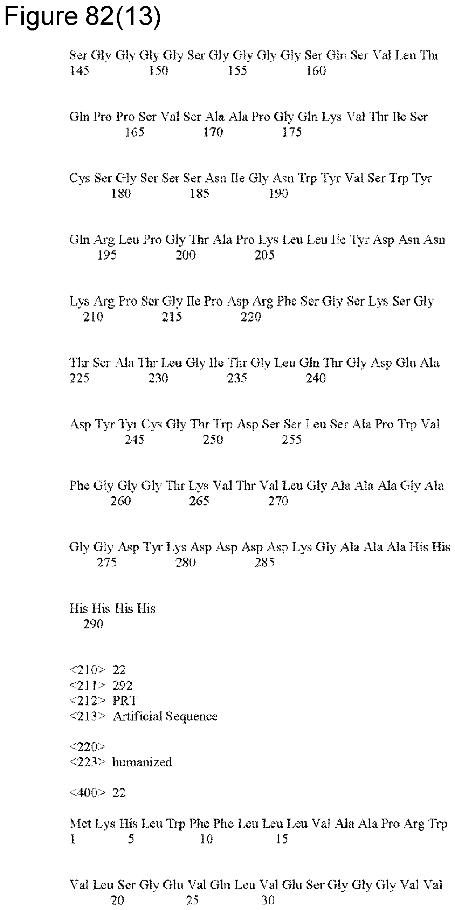

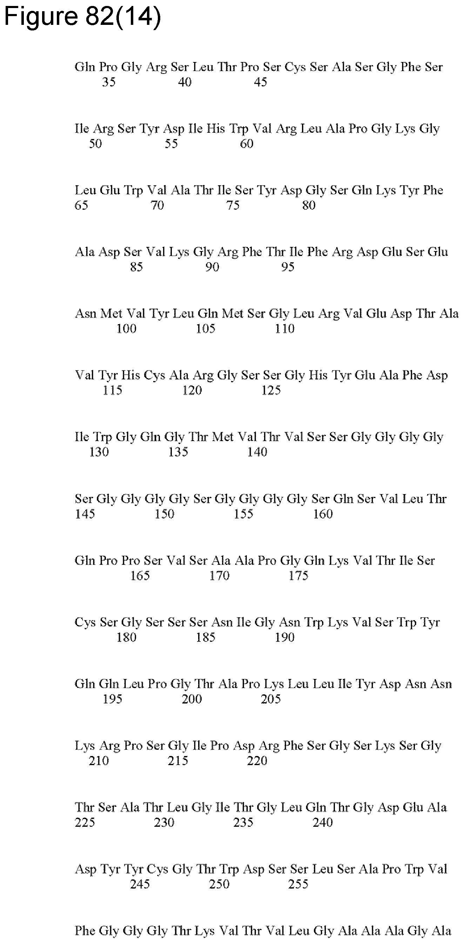

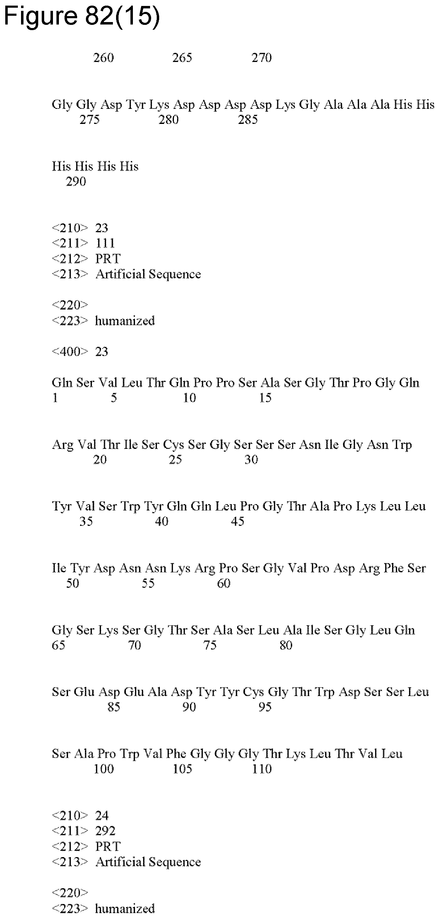

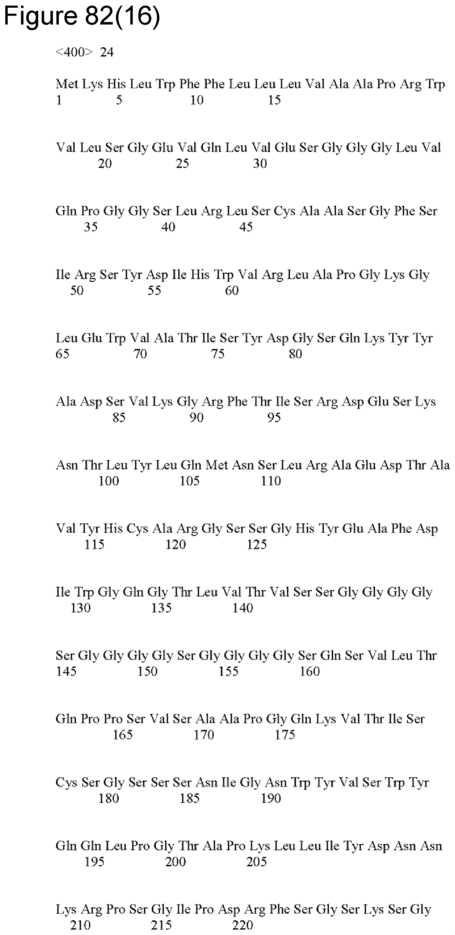

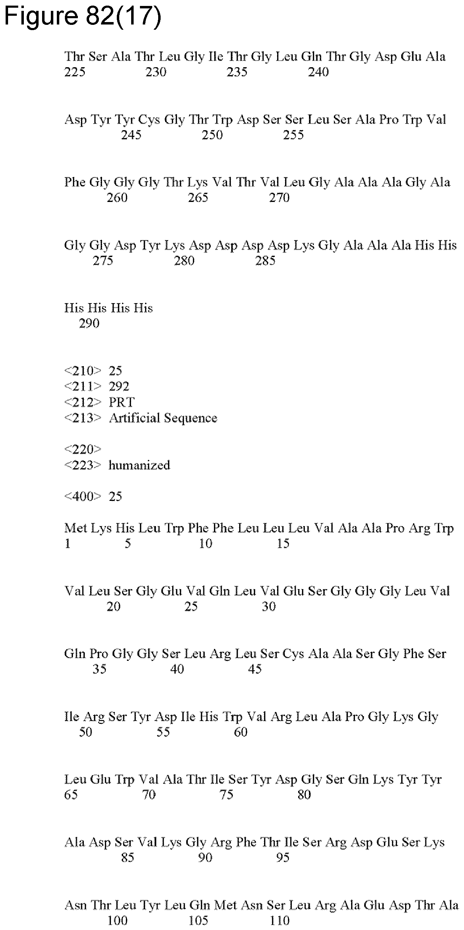

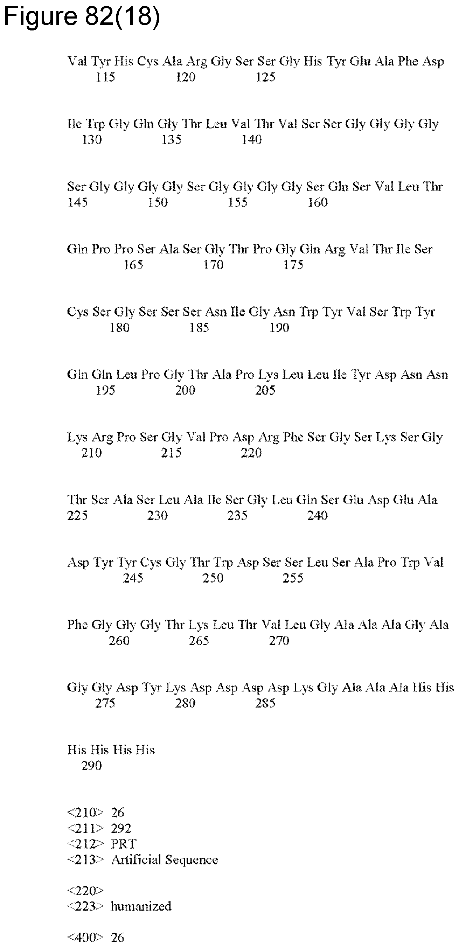

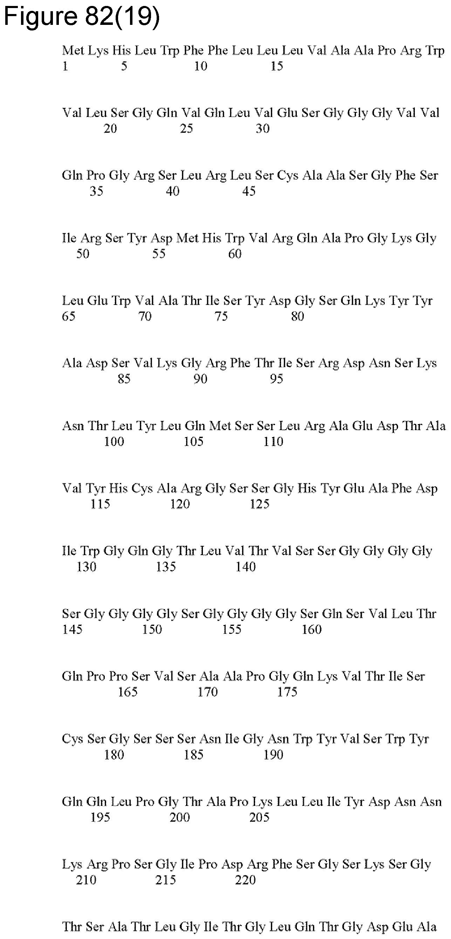

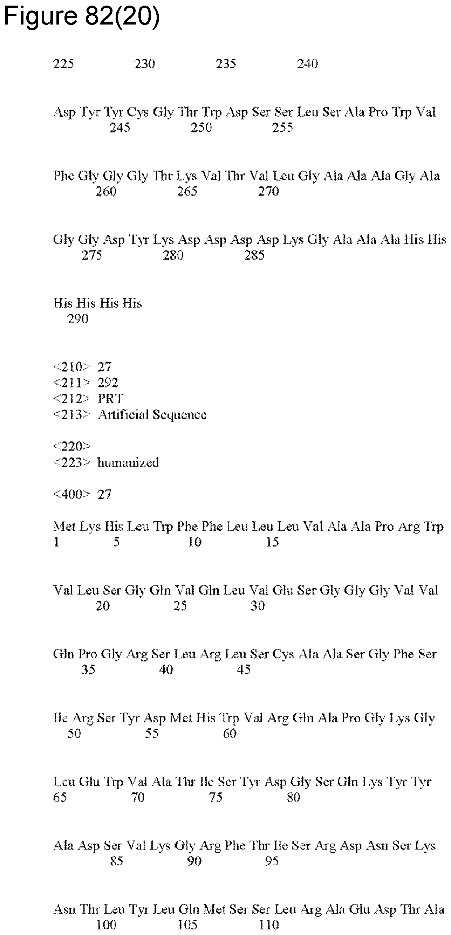

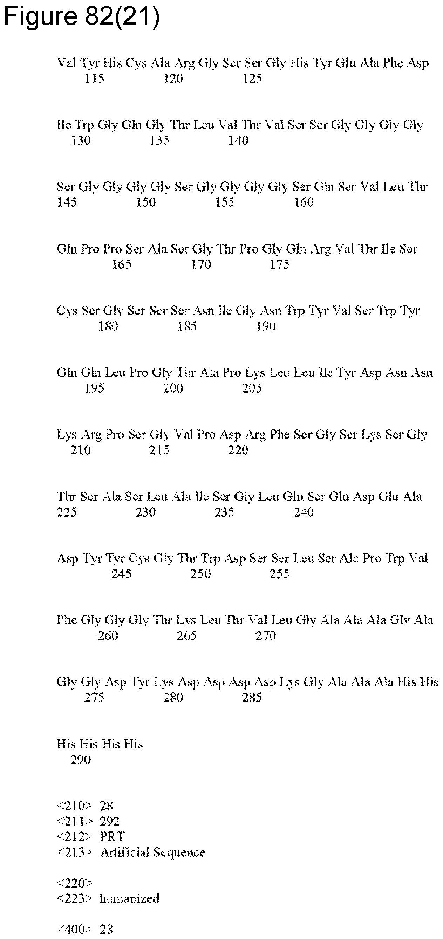

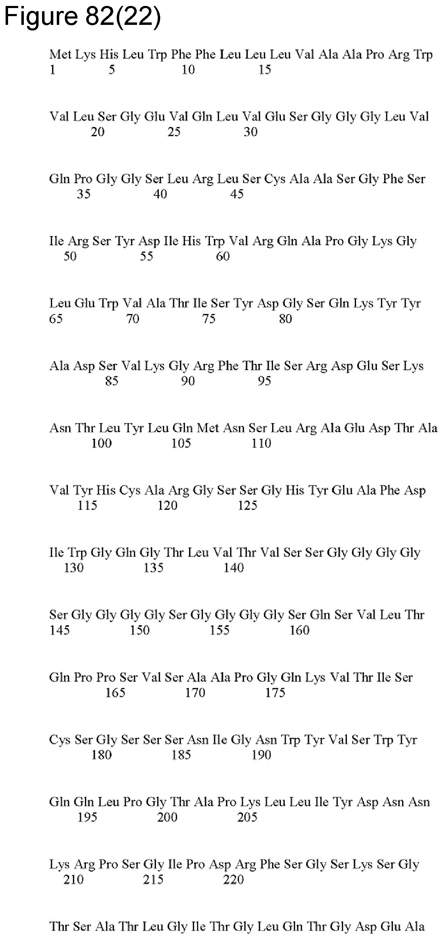

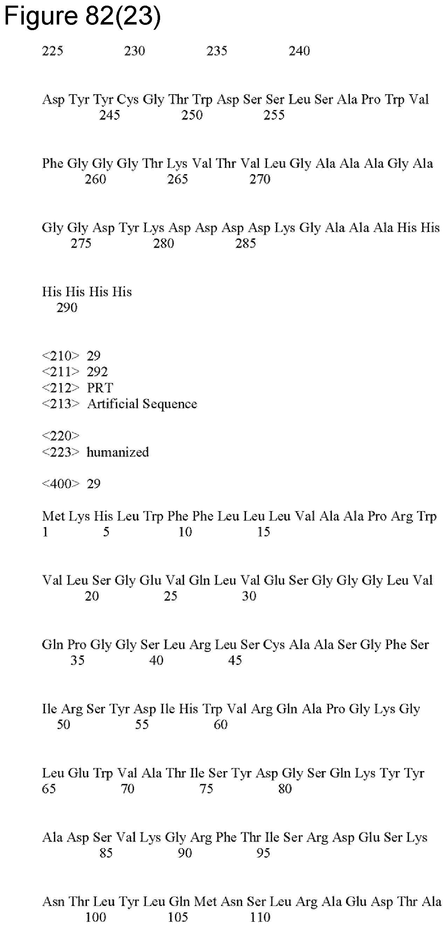

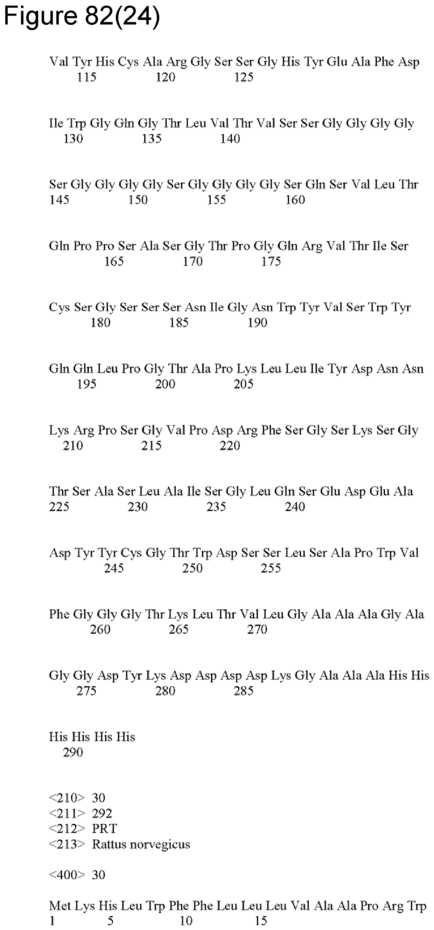

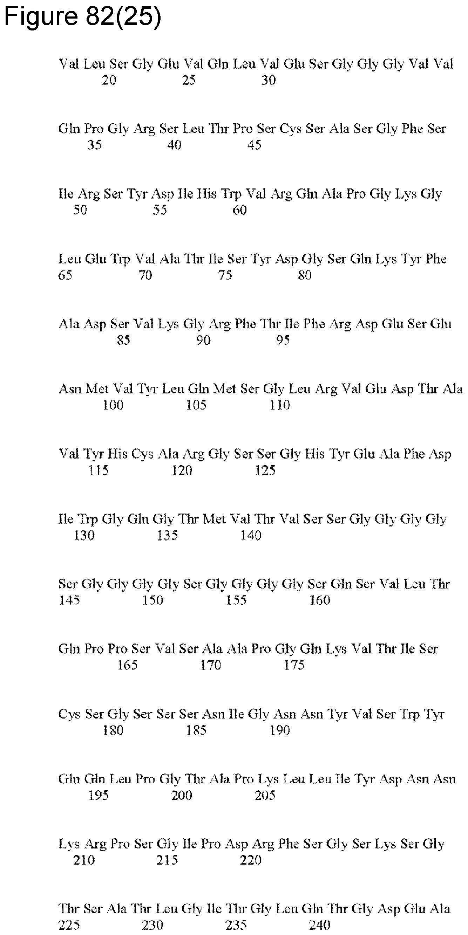

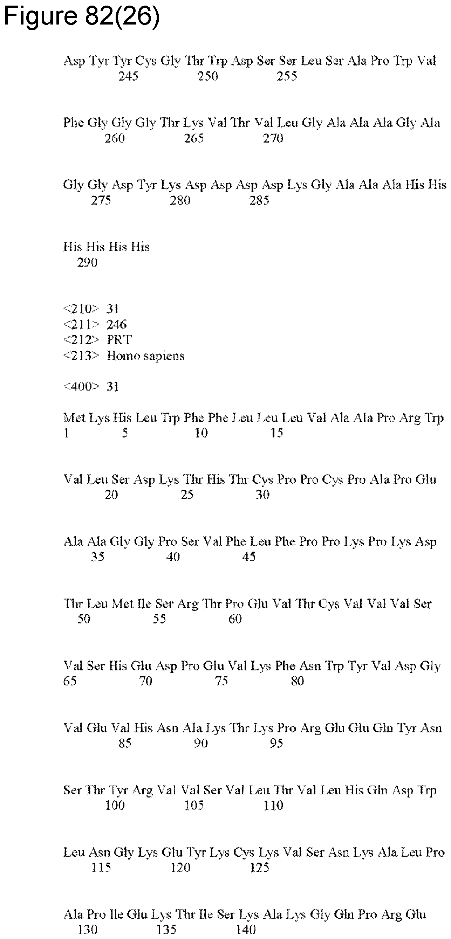

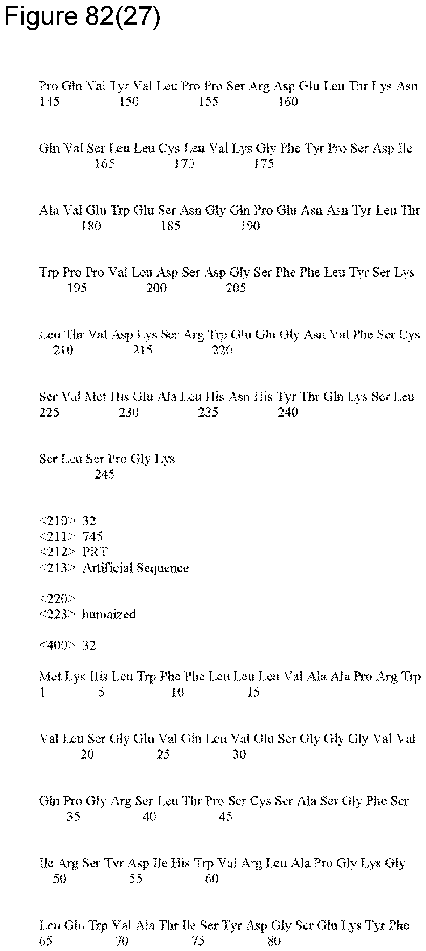

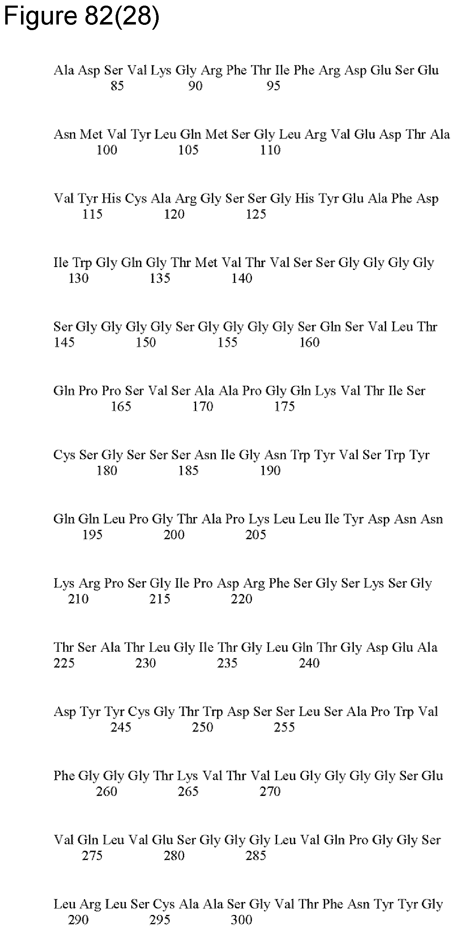

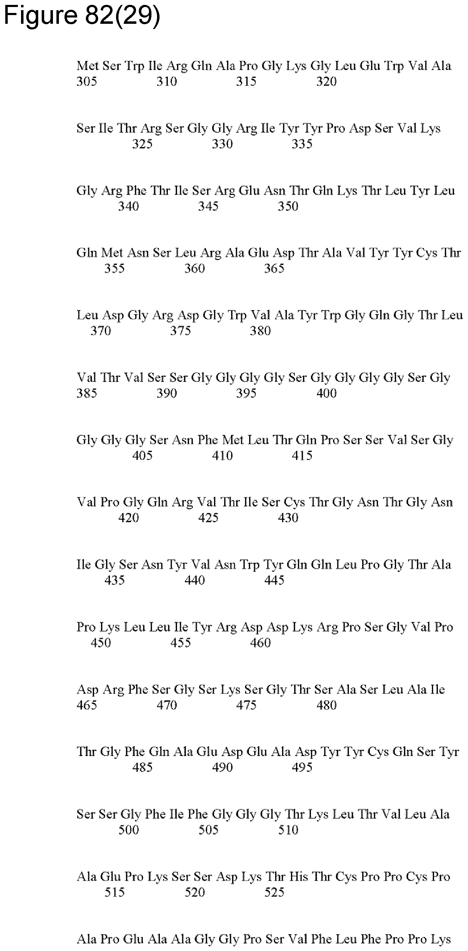

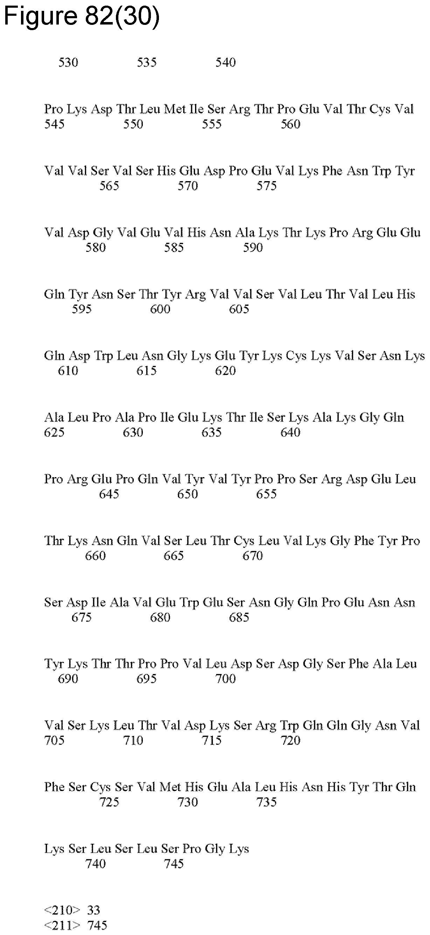

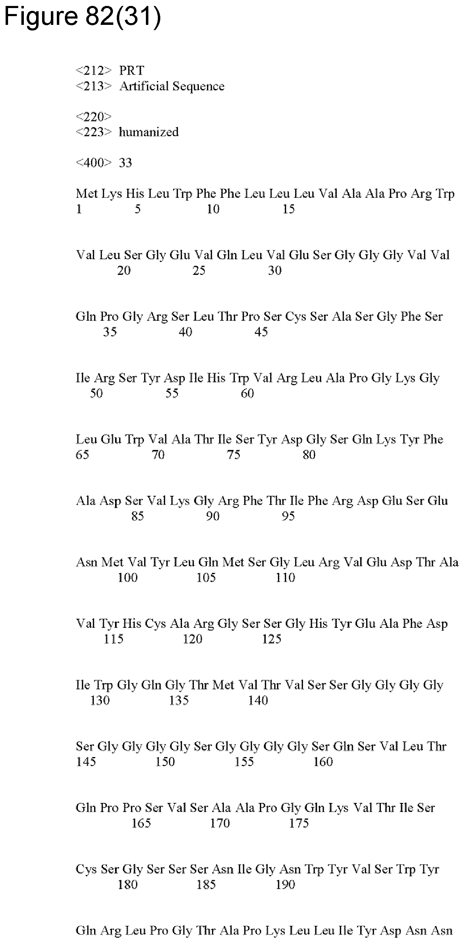

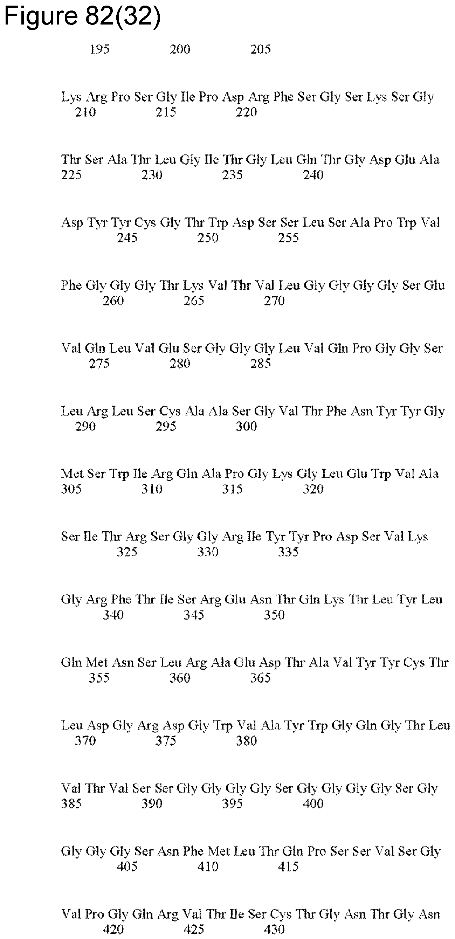

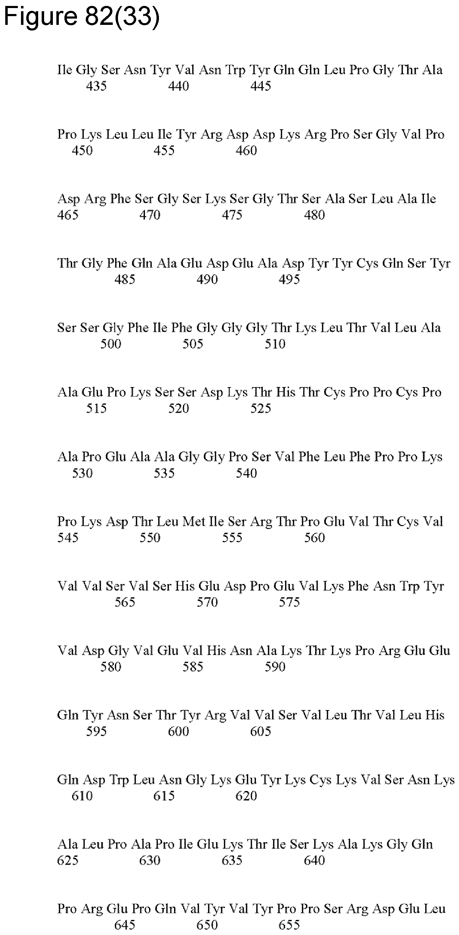

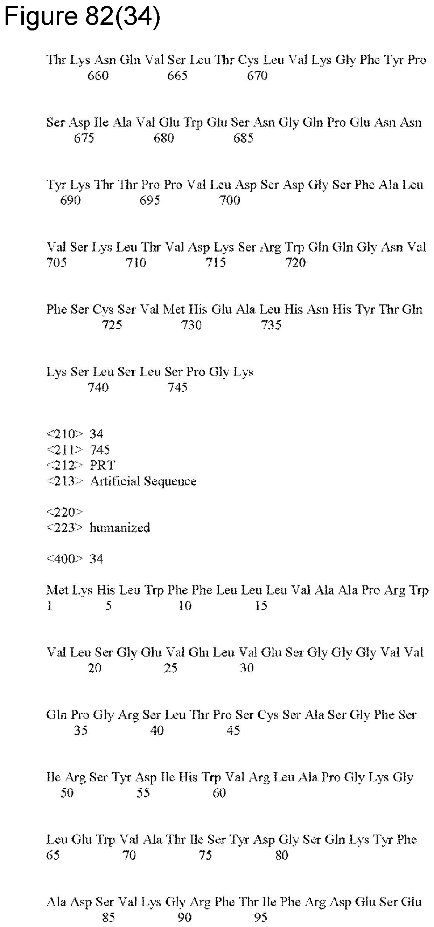

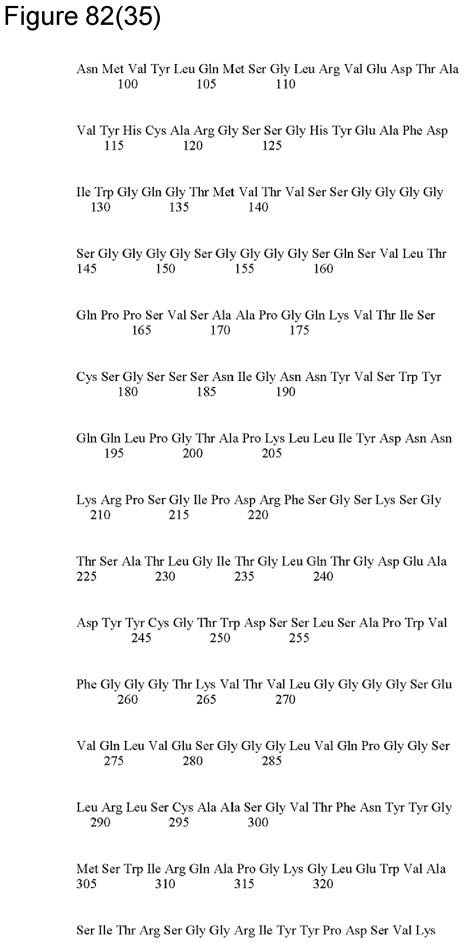

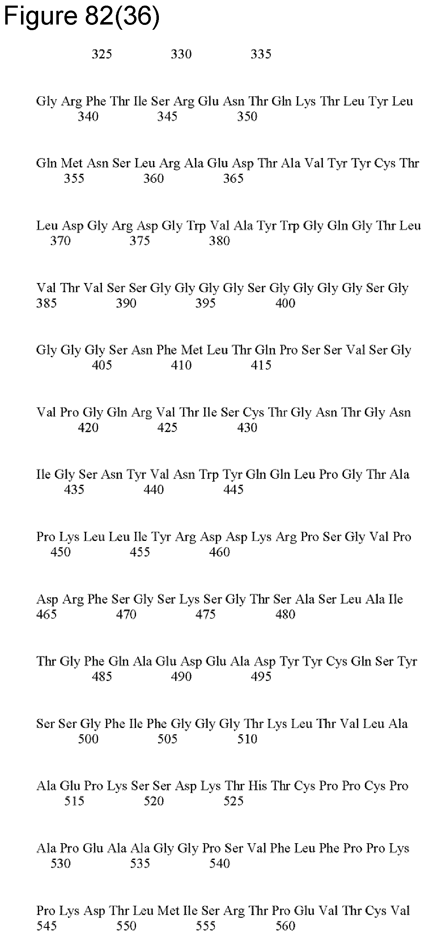

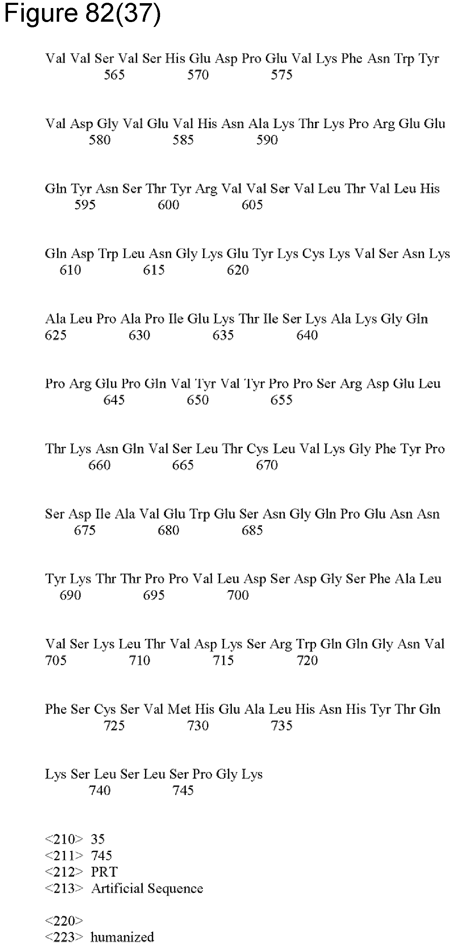

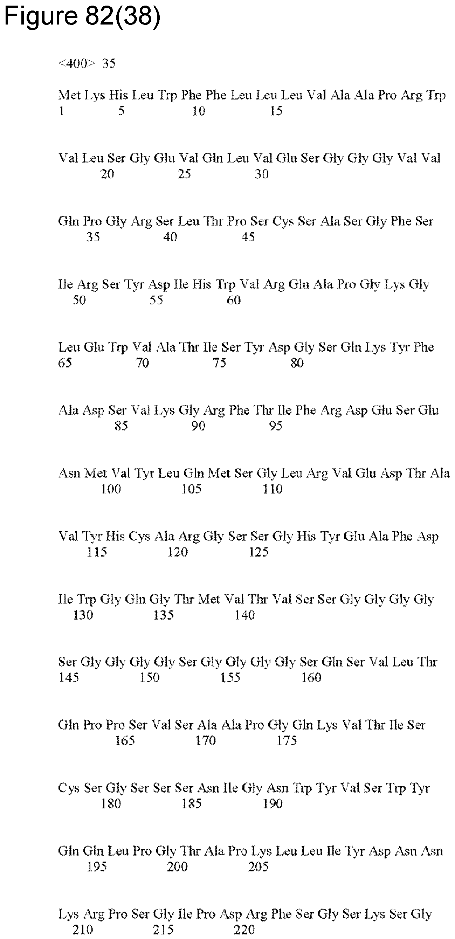

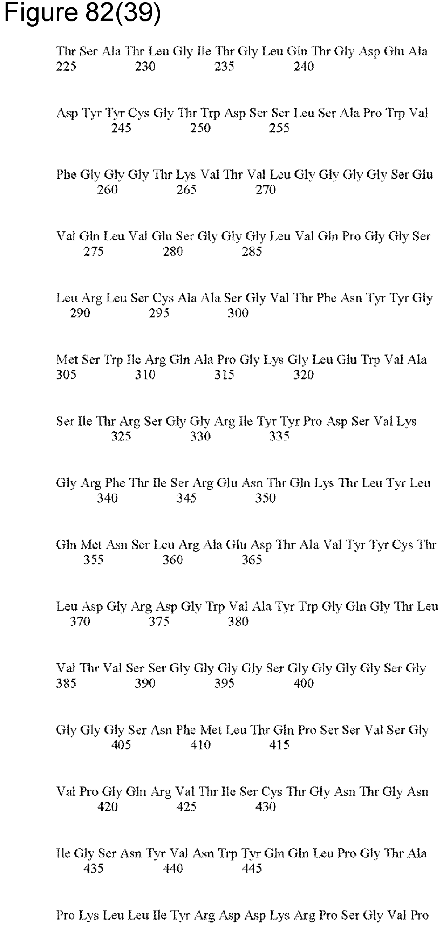

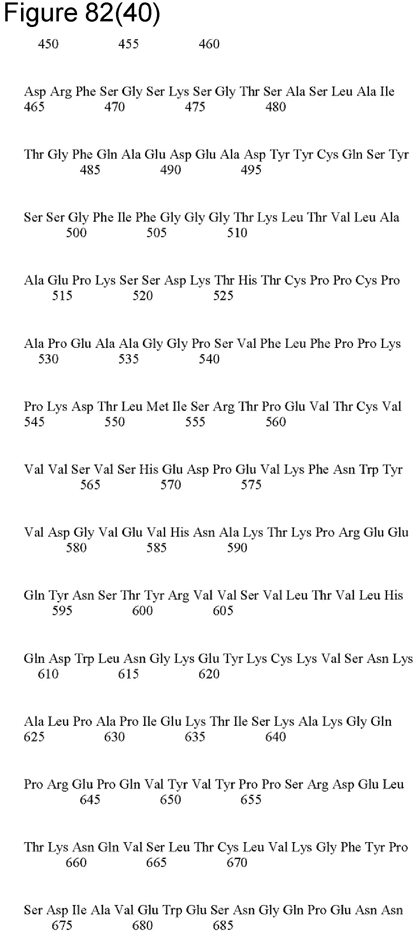

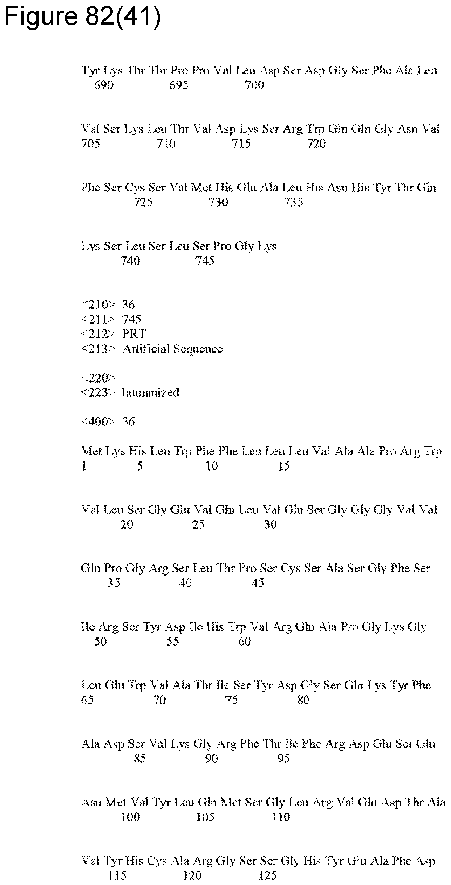

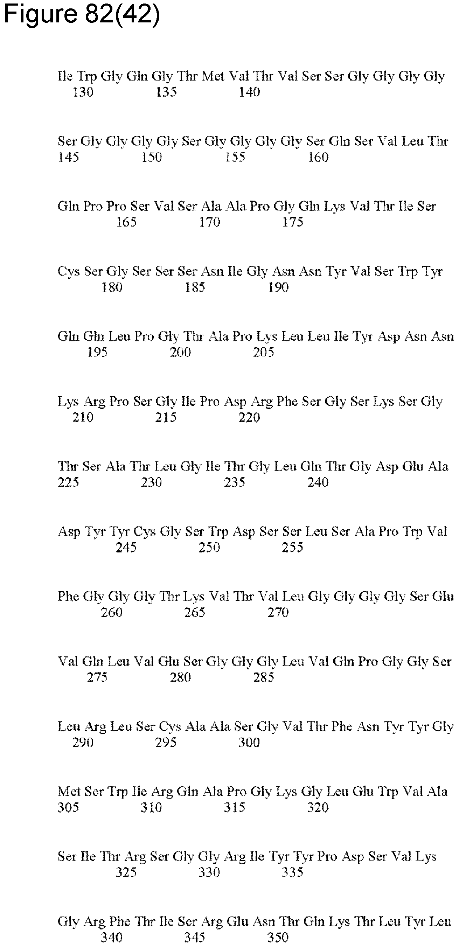

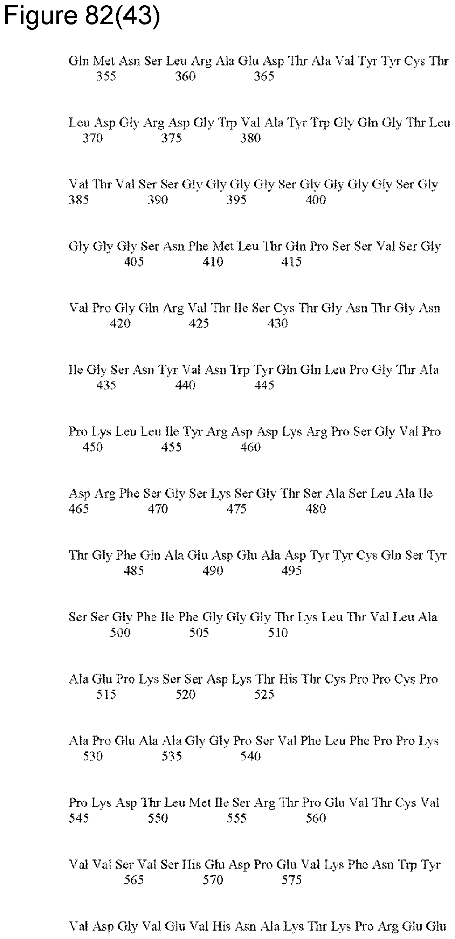

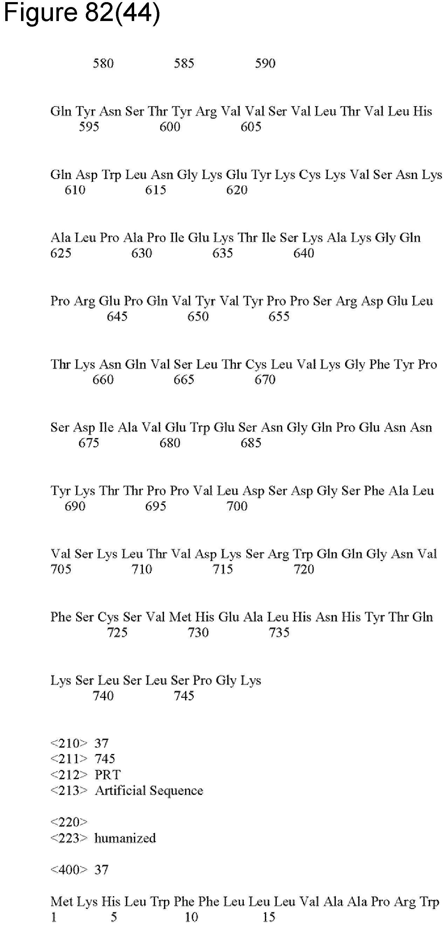

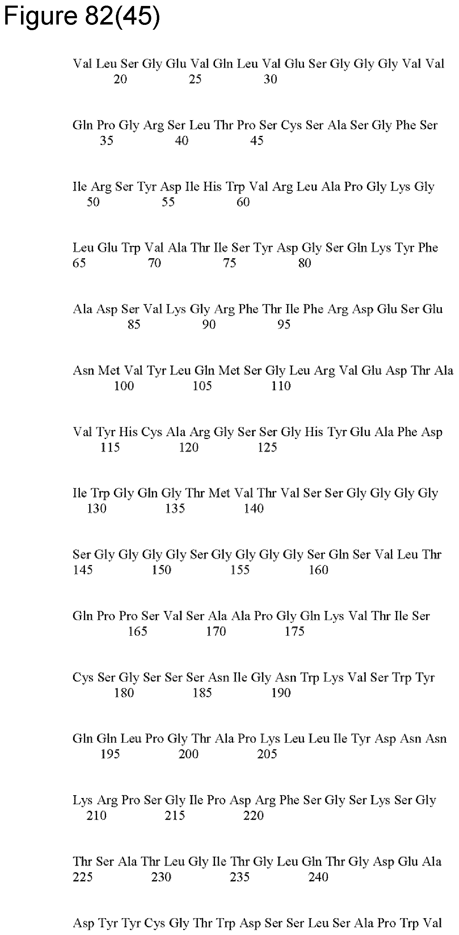

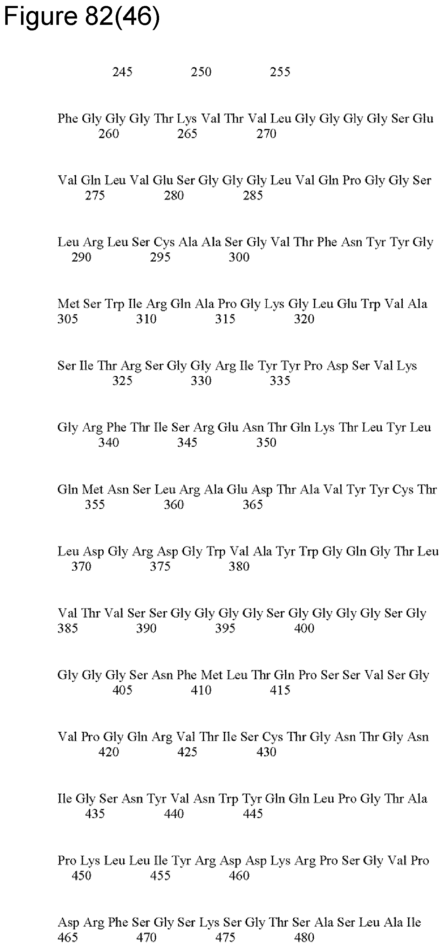

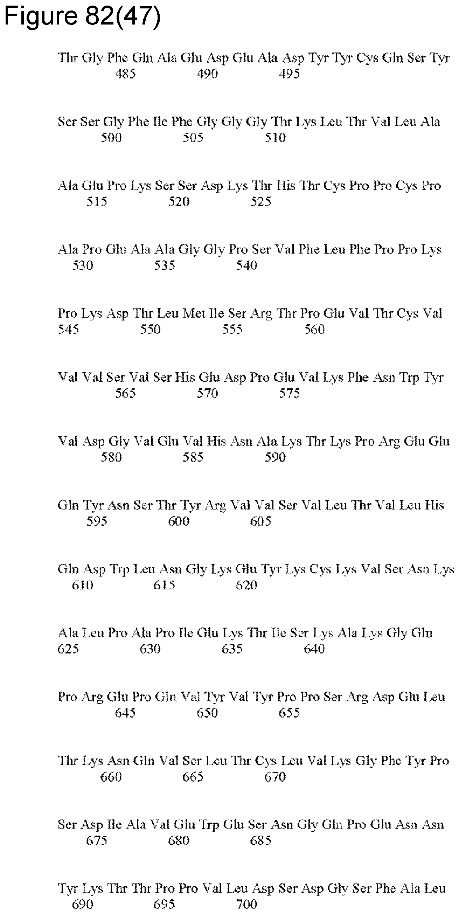

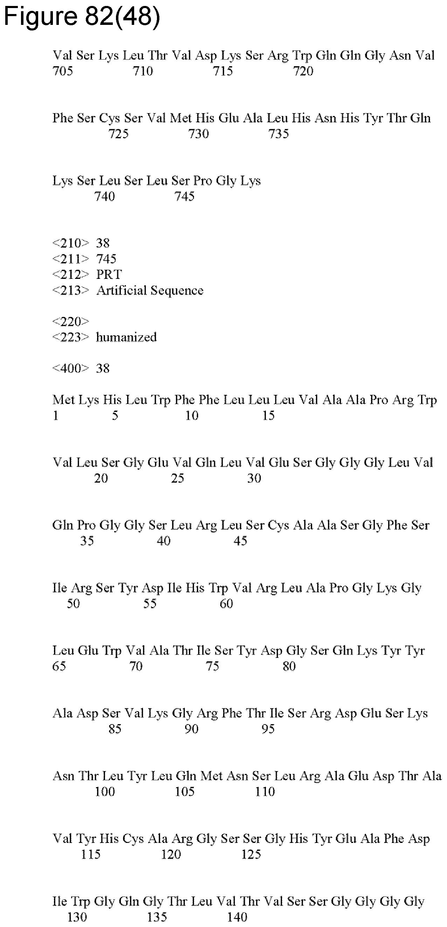

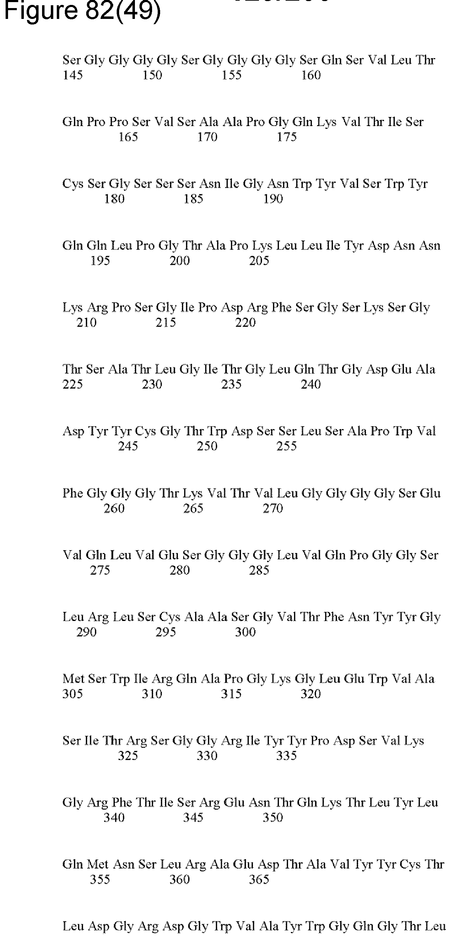

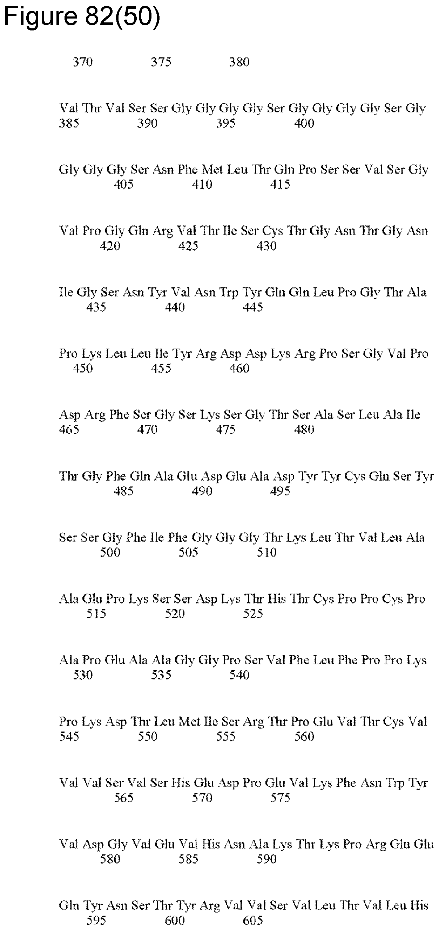

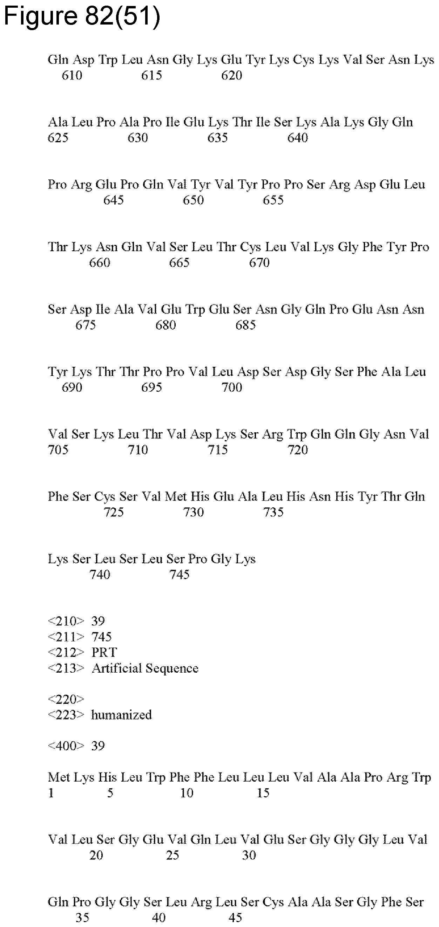

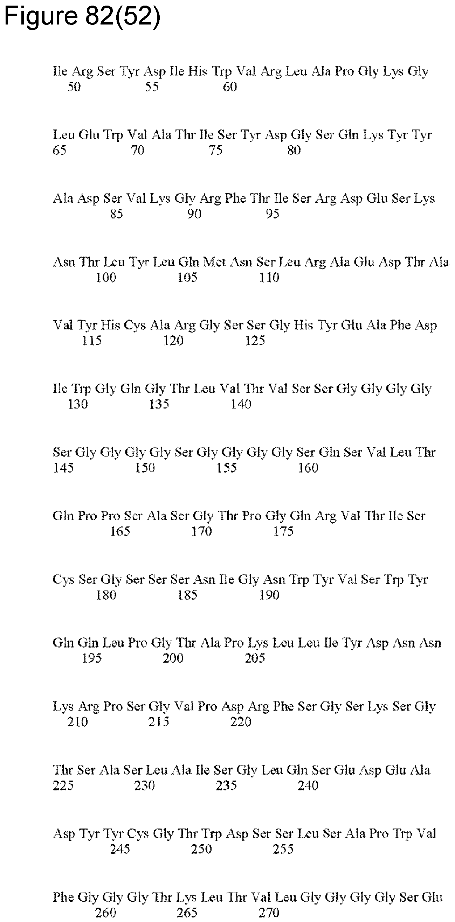

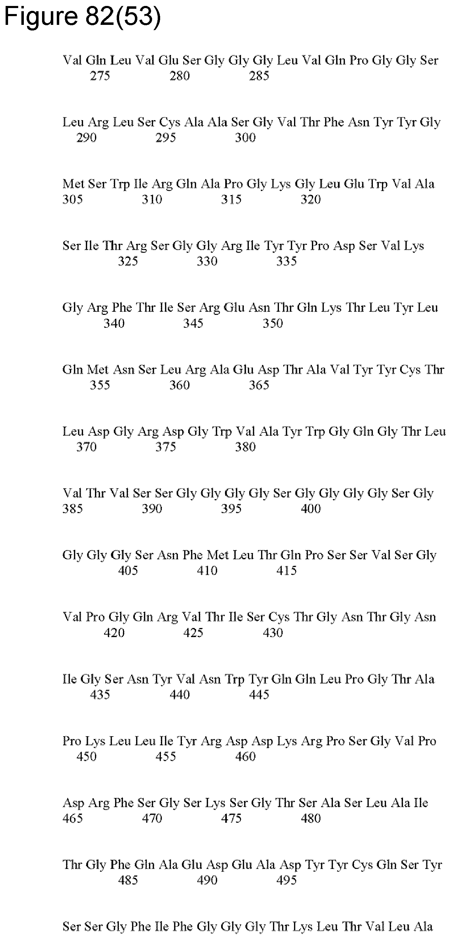

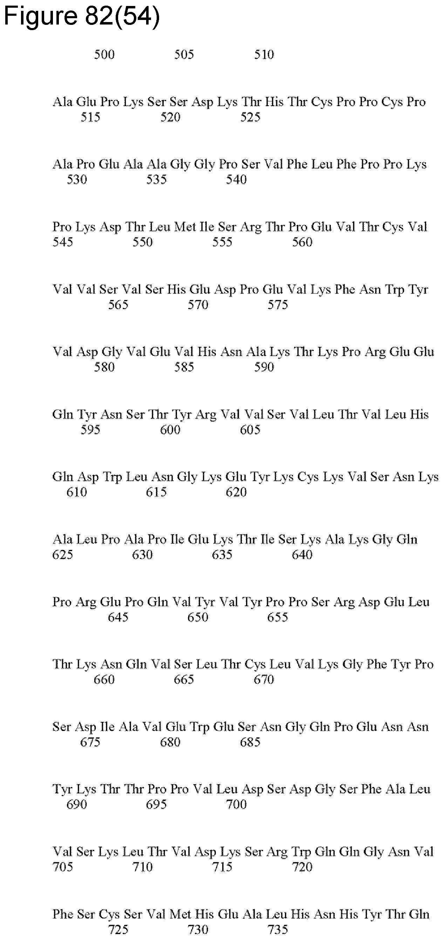

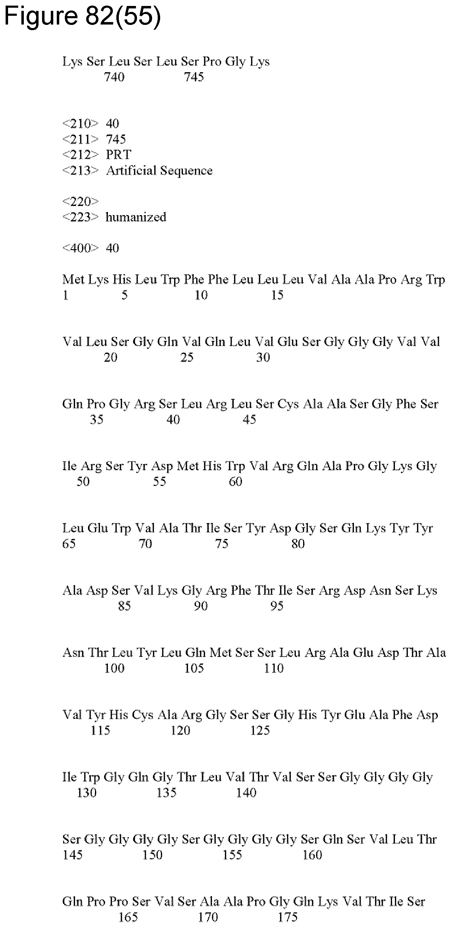

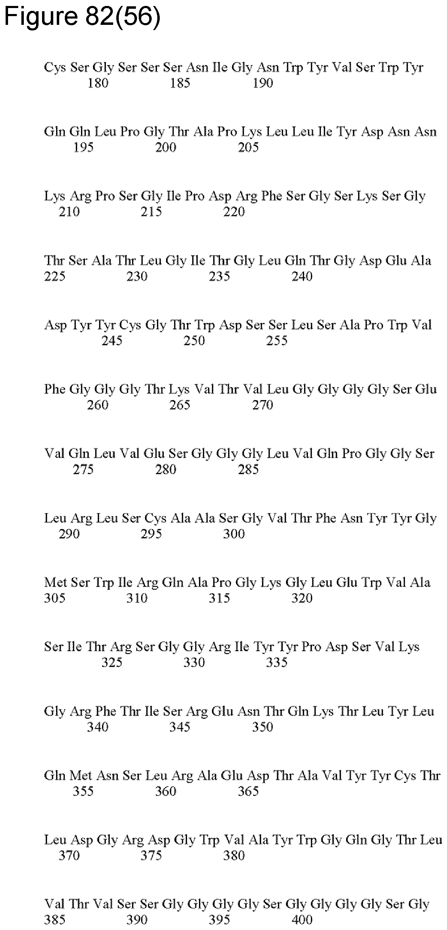

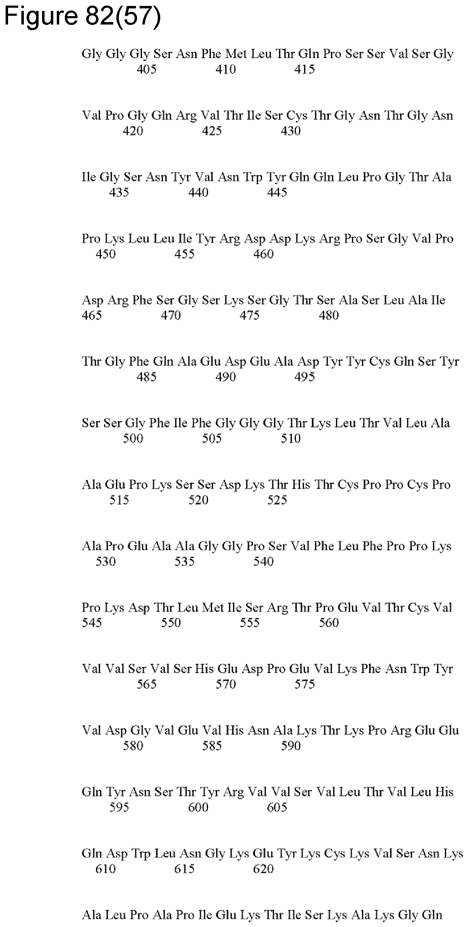

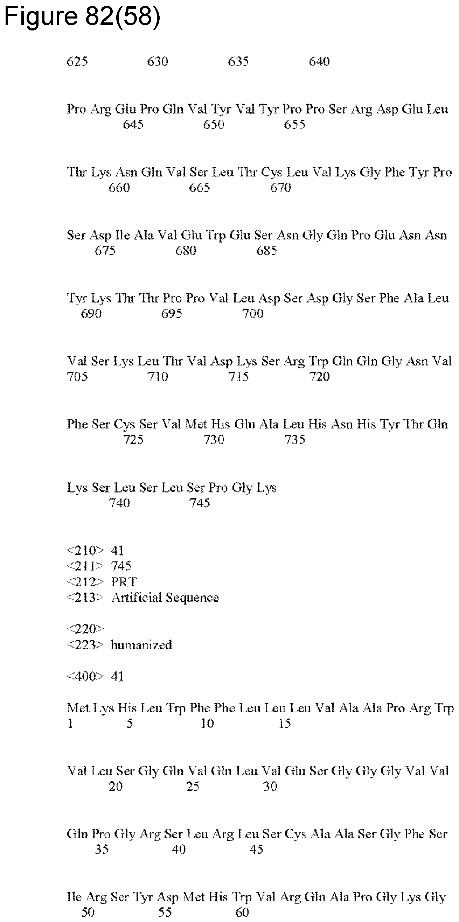

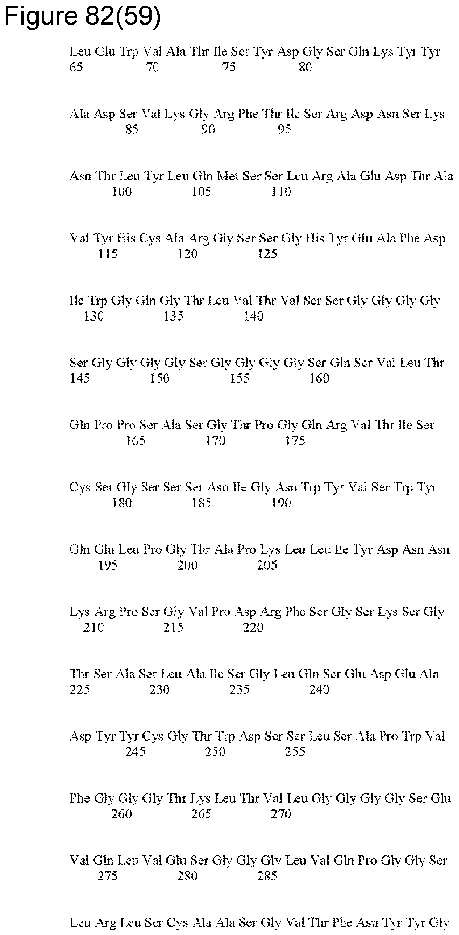

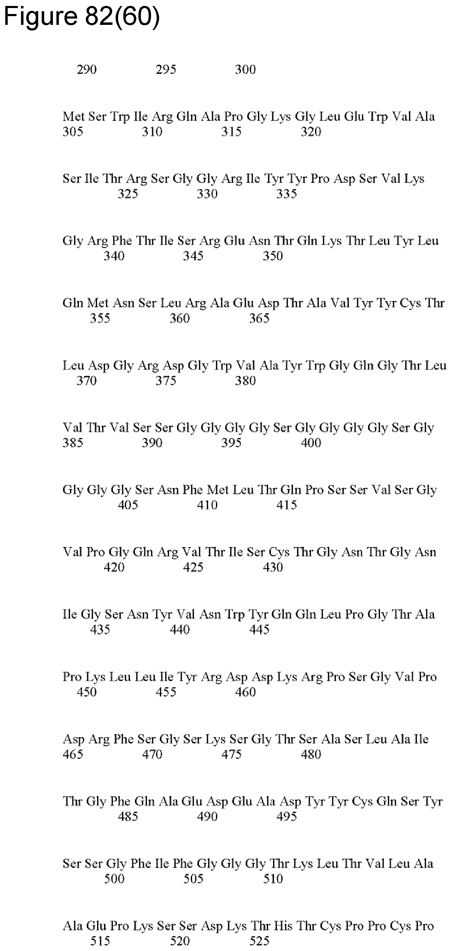

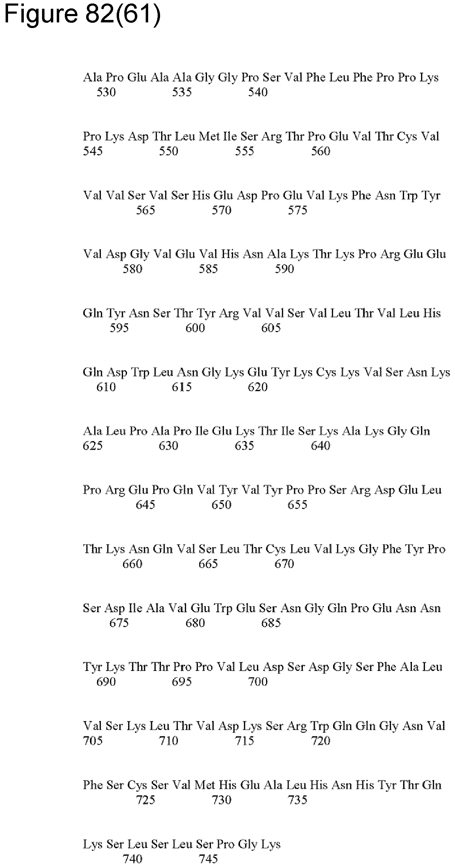

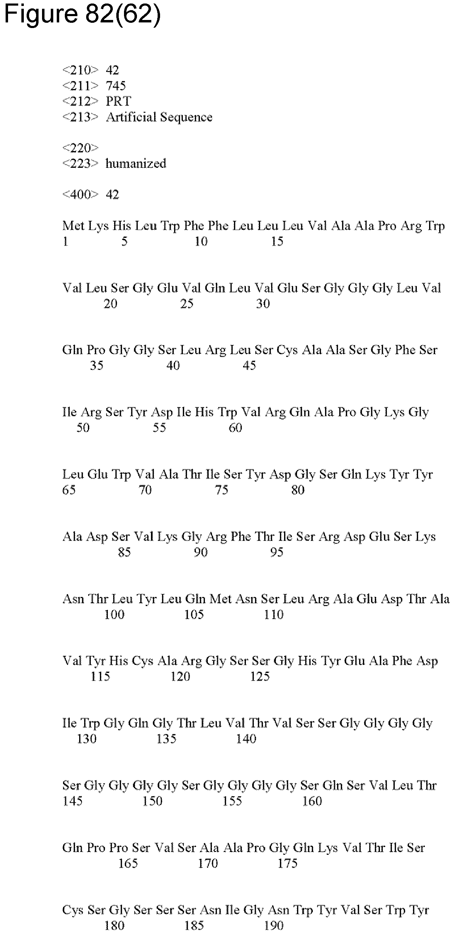

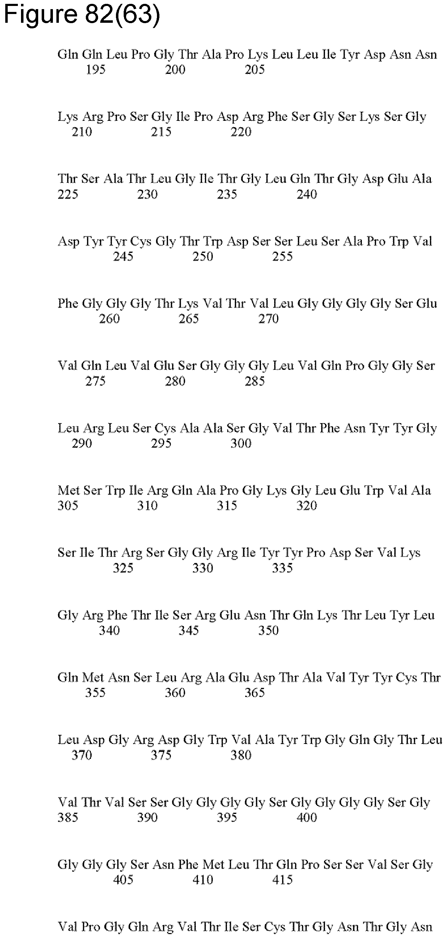

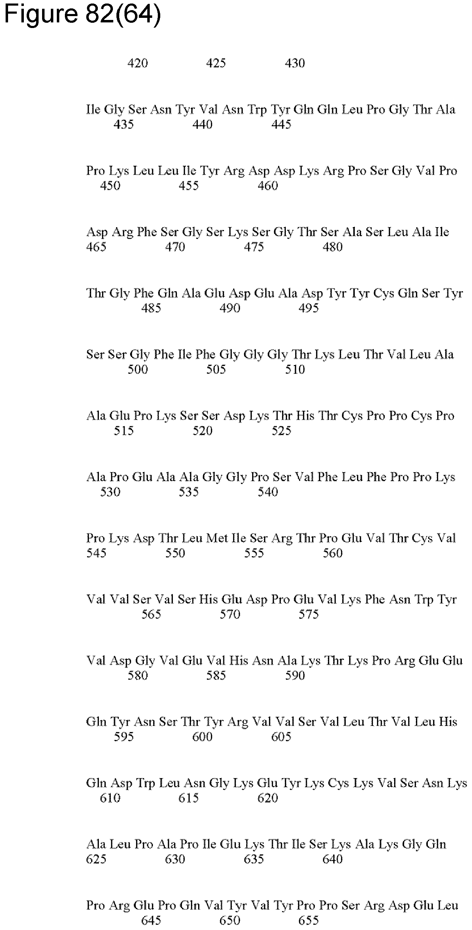

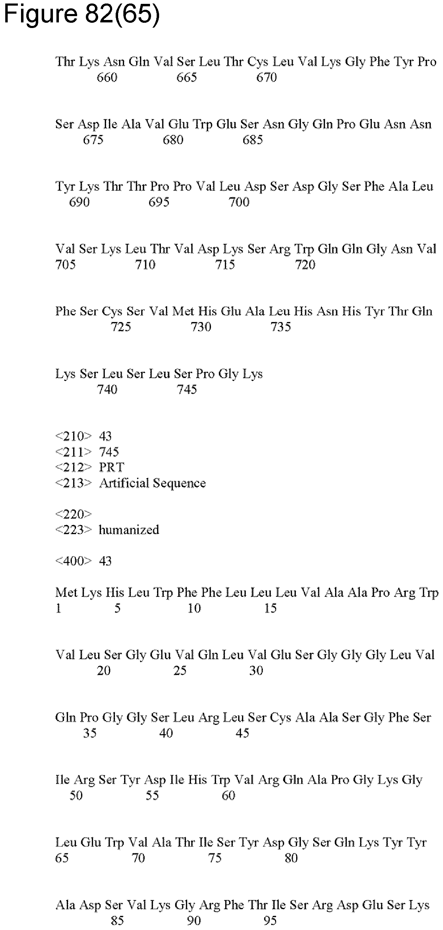

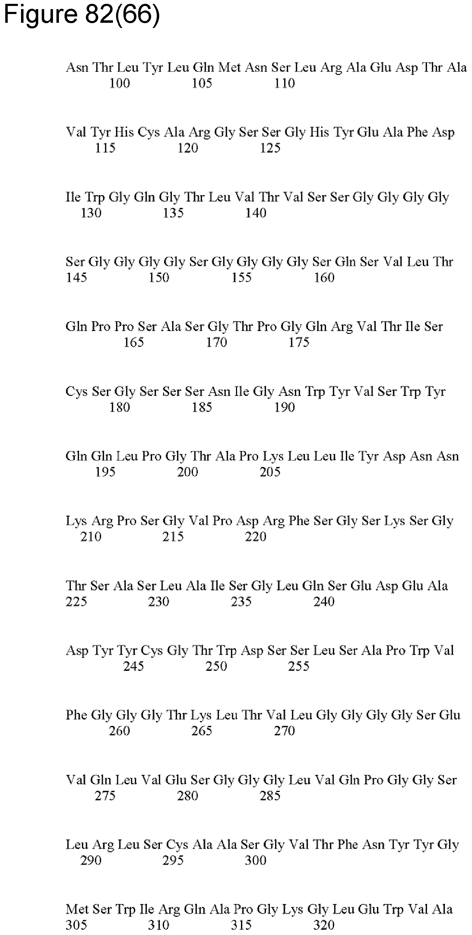

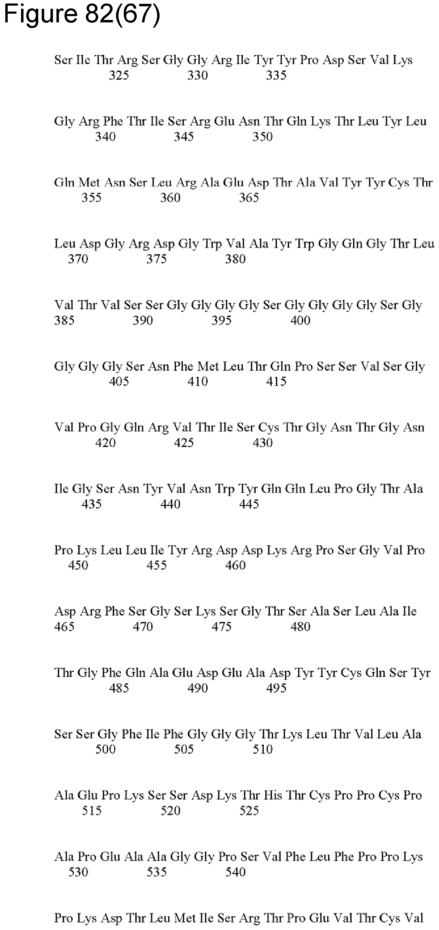

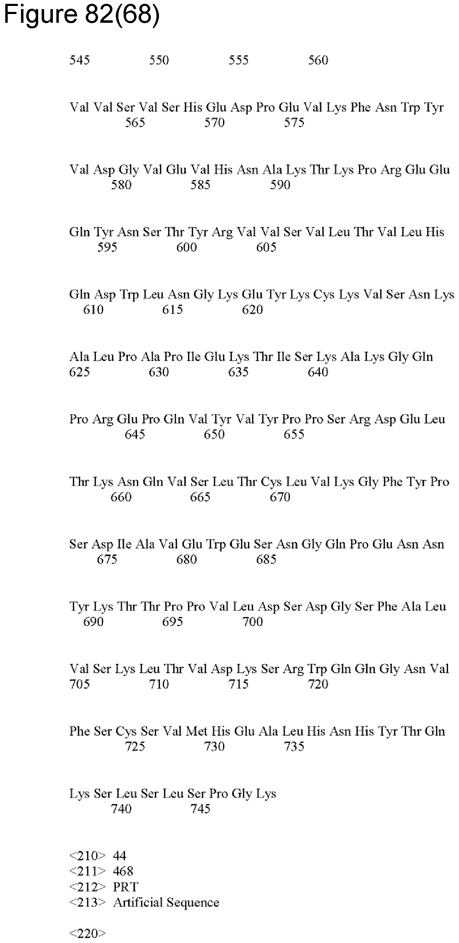

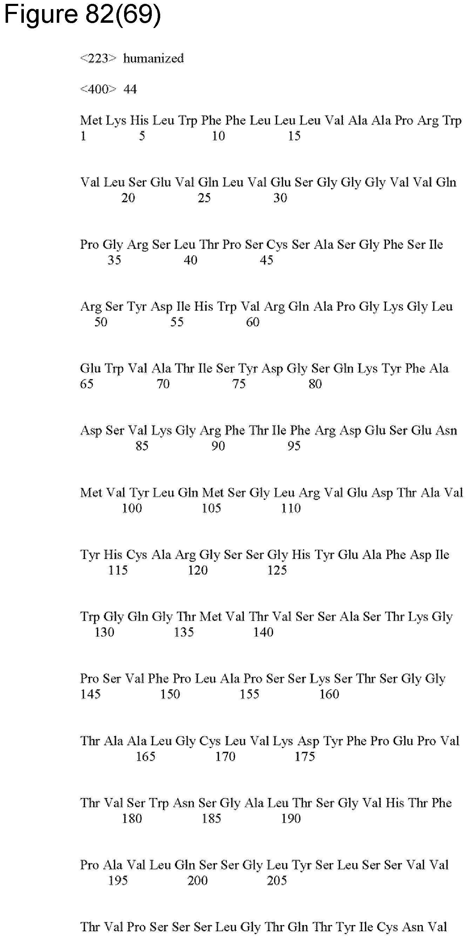

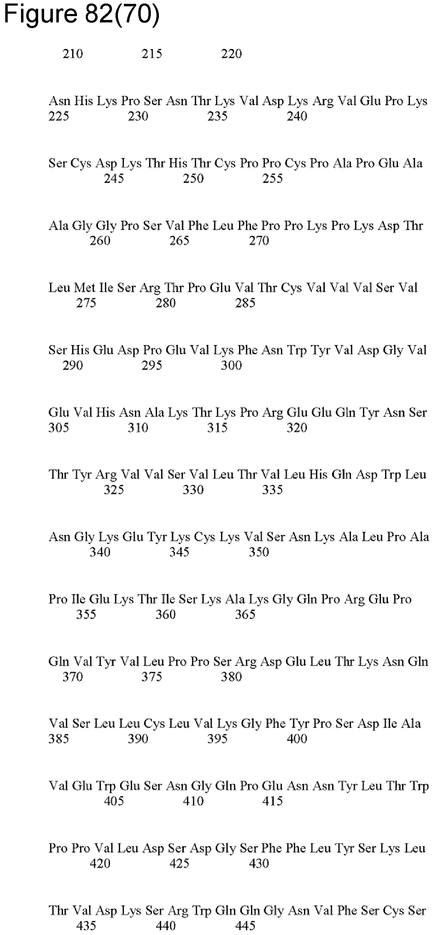

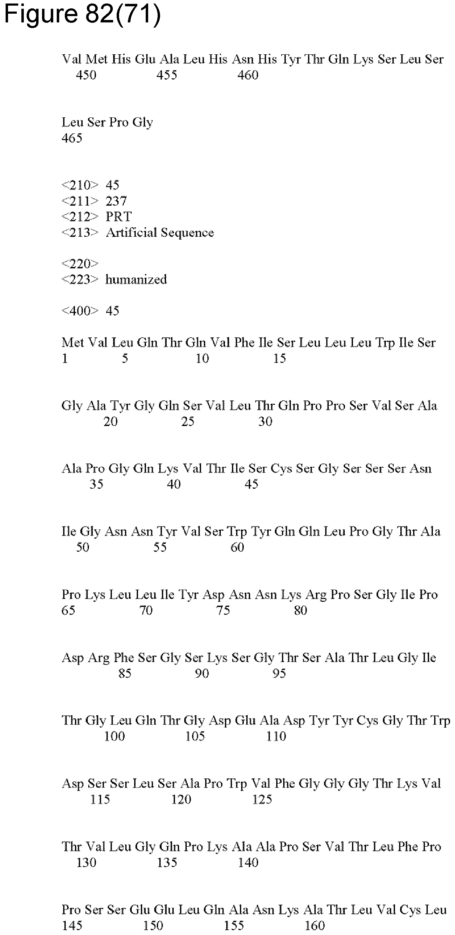

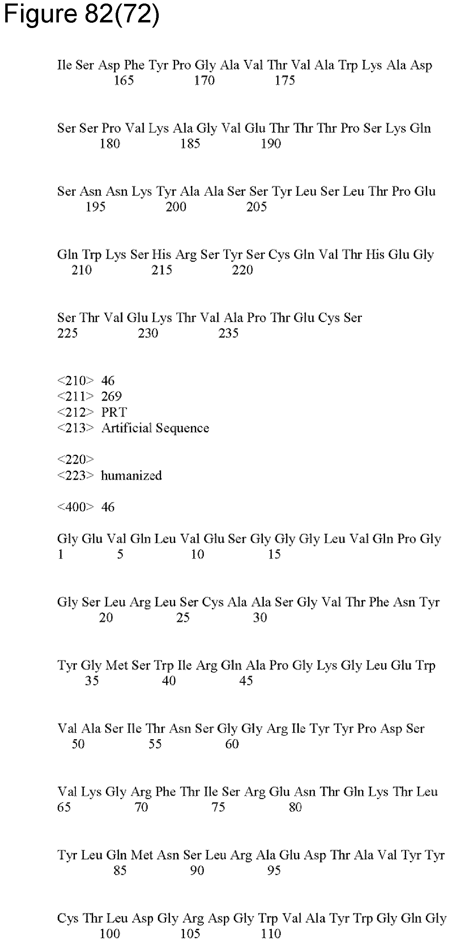

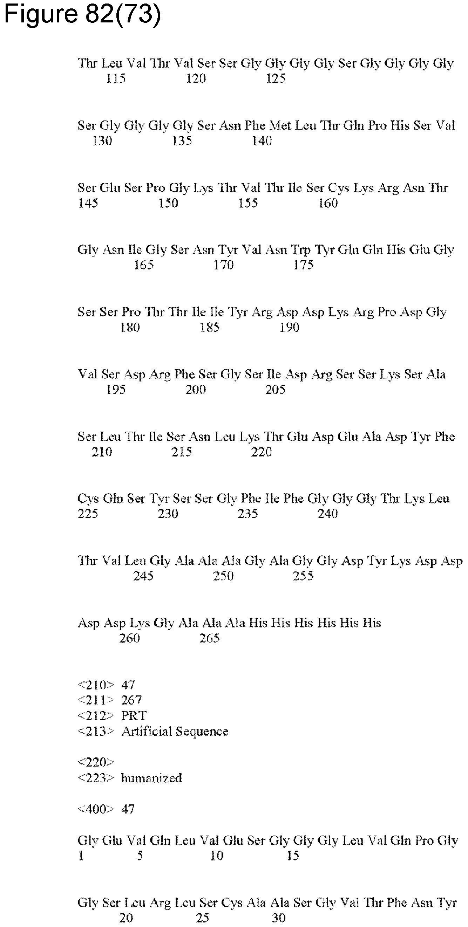

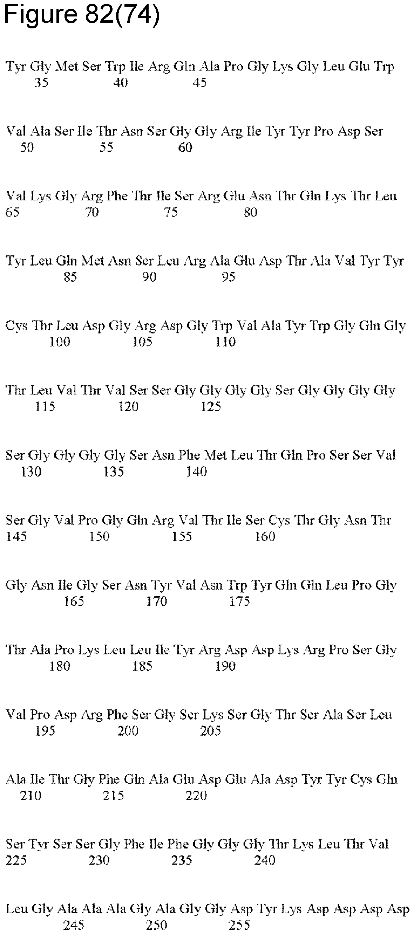

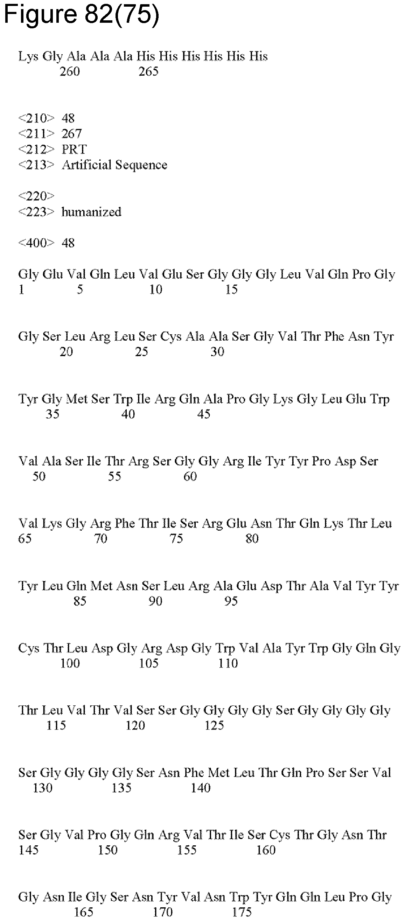

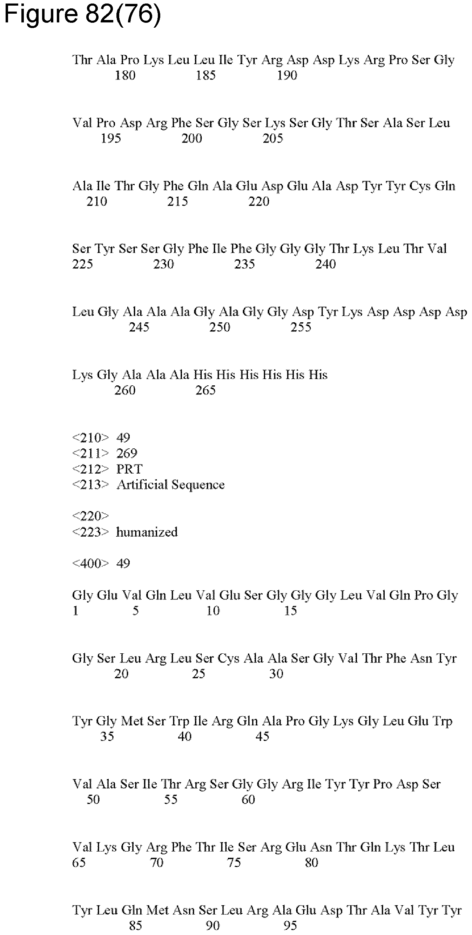

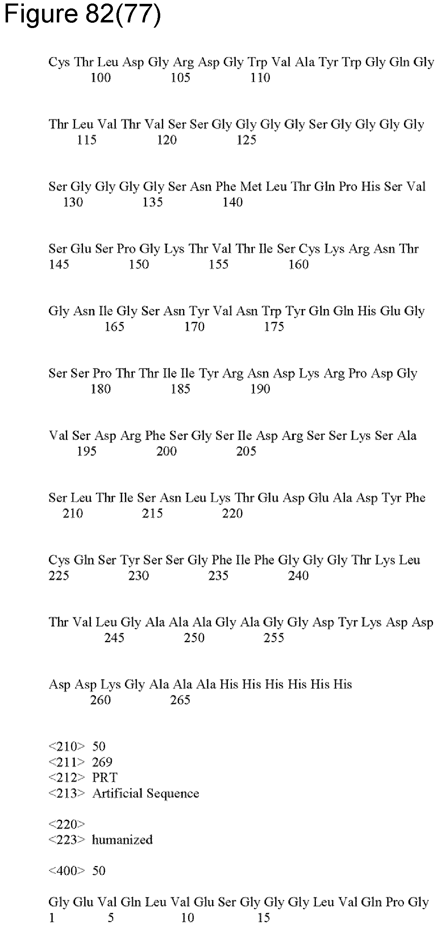

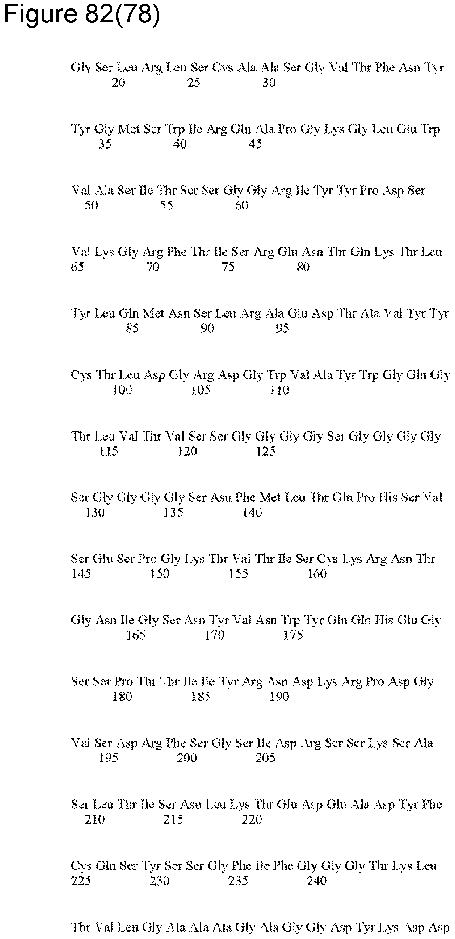

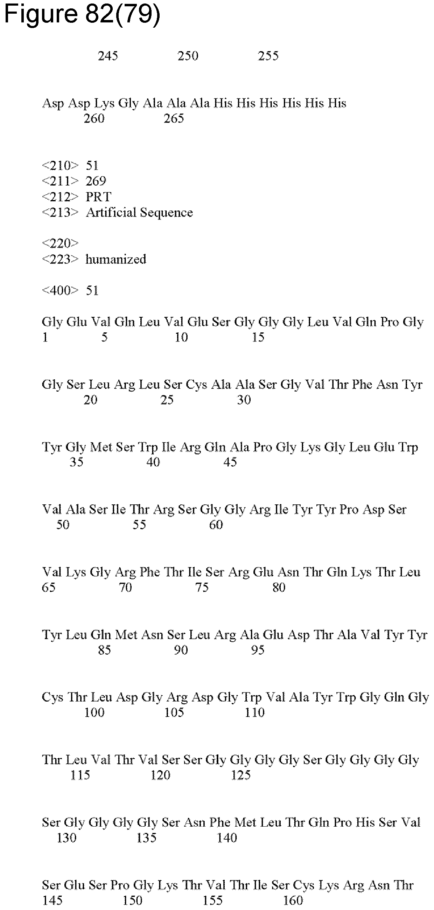

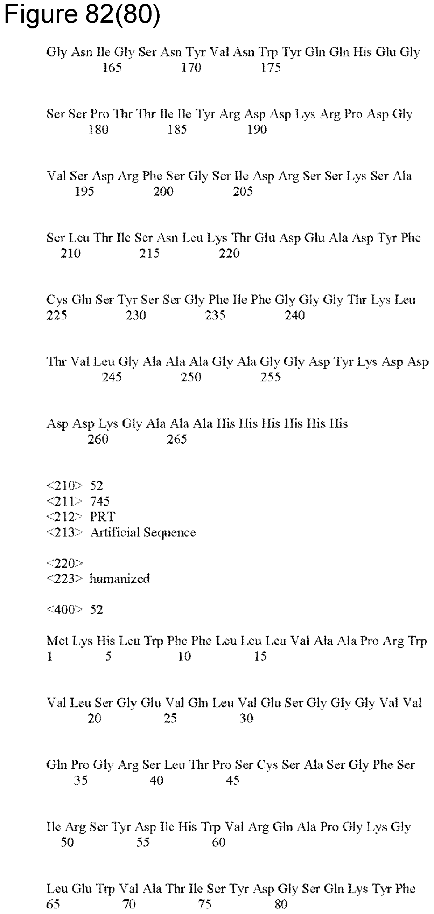

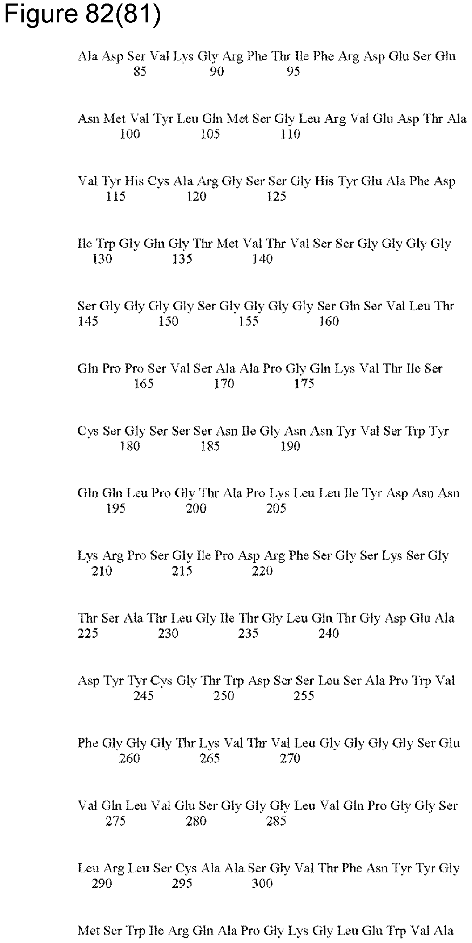

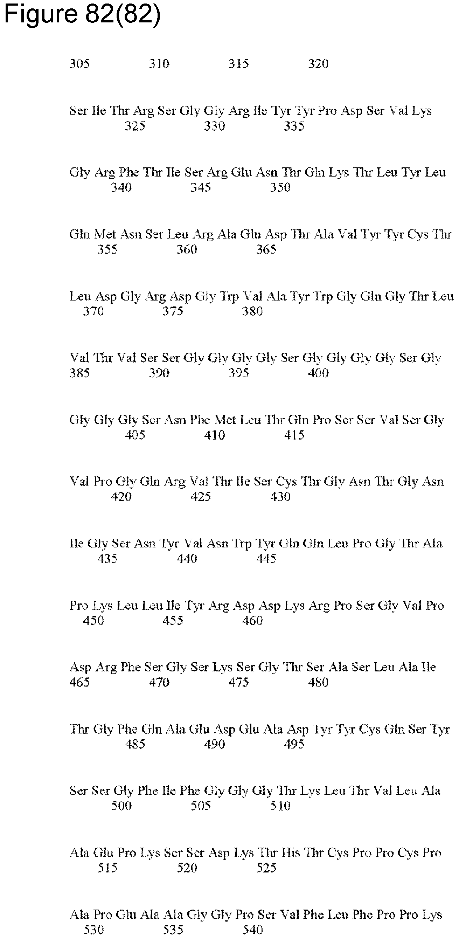

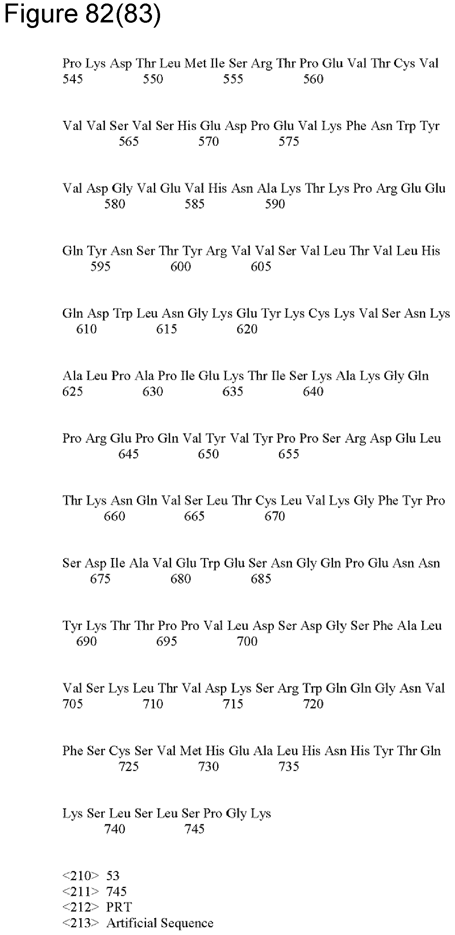

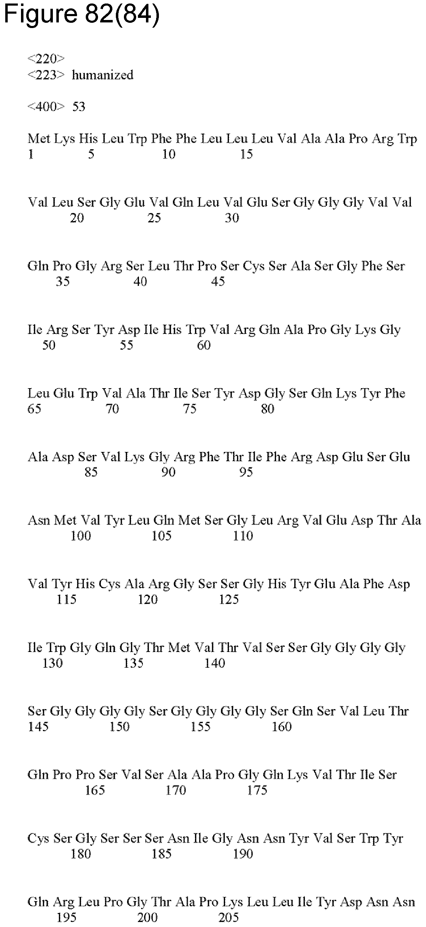

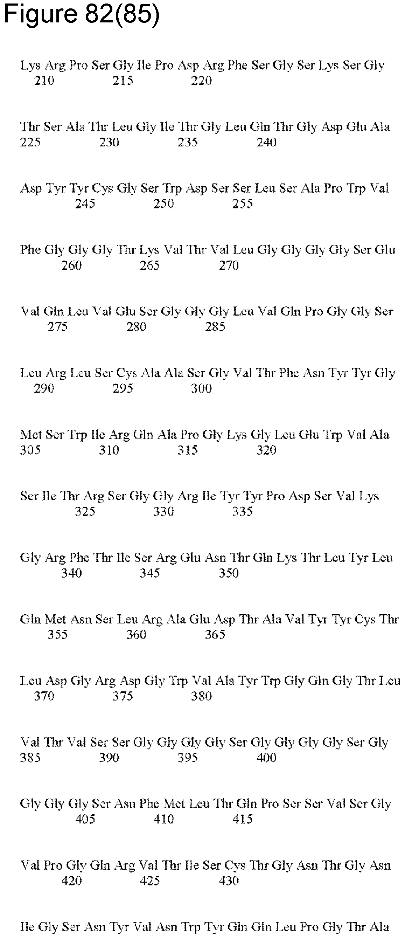

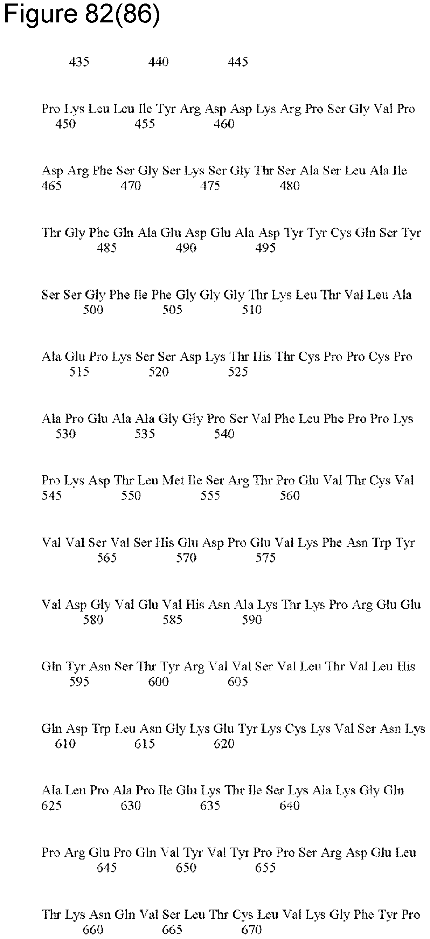

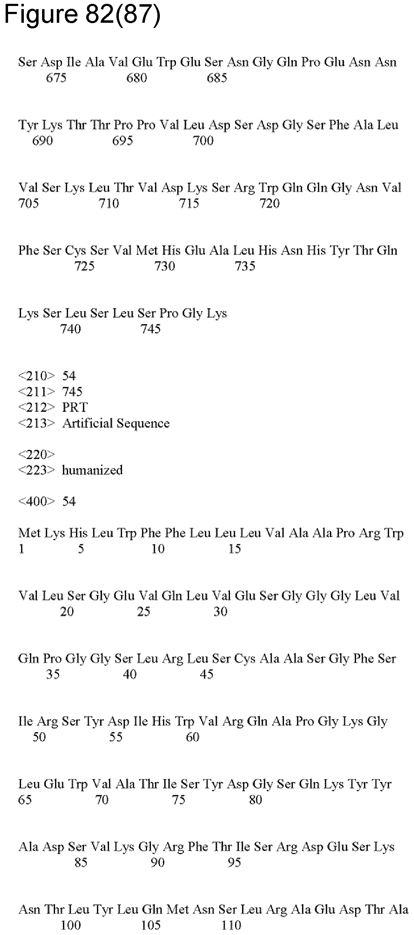

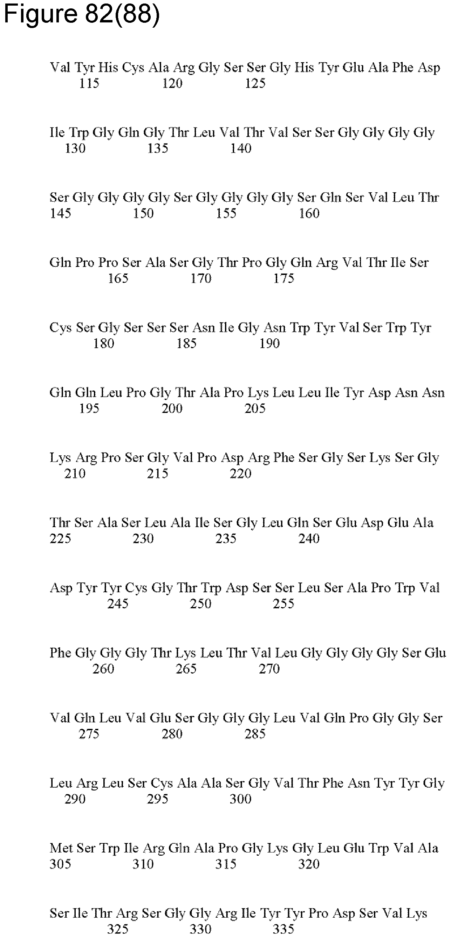

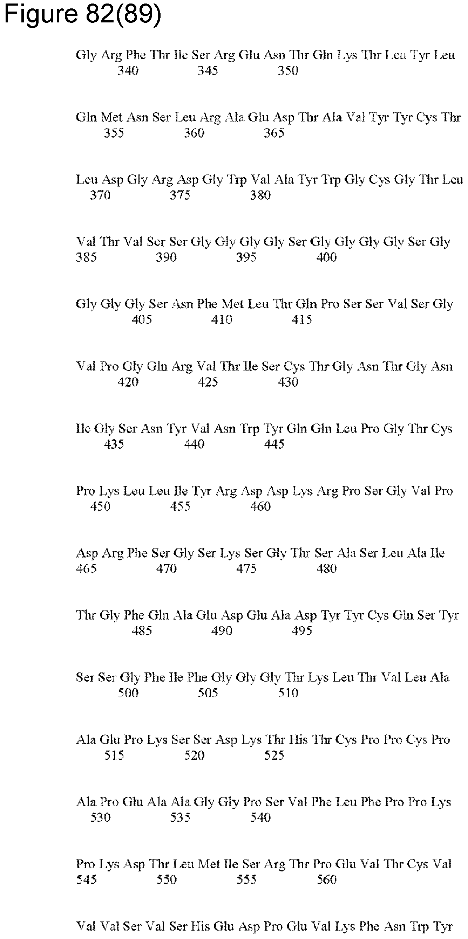

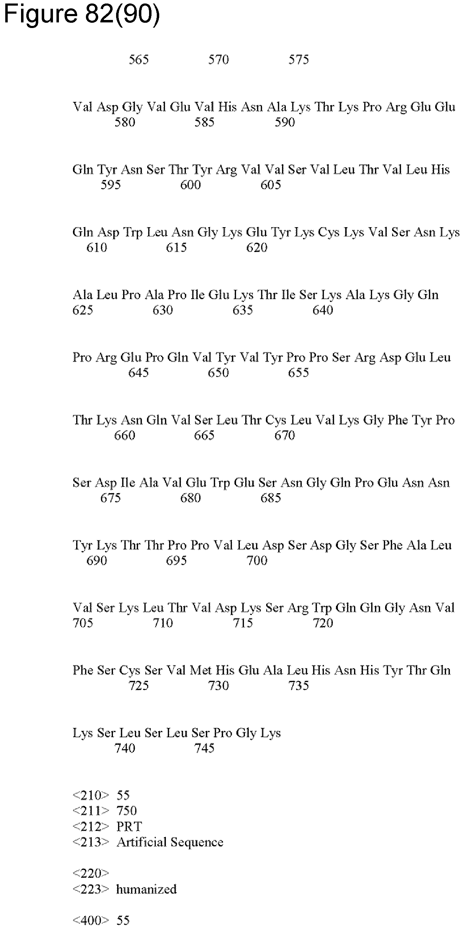

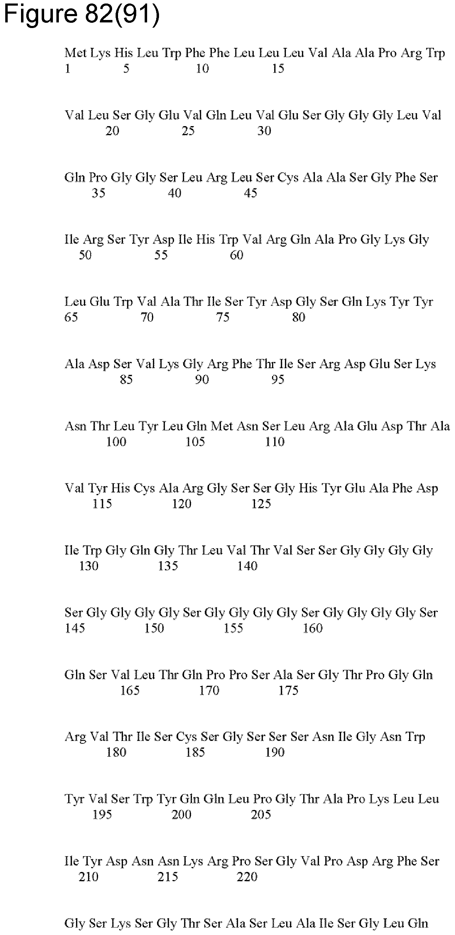

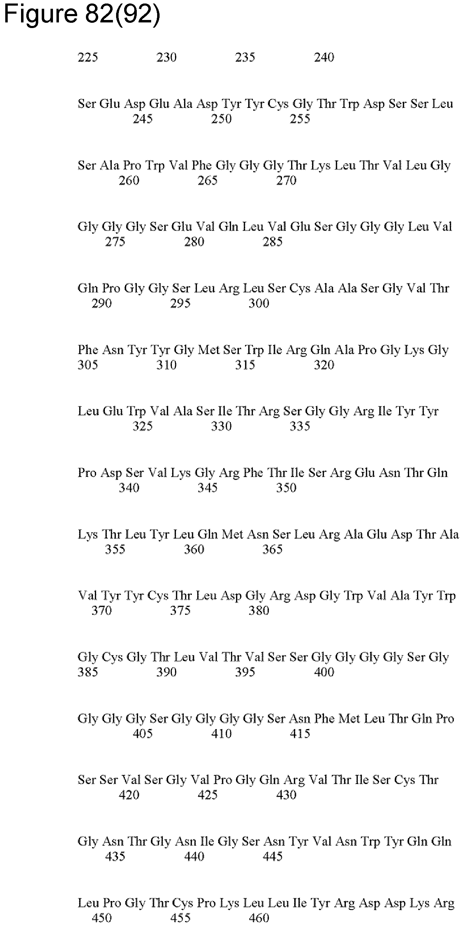

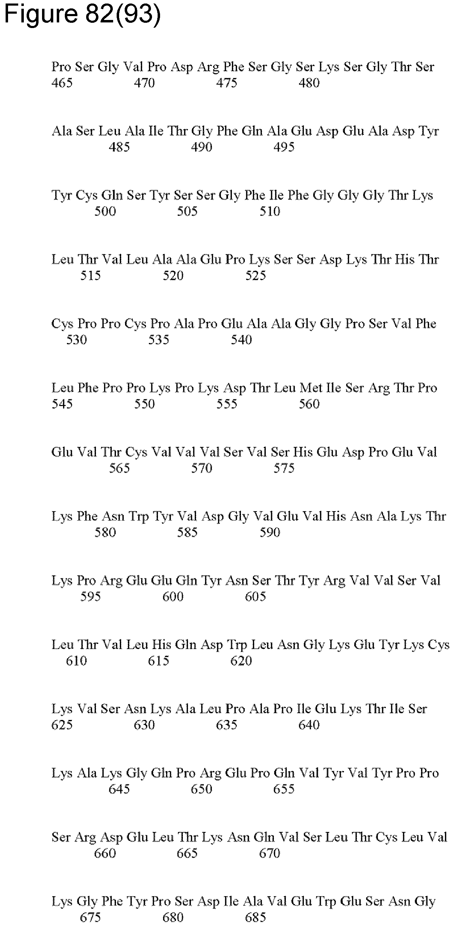

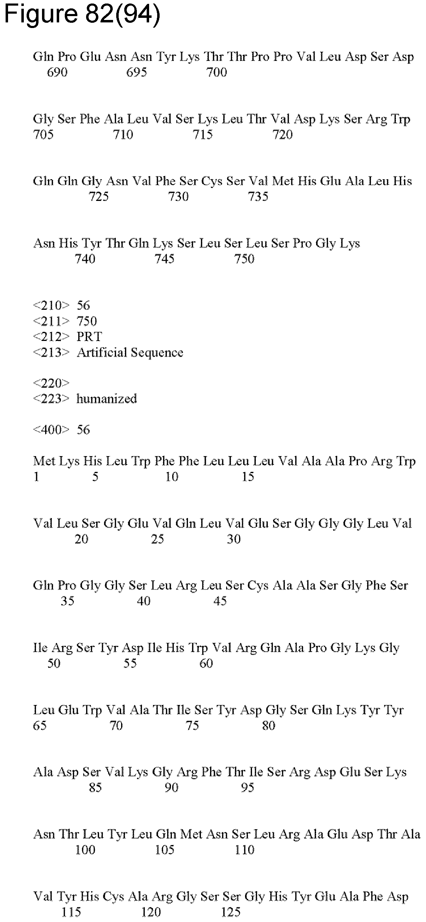

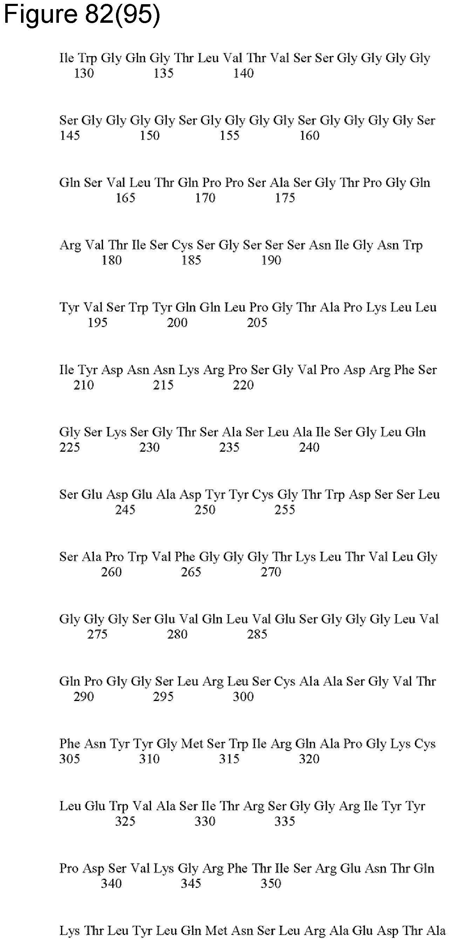

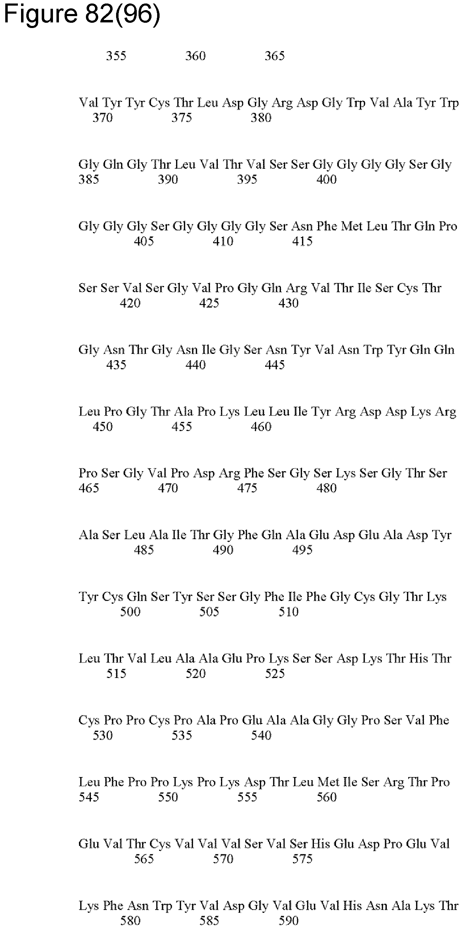

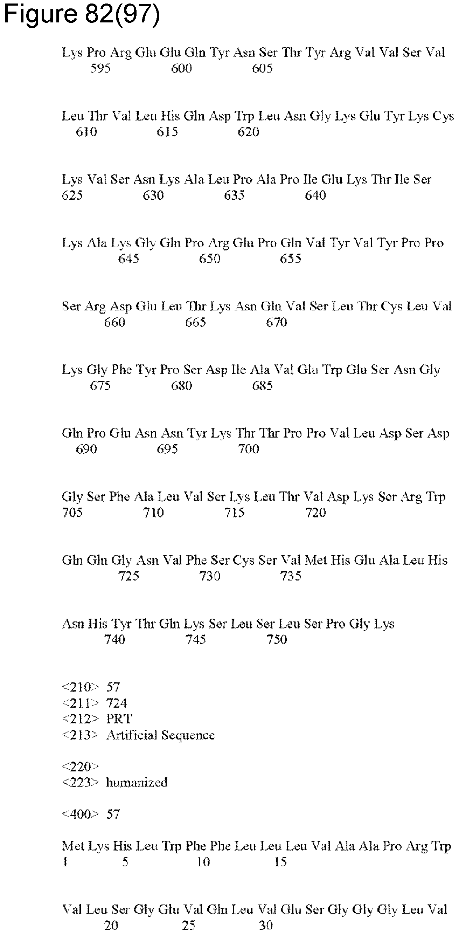

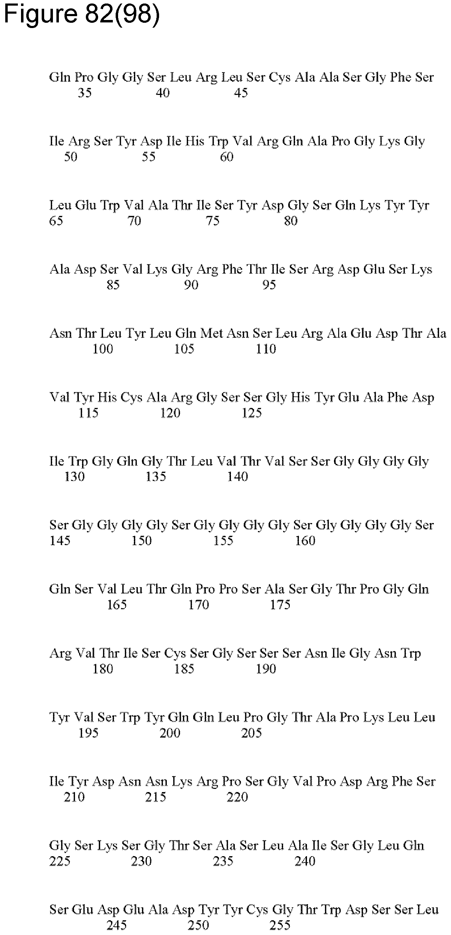

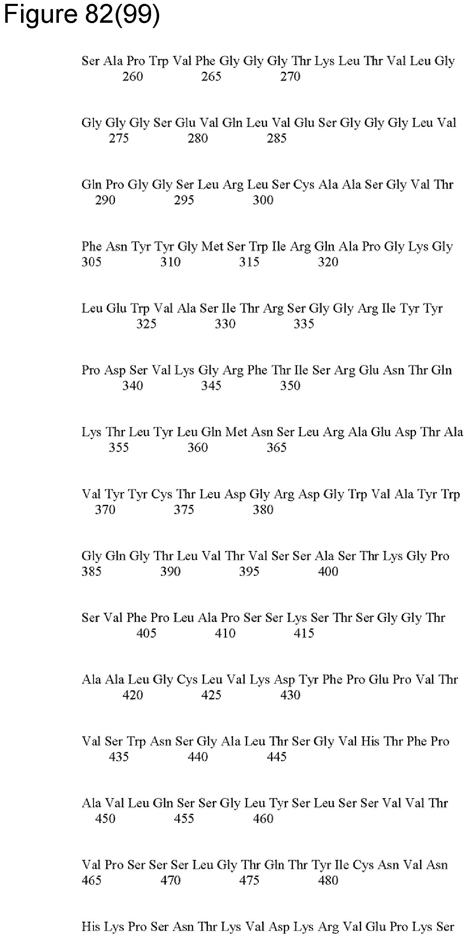

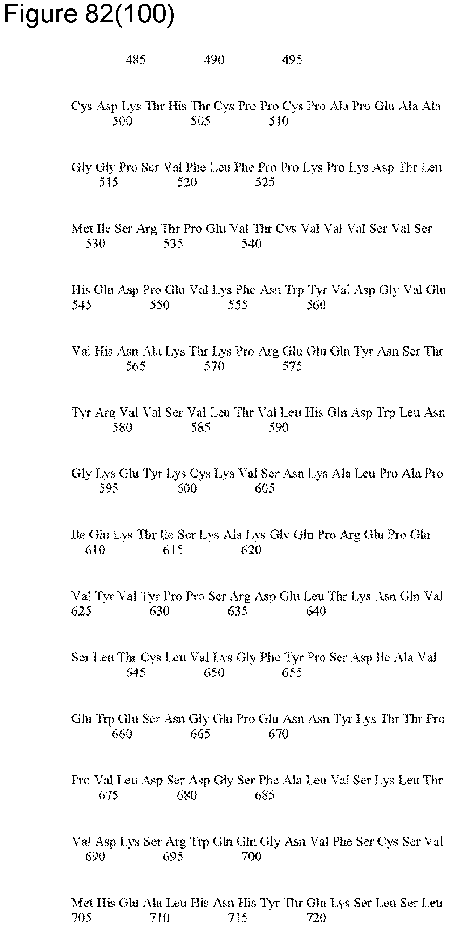

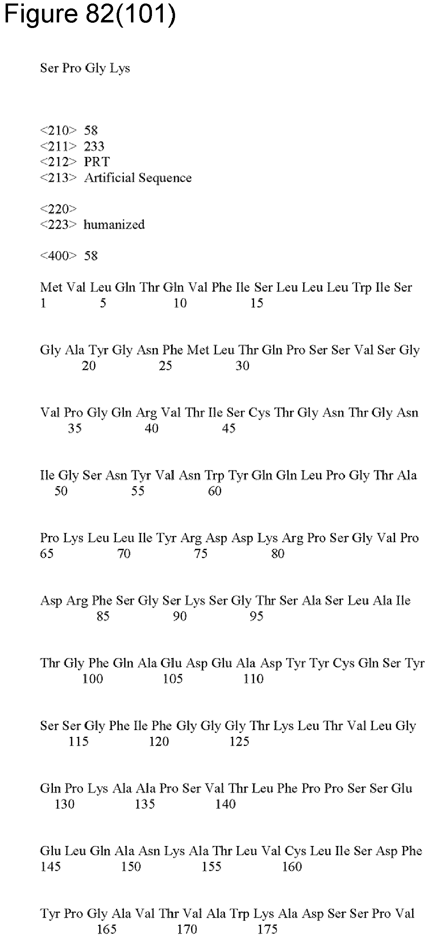

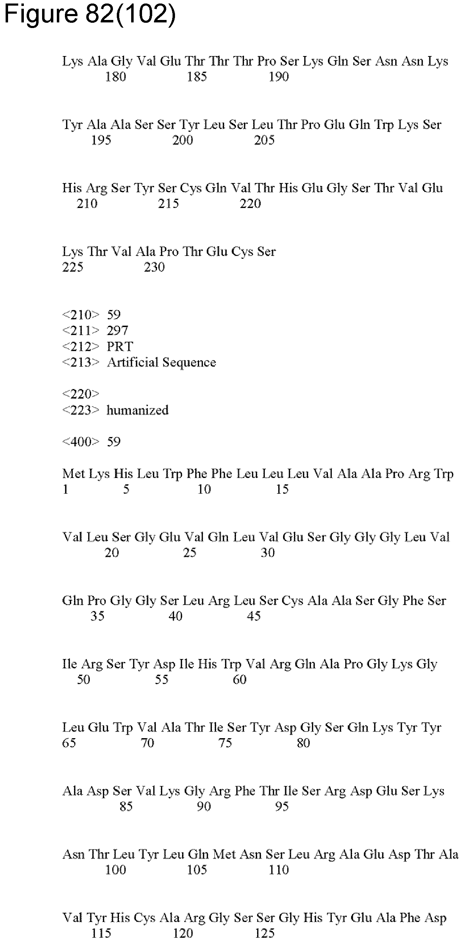

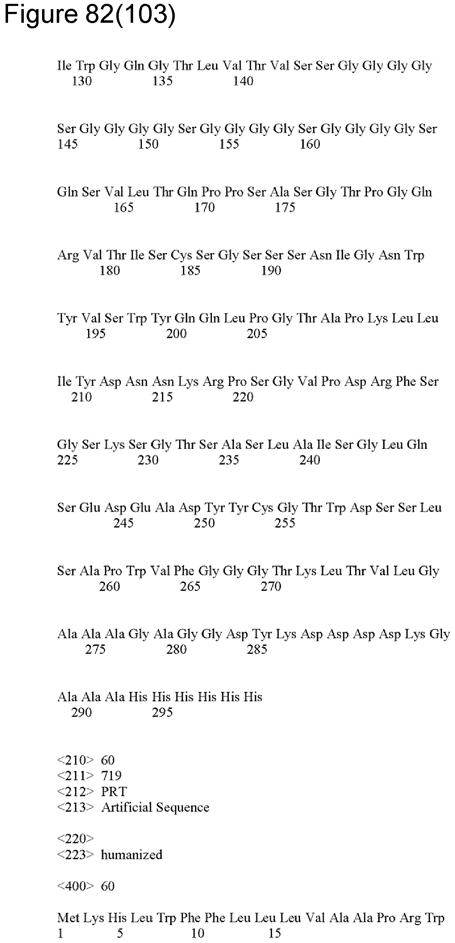

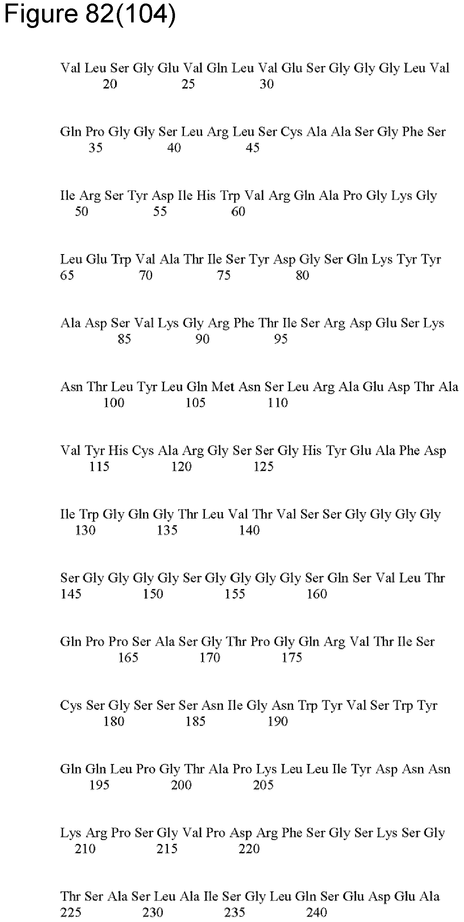

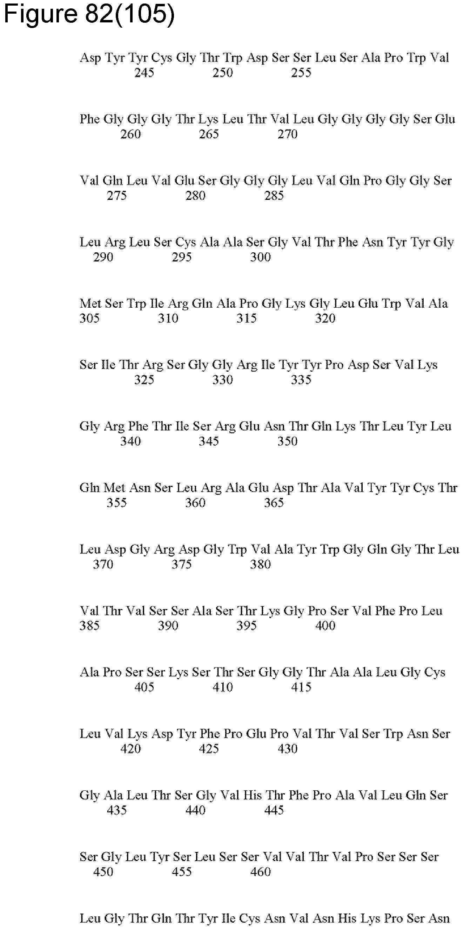

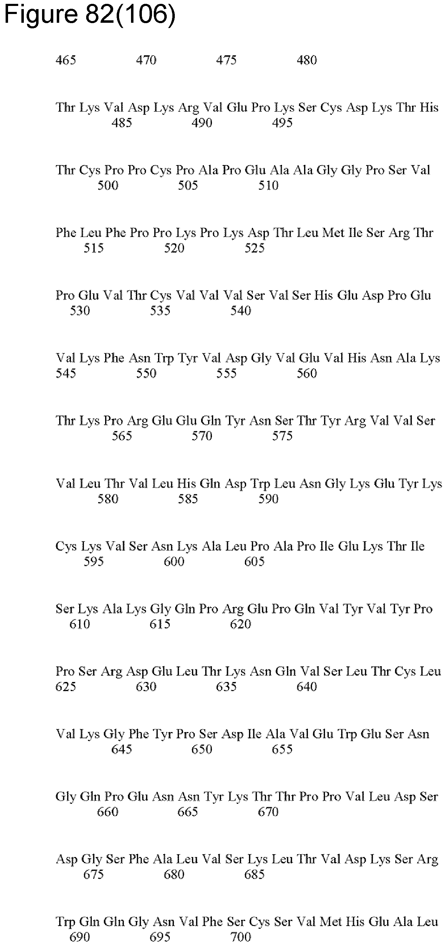

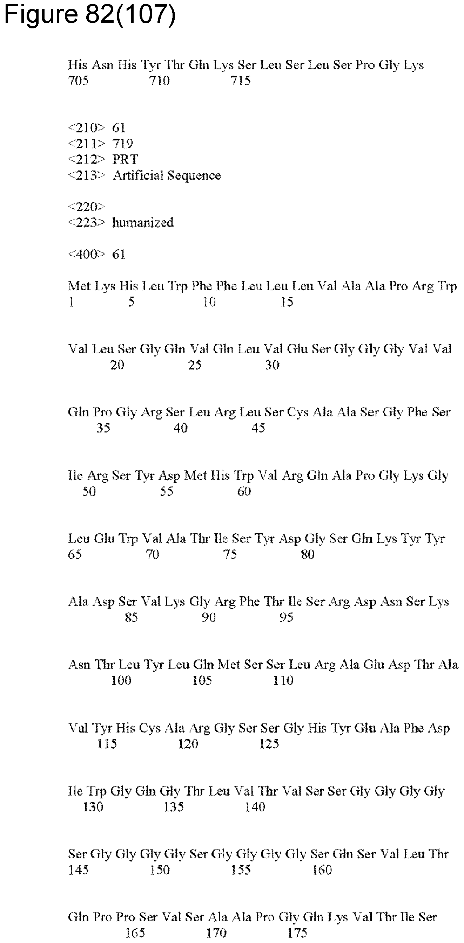

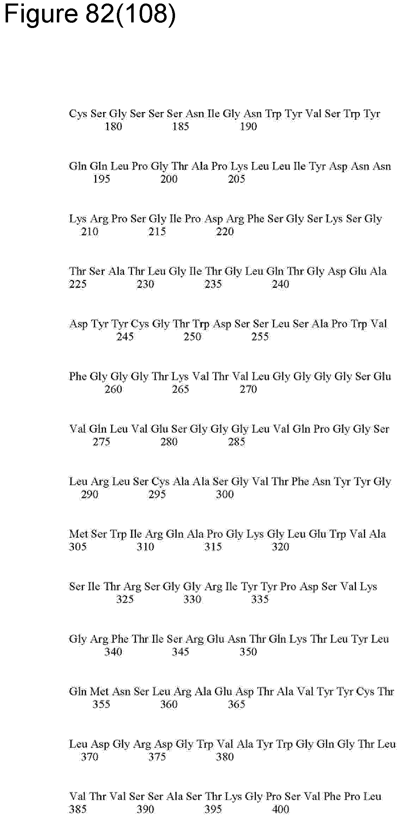

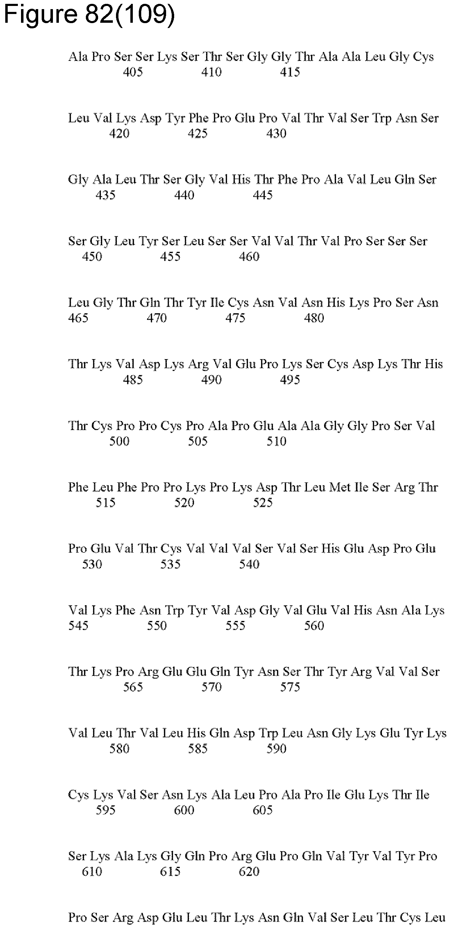

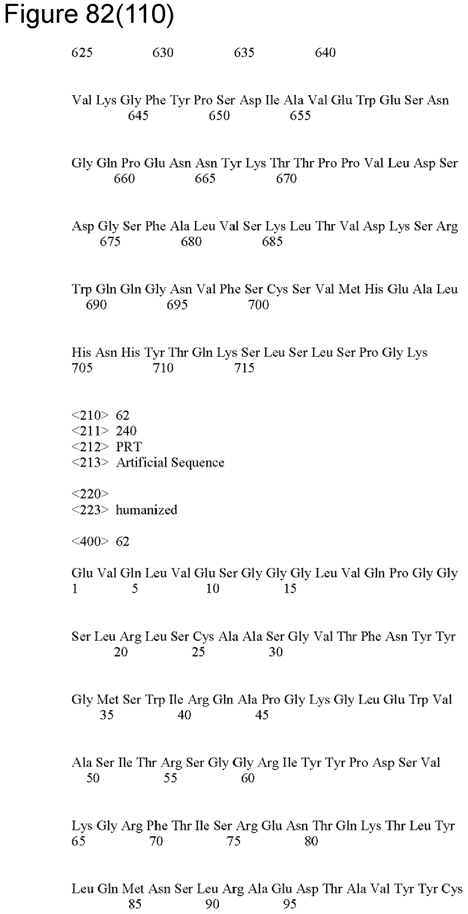

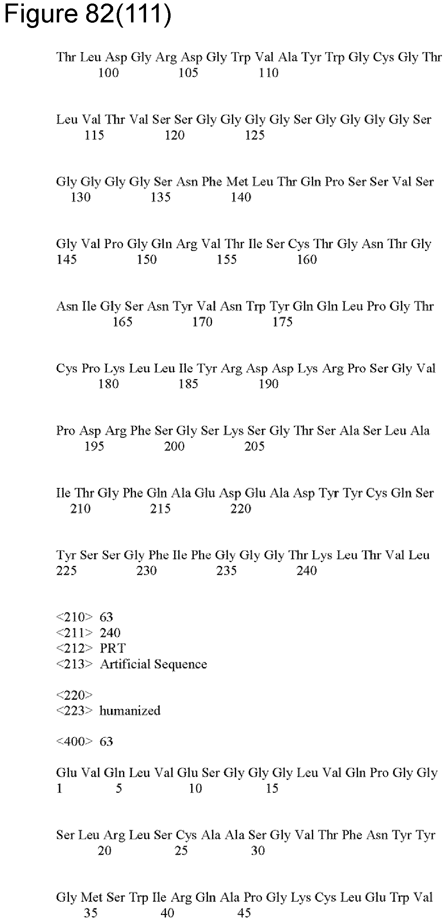

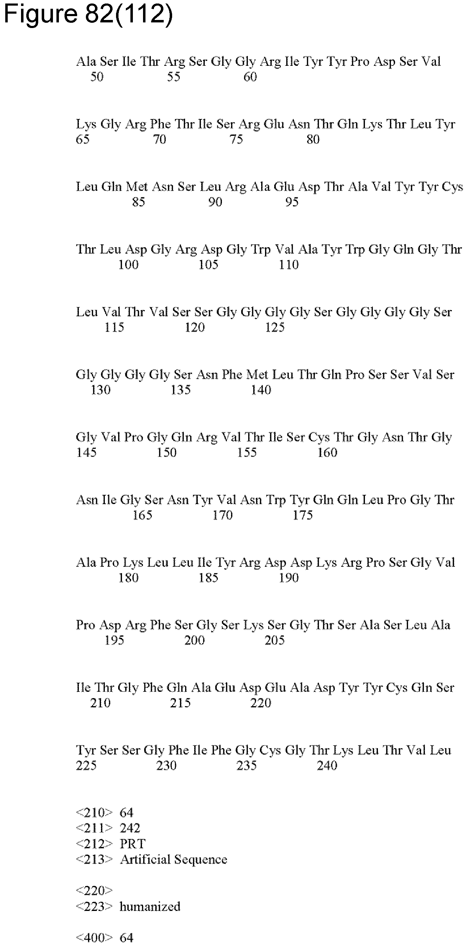

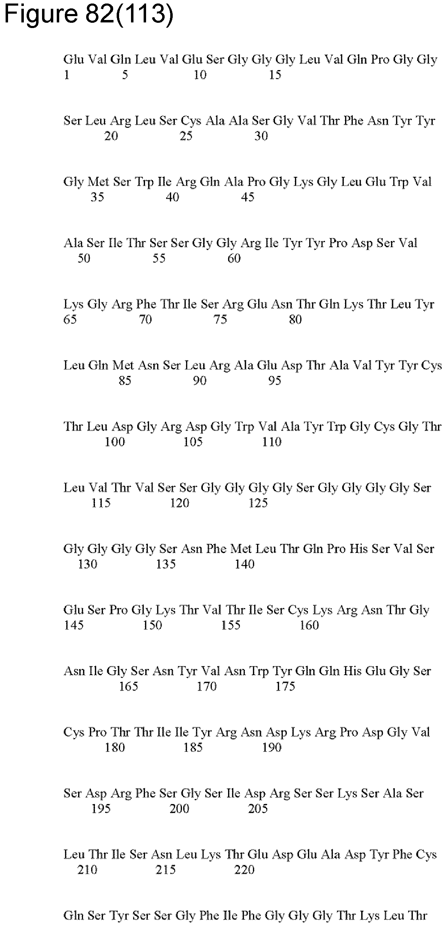

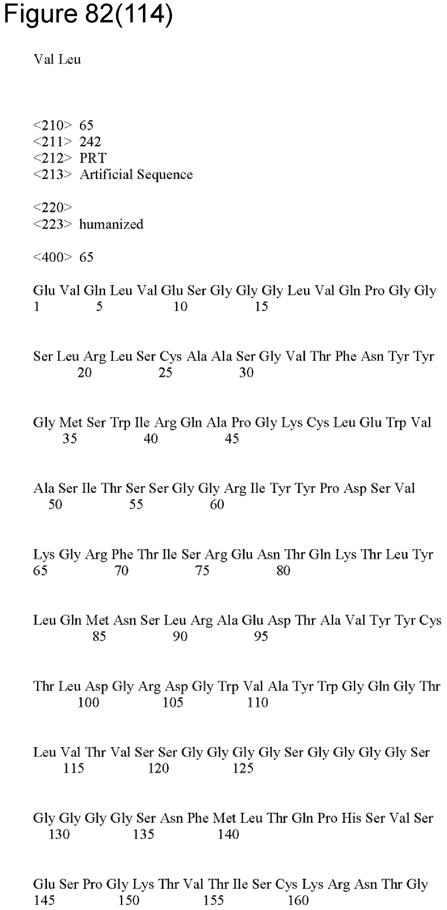

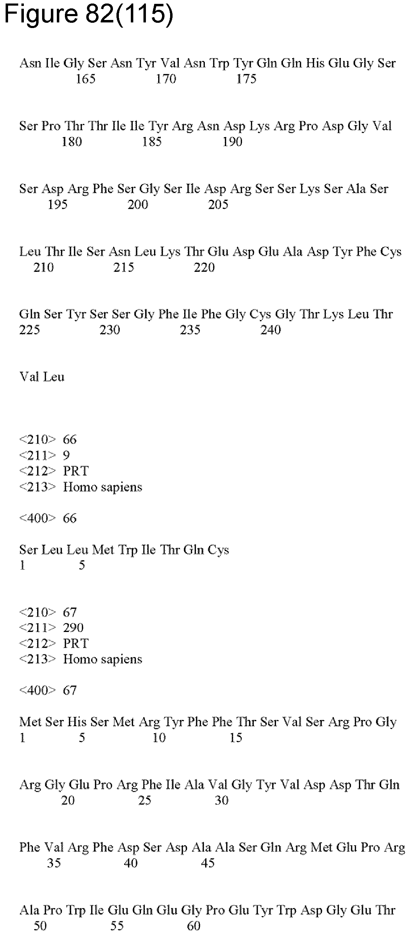

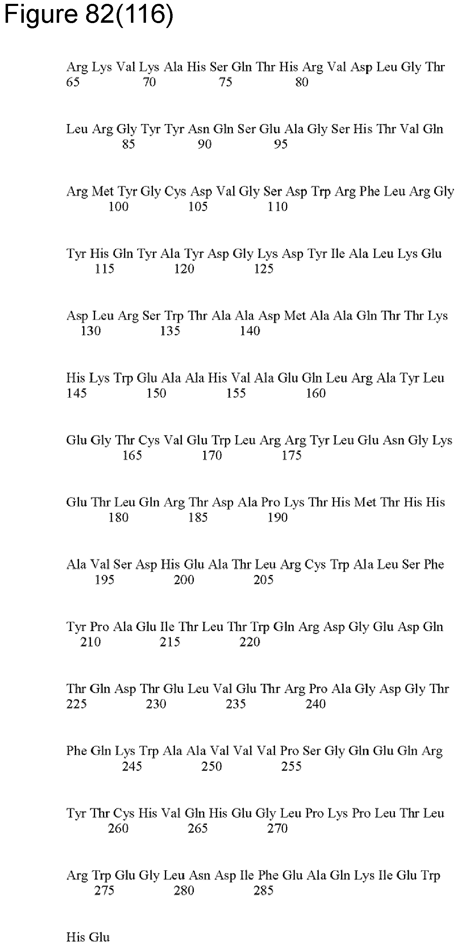

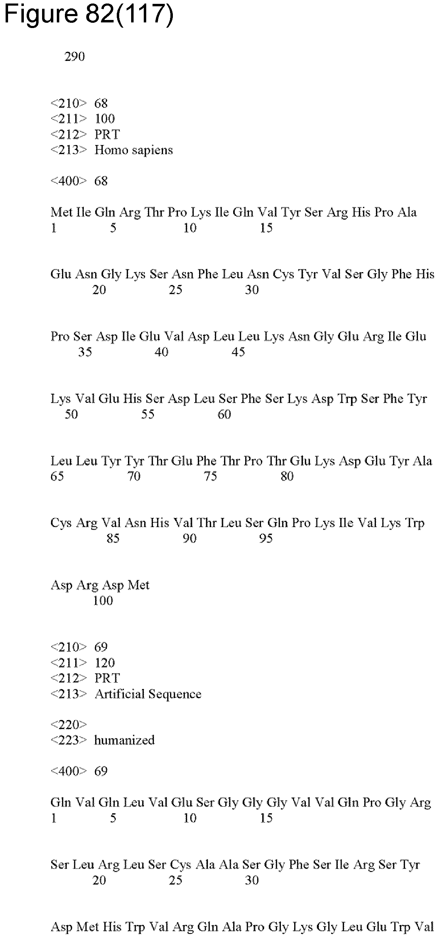

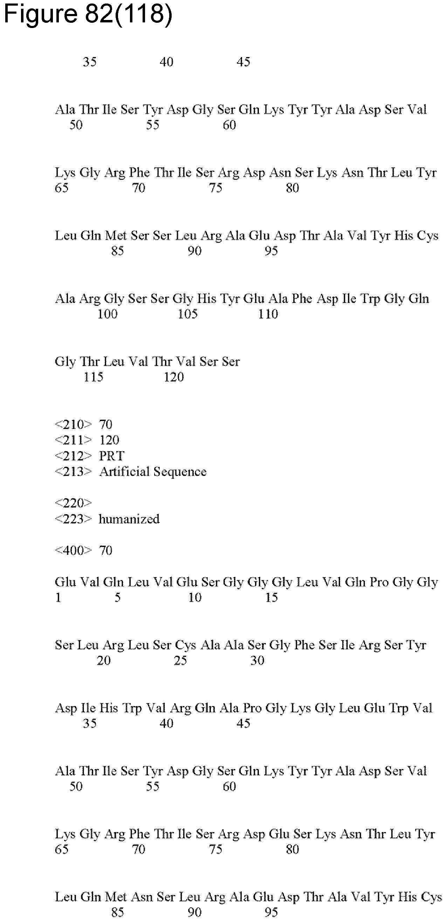

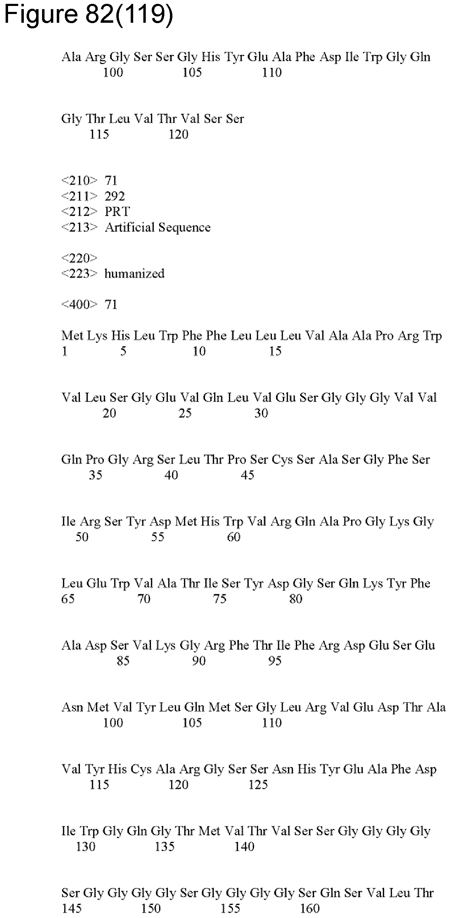

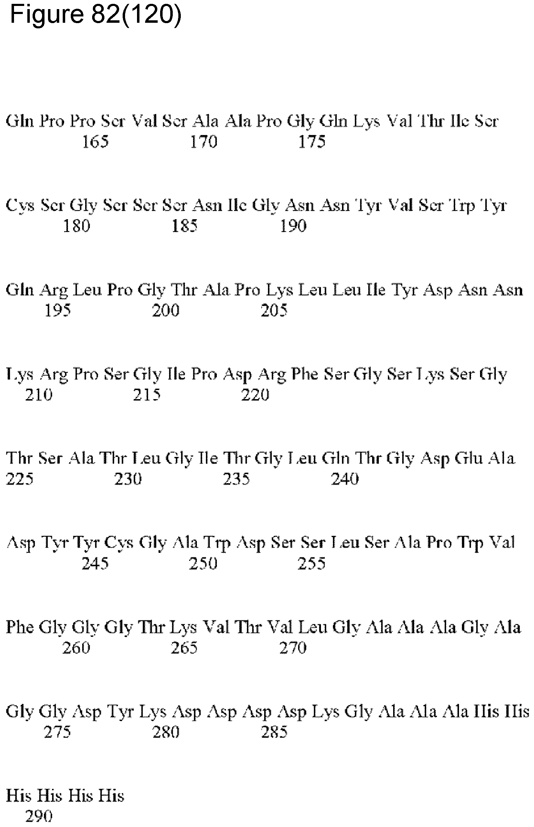

The present invention relates to
(1) A pharmaceutical composition for the treatment and/or prevention of a cancer, comprising the following multispecific antibody [i], the pharmaceutical composition being used in combination with the following compound [ii]:
   [i]
      a multispecific antibody comprising
      an antibody that binds to HLA/NY-ESO or an antigen-binding fragment thereof, comprising
         a heavy chain CDRH1 consisting of the amino acid sequence represented by SEQ ID NO: 1,
         a heavy chain CDRH2 consisting of the amino acid sequence represented by SEQ ID NO: 2,
         a heavy chain CDRH3 consisting of the amino acid sequence represented by SEQ ID NO: 3, or a heavy chain CDRH3 consisting of the amino acid sequence represented by SEQ ID NO: 3 in which the amino acid at position 6 is N;
         a light chain CDRL1 consisting of the amino acid sequence represented by SEQ ID NO: 4, or a light chain CDRL1 consisting of the amino acid sequence represented by SEQ ID NO: 4 in which the amino acid at position 7 is W and/or the amino acid at position 8 is K,
         a light chain CDRL2 consisting of the amino acid sequence represented by Asp Asn Asn (the 5th sequence from the top in Figure 11; SEQ ID NO: 5 in Figure 82), and a light chain CDRL3 consisting of the amino acid sequence represented by SEQ ID NO: 6, or a light chain CDRL3 consisting of the amino acid sequence represented by SEQ ID NO: 6 in which the amino acid at position 2 is A or S; and
      an antibody that binds to CD3 or an antigen-binding fragment thereof, comprising
         a heavy chain CDRH1 consisting of the amino acid sequence represented by SEQ ID NO: 7,
         a heavy chain CDRH2 consisting of the amino acid sequence represented by SEQ ID NO: 8, or a heavy chain CDRH2 consisting of the amino acid sequence represented by SEQ ID NO: 8 in which the amino acid at position 3 is N or **S,** a heavy chain CDRH3 consisting of the amino acid sequence represented by SEQ ID NO: 9,
         a light chain CDRL1 consisting of the amino acid sequence represented by SEQ ID NO: 10,
         a light chain CDRL2 consisting of the amino acid sequence represented by Arg Asp Asp (the 5th sequence from the top in Figure 12; SEQ ID NO: 11 in Figure 82), or a light chain CDRL2 consisting of the amino acid sequence represented by Arg Asp Asp (the 5th sequence from the top in Figure 12; SEQ ID NO: 11 in Figure 82) in which the amino acid at position 2 is G, Q, N, S, or A, and a light chain CDRL3 consisting of the amino acid sequence represented by SEQ ID NO: 12;
   [ii] a compound that inhibits an immune checkpoint molecule or a chemotherapeutic.
(2) The pharmaceutical composition according to (1), wherein the multispecific antibody is a bispecific antibody.
(3) The pharmaceutical composition according to (1) or (2), wherein the antibody that binds to HLA/NY-ESO or the antigen-binding fragment thereof comprises one or two or more sets of CDRH1 to CDRH3 and CDRL1 to CDRL3 selected from the group consisting of the following sets (i) to (v):
   (i)
      a heavy chain CDRH1 consisting of the amino acid sequence represented by SEQ ID NO: 1,
      a heavy chain CDRH2 consisting of the amino acid sequence represented by SEQ ID NO: 2,
      a heavy chain CDRH3 consisting of the amino acid sequence represented by SEQ ID NO: 3,
      a light chain CDRL1 consisting of the amino acid sequence represented by SEQ ID NO: 4,
      a light chain CDRL2 consisting of the amino acid sequence represented by Asp Asn Asn (the 5th sequence from the top in Figure 11; SEQ ID NO: 5 in Figure 82), and a light chain CDRL3 consisting of the amino acid sequence represented by SEQ ID NO: 6,
   (ii)
      a heavy chain CDRH1 consisting of the amino acid sequence represented by SEQ ID NO: 1,
      a heavy chain CDRH2 consisting of the amino acid sequence represented by SEQ ID NO: 2,
      a heavy chain CDRH3 consisting of the amino acid sequence represented by SEQ ID NO: 3,
      a light chain CDRL1 consisting of the amino acid sequence represented by SEQ ID NO: 4 in which the amino acid at position 7 is W,
      a light chain CDRL2 consisting of the amino acid sequence represented by Asp Asn Asn (the 5th sequence from the top in Figure 11; SEQ ID NO: 5 in Figure 82), and a light chain CDRL3 consisting of the amino acid sequence represented by SEQ ID NO: 6,
   (iii)
      a heavy chain CDRH1 consisting of the amino acid sequence represented by SEQ ID NO: 1,
      a heavy chain CDRH2 consisting of the amino acid sequence represented by SEQ ID NO: 2,
      a heavy chain CDRH3 consisting of the amino acid sequence represented by SEQ ID NO: 3 in which the amino acid at position 6 is N,
      a light chain CDRL1 consisting of the amino acid sequence represented by SEQ ID NO: 4,
      a light chain CDRL2 consisting of the amino acid sequence represented by Asp Asn Asn (the 5th sequence from the top in Figure 11; SEQ ID NO: 5 in Figure 82), and a light chain CDRL3 consisting of the amino acid sequence represented by SEQ ID NO: 6 in which the amino acid at position 2 is A,
   (iv)
      a heavy chain CDRH1 consisting of the amino acid sequence represented by SEQ ID NO: 1,
      a heavy chain CDRH2 consisting of the amino acid sequence represented by SEQ ID NO: 2,
      a heavy chain CDRH3 consisting of the amino acid sequence represented by SEQ ID NO: 3,
      a light chain CDRL1 consisting of the amino acid sequence represented by SEQ ID NO: 4,
      a light chain CDRL2 consisting of the amino acid sequence represented by Asp Asn Asn (the 5th sequence from the top in Figure 11; SEQ ID NO: 5 in Figure 82), and
      a light chain CDRL3 consisting of the amino acid sequence represented by SEQ ID NO: 6 in which the amino acid at position 2 is S, and
   (v)
      a heavy chain CDRH1 consisting of the amino acid sequence represented by SEQ ID NO: 1,
      a heavy chain CDRH2 consisting of the amino acid sequence represented by SEQ ID NO: 2,
      a heavy chain CDRH3 consisting of the amino acid sequence represented by SEQ ID NO: 3,
      a light chain CDRL1 consisting of the amino acid sequence represented by SEQ ID NO: 4 in which the amino acid at position 7 is W and the amino acid at position 8 is K,
      a light chain CDRL2 consisting of the amino acid sequence represented by Asp Asn Asn (the 5th sequence from the top in Figure 11; SEQ ID NO: 5 in Figure 82), and a light chain CDRL3 consisting of the amino acid sequence represented by SEQ ID NO: 6 in which the amino acid at position 2 is A.
(4) The pharmaceutical composition according to any one of (1) to (3), wherein the antibody that binds to HLA/NY-ESO or the antigen-binding fragment thereof comprises a heavy chain variable region consisting of an amino acid sequence having 95% or higher sequence identity to an amino acid sequence corresponding to positions 21 to 140 of the amino acid sequence represented by SEQ ID NO: 18, or the amino acid sequence represented by SEQ ID NO: 69 or SEQ ID NO: 70, and a light chain variable region consisting of an amino acid sequence having 95% or higher sequence identity to an amino acid sequence corresponding to positions 156 to 266 of the amino acid sequence represented by SEQ ID NO: 18 or SEQ ID NO: 28, or the amino acid sequence represented by SEQ ID NO: 23.
(5) The pharmaceutical composition according to any one of (1) to (4), wherein the antibody that binds to HLA/NY-ESO or the antigen-binding fragment thereof comprises
   a heavy chain variable region consisting of the amino acid sequence represented by SEQ ID NO: 13,
   a heavy chain variable region consisting of the amino acid sequence represented by SEQ ID NO: 15,
   a heavy chain variable region consisting of an amino acid sequence corresponding to positions 21 to 140 of the amino acid sequence represented by SEQ ID NO: 20,
   a heavy chain variable region consisting of an amino acid sequence corresponding to positions 21 to 140 of the amino acid sequence represented by SEQ ID NO: 17,
   a heavy chain variable region consisting of an amino acid sequence corresponding to positions 21 to 140 of the amino acid sequence represented by SEQ ID NO: 18,
   a heavy chain variable region consisting of an amino acid sequence corresponding to positions 21 to 140 of the amino acid sequence represented by SEQ ID NO: 19,
   a heavy chain variable region consisting of an amino acid sequence corresponding to positions 21 to 140 of the amino acid sequence represented by SEQ ID NO: 22,
   a heavy chain variable region consisting of an amino acid sequence corresponding to positions 21 to 140 of the amino acid sequence represented by SEQ ID NO: 24,
   a heavy chain variable region consisting of an amino acid sequence corresponding to positions 21 to 140 of the amino acid sequence represented by SEQ ID NO: 25,
   a heavy chain variable region consisting of an amino acid sequence corresponding to positions 21 to 140 of the amino acid sequence represented by SEQ ID NO: 26,
   a heavy chain variable region consisting of an amino acid sequence corresponding to positions 21 to 140 of the amino acid sequence represented by SEQ ID NO: 27,
   a heavy chain variable region consisting of an amino acid sequence corresponding to positions 21 to 140 of the amino acid sequence represented by SEQ ID NO: 28,
   a heavy chain variable region consisting of an amino acid sequence corresponding to positions 21 to 140 of the amino acid sequence represented by SEQ ID NO: 29,
   a heavy chain variable region consisting of an amino acid sequence corresponding to from positions 21 to 140 of the amino acid sequence represented by SEQ ID NO: 21,
   a heavy chain variable region consisting of an amino acid sequence corresponding to positions 21 to 140 of the amino acid sequence represented by SEQ ID NO: 55, or
   a heavy chain variable region consisting of an amino acid sequence corresponding to positions 21 to 140 of the amino acid sequence represented by SEQ ID NO: 71; and
   a light chain variable region consisting of the amino acid sequence represented by SEQ ID NO: 14,
   a light chain variable region consisting of the amino acid sequence represented by SEQ ID NO: 16,
   a light chain variable region consisting of an amino acid sequence corresponding to positions 156 to 266 of the amino acid sequence represented by SEQ ID NO: 20,
   a light chain variable region consisting of an amino acid sequence corresponding to positions 156 to 266 of the amino acid sequence represented by SEQ ID NO: 17,
   a light chain variable region consisting of an amino acid sequence corresponding to positions 156 to 266 of the amino acid sequence represented by SEQ ID NO: 18,
   a light chain variable region consisting of an amino acid sequence corresponding to positions 156 to 266 of the amino acid sequence represented by SEQ ID NO: 19,
   a light chain variable region consisting of an amino acid sequence corresponding to positions 156 to 266 of the amino acid sequence represented by SEQ ID NO: 22,
   a light chain variable region consisting of an amino acid sequence corresponding to positions 156 to 266 of the amino acid sequence represented by SEQ ID NO: 24,
   a light chain variable region consisting of an amino acid sequence corresponding to positions 156 to 266 of the amino acid sequence represented by SEQ ID NO: 25,
   a light chain variable region consisting of an amino acid sequence corresponding to positions 156 to 266 of the amino acid sequence represented by SEQ ID NO: 26,
   a light chain variable region consisting of an amino acid sequence corresponding to positions 156 to 266 of the amino acid sequence represented by SEQ ID NO: 27,
   a light chain variable region consisting of an amino acid sequence corresponding to positions 156 to 266 of the amino acid sequence represented by SEQ ID NO: 28,
   a light chain variable region consisting of an amino acid sequence corresponding to positions 156 to 266 of the amino acid sequence represented by SEQ ID NO: 29,
   a light chain variable region consisting of an amino acid sequence corresponding to positions 156 to 266 of the amino acid sequence represented by SEQ ID NO: 21,
   a light chain variable region consisting of an amino acid sequence corresponding to positions 161 to 271 of the amino acid sequence represented by SEQ ID NO: 55, or
   a light chain variable region consisting of an amino acid sequence corresponding to positions 156 to 266 of the amino acid sequence represented by SEQ ID NO: 71.
(6) The pharmaceutical composition according to any one of (1) to (5), wherein the antibody that binds to HLA/NY-ESO or the antigen-binding fragment thereof comprises
   a heavy chain variable region consisting of the amino acid sequence represented by SEQ ID NO: 13 and a light chain variable region consisting of the amino acid sequence represented by SEQ ID NO: 14,
   a heavy chain variable region consisting of the amino acid sequence represented by SEQ ID NO: 15 and a light chain variable region consisting of the amino acid sequence represented by SEQ ID NO: 16,
   a heavy chain variable region consisting of an amino acid sequence corresponding to positions 21 to 140 of the amino acid sequence represented by SEQ ID NO: 20 and a light chain variable region consisting of an amino acid sequence corresponding to positions 156 to 266 of the amino acid sequence represented by SEQ ID NO: 20,
   a heavy chain variable region consisting of an amino acid sequence corresponding to positions 21 to 140 of the amino acid sequence represented by SEQ ID NO: 17 and a light chain variable region consisting of an amino acid sequence corresponding to positions 156 to 266 of the amino acid sequence represented by SEQ ID NO: 17,
   a heavy chain variable region consisting of an amino acid sequence corresponding to positions 21 to 140 of the amino acid sequence represented by SEQ ID NO: 18 and a light chain variable region consisting of an amino acid sequence corresponding to positions 156 to 266 of the amino acid sequence represented by SEQ ID NO: 18,
   a heavy chain variable region consisting of an amino acid sequence corresponding to positions 21 to 140 of the amino acid sequence represented by SEQ ID NO: 19 and a light chain variable region consisting of an amino acid sequence corresponding to positions 156 to 266 of the amino acid sequence represented by SEQ ID NO: 19,
   a heavy chain variable region consisting of an amino acid sequence corresponding to positions 21 to 140 of the amino acid sequence represented by SEQ ID NO: 22 and a light chain variable region consisting of an amino acid sequence corresponding to positions 156 to 266 of the amino acid sequence represented by SEQ ID NO: 22,
   a heavy chain variable region consisting of an amino acid sequence corresponding to positions 21 to 140 of the amino acid sequence represented by SEQ ID NO: 24 and a light chain variable region consisting of an amino acid sequence corresponding to positions 156 to 266 of the amino acid sequence represented by SEQ ID NO: 24,
   a heavy chain variable region consisting of an amino acid sequence corresponding to positions 21 to 140 of the amino acid sequence represented by SEQ ID NO: 25 and a light chain variable region consisting of an amino acid sequence corresponding to positions 156 to 266 of the amino acid sequence represented by SEQ ID NO: 25,
   a heavy chain variable region consisting of an amino acid sequence corresponding to positions 21 to 140 of the amino acid sequence represented by SEQ ID NO: 26 and a light chain variable region consisting of an amino acid sequence corresponding to positions 156 to 266 of the amino acid sequence represented by SEQ ID NO: 26,
   a heavy chain variable region consisting of an amino acid sequence corresponding to positions 21 to 140 of the amino acid sequence represented by SEQ ID NO: 27 and a light chain variable region consisting of an amino acid sequence corresponding to positions 156 to 266 of the amino acid sequence represented by SEQ ID NO: 27,
   a heavy chain variable region consisting of an amino acid sequence corresponding to positions 21 to 140 of the amino acid sequence represented by SEQ ID NO: 28 and a light chain variable region consisting of an amino acid sequence corresponding to positions 156 to 266 of the amino acid sequence represented by SEQ ID NO: 28,
   a heavy chain variable region consisting of an amino acid sequence corresponding to positions 21 to 140 of the amino acid sequence represented by SEQ ID NO: 29 and a light chain variable region consisting of an amino acid sequence corresponding to positions 156 to 266 of the amino acid sequence represented by SEQ ID NO: 29,
   a heavy chain variable region consisting of an amino acid sequence corresponding to positions 21 to 140 of the amino acid sequence represented by SEQ ID NO: 21 and a light chain variable region consisting of an amino acid sequence corresponding to positions 156 to 266 of the amino acid sequence represented by SEQ ID NO: 21,
   a heavy chain variable region consisting of an amino acid sequence corresponding to positions 21 to 140 of the amino acid sequence represented by SEQ ID NO: 55 and a light chain variable region consisting of an amino acid sequence corresponding to positions 161 to 271 of the amino acid sequence represented by SEQ ID NO: 55, or a heavy chain variable region consisting of an amino acid sequence corresponding to positions 21 to 140 of the amino acid sequence represented by SEQ ID NO: 71 and a light chain variable region consisting of an amino acid sequence corresponding to positions 156 to 266 of the amino acid sequence represented by SEQ ID NO: 71.
(7) The pharmaceutical composition according to any one of (1) to (6), wherein the antibody that binds to HLA/NY-ESO or the antigen-binding fragment thereof is a scFv.
(8) The pharmaceutical composition according to any one of (1) to (7), wherein the antibody that binds to HLA/NY-ESO or the antigen-binding fragment thereof is a scFv consisting of an amino acid sequence from positions 21 to 266 of the amino acid sequence represented by SEQ ID NO: 30,
   a scFv comprising a heavy chain variable region consisting of the amino acid sequence represented by SEQ ID NO: 15 and a light chain variable region consisting of the amino acid sequence represented by SEQ ID NO: 16,
   a scFv consisting of an amino acid sequence corresponding to positions 21 to 266 of the amino acid sequence represented by SEQ ID NO: 20,
   a scFv consisting of an amino acid sequence corresponding to positions 21 to 266 of the amino acid sequence represented by SEQ ID NO: 17,
   a scFv consisting of an amino acid sequence corresponding to positions 21 to 266 of the amino acid sequence represented by SEQ ID NO: 18,
   a scFv consisting of an amino acid sequence corresponding to positions 21 to 266 of the amino acid sequence represented by SEQ ID NO: 19,
   a scFv consisting of an amino acid sequence corresponding to positions 21 to 266 of the amino acid sequence represented by SEQ ID NO: 22,
   a scFv consisting of an amino acid sequence corresponding to positions 21 to 266 of the amino acid sequence represented by SEQ ID NO: 24,
   a scFv consisting of an amino acid sequence corresponding to positions 21 to 266 of the amino acid sequence represented by SEQ ID NO: 25,
   a scFv consisting of an amino acid sequence corresponding to positions 21 to 266 of the amino acid sequence represented by SEQ ID NO: 26,
   a scFv consisting of an amino acid sequence corresponding to positions 21 to 266 of the amino acid sequence represented by SEQ ID NO: 27,
   a scFv consisting of an amino acid sequence corresponding to positions 21 to 266 of the amino acid sequence represented by SEQ ID NO: 28,
   a scFv consisting of an amino acid sequence corresponding to positions 21 to 266 of the amino acid sequence represented by SEQ ID NO: 29,
   a scFv consisting of an amino acid sequence corresponding to positions 21 to 266 of the amino acid sequence represented by SEQ ID NO: 21,
   a scFv consisting of an amino acid sequence corresponding to positions 21 to 271 of the amino acid sequence represented by SEQ ID NO: 55, or
   a scFv consisting of an amino acid sequence corresponding to positions 21 to 266 of the amino acid sequence represented by SEQ ID NO: 71.
(9) The pharmaceutical composition according to any one of (1) to (8), wherein the antibody that binds to CD3 or the antigen-binding fragment thereof comprises one or two or more sets of CDRH1 to CDRH3 and CDRL1 to CDRL3 selected from the group consisting of the following sets (i) to (x):
   (i)
      a heavy chain CDRH1 consisting of the amino acid sequence represented by SEQ ID NO: 7,
      a heavy chain CDRH2 consisting of the amino acid sequence represented by SEQ ID NO: 8,
      a heavy chain CDRH3 consisting of the amino acid sequence represented by SEQ ID NO: 9,
      a light chain CDRL1 consisting of the amino acid sequence represented by SEQ ID NO: 10,
      a light chain CDRL2 consisting of the amino acid sequence represented by Arg Asp Asp (the 5th sequence from the top in Figure 12; SEQ ID NO: 11 in Figure 82), and
      a light chain CDRL3 consisting of the amino acid sequence represented by SEQ ID NO: 12,
   (ii)
      a heavy chain CDRH1 consisting of the amino acid sequence represented by SEQ ID NO: 7,
      a heavy chain CDRH2 consisting of the amino acid sequence represented by SEQ ID NO: 8 in which the amino acid at position 3 is N,
      a heavy chain CDRH3 consisting of the amino acid sequence represented by SEQ ID NO: 9,
      a light chain CDRL1 consisting of the amino acid sequence represented by SEQ ID NO: 10,
      a light chain CDRL2 consisting of the amino acid sequence represented by Arg Asp Asp (the 5th sequence from the top in Figure 12; SEQ ID NO: 11 in Figure 82), and a light chain CDRL3 consisting of the amino acid sequence represented by SEQ ID NO: 12,
   (iii)
      a heavy chain CDRH1 consisting of the amino acid sequence represented by SEQ ID NO: 7,
      a heavy chain CDRH2 consisting of the amino acid sequence represented by SEQ ID NO: 8 in which the amino acid at position 3 is S,
      a heavy chain CDRH3 consisting of the amino acid sequence represented by SEQ ID NO: 9,
      a light chain CDRL1 consisting of the amino acid sequence represented by SEQ ID NO: 10,
      a light chain CDRL2 consisting of the amino acid sequence represented by Arg Asp Asp (the 5th sequence from the top in Figure 12; SEQ ID NO: 11 in Figure 82), and a light chain CDRL3 consisting of the amino acid sequence represented by SEQ ID NO: 12,
   (iv)
      a heavy chain CDRH1 consisting of the amino acid sequence represented by SEQ ID NO: 7,
      a heavy chain CDRH2 consisting of the amino acid sequence represented by SEQ ID NO: 8,
      a heavy chain CDRH3 consisting of the amino acid sequence represented by SEQ ID NO: 9,
      a light chain CDRL1 consisting of the amino acid sequence represented by SEQ ID NO: 10,
      a light chain CDRL2 consisting of the amino acid sequence represented by Arg Asp Asp (the 5th sequence from the top in Figure 12; SEQ ID NO: 11 in Figure 82) in which the amino acid at position 2 is G, and
      a light chain CDRL3 consisting of the amino acid sequence represented by SEQ ID NO: 12,
   (v)
      a heavy chain CDRH1 consisting of the amino acid sequence represented by SEQ ID NO: 7,
      a heavy chain CDRH2 consisting of the amino acid sequence represented by SEQ ID NO: 8,
      a heavy chain CDRH3 consisting of the amino acid sequence represented by SEQ ID NO: 9,
      a light chain CDRL1 consisting of the amino acid sequence represented by SEQ ID NO: 10,
      a light chain CDRL2 consisting of the amino acid sequence represented by Arg Asp Asp (the 5th sequence from the top in Figure 12' SEQ ID NO: 11 in Figure 82) in which the amino acid at position 2 is Q, and
      a light chain CDRL3 consisting of the amino acid sequence represented by SEQ ID NO: 12,
   (vi)
      a heavy chain CDRH1 consisting of the amino acid sequence represented by SEQ ID NO: 7,
      a heavy chain CDRH2 consisting of the amino acid sequence represented by SEQ ID NO: 8,
      a heavy chain CDRH3 consisting of the amino acid sequence represented by SEQ ID NO: 9,
      a light chain CDRL1 consisting of the amino acid sequence represented by SEQ ID NO: 10,
      a light chain CDRL2 consisting of the amino acid sequence represented by Arg Asp Asp (the 5th sequence from the top in Figure 12; SEQ ID NO: 11 in Figure 82) in which the amino acid at position 2 is N, and
      a light chain CDRL3 consisting of the amino acid sequence represented by SEQ ID NO: 12,
   (vii)
      a heavy chain CDRH1 consisting of the amino acid sequence represented by SEQ ID NO: 7,
      a heavy chain CDRH2 consisting of the amino acid sequence represented by SEQ ID NO: 8,
      a heavy chain CDRH3 consisting of the amino acid sequence represented by SEQ ID NO: 9,
      a light chain CDRL1 consisting of the amino acid sequence represented by SEQ ID NO: 10,
      a light chain CDRL2 consisting of the amino acid sequence represented by Arg Asp Asp (the 5th sequence from the top in Figure 12; SEQ ID NO: 11 in Figure 82) in which the amino acid at position 2 is S, and
      a light chain CDRL3 consisting of the amino acid sequence represented by SEQ ID NO: 12,
   (viii)
      a heavy chain CDRH1 consisting of the amino acid sequence represented by SEQ ID NO: 7,
      a heavy chain CDRH2 consisting of the amino acid sequence represented by SEQ ID NO: 8,
      a heavy chain CDRH3 consisting of the amino acid sequence represented by SEQ ID NO: 9,
      a light chain CDRL1 consisting of the amino acid sequence represented by SEQ ID NO: 10,
      a light chain CDRL2 consisting of the amino acid sequence represented by Arg Asp Asp (the 5th sequence from the top in Figure 12; SEQ ID NO: 11 in Figure 82) in which the amino acid at position 2 is A, and
      a light chain CDRL3 consisting of the amino acid sequence represented by SEQ ID NO: 12,
   (ix)
      a heavy chain CDRH1 consisting of the amino acid sequence represented by SEQ ID NO: 7,
      a heavy chain CDRH2 consisting of the amino acid sequence represented by SEQ ID NO: 8 in which the amino acid at position 3 is S,
      a heavy chain CDRH3 consisting of the amino acid sequence represented by SEQ ID NO: 9,
      a light chain CDRL1 consisting of the amino acid sequence represented by SEQ ID NO: 10,
      a light chain CDRL2 consisting of the amino acid sequence represented by Arg Asp Asp (the 5th sequence from the top
      in Figure 12; SEQ ID NO: 11 in Figure 82) in which the amino acid at position 2 is N, and
      a light chain CDRL3 consisting of the amino acid sequence represented by SEQ ID NO: 12
         and
   (x)
      a heavy chain CDRH1 consisting of the amino acid sequence represented by SEQ ID NO: 7,
      a heavy chain CDRH2 consisting of the amino acid sequence represented by SEQ ID NO: 8 in which the amino acid at position 3 is S,
      a heavy chain CDRH3 consisting of the amino acid sequence represented by SEQ ID NO: 9,
      a light chain CDRL1 consisting of the amino acid sequence represented by SEQ ID NO: 10,
      a light chain CDRL2 consisting of the amino acid sequence represented by Arg Asp Asp (the 5th sequence from the top in Figure 12; SEQ ID NO: 11 in Figure 82) in which the amino acid at position 2 is S, and
      a light chain CDRL3 consisting of the amino acid sequence represented by SEQ ID NO: 12.
(10) The pharmaceutical composition according to (9), wherein
   the antibody that binds to CD3 or the antigen-binding fragment thereof comprises
   a heavy chain variable region consisting of an amino acid sequence corresponding to positions 2 to 119 of the amino acid sequence represented by SEQ ID NO: 46,
   a heavy chain variable region consisting of an amino acid sequence corresponding to positions 2 to 119 of the amino acid sequence represented by SEQ ID NO: 47,
   a heavy chain variable region consisting of an amino acid sequence corresponding to positions 2 to 119 of the amino acid sequence represented by SEQ ID NO: 51,
   a heavy chain variable region consisting of an amino acid sequence corresponding to positions 2 to 119 of the amino acid sequence represented by SEQ ID NO: 48,
   a heavy chain variable region consisting of an amino acid sequence corresponding to positions 2 to 119 of the amino acid sequence represented by SEQ ID NO: 49,
   a heavy chain variable region consisting of an amino acid sequence corresponding to positions 2 to 119 of the amino acid sequence represented by SEQ ID NO: 50,
   a heavy chain variable region consisting of an amino acid sequence corresponding to positions 272 to 389 of the amino acid sequence represented by SEQ ID NO: 54,
   a heavy chain variable region consisting of an amino acid sequence corresponding to positions 277 to 394 of the amino acid sequence represented by SEQ ID NO: 55, or
   a heavy chain variable region consisting of an amino acid sequence from corresponding to 277 to 394 of the amino acid sequence represented by SEQ ID NO: 56, and
   a light chain variable region consisting of an amino acid sequence corresponding to positions 135 to 243 of the amino acid sequence represented by SEQ ID NO: 46,
   a light chain variable region consisting of an amino acid sequence corresponding to positions 135 to 241 of the amino acid sequence represented by SEQ ID NO: 47,
   a light chain variable region consisting of an amino acid sequence corresponding to positions 135 to 243 of the amino acid sequence represented by SEQ ID NO: 51,
   a light chain variable region consisting of an amino acid sequence corresponding to positions 135 to 241 of the amino acid sequence represented by SEQ ID NO: 48,
   a light chain variable region consisting of an amino acid sequence corresponding to positions 135 to 243 of the amino acid sequence represented by SEQ ID NO: 49,
   a light chain variable region consisting of an amino acid sequence corresponding to from positions 135 to 243 of the amino acid sequence represented by SEQ ID NO: 50,
   a light chain variable region consisting of an amino acid sequence corresponding to positions 405 to 511 of the amino acid sequence represented by SEQ ID NO: 54,
   a light chain variable region consisting of an amino acid sequence corresponding to positions 410 to 516 of the amino acid sequence represented by SEQ ID NO: 55, or
   a light chain variable region consisting of an amino acid sequence corresponding to positions 410 to 516 of the amino acid sequence represented by SEQ ID NO: 56.
(11) The pharmaceutical composition according to (8) or (9), wherein
   the antibody that binds to CD3 or the antigen-binding fragment thereof comprises
   a heavy chain variable region consisting of an amino acid sequence corresponding to positions 2 to 119 of the amino acid sequence represented by SEQ ID NO: 46 and a light chain variable region consisting of an amino acid sequence corresponding to positions 135 to 243 of the amino acid sequence represented by SEQ ID NO: 46,
   a heavy chain variable region consisting of an amino acid sequence corresponding to positions 2 to 119 of the amino acid sequence represented by SEQ ID NO: 47 and a light chain variable region consisting of an amino acid sequence corresponding to positions 135 to 241 of the amino acid sequence represented by SEQ ID NO: 47,
   a heavy chain variable region consisting of an amino acid sequence corresponding to positions 2 to 119 of the amino acid sequence represented by SEQ ID NO: 51 and a light chain variable region consisting of an amino acid sequence corresponding to positions 135 to 243 of the amino acid sequence represented by SEQ ID NO: 51,
   a heavy chain variable region consisting of an amino acid sequence corresponding to positions 2 to 119 of the amino acid sequence represented by SEQ ID NO: 48 and a light chain variable region consisting of an amino acid sequence corresponding to positions 135 to 241 of the amino acid sequence represented by SEQ ID NO: 48,
   a heavy chain variable region consisting of an amino acid sequence corresponding to positions 2 to 119 of the amino acid sequence represented by SEQ ID NO: 49 and a light chain variable region consisting of an amino acid sequence corresponding to positions 135 to 243 of the amino acid sequence represented by SEQ ID NO: 49,
   a heavy chain variable region consisting of an amino acid sequence corresponding to positions 2 to 119 of the amino acid sequence represented by SEQ ID NO: 50 and a light chain variable region consisting of an amino acid sequence corresponding to positions 135 to 243 of the amino acid sequence represented by SEQ ID NO: 50,
   a heavy chain variable region consisting of an amino acid sequence corresponding to positions 272 to 389 of the amino acid sequence represented by SEQ ID NO: 54 and a light chain variable region consisting of an amino acid sequence corresponding to positions 405 to 511 of the amino acid sequence represented by SEQ ID NO: 54,
   a heavy chain variable region consisting of an amino acid sequence corresponding to positions 277 to 394 of the amino acid sequence represented by SEQ ID NO: 55 and a light chain variable region consisting of an amino acid sequence corresponding to positions 410 to 516 of the amino acid sequence represented by SEQ ID NO: 55, or
   a heavy chain variable region consisting of an amino acid sequence corresponding to positions 277 to 394 of the amino acid sequence represented by SEQ ID NO: 56 and a light chain variable region consisting of an amino acid sequence corresponding to positions 410 to 516 of the amino acid sequence represented by SEQ ID NO: 56.
(12) The pharmaceutical composition according to any one of (1) to (11), wherein the antibody that binds to CD3 or the antigen-binding fragment thereof is a scFv.
(13) The pharmaceutical composition according to any one of (1) to (12), wherein the antibody that binds to CD3 or the antigen-binding fragment thereof is
   a scFv consisting of an amino acid sequence corresponding to positions 2 to 243 of the amino acid sequence represented by SEQ ID NO: 46,
   a scFv consisting of an amino acid sequence corresponding to positions 2 to 241 of the amino acid sequence represented by SEQ ID NO: 47,
   a scFv consisting of an amino acid sequence corresponding to positions 2 to 243 of the amino acid sequence represented by SEQ ID NO: 51,
   a scFv consisting of an amino acid sequence corresponding to positions 2 to 241 of the amino acid sequence represented by SEQ ID NO: 48,
   a scFv consisting of an amino acid sequence corresponding to positions 2 to 243 of the amino acid sequence represented by SEQ ID NO: 49,
   a scFv consisting of an amino acid sequence corresponding to positions 2 to 243 of the amino acid sequence represented by SEQ ID NO: 50,
   a scFv consisting of an amino acid sequence corresponding to positions 272 to 511 of the amino acid sequence represented by SEQ ID NO: 54,
   a scFv consisting of an amino acid sequence corresponding to positions 277 to 516 of the amino acid sequence represented by SEQ ID NO: 55, or
   a scFv consisting of an amino acid sequence corresponding to positions 277 to 516 of the amino acid sequence represented by SEQ ID NO: 56.
(14) The pharmaceutical composition according to any one of (1) to (13), wherein the pharmaceutical composition comprises: a first polypeptide comprising the antibody that binds to HLA/NY-ESO or the antigen-binding fragment thereof which is a scFv, the antibody that binds to CD3 or the antigen-binding fragment thereof which is a scFv, and an Fc region (i) in that order from the N-terminus towards the C-terminus; and a second polypeptide comprising an Fc region (ii), wherein preferably, the first polypeptide and the second polypeptide are associated with each other via the Fc region (i) and the Fc region (ii).
(15) The pharmaceutical composition according to (14), wherein the first polypeptide comprises an amino acid sequence corresponding to positions 21 to 511 of the amino acid sequence represented by SEQ ID NO: 32, an amino acid sequence corresponding to positions 21 to 511 of the amino acid sequence represented by SEQ ID NO: 34, an amino acid sequence corresponding to positions 21 to 511 of the amino acid sequence represented by SEQ ID NO: 35, an amino acid sequence corresponding to positions 21 to 511 of the amino acid sequence represented by SEQ ID NO: 36, an amino acid sequence corresponding to positions 21 to 511 of the amino acid sequence represented by SEQ ID NO: 37, an amino acid sequence corresponding to positions 21 to 511 of the amino acid sequence represented by SEQ ID NO: 38, an amino acid sequence corresponding to positions 21 to 511 of the amino acid sequence represented by SEQ ID NO: 39, an amino acid sequence corresponding to positions 21 to 511 of the amino acid sequence represented by SEQ ID NO: 40, an amino acid sequence corresponding to positions 21 to 511 of the amino acid sequence represented by SEQ ID NO: 41, an amino acid sequence corresponding to positions 21 to 511 of the amino acid sequence represented by SEQ ID NO: 42, an amino acid sequence corresponding to positions 21 to 511 of the amino acid sequence represented by SEQ ID NO: 43, an amino acid sequence corresponding to positions 21 to 511 of the amino acid sequence represented by SEQ ID NO: 33, an amino acid sequence corresponding to positions 21 to 511 of the amino acid sequence represented by SEQ ID NO: 52, an amino acid sequence corresponding to positions 21 to 511 of the amino acid sequence represented by SEQ ID NO: 53, an amino acid sequence corresponding to positions 21 to 511 of the amino acid sequence represented by SEQ ID NO: 54, an amino acid sequence corresponding to positions 21 to 516 of the amino acid sequence represented by SEQ ID NO: 55, or an amino acid sequence corresponding to positions 21 to 516 of the amino acid sequence represented by SEQ ID NO: 56.
(16) The pharmaceutical composition according to (14) or (15), wherein the first polypeptide comprises an amino acid sequence corresponding to positions 529 to 745 of the amino acid sequence represented by SEQ ID NO: 32, 34, 35, 36, 37, 38, 39, 40, 41, 42, 43, 33, 52, 53, or 54, or an amino acid sequence corresponding to positions 534 to 750 of the amino acid sequence represented by SEQ ID NO: 55 or 56.
(17) The pharmaceutical composition according to any one of (14) to (16), wherein the first polypeptide consists of an amino acid sequence corresponding to positions 20 to 745 of the amino acid sequence represented by SEQ ID NO: 32, an amino acid sequence corresponding to positions 20 to 745 of the amino acid sequence represented by SEQ ID NO: 34, an amino acid sequence corresponding to positions 20 to 745 of the amino acid sequence represented by SEQ ID NO: 35, an amino acid sequence corresponding to positions 20 to 745 of the amino acid sequence represented by SEQ ID NO: 36, an amino acid sequence corresponding to positions 20 to 745 of the amino acid sequence represented by SEQ ID NO: 37, an amino acid sequence corresponding to positions 20 to 745 of the amino acid sequence represented by SEQ ID NO: 38, an amino acid sequence corresponding to positions 20 to 745 of the amino acid sequence represented by SEQ ID NO: 39, an amino acid sequence corresponding to positions 20 to 745 of the amino acid sequence represented by SEQ ID NO: 40, an amino acid sequence corresponding to positions 20 to 745 of the amino acid sequence represented by SEQ ID NO: 41, an amino acid sequence corresponding to positions 20 to 745 of the amino acid sequence represented by SEQ ID NO: 42, an amino acid sequence corresponding to positions 20 to 745 of the amino acid sequence represented by SEQ ID NO: 43, an amino acid sequence corresponding to positions 20 to 745 of the amino acid sequence represented by SEQ ID NO: 33, an amino acid sequence corresponding to positions 20 to 745 of the amino acid sequence represented by SEQ ID NO: 52, an amino acid sequence corresponding to positions 20 to 745 of the amino acid sequence represented by SEQ ID NO: 53, an amino acid sequence corresponding to positions 20 to 745 of the amino acid sequence represented by SEQ ID NO: 54, an amino acid sequence corresponding to positions 20 to 750 of the amino acid sequence represented by SEQ ID NO: 55, or an amino acid sequence corresponding to positions 20 to 750 of the amino acid sequence represented by SEQ ID NO: 56.
(18) The pharmaceutical composition according to any one of (14) to (17), wherein the second polypeptide comprises an amino acid sequence corresponding to positions 20 to 246 of the amino acid sequence represented by SEQ ID NO: 31.
(19) The pharmaceutical composition according to any one of (1) to (13), wherein the pharmaceutical composition comprises a first polypeptide, a second polypeptide, and a third polypeptide,
   the first polypeptide comprising the antibody that binds to HLA/NY-ESO or the antigen-binding fragment thereof which is a scFv, an antibody that specifically binds to CD3 or an antigen-binding fragment thereof which is a scFv, and an Fc region (i) in that order from the N-terminus towards the C-terminus,
   the second polypeptide consisting of an immunoglobulin heavy chain comprising an Fc region (ii), and
   the third polypeptide consisting of an immunoglobulin light chain, wherein
   preferably, the second polypeptide and the third polypeptide are associated with each other, and
   the first polypeptide and the second peptide are associated with each other via their respective Fc regions.
(20) The pharmaceutical composition according to (19), wherein the second polypeptide comprises an amino acid sequence corresponding to amino acid positions 20 to 242 of the amino acid sequence represented by SEQ ID NO: 44.
(21) The pharmaceutical composition according to (19) or (20), wherein the third polypeptide comprises the amino acid sequence represented by SEQ ID NO: 45.
(22) The pharmaceutical composition according to any one of (19) to (21), wherein the first polypeptide comprises an amino acid sequence corresponding to positions 21 to 511 of the amino acid sequence represented by SEQ ID NO: 32, an amino acid sequence corresponding to positions 21 to 511 of the amino acid sequence represented by SEQ ID NO: 34, an amino acid sequence corresponding to positions 21 to 511 of the amino acid sequence represented by SEQ ID NO: 35, an amino acid sequence corresponding to positions 21 to 511 of the amino acid sequence represented by SEQ ID NO: 36, an amino acid sequence corresponding to positions 21 to 511 of the amino acid sequence represented by SEQ ID NO: 37, an amino acid sequence corresponding to positions 21 to 511 of the amino acid sequence represented by SEQ ID NO: 38, an amino acid sequence corresponding to positions 21 to 511 of the amino acid sequence represented by SEQ ID NO: 39, an amino acid sequence corresponding to positions 21 to 511 of the amino acid sequence represented by SEQ ID NO: 40, an amino acid sequence corresponding to positions 21 to 511 of the amino acid sequence represented by SEQ ID NO: 41, an amino acid sequence corresponding to positions 21 to 511 of the amino acid sequence represented by SEQ ID NO: 42, an amino acid sequence corresponding to positions 21 to 511 of the amino acid sequence represented by SEQ ID NO: 43, an amino acid sequence corresponding to positions 21 to 511 of the amino acid sequence represented by SEQ ID NO: 33, an amino acid sequence corresponding to positions 21 to 511 of the amino acid sequence represented by SEQ ID NO: 52, an amino acid sequence corresponding to positions 21 to 511 of the amino acid sequence represented by SEQ ID NO: 53, an amino acid sequence corresponding to positions 21 to 511 of the amino acid sequence represented by SEQ ID NO: 54, an amino acid sequence corresponding to positions 21 to 516 of the amino acid sequence represented by SEQ ID NO: 55, or an amino acid sequence corresponding to positions 21 to 516 of the amino acid sequence represented by SEQ ID NO: 56.
(23) The pharmaceutical composition according to any one of (19) to (22), wherein the first polypeptide consists of an amino acid sequence corresponding to positions 20 to 745 of the amino acid sequence represented by SEQ ID NO: 32, an amino acid sequence corresponding to positions 20 to 745 of the amino acid sequence represented by SEQ ID NO: 34, an amino acid sequence corresponding to positions 20 to 745 of the amino acid sequence represented by SEQ ID NO: 35, an amino acid sequence corresponding to positions 20 to 745 of the amino acid sequence represented by SEQ ID NO: 36, an amino acid sequence corresponding to positions 20 to 745 of the amino acid sequence represented by SEQ ID NO: 37, an amino acid sequence corresponding to positions 20 to 745 of the amino acid sequence represented by SEQ ID NO: 38, an amino acid sequence corresponding to positions 20 to 745 of the amino acid sequence represented by SEQ ID NO: 39, an amino acid sequence corresponding to positions 20 to 745 of the amino acid sequence represented by SEQ ID NO: 40, an amino acid sequence corresponding to positions 20 to 745 of the amino acid sequence represented by SEQ ID NO: 41, an amino acid sequence corresponding to positions 20 to 745 of the amino acid sequence represented by SEQ ID NO: 42, an amino acid sequence corresponding to positions 20 to 745 of the amino acid sequence represented by SEQ ID NO: 43, an amino acid sequence corresponding to positions 20 to 745 of the amino acid sequence represented by SEQ ID NO: 33, an amino acid sequence corresponding to positions 20 to 745 of the amino acid sequence represented by SEQ ID NO: 52, an amino acid sequence corresponding to positions 20 to 745 of the amino acid sequence represented by SEQ ID NO: 53, an amino acid sequence corresponding to positions 20 to 745 of the amino acid sequence represented by SEQ ID NO: 54, an amino acid sequence corresponding to positions 20 to 750 of the amino acid sequence represented by SEQ ID NO: 55, or an amino acid sequence corresponding to positions 20 to 750 of the amino acid sequence represented by SEQ ID NO: 56.
(24) The pharmaceutical composition according to any one of (9) to (11), wherein the pharmaceutical composition comprises:
   a first polypeptide comprising an antibody that specifically binds to HLA/NY-ESO or an antigen-binding fragment thereof which is a scFv, a heavy chain variable region and constant region CH1 of the antibody that binds to CD3, and an immunoglobulin Fc region (i) in that order from the N-terminus towards the C-terminus;
   a second polypeptide comprising an immunoglobulin hinge region and Fc region (ii); and
   a third polypeptide comprising a light chain of the antibody that binds to CD3, the light chain consisting of a variable region and a constant region, wherein preferably, the first polypeptide and the second polypeptide are associated with each other via the Fc region (i) and the Fc region (ii), and the first polypeptide is associated with the third polypeptide at the antibody heavy chain variable region and constant region CH1.
(25) The pharmaceutical composition according to any one of (9) to (11) and (24), wherein the first polypeptide comprises an amino acid sequence corresponding to positions 20 to 724 of the amino acid sequence represented by SEQ ID NO: 57, an amino acid sequence corresponding to positions 20 to 719 of the amino acid sequence represented by SEQ ID NO: 60, or an amino acid sequence corresponding to positions 20 to 719 of the amino acid sequence represented by SEQ ID NO: 61.
(26) The pharmaceutical composition according to any one of (9) to (11), (24) and (25), wherein the second polypeptide comprises an amino acid sequence corresponding to positions 20 to 246 of the amino acid sequence represented by SEQ ID NO: 31.
(27) The pharmaceutical composition according to any one of (9) to (11) and (24) to (26), wherein the third polypeptide comprises an amino acid sequence corresponding to positions 21 to 127 of the amino acid sequence represented by SEQ ID NO: 58.
(28) The pharmaceutical composition according to any one of (9) to (11) and (24) to (27), wherein the third polypeptide comprises an amino acid sequence corresponding to positions 21 to 233 of the amino acid sequence represented by SEQ ID NO: 58.
(29) The pharmaceutical composition according to any one of (1) to (28), wherein the amino acid sequences of one, two or more polypeptides contained in the pharmaceutical composition lack one or two carboxy-terminal amino acids.
(30) The pharmaceutical composition according to any one of (1) to (29), wherein the cancer is one,two or more members selected from the group consisting of kidney cancer, melanoma, squamous cell cancer, basal cell cancer, conjunctival cancer, oral cancer, larynx cancer, throat cancer, thyroid cancer, lung cancer (non-small cell lung cancer (adenocarcinoma, squamous cell cancer, and large-cell cancer) and small-cell lung cancer), breast cancer, esophageal cancer, stomach cancer, duodenum cancer, small intestine cancer, large intestine cancer, rectal cancer, appendix cancer, anus cancer, liver cancer, gallbladder cancer, bile duct cancer, pancreatic cancer, adrenal cancer, urinary bladder cancer, prostate cancer, uterine cancer, vaginal cancer, liposarcoma, angiosarcoma, chondrosarcoma, rhabdomyosarcoma, Ewing's sarcoma, osteosarcoma, undifferentiated pleomorphic sarcoma, myxofibrosarcoma, malignant peripheral nerve sheath tumor, retroperitoneal sarcoma, synovial sarcoma, uterine sarcoma, gastrointestinal stromal tumor, leiomyosarcoma, epithelioid sarcoma, B cell lymphoma, T/NK cell lymphoma, Hodgkin's lymphoma, myeloid leukemia, lymphoid leukemia, myeloproliferative disease, myelodysplastic syndrome, multiple myeloma, testicle cancer, and ovary cancer.
(31) The pharmaceutical composition according to any one of (1) to (30), wherein the pharmaceutical composition is administered after the compound that inhibits an immune checkpoint molecule or the chemotherapeutic.
(32) The pharmaceutical composition according to any one of (1) to (30), wherein the pharmaceutical composition is administered before the compound that inhibits an immune checkpoint molecule or the chemotherapeutic.
(33) The pharmaceutical composition according to any one of (1) to (30), wherein the pharmaceutical composition is administered concurrently with the compound that inhibits an immune checkpoint molecule or the chemotherapeutic.
(34) The pharmaceutical composition according to any one of (1) to (30) and (33), wherein the pharmaceutical composition comprises the compound that inhibits an immune checkpoint molecule or the chemotherapeutic.
(35) The pharmaceutical composition according to any one of (1) to (34), wherein the pharmaceutical composition is used in combination with one,two or more additional medicaments and/or therapies.
(36) The pharmaceutical composition according to any one of (1) to (35), wherein compound [ii] is a compound that inhibits an immune checkpoint molecule.
(37) The pharmaceutical composition according to any one of (1) to (36), wherein the immune checkpoint molecule is selected from the group consisting of PD-1, PD-L1, PD-L2, CTLA-4, and TIGIT.
(38) The pharmaceutical composition according to any one of (1) to (37), wherein the immune checkpoint molecule is PD-1.
(39) The pharmaceutical composition according to (38), wherein the compound that inhibits the immune checkpoint molecule is nivolumab or pembrolizumab, an antigen-binding fragment thereof, or a compound comprising the antigen-binding fragment thereof.
(40) The pharmaceutical composition according to any one of (1) to (37), wherein the immune checkpoint molecule is PD-L1.
(41) The pharmaceutical composition according to (40), wherein the compound that inhibits the immune checkpoint molecule is atezolizumab, durvalumab, or avelumab, an antigen-binding fragment thereof, or a compound comprising the antigen-binding fragment thereof.
(42) The pharmaceutical composition according to any one of (1) to (37), wherein the immune checkpoint molecule is CTLA-4.
(43) The pharmaceutical composition according to (42), wherein the compound that inhibits the immune checkpoint molecule is ipilimumab, tremelimumab, spartalizumab, or cemiplimab, an antigen-binding fragment thereof, or a compound comprising the antigen-binding fragment thereof.
(44) The pharmaceutical composition according to any one of (1) to (37), wherein the immune checkpoint molecule is TIGIT.
(45) The pharmaceutical composition according to (44), wherein the compound that inhibits the immune checkpoint molecule is tiragolumab or vibostolimab, an antigen-binding fragment thereof, or a compound comprising the antigen-binding fragment thereof.
(46) The pharmaceutical composition according to any one of (1) to (36), wherein the compound that inhibits an immune checkpoint molecule is pembrolizumab, nivolumab, spartalizumab, cemiplimab, avelumab, atezolizumab, durvalumab, ipilimumab, or tremelimumab.
(47) The pharmaceutical composition according to any one of (1) to (39), wherein the compound that inhibits an immune checkpoint molecule is pembrolizumab, an antigen-binding fragment of pembrolizumab, or a compound comprising the antigen-binding fragment thereof.
(48) The pharmaceutical composition according to any one of (1) to (35), wherein compound [ii] is a chemotherapeutic.
(49) The pharmaceutical composition according to any one of (1) to (35) and (48), wherein the chemotherapeutic is a microtubule inhibitor.
(50) The pharmaceutical composition according to any one of (1) to (35), (48) and (49), wherein the chemotherapeutic is a taxane-based microtubule inhibitor.
(51) The pharmaceutical composition according to any one of (1) to (35) and (48) to (50), wherein the chemotherapeutic is selected from the group consisting of paclitaxel, docetaxel, vincristine, vinblastine, vindesine, eribulin, vinorelbine, albumin-bound paclitaxel, oxaliplatin, carboplatin, cisplatin, nedaplatin, azacytidine, decitabine, doxorubicin, bleomycin, liposomal doxorubicin, ifosfamide, cyclophosphamide, and dacarbazine.
(52) The pharmaceutical composition according to any one of (1) to (35) and (48) to (51), wherein the chemotherapeutic is paclitaxel or albumin-bound paclitaxel.
(53) The pharmaceutical composition according to any one of (1) to (52), wherein the pharmaceutical composition is further used in combination with a multikinase inhibitor.
(54) The pharmaceutical composition according to (53), wherein the multikinase inhibitor is one,two or more members selected from the group consisting of sorafenib, sunitinib, pazopanib, regorafenib, and lenvatinib.
(55) The pharmaceutical composition according to (53) or (54), wherein the multikinase inhibitor is lenvatinib.
(56)The pharmaceutical composition according to any one of (53) to (55), wherein the compound [ii] is the compound that inhibits an immune checkpoint molecule, preferably pembrolizumab, an antigen-binding fragment of pembrolizumab, or a compound comprising the antigen-binding fragment thereof.
(57) The pharmaceutical composition according to any one of (53) to (56), wherein the compound that inhibits an immune checkpoint molecule is pembrolizumab, an antigen-binding fragment of pembrolizumab, or a compound comprising the antigen-binding fragment thereof, and the multikinase inhibitor is lenvatinib.
(58) The pharmaceutical composition according to any one of (1) to (35) and (48) to (52), wherein the chemotherapeutic is a taxane-based microtubule inhibitor selected from the group consisting of paclitaxel, docetaxel, vincristine, vinblastine, vindesine, eribulin, vinorelbine, and albumin-bound paclitaxel.
(59) [i] A multispecific antibody and [ii] a compound that inhibits an immune checkpoint molecule or a chemotherapeutic according to any one of (1) to (47) for the treatment and/or prevention of a cancer.
(60) [i] A multispecific antibody and [ii] a compound that inhibits an immune checkpoint molecule or a chemotherapeutic, and a multikinase inhibitor according to any one of (53) to (57) for the treatment and/or prevention of a cancer.
(61) [i] A multispecific antibody and [ii] a compound that inhibits an immune checkpoint molecule or a chemotherapeutic according to any one of (1) to (47) for the treatment and/or prevention of a cancer.
(62) Use of [i] a multispecific antibody and [ii] a compound that inhibits an immune checkpoint molecule or a chemotherapeutic, and a multikinase inhibitor according to any one of (53) to (57) for preparing a pharmaceutical composition for the treatment and/or prevention of a cancer.
(63) A method for treating and/or preventing a cancer, comprising administering [i] a multispecific antibody and [ii] a compound that inhibits an immune checkpoint molecule or a chemotherapeutic according to any one of (1) to (47).
(64) A method for treating and/or preventing a cancer, comprising administering [i] a multispecific antibody and [ii] a compound that inhibits an immune checkpoint molecule or a chemotherapeutic, and a multikinase inhibitor according to any one of (53) to (57) or the like.

### Advantageous Effects of Invention

The combination of an antibody and another agent provided in accordance with the present invention enables various cancers to be treated or prevented.

### Brief Description of Drawings

[Figure 1(1)] Figure 1(1) is a diagram showing antitumor activity achieved by single administration of an Fc-conjugated type anti-HLA-A2/NY-ESO-anti-CD3 bispecific molecule NYZ-1010, single administration of paclitaxel, and combined administration of NYZ-1010 and paclitaxel to human T cell transfer models. The error bar in the diagram represents standard deviation (n = 5).

[Figure 1(2)] Figure 1(2) is a diagram showing the percentage change in tumor volume on Day 31 of each individual mouse using a tumor volume on Day 12 as a baseline following single administration of an Fc-conjugated type anti-HLA-A2/NY-ESO-anti-CD3 bispecific molecule NYZ-1010, single administration of paclitaxel, and combined administration of NYZ-1010 and paclitaxel to human T cell transfer models.

[Figure 2(1)] Figure 2(1) is a diagram showing antitumor activity achieved by single administration of an Fc-conjugated type anti-HLA-A2/NY-ESO-anti-CD3 bispecific molecule NYZ-1010, single administration of Nab paclitaxel, and combined administration of NYZ-1010 and Nab paclitaxel to human T cell transfer models. The error bar in the diagram represents standard deviation (n = 5).

[Figure 2(2)] Figure 2(2) is a diagram showing the percentage change in tumor volume on Day 38 of each individual mouse using a tumor volume on Day 13 as a baseline following single administration of an Fc-conjugated type anti-HLA-A2/NY-ESO-anti-CD3 bispecific molecule NYZ-1010, single administration of Nab paclitaxel, and combined administration of NYZ-1010 and Nab paclitaxel to human T cell transfer models.

[Figure 3] Figure 3 is a diagram showing antitumor activity achieved by single administration of an Fc-conjugated type anti-HLA-A2/NY-ESO-anti-CD3 bispecific molecule NYZ-1010, single administration of pembrolizumab, and combined administration of NYZ-1010 and pembrolizumab to human T cell transfer models. The error bar in the diagram represents standard deviation (n = 5).

[Figure 4] Figure 4 is a diagram showing antitumor activity achieved by single administration of an Fc-conjugated type anti-HLA-A2/NY-ESO-anti-CD3 bispecific molecule NYF-0016, single administration of an anti-mouse PD-1 antibody, and combined administration of NYF-0016 and an anti-mouse PD-1 antibody to human CD3ε knock-in mouse models. The error bar in the diagram represents standard deviation (n = 5).

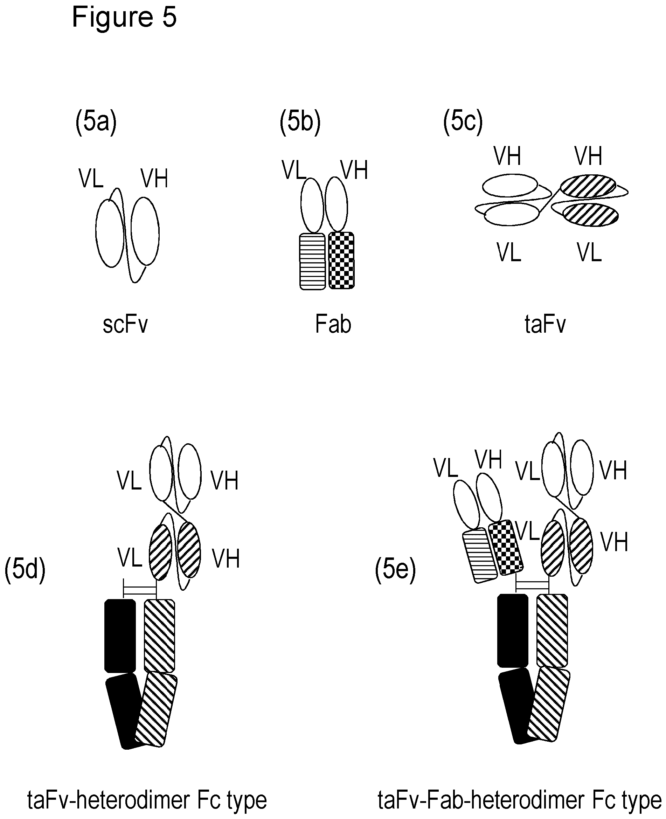

[Figure 5] Figure 5 is a diagram showing antibody formats which may be used in accordance with the present invention. (5a) scFv: a format of an antibody H chain variable region (VH, mentioned below) and an antibody L chain variable region (VL, mentioned below) (both unfilled) connected by a linker. In Examples of the present invention, anti-HLA-A2/NY-ESO scFv and anti-CD3 scFv were used in evaluation. (5b) Fab: a format consisting of VH (unfilled)-antibody H chain constant region (CH1: checked) and VL (unfilled)-antibody L chain constant region (horizontal line). In Examples of the present invention, anti-HLA-A2/NY-ESO Fab and the like were used in evaluation. (5c) taFv: a format of two types of scFv molecules (unfilled and positively hatched, respectively) connected via linkers. In Examples of the present invention,a taFv comprising anti-HLA-A2/NY-ESO scFv and anti-CD3 scFv was used in evaluation. (5d) taFv-heterodimer Fc type: a format in which Fc which is mutated to form a heterodimer (negatively hatched) is conjugated to the C-terminus of taFv (also referred to as a first polypeptide) and hetero-associated with another Fc (solid: also referred to as a second polypeptide). In Examples of the present invention,a taFv-heterodimer Fc comprising anti-HLA-A2/NY-ESO scFv and anti-CD3 scFv was used. (5e) taFv-Fab-heterodimer Fc type: a format of Fab added to the taFv-heterodimer Fc type. In Examples of the present invention, a taFv-Fab-heterodimer Fc obtained using taFv comprising anti-HLA-A2/NY-ESO scFv and anti-CD3 scFv, and HLA-A2/NY-ESO Fab, and the like were used in evaluation.

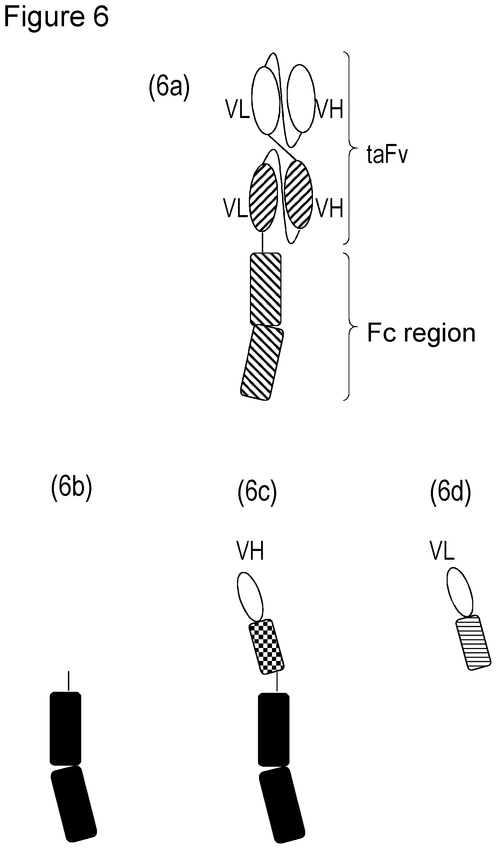

[Figure 6] Figure 6 is a diagram showing antibody formats which may be used in accordance with the present invention. (6a) A first polypeptide is shown which is common to the taFv-heterodimer Fc type and taFv-Fab-heterodimer Fc type. The first polypeptide comprises a scFv that specifically binds to human HLA/NY-ESO, a scFv that specifically binds to CD3, and an Fc region (i) in that order from the N-terminus towards the C-terminus. (6b) A second polypeptide in the taFv-heterodimer Fc type is shown. The second polypeptide comprises a hinge region and an Fc region (ii). (6c) A second polypeptide in the taFv-Fab-heterodimer Fc type is shown. The second polypeptide comprises an immunoglobulin heavy chain comprising a hinge region and an Fc region (ii). (6d) A third polypeptide in the taFv-Fab-heterodimer Fc type is shown. The third polypeptide comprises an immunoglobulin light chain.

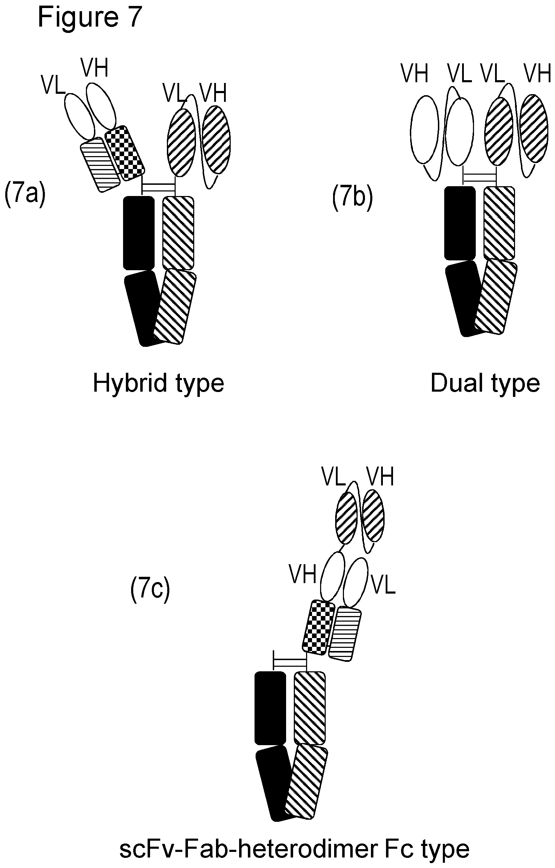

[Figure 7] Figure 7 is a diagram showing antibody formats which may be used in accordance with the present invention. (7a) Hybrid type: a format in which Fc mutated to form a heterodimer (negatively hatched and solid, respectively) is conjugated to the C-terminus of each of Fab and scFv and these two Fc molecules are associated with each other. In Examples of the present invention, a hybrid type comprising anti-HLA-A2/NY-ESO Fab and anti-CD3 scFv was used in evaluation. (7b) Dual type: a format in which Fc which is mutated to form a heterodimer (negatively hatched and solid) is conjugated to the C-terminus of each of two types of scFv molecules and these two Fc molecules are hetero-associated with each other. In Examples of the present invention, a dual type format comprising anti-HLA-A2/NY-ESO scFv and anti-CD3 scFv was used in evaluation. (7c) scFv-Fab-heterodimer Fc type: a Fc which is mutated to form a heterodimer (negatively hatched) is conjugated to the C-terminus of scFv and Fab and connected by a linker, and associated with another Fc (solid). In Examples of the present invention, a scFv-Fab-heterodimer Fc type comprising anti-CD3 scFv (positively hatched) and anti-HLA-A2/NY-ESO Fab was used in evaluation. Also, a scFv-Fab-heterodimer Fc type comprising anti-HLA-A2/NY-ESO scFv (positively hatched) and anti-CD3 Fab was used in evaluation.

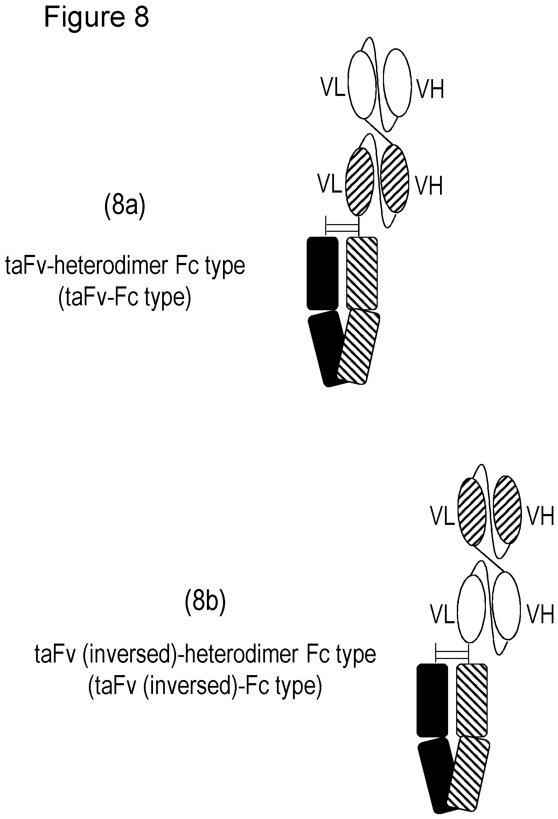

[Figure 8] Figure 8 is a diagram showing antibody formats which may be used in accordance with the present invention. (8a) taFv-heterodimer Fc type: the same as in Figure 5(5d). In this format, a Fc which is mutated to form a heterodimer (negatively hatched) is conjugated to the C-terminus of taFv and hetero-associated with another Fc (solid). In Examples of the present invention, taFv-heterodimer Fc type consisting of anti-HLA-A2/NY-ESO scFv and anti-CD3 scFv was used in evaluation. (8b) taFv (inversed)-heterodimer Fc type: the order of two scFv molecules in the taFv-heterodimer Fc type is reversed with respect to 8(a).

[Figure 9] Figure 9 is a diagram showing antibody formats which may be used in accordance with the present invention. (9a) A first polypeptide in a taFv (inversed)-heterodimer Fc type is shown. The first polypeptide comprises a scFv that specifically binds to CD3, a scFv that specifically binds to human HLA/NY-ESO, and an Fc region (i) in that order from the N-terminus towards the C-terminus. (9b) A second polypeptide in the taFv (inversed)-heterodimer Fc type is shown. The second polypeptide comprises a hinge region and an Fc region (ii).

[Figure 10] Figure 10 shows the amino acid sequence of human CD3ε. This sequence is registered under accession No: NP_000724 (NM_000724.1) in NCBI/GenPept.

[Figure 11] Figure 11 shows the amino acid sequences (SEQ ID NOs: 1 to 4 and 6; the 5th sequence is DNN and corresponds to SEQ ID NO: 5 in Figure 82) of CDRH1 to CDRH3 and CDRL1 to CDRL3 contained in NYA-0001.

[Figure 12] Figure 12 shows the amino acid sequences (SEQ ID NOs: 7 to 10 and 12; the 11th sequence is RDD and corresponds to SEQ ID NO: 11 in Figure 82) of heavy chain CDRH1 to CDRH3 and light chain CDRL1 to CDRL3 of C3E-7085.

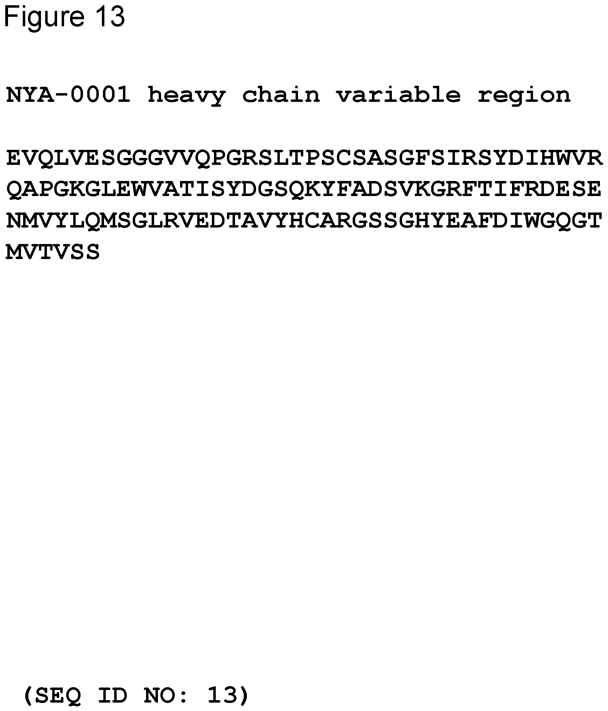

[Figure 13] Figure 13 shows the amino acid sequence (SEQ ID NO: 13) of the heavy chain variable region of NYA-0001.

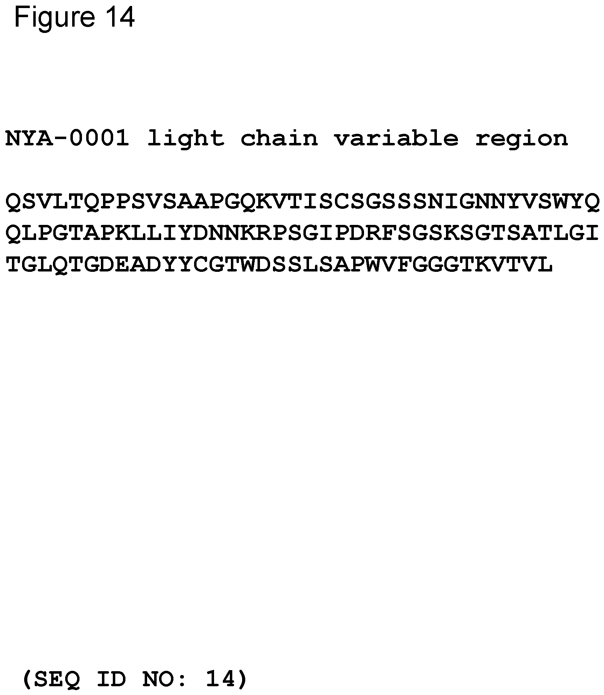

[Figure 14] Figure 14 shows the amino acid sequence (SEQ ID NO: 14) of the light chain variable region of NYA-0001.

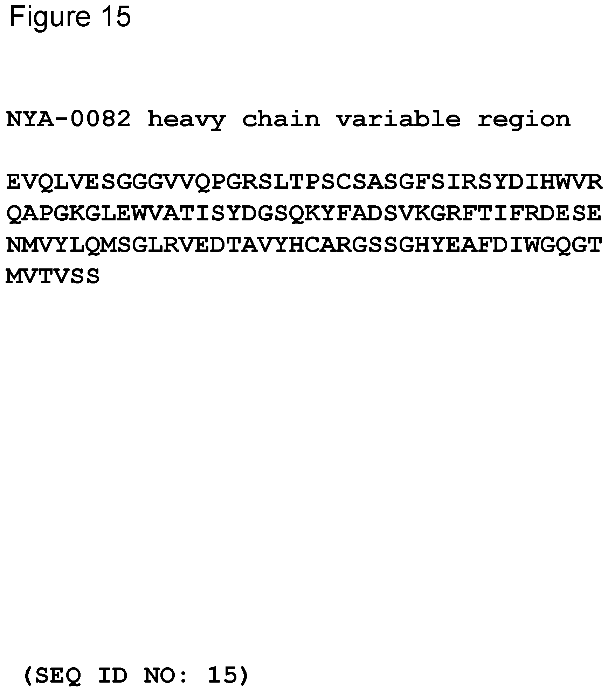

[Figure 15] Figure 15 shows the amino acid sequence (SEQ ID NO: 15) of the heavy chain variable region of NYA-0082.

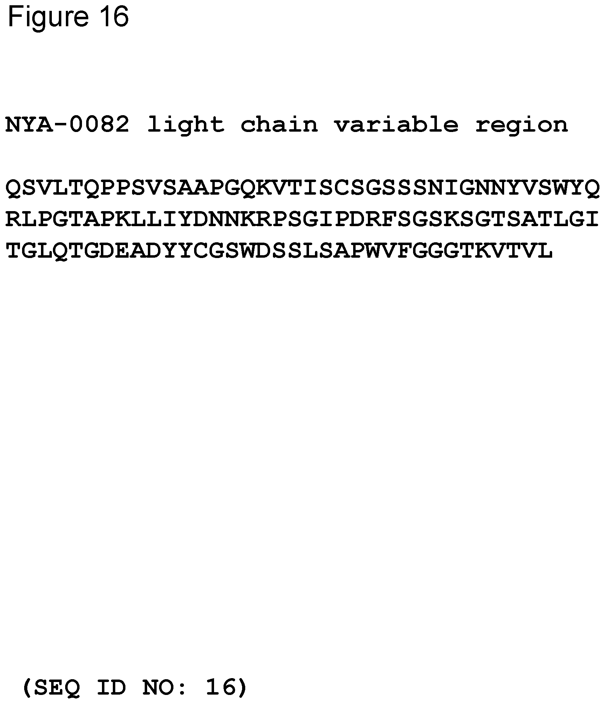

[Figure 16] Figure 16 shows the amino acid sequence (SEQ ID NO**:** 16) of the light chain variable region of NYA-0082.

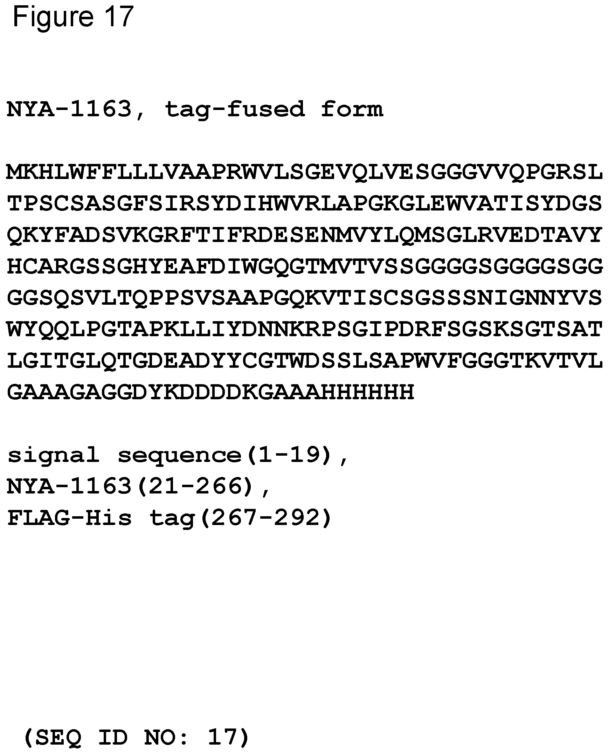

[Figure 17] Figure 17 shows the full-length amino acid sequence (SEQ ID NO**:** 17) of NYA-1163. This includes a signal sequence (1-19), NYA-1163 (21-266), and FLAG-His tag (267-292).

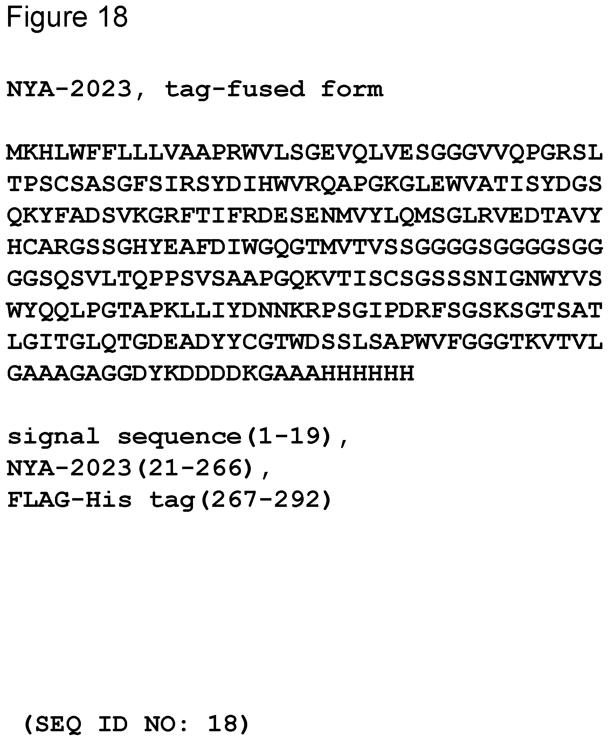

[Figure 18] Figure 18 shows the full-length amino acid sequence (SEQ ID NO**:** 18) of NYA-2023. This includes a aignal sequence (1-19), NYA-2023 (21-266), and FLAG-His tag (267-292).

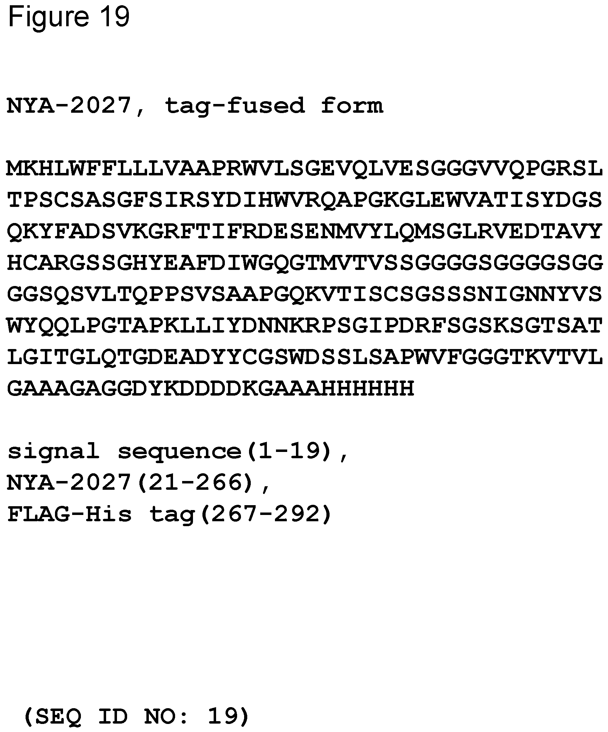

[Figure 19] Figure 19 shows the full-length amino acid sequence (SEQ ID NO**:** 19) of NYA-2027. This includes a aignal sequence (1-19), NYA-2027 (21-266), and FLAG-His tag (267-292).

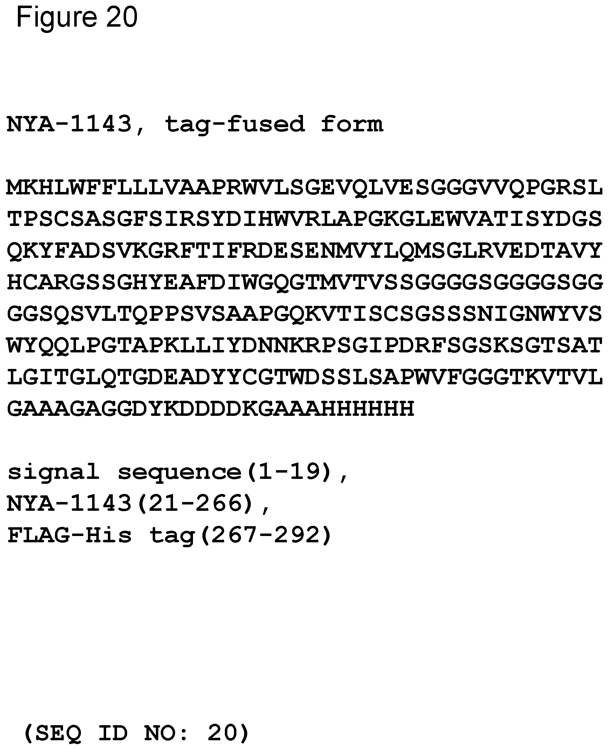

[Figure 20] Figure 20 shows the full-length amino acid sequence (SEQ ID NO**:** 20) of NYA-1143. This includes a aignal sequence (1-19), NYA-1143 (21-266), and FLAG-His tag (267-292).

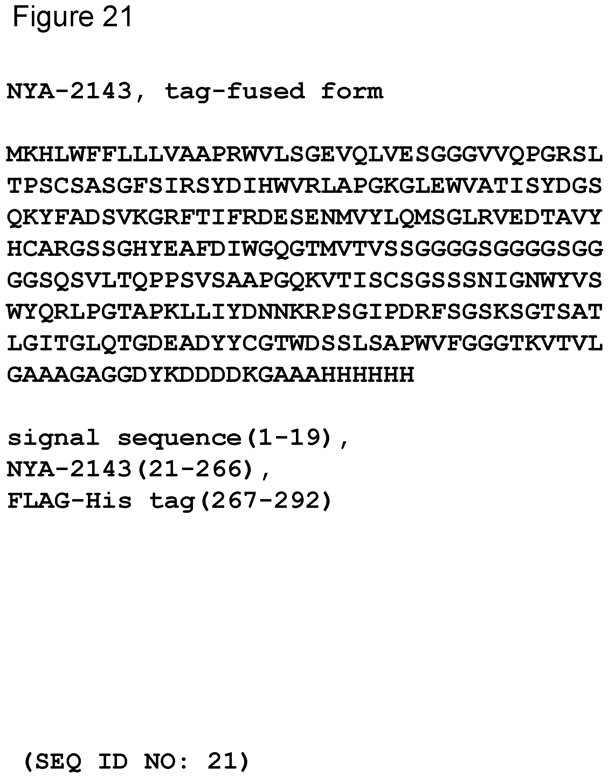

[Figure 21] Figure 21 shows the full-length amino acid sequence (SEQ ID **NO:** 21) of NYA-2143. This includes a signal sequence (1-19), NYA-2143 (21-266), and FLAG-His tag (267-292).

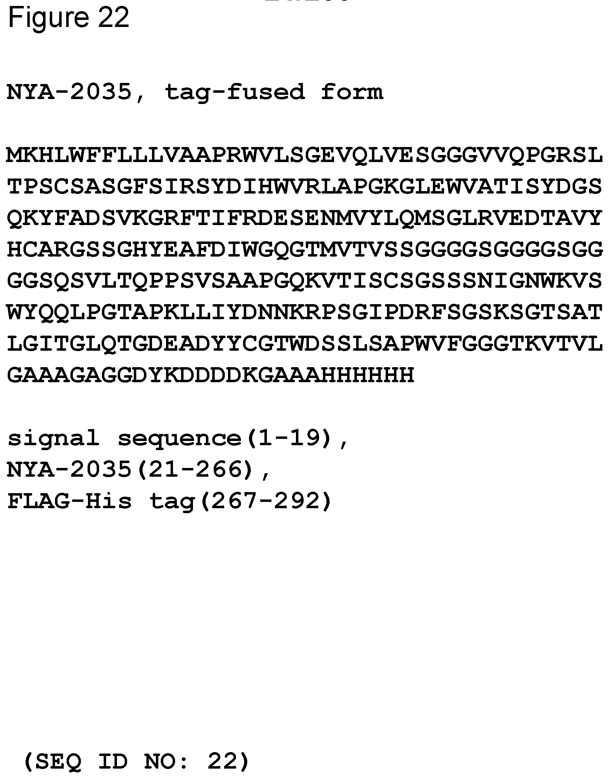

[Figure 22] Figure 22 shows the full-length amino acid sequence (SEQ ID **NO:** 22) of NYA-2035. This includes a signal sequence (1-19), NYA-2035 (21-266), and FLAG-His tag (267-292).

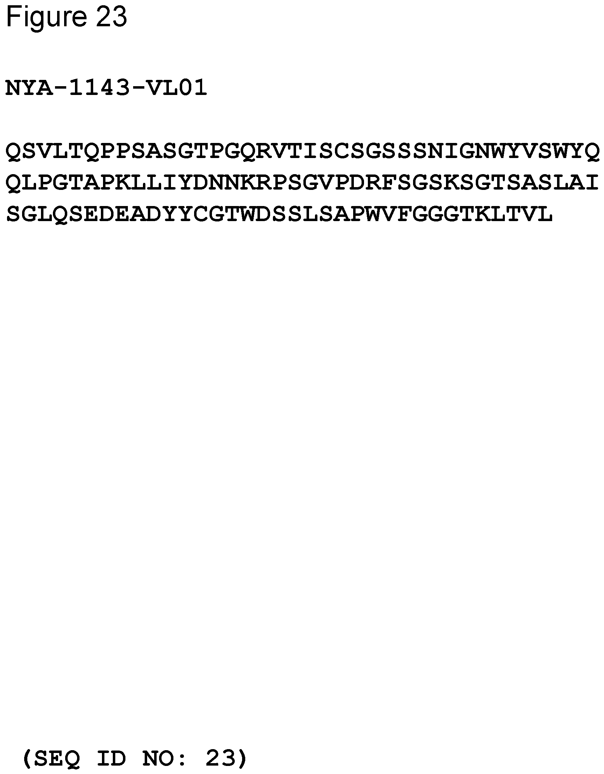

[Figure 23] Figure 23 shows the amino acid sequence (SEQ **ID NO:** 23) of NYA-1143-VL01.

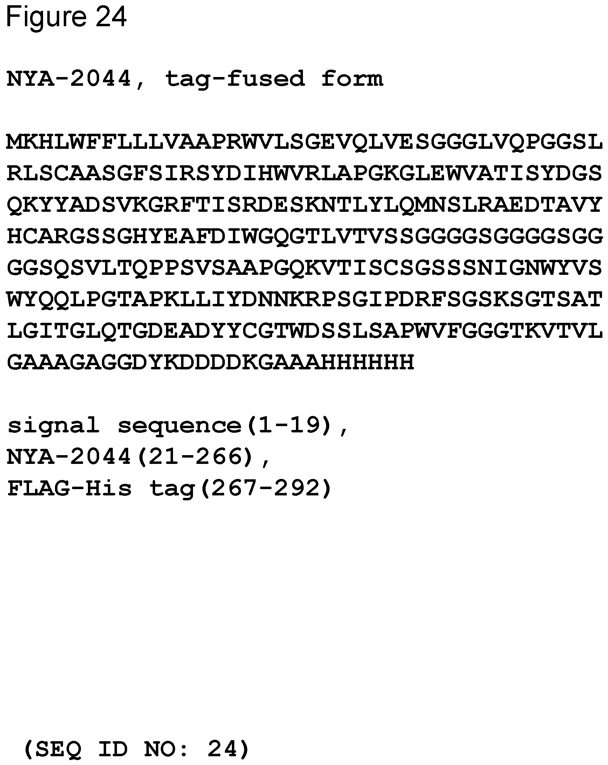

[Figure 24] Figure 24 shows the full-length amino acid sequence (SEQ ID **NO:** 24) of NYA-2044. This includes a aignal sequence (1-19), NYA-2044 (21-266), and FLAG-His tag (267-292).

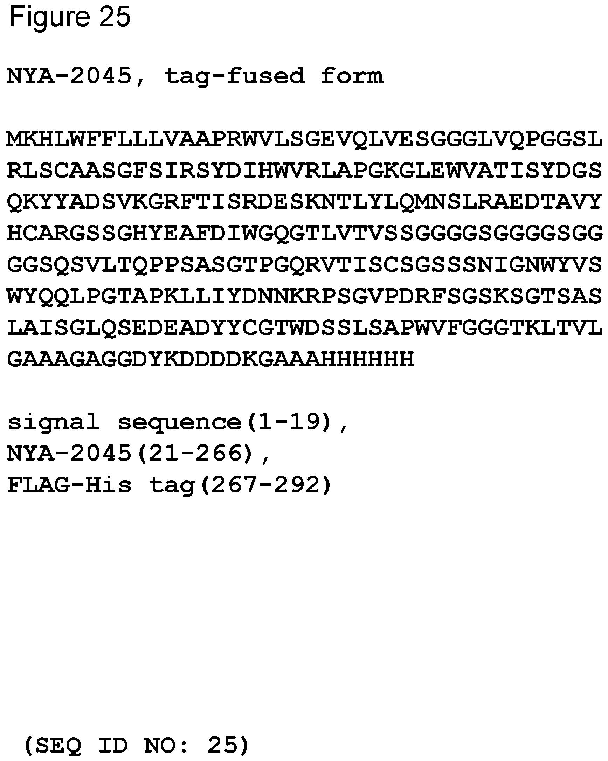

[Figure 25] Figure 25 shows the full-length amino acid sequence (SEQ ID **NO:** 25) of NYA-2045. This includes a signal sequence (1-19), NYA-2045 (21-266), and FLAG-His tag (267-292).

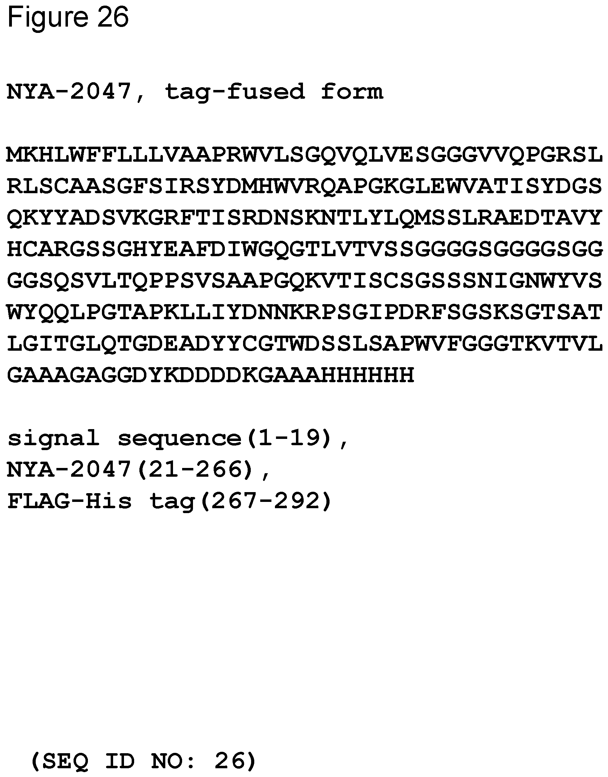

[Figure 26] Figure 26 shows the full-length amino acid sequence (SEQ ID **NO:** 26) of NYA-2047. This includes a signal sequence (1-19), NYA-2047 (21-266), and FLAG-His tag (267-292).

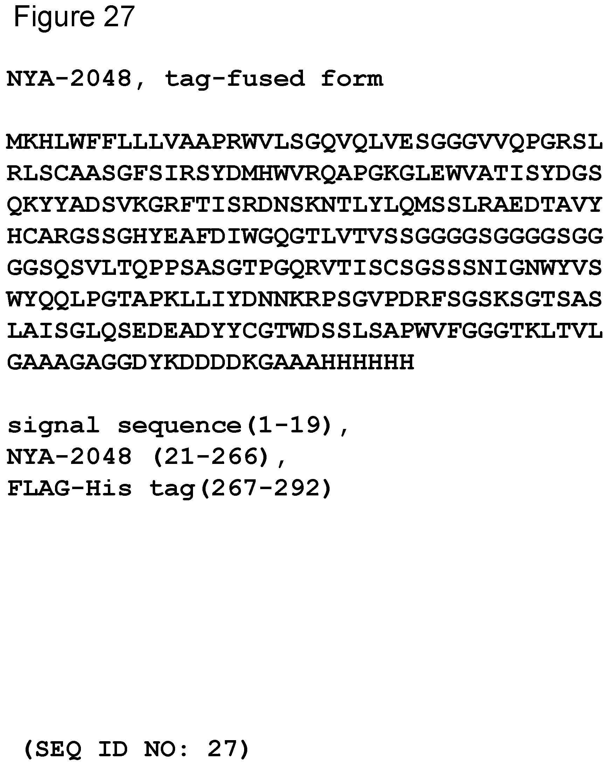

[Figure 27] Figure 27 shows the full-length amino acid sequence (SEQ ID **NO:** 27) of NYA-2048. This includes a signal sequence (1-19), NYA-2048 (21-266), and FLAG-His tag (267-292)

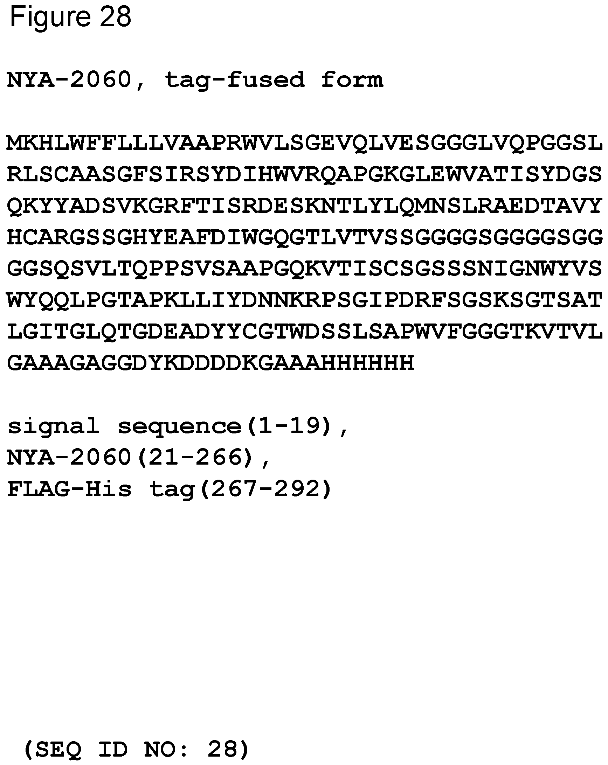

[Figure 28] Figure 28 shows the full-length amino acid sequence (SEQ ID **NO:** 28) of NYA-2060. This includes a signal sequence (1-19), NYA-2060 (21-266), and FLAG-His tag (267-292).

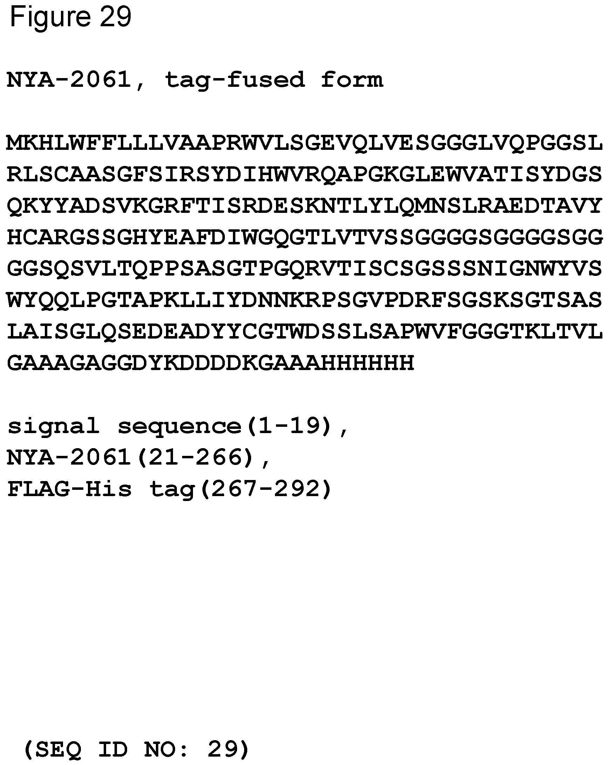

[Figure 29] Figure 29 shows the full-length amino acid sequence (SEQ ID NO: 29) of NYA-2061. This includes a signal sequence (1-19), NYA-2061 (21-266), and FLAG-His tag (267-292).

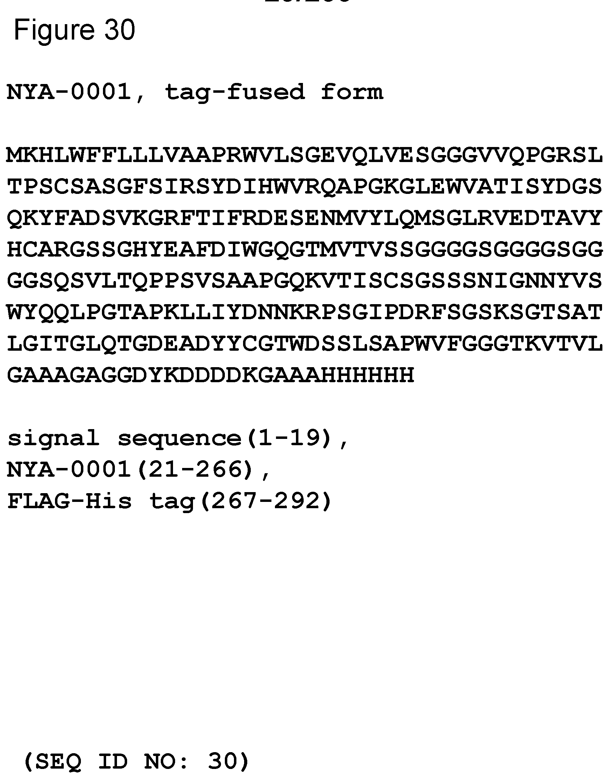

[Figure 30] Figure 30 shows the full-length amino acid sequence (SEQ ID NO: 30) of NYA-0001. This includes a signal sequence (1-19), NYA-0001 (21-266), and FLAG-His tag (267-292)

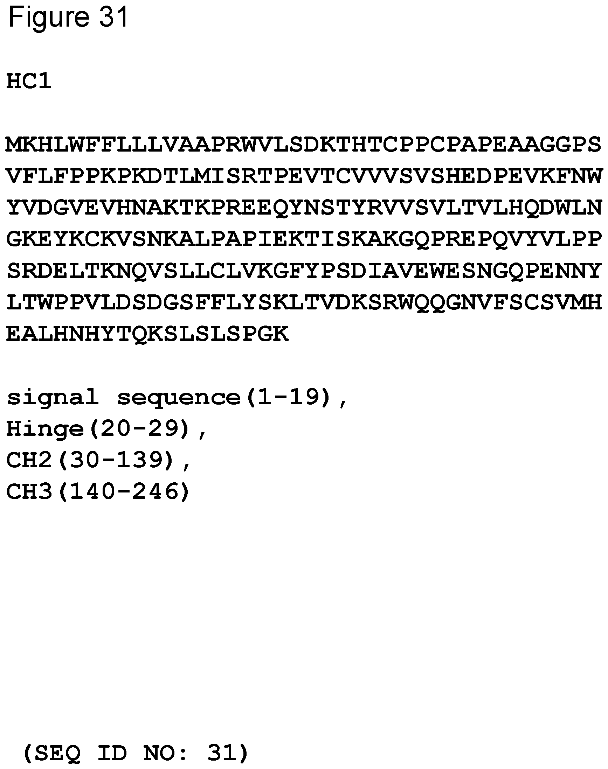

[Figure 31] Figure 31 shows the full-length amino acid sequence (SEQ ID NO: 31) of HC1. This includes a signal sequence (1-19), Hinge (20-29), CH2 (30-139), and CH3 (140-246).

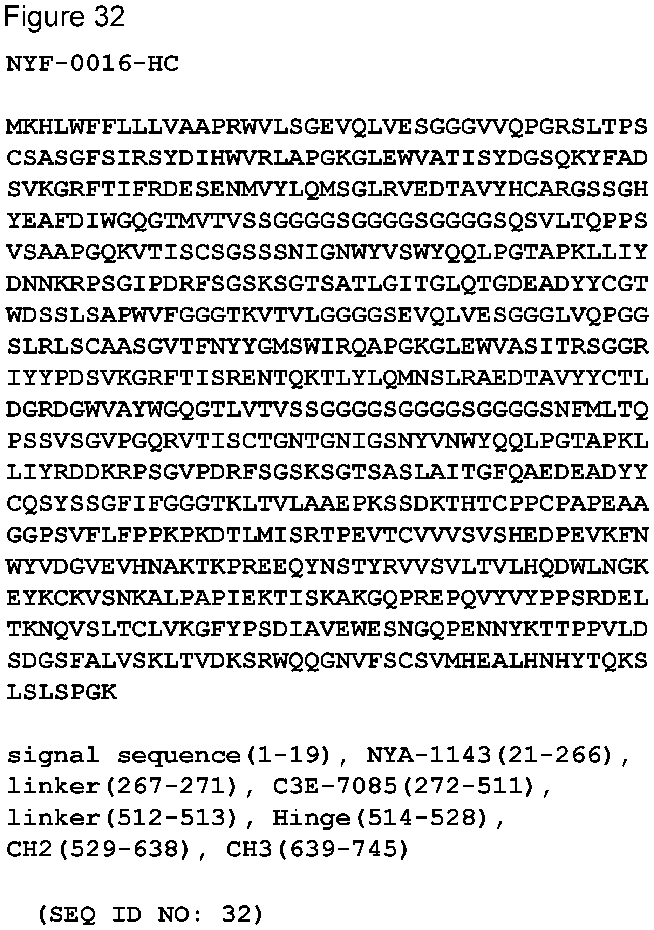

[Figure 32] Figure 32 shows the full-length amino acid sequence (SEQ ID NO: 32) of NYF-0016-HC2. This includes a signal sequence (1-19), NYA-1143 (21-266), linker (267-271), C3E-7085 (272-511), linker (512-513), Hinge (514-528), CH2 (529-638), and CH3 (639-745).

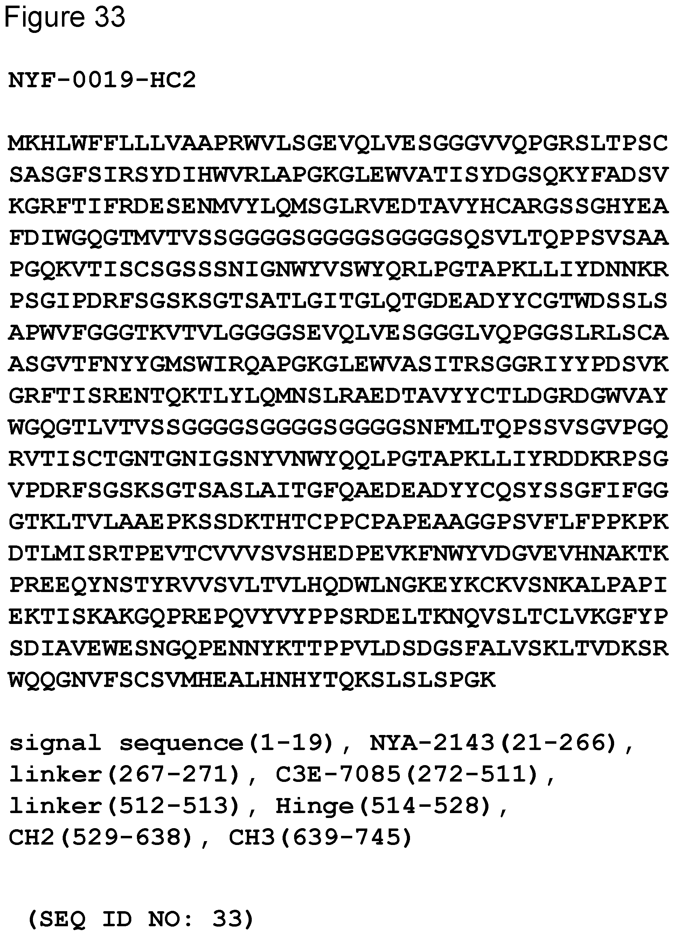

[Figure 33] Figure 33 shows the full-length amino acid sequence (SEQ ID NO: 33) of NYF-0019-HC2. This includes a signal sequence (1-19), NYA-2143 (21-266), linker (267-271), C3E-7085 (272-511), linker (512-513), Hinge (514-528), CH2 (529-638), and CH3 (639-745).

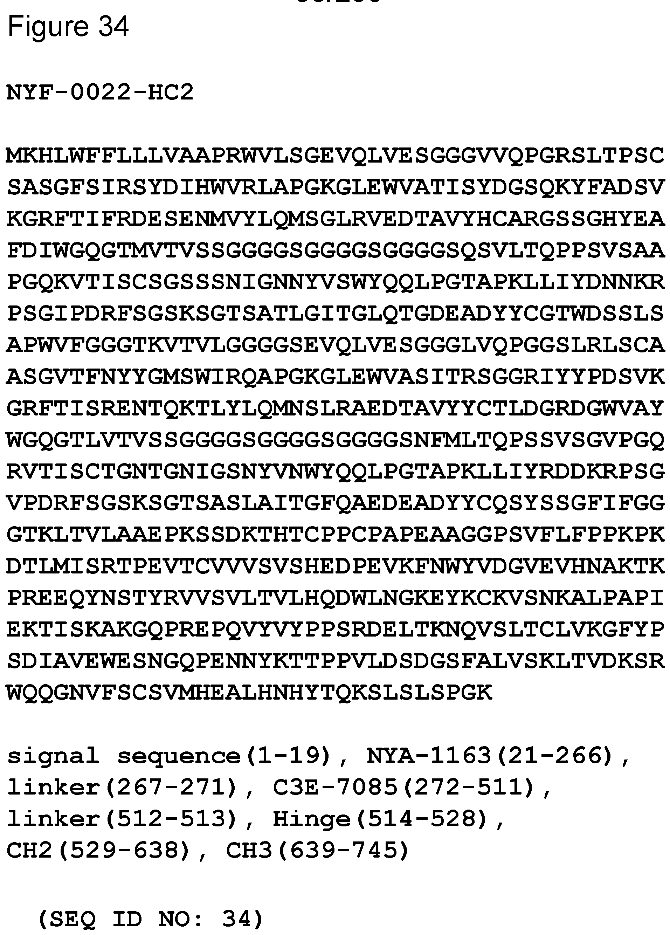

[Figure 34] Figure 34 shows the full-length amino acid sequence (SEQ ID NO: 34) of NYF-0022-HC2. This includes a signal sequence (1-19), NYA-1163 (21-266), linker (267-271), C3E-7085 (272-511), linker (512-513), Hinge (514-528), CH2 (529-638), and CH3 (639-745).

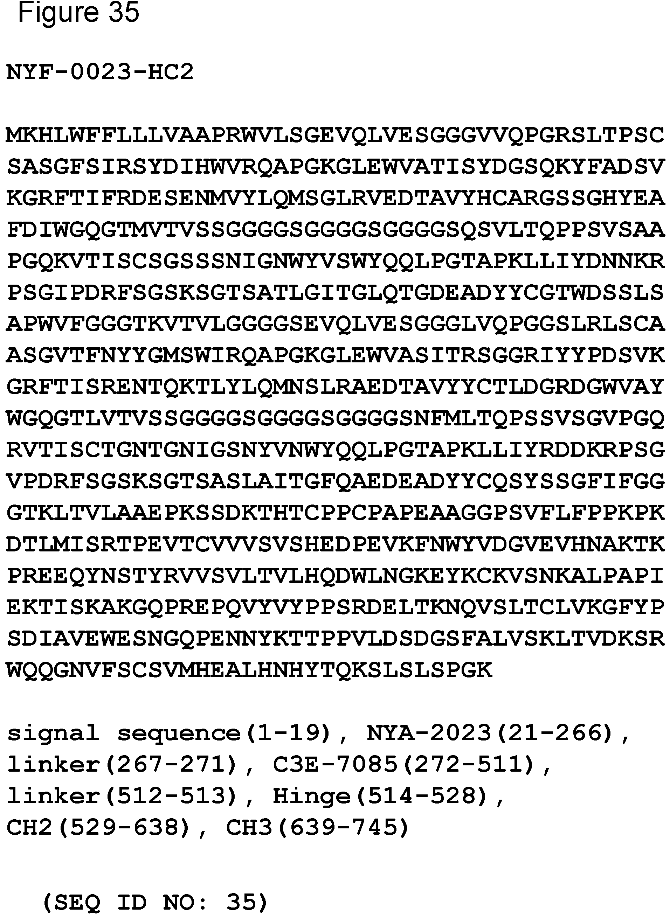

[Figure 35] Figure 35 shows the full-length amino acid sequence (SEQ ID **NO:** 35) of NYF-0023-HC2. This includes a signal sequence (1-19), NYA-2023 (21-266), linker (267-271), C3E-7085 (272-511), linker (512-513), Hinge (514-528), CH2 (529-638), and CH3 (639-745).

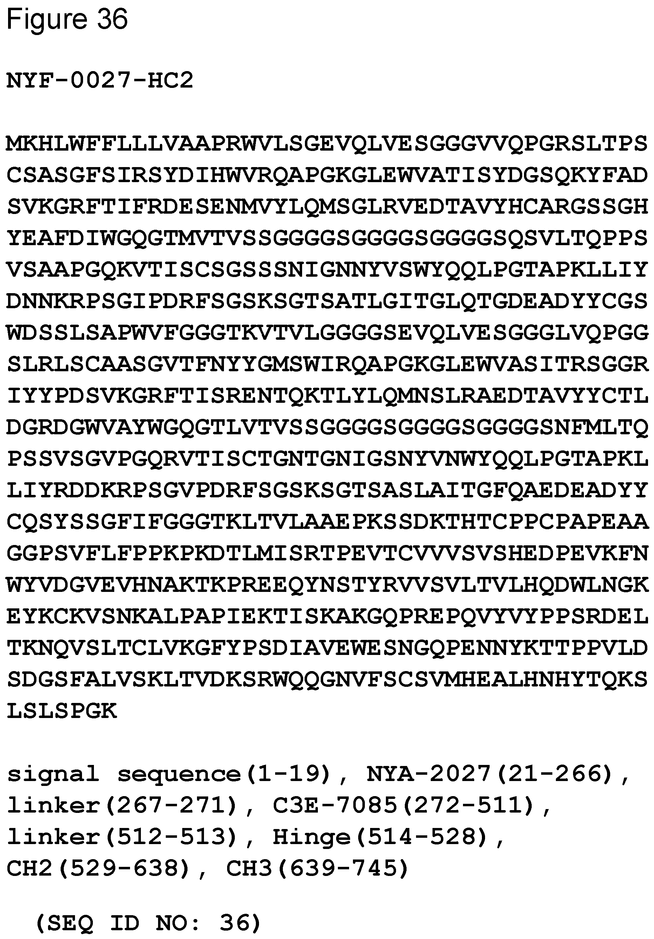

[Figure 36] Figure 36 shows the full-length amino acid sequence (SEQ ID **NO:** 36) of NYF-0027-HC2. This includes a signal sequence (1-19), NYA-2027 (21-266), linker (267-271), C3E-7085 (272-511), linker (512-513), Hinge (514-528), CH2 (529-638), and CH3 (639-745).

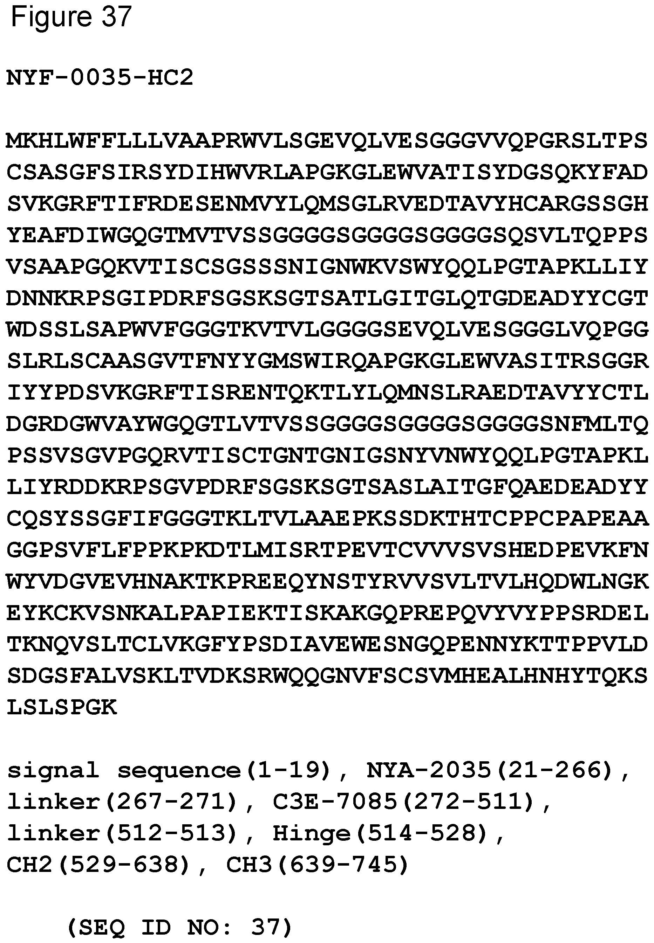

[Figure 37] Figure 37 shows the full-length amino acid sequence (SEQ ID **NO:** 37) of NYF-0035-HC2. This includes a signal sequence (1-19), NYA-2035 (21-266), linker (267-271), C3E-7085 (272-511), linker (512-513), Hinge (514-528), CH2 (529-638), and CH3 (639-745).

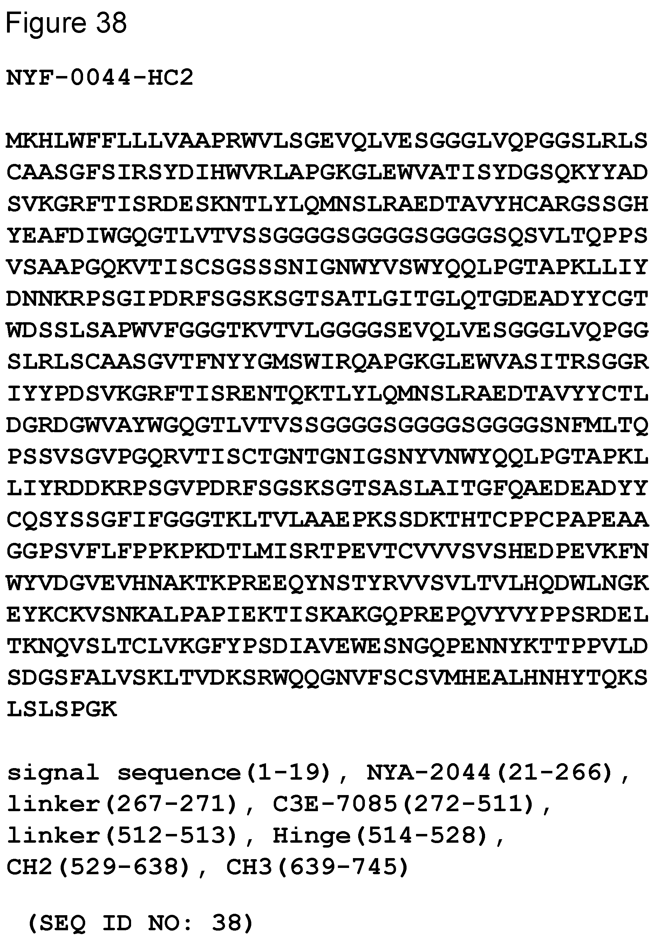

[Figure 38] Figure 38 shows the full-length amino acid sequence (SEQ ID **NO:** 38) of NYF-0044-HC2. This includes a signal sequence (1-19), NYA-2044 (21-266), linker (267-271), C3E-7085 (272-511), linker (512-513), Hinge (514-528), CH2 (529-638), and CH3 (639-745).

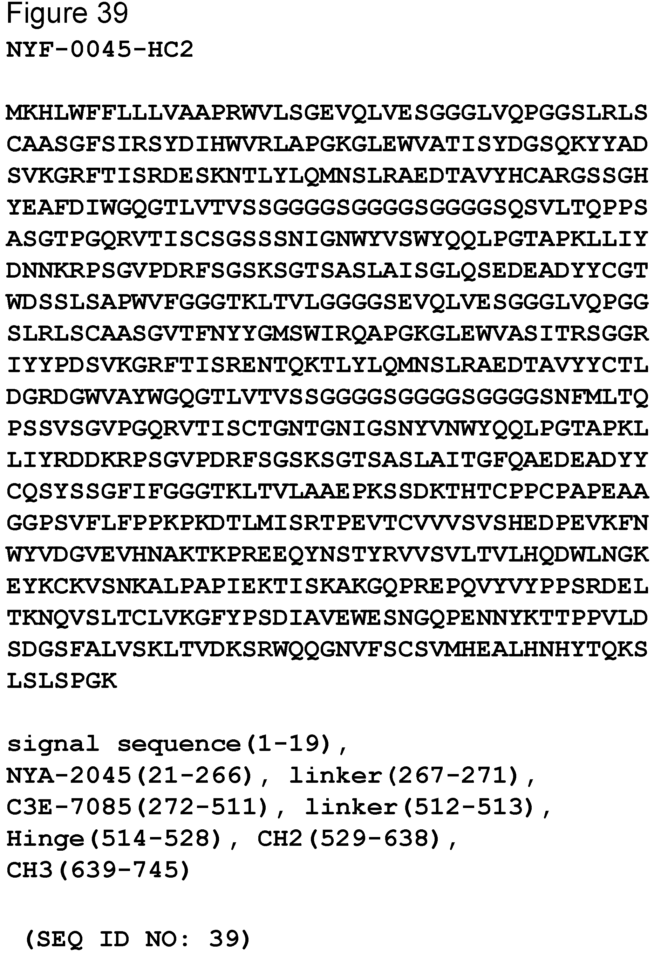

[Figure 39] Figure 39 shows the full-length amino acid sequence (SEQ ID **NO:** 39) of NYF-0045-HC2. This includes a signal sequence (1-19), NYA-2045 (21-266), linker (267-271), C3E-7085 (272-511), linker (512-513), Hinge (514-528), CH2 (529-638), and CH3 (639-745).

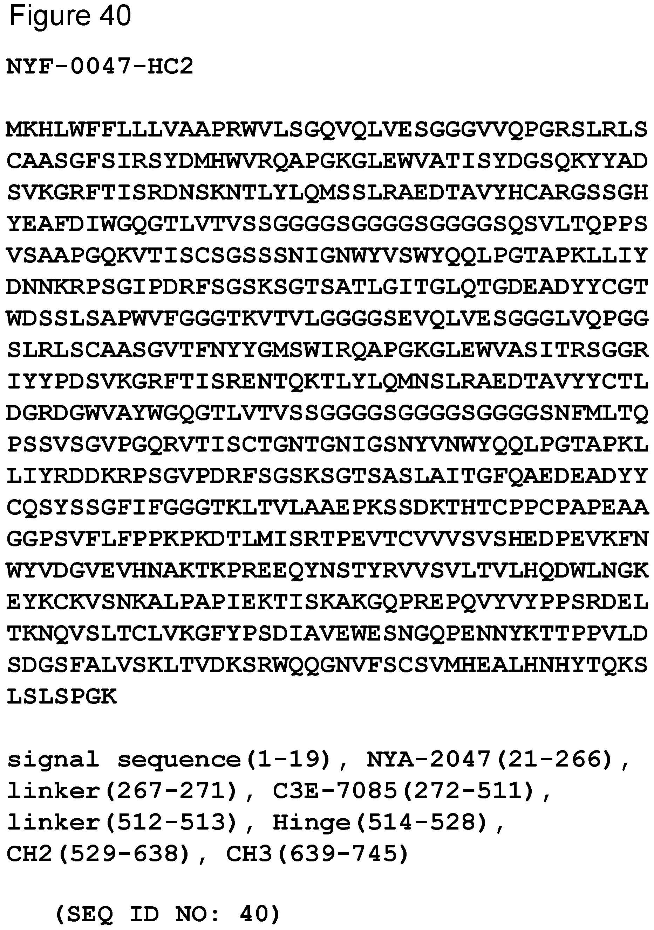

[Figure 40] Figure 40 shows the full-length amino acid sequence (SEQ ID **NO:** 40) of NYF-0047-HC2. This includes a signal sequence (1-19), NYA-2047 (21-266), linker (267-271), C3E-7085 (272-511), linker (512-513), Hinge (514-528), CH2 (529-638), and CH3 (639-745).

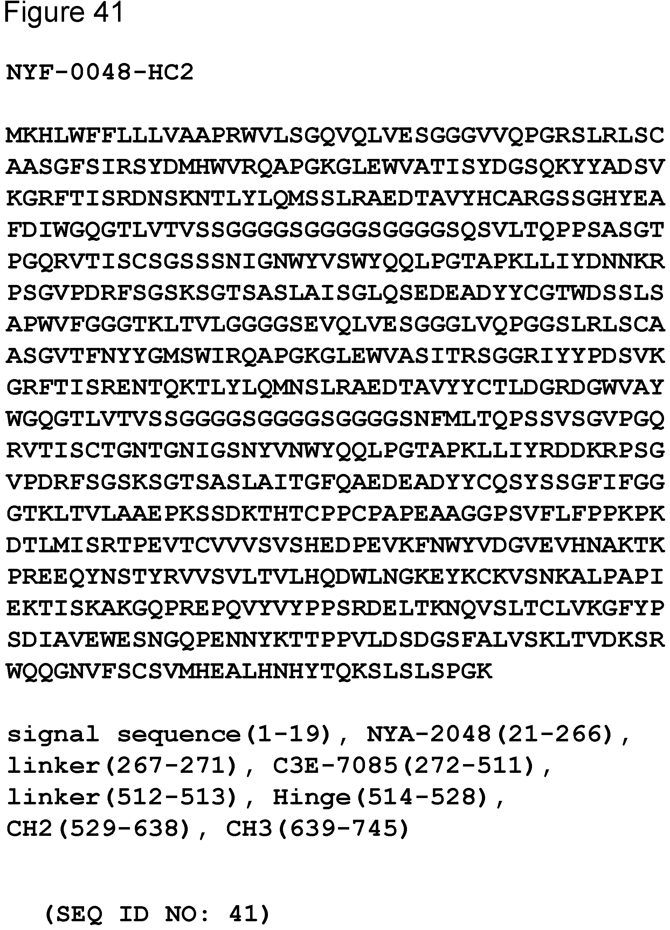

[Figure 41] Figure 41 shows the full-length amino acid sequence (SEQ ID **NO:** 41) of NYF-0048-HC2. This includes a signal sequence (1-19), NYA-2048 (21-266), linker (267-271), C3E-7085 (272-511), linker (512-513), Hinge (514-528), CH2 (529-638), and CH3 (639-745).

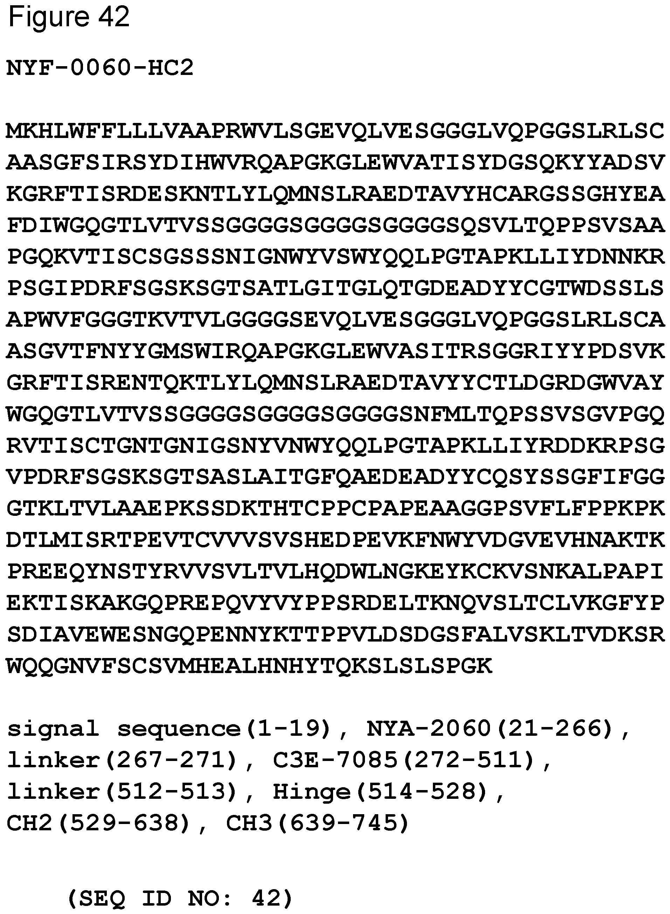

[Figure 42] Figure 42 shows the full-length amino acid sequence (SEQ ID **NO:** 42) of NYF-0060-HC2. This includes a signal sequence (1-19), NYA-2060 (21-266), linker (267-271), C3E-7085 (272-511), linker (512-513), Hinge (514-528), CH2 (529-638), and CH3 (639-745).

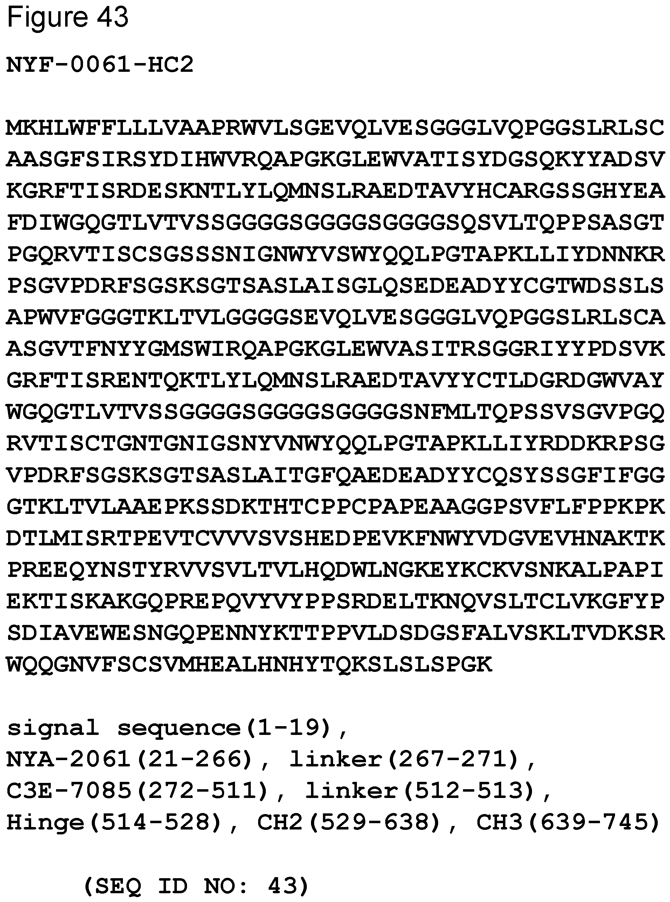

[Figure 43] Figure 43 shows the full-length amino acid sequence (SEQ ID **NO:** 43) of NYF-0061-HC2. This includes a signal sequence (1-19), NYA-2061 (21-266), linker (267-271), C3E-7085 (272-511), linker (512-513), Hinge (514-528), CH2 (529-638), and CH3 (639-745).

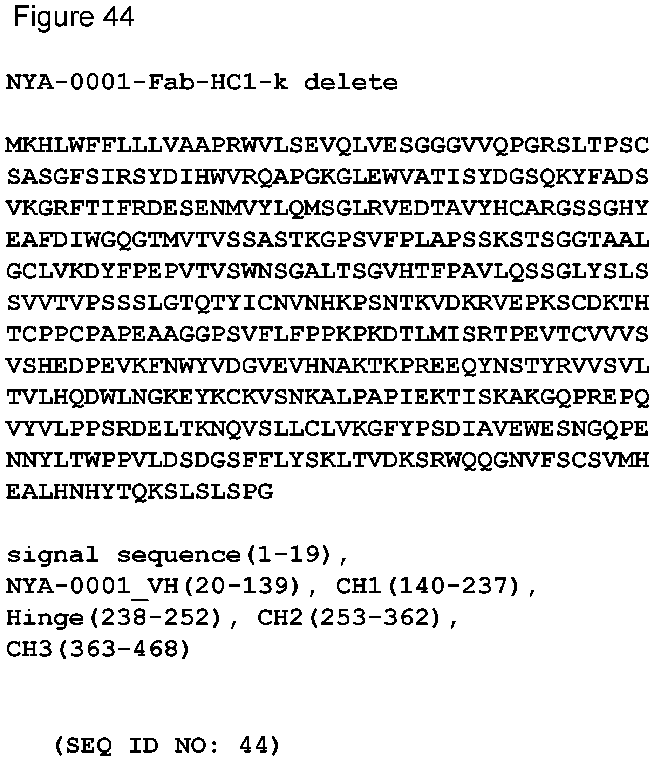

[Figure 44] Figure 44 shows the full-length amino acid sequence (SEQ ID **NO:** 44) of NYA-0001-Fab-HC1-κ delete. This includes a signal sequence (1-19), NYA-0001_VH (20-139), CH1 (140-237), Hinge (238-252), CH2 (253-362), and CH3 (363-468).

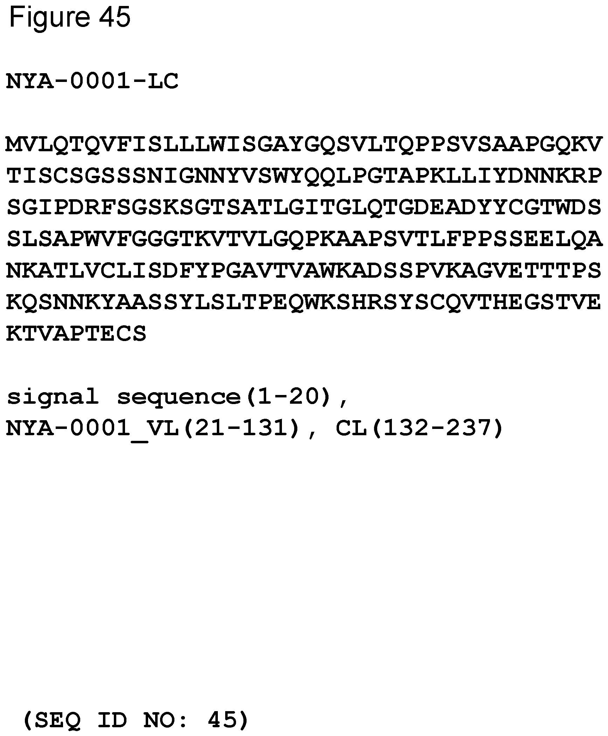

[Figure 45] Figure 45 shows the full-length amino acid sequence (SEQ ID **NO:** 45) of NYA-0001-LC. This includes a signal sequence (1-20), NYA-0001_VL (21-131), and CL (132-237).

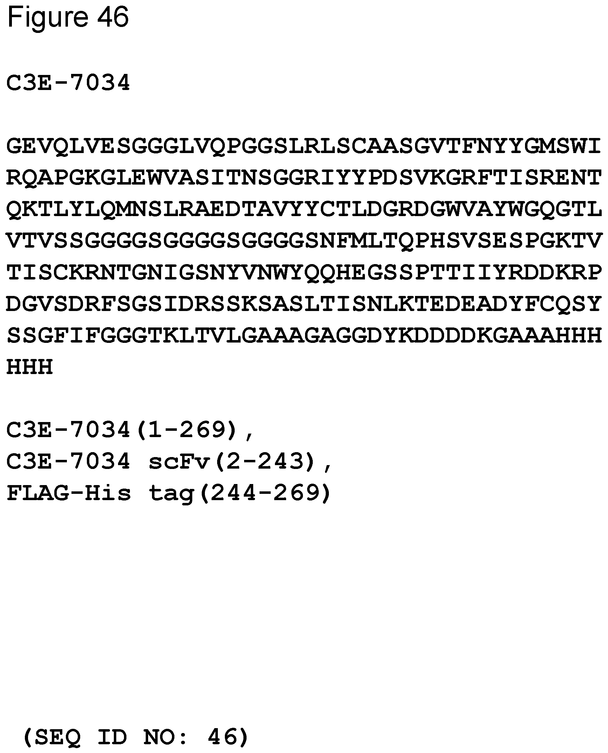

[Figure 46] Figure 46 shows the amino acid sequence (SEQ ID NO: 46) of C3E-7034 (1-269). This includes a scFv (2-241), and a FLAG-His tag (244-269).

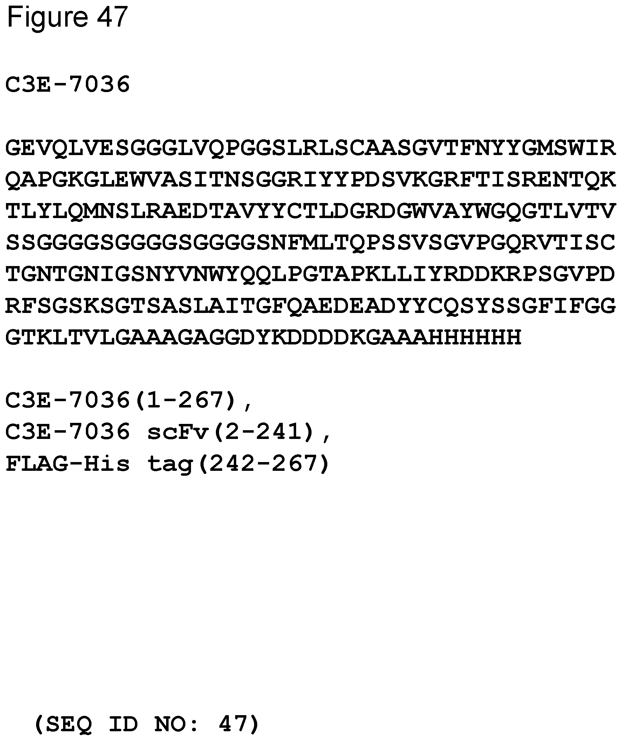

[Figure 47] Figure 47 shows the amino acid sequence (SEQ ID NO: 47) of C3E-7036 (1-267). This includes a scFv (2-241) and a FLAG-His tag (242-267).

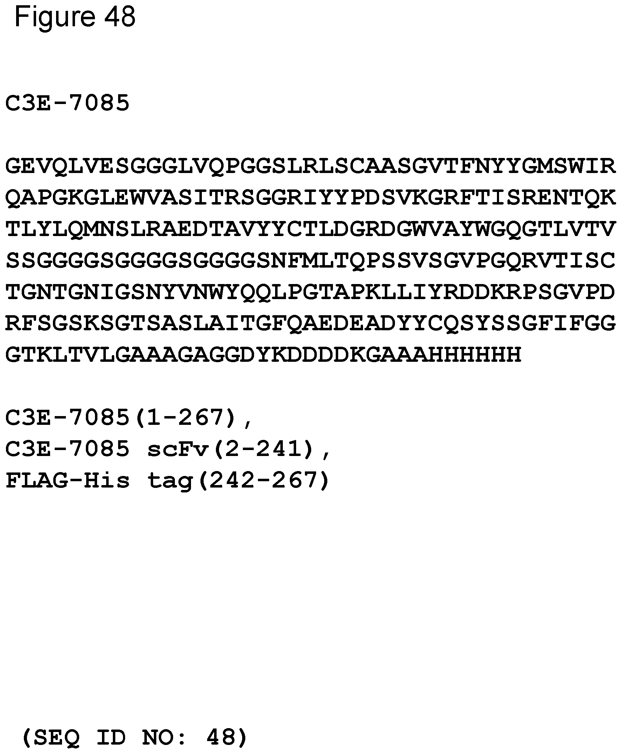

[Figure 48] Figure 48 shows the amino acid sequence (SEQ ID NO: 48) of C3E-7085 (1-267). This includes a scFv (2-241), and a FLAG-His tag (242-267).

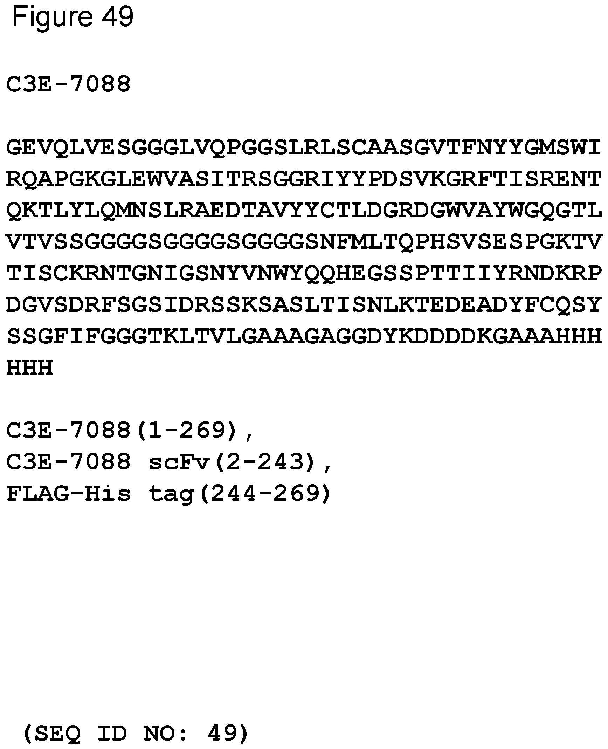

[Figure 49] Figure 49 shows the amino acid sequence (SEQ ID NO: 49) of C3E-7088 (1-269). This includes a scFv (2-243) and a FLAG-His tag (244-269).

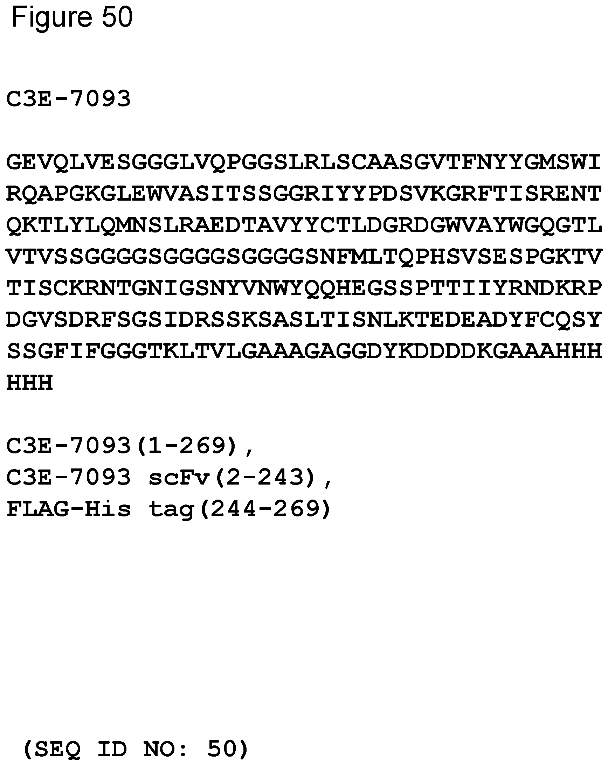

[Figure 50] Figure 50 shows the amino acid sequence (SEQ ID NO: 50) of C3E-7093 (1-269). This includes a scFv (2-243) and a FLAG-His tag (244-269).

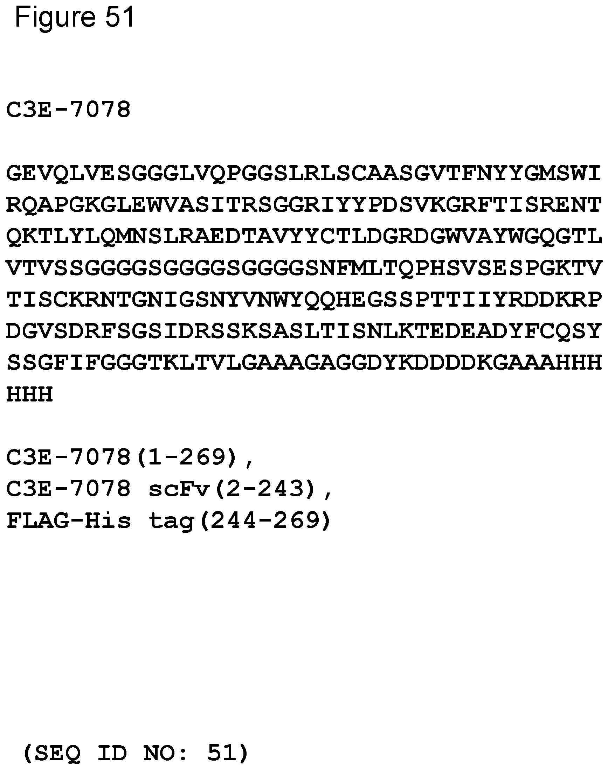

[Figure 51] Figure 51 shows the amino acid sequence (SEQ ID NO: 51) of C3E-7078(1-269). This includes a scFv (2-243) and a FLAG-His tag (244-269).

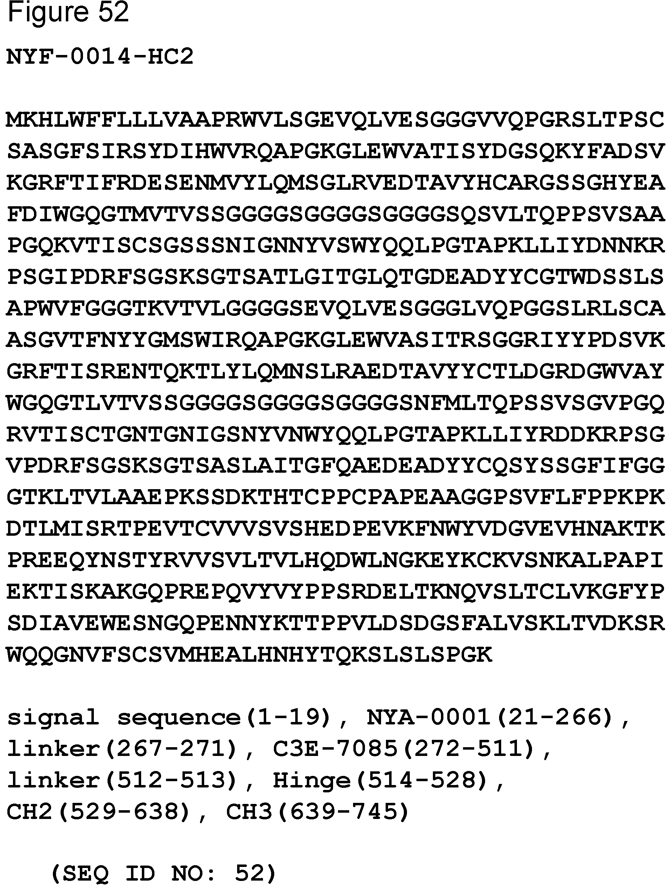

[Figure 52] Figure 52 shows the full-length amino acid sequence (SEQ ID **NO:** 52) of NYF-0014-HC2. This includes a signal sequence (1-19), NYA-0001 (21-266), linker (267-271), C3E-7085 (272-511), linker (512-513), Hinge (514-528), CH2 (529-638), and CH3 (639-745).

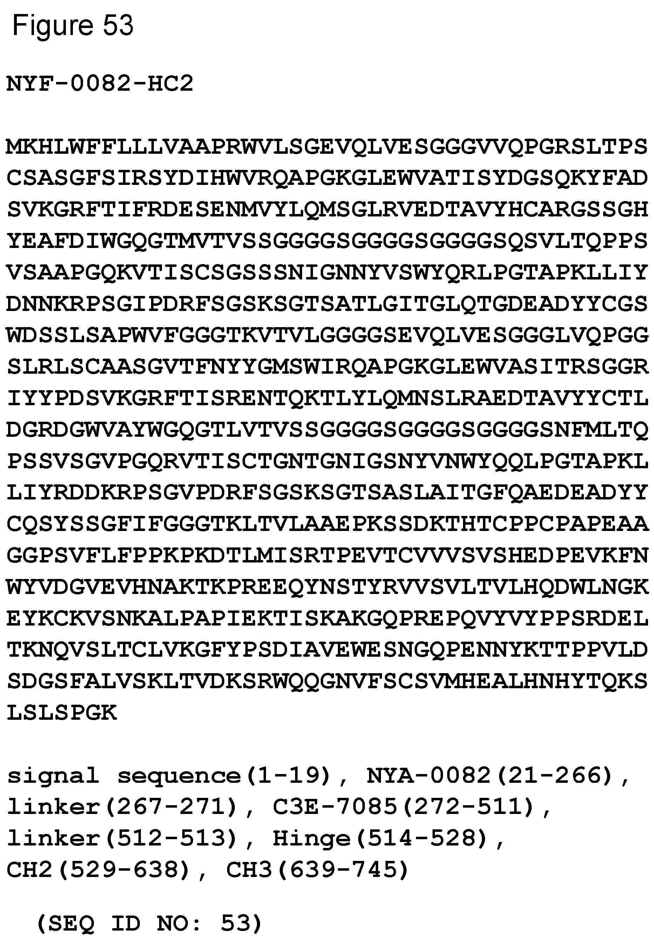

[Figure 53] Figure 53 shows the full-length amino acid sequence (SEQ ID **NO:** 53) of NYF-0082-HC2. This includes a signal sequence (1-19), NYA-0082 (21-266), linker (267-271), C3E-7085 (272-511), linker (512-513), Hinge (514-528), CH2 (529-638), and CH3 (639-745).

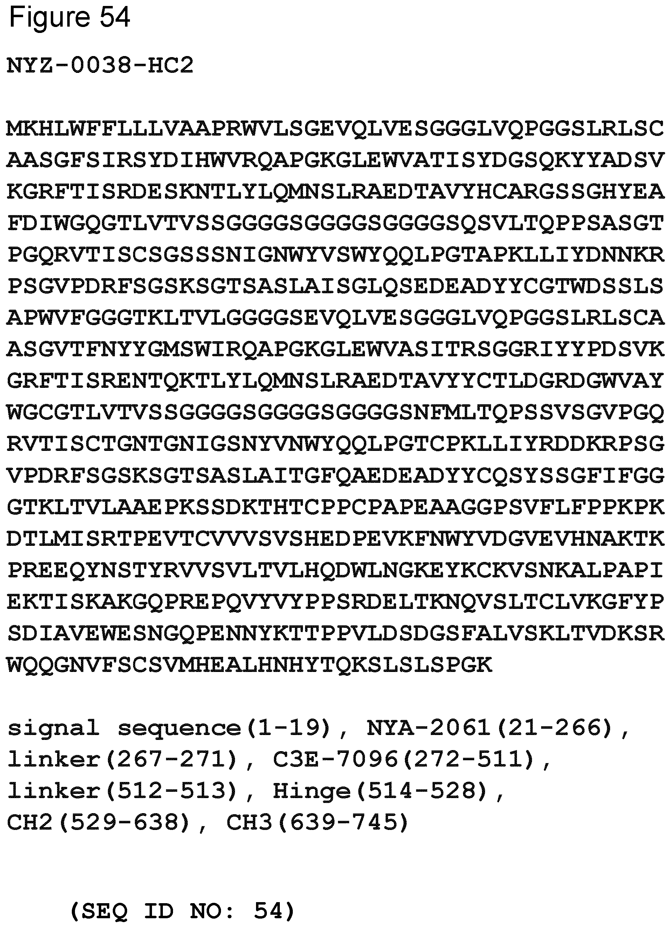

[Figure 54] Figure 54 shows the full-length amino acid sequence (SEQ ID **NO:** 54) of NYF-0083-HC2. This includes a signal sequence (1-19), NYA-2061 (21-266), linker (267-271), C3E-7095 (272-511), linker (512-513), Hinge (514-528), CH2 (529-638), and CH3 (639-745).

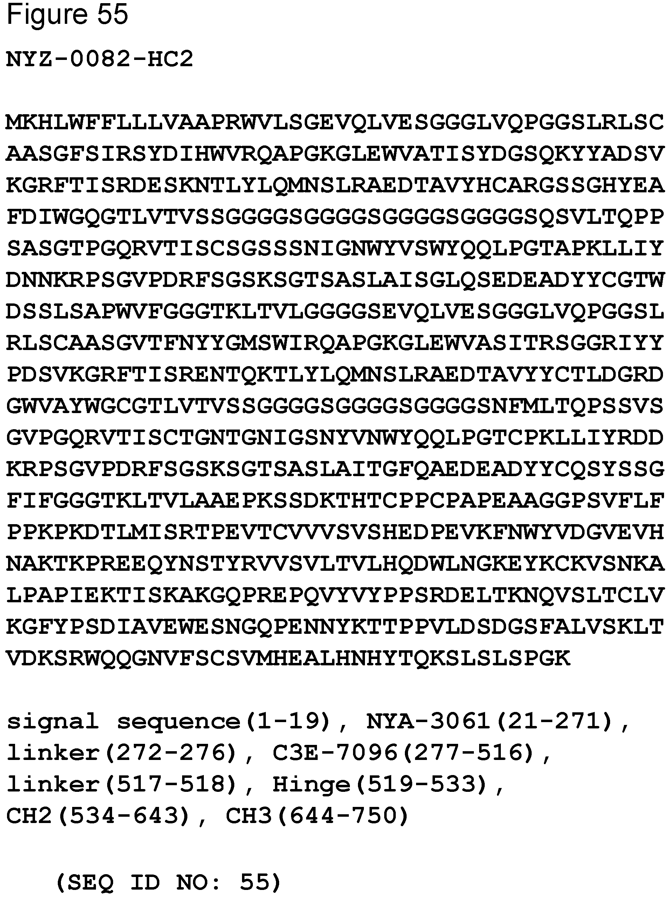

[Figure 55] Figure 55 shows the full-length amino acid sequence (SEQ ID **NO:** 55) of NYZ-0082-HC2. This includes a signal sequence (1-19), NYA-3061 (21-271), linker (272-276), C3E-7096 (277-516), linker (517-518), Hinge (519-533), CH2 (534-643), and CH3 (644-750).

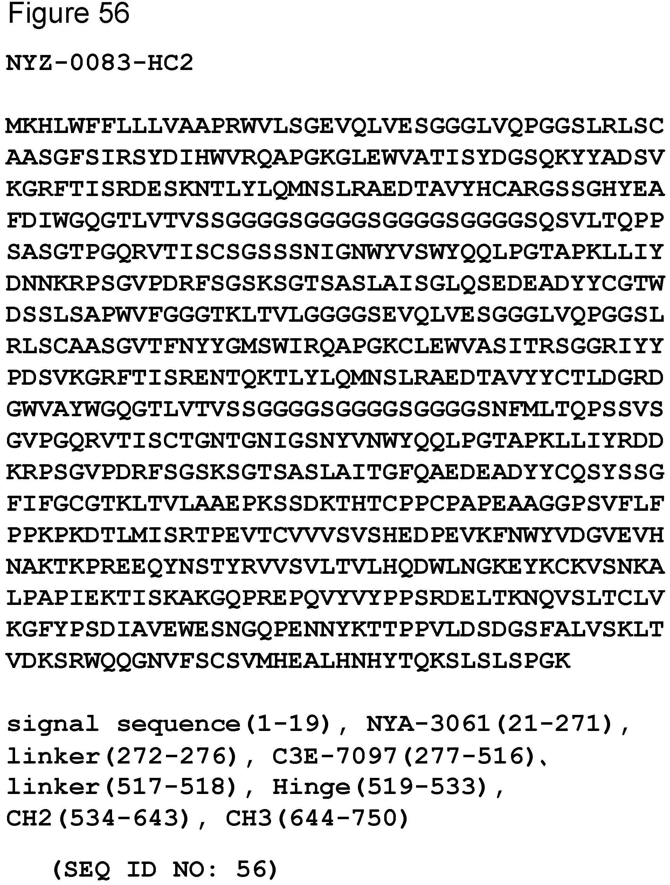

[Figure 56] Figure 56 shows the full-length amino acid sequence (SEQ ID **NO:** 56) of NYZ-0083-HC2. This includes a signal sequence (1-19), NYA-3061 (21-271), linker (272-276), C3E-7097 (277-516), linker (517-518), Hinge (519-533), CH2 (534-643), and CH3 (644-750).

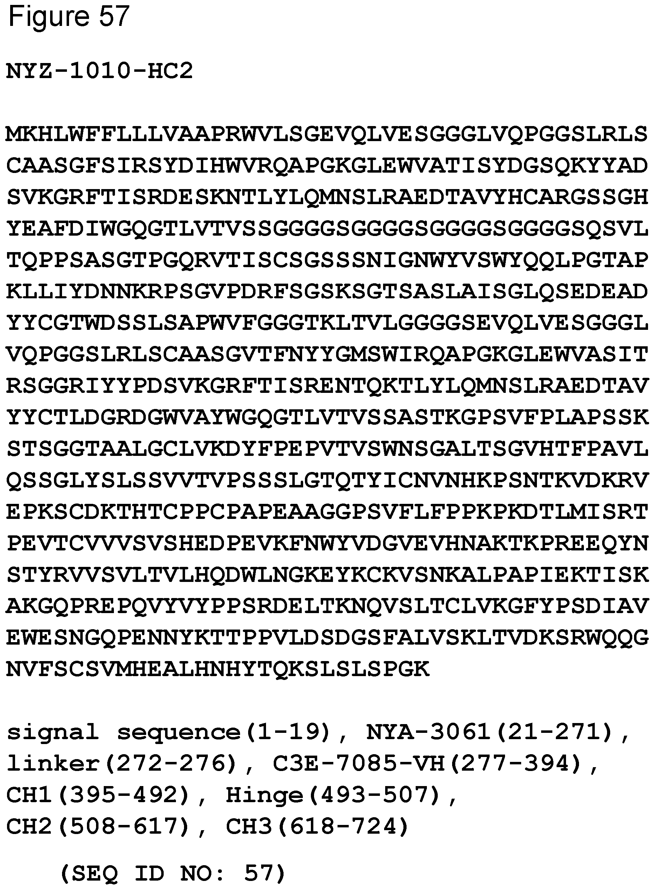

[Figure 57] Figure 57 shows the full-length amino acid sequence (SEQ ID **NO:** 57) of NYZ-1010-HC2. This includes a signal sequence (1-19), NYA-3061 (21-271), linker (272-276), C3E-7085-VH (277-394), CH1 (395-492)Hinge, (493-507), CH2 (508-617), and CH3 (618-724).

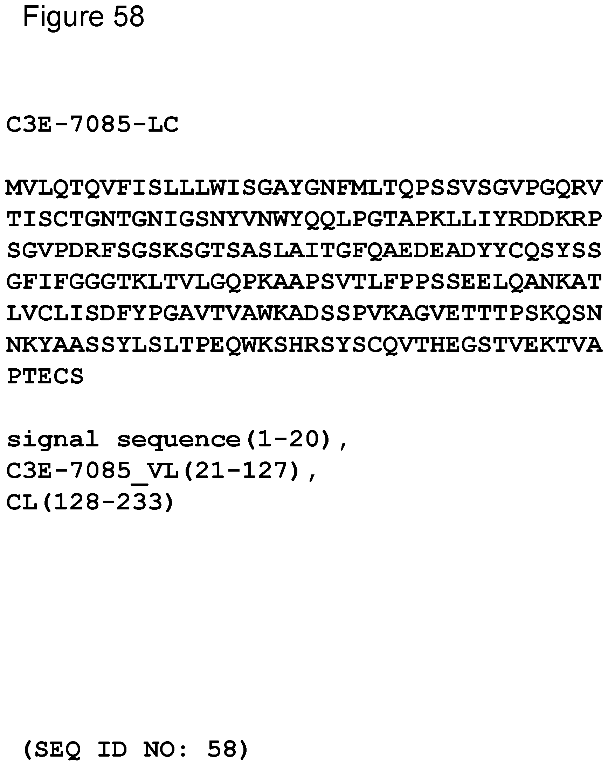

[Figure 58] Figure 58 shows the full-length amino acid sequence (SEQ ID **NO:** 58) of C3E-7085-LC. This includes a signal sequence (1-20), C3E-7085_VL (21-127), and CL (128-233).

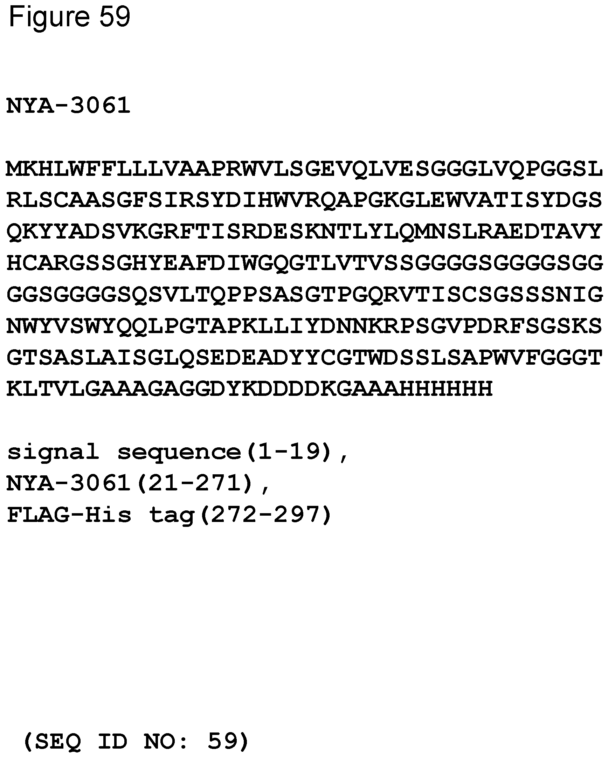

[Figure 59] Figure 59 shows the full-length amino acid sequence (SEQ ID NO: 59) of NYA-3061. This includes a signal sequence (1-19), NYA-3061 (21-271), and FLAG-His tag (272-297).

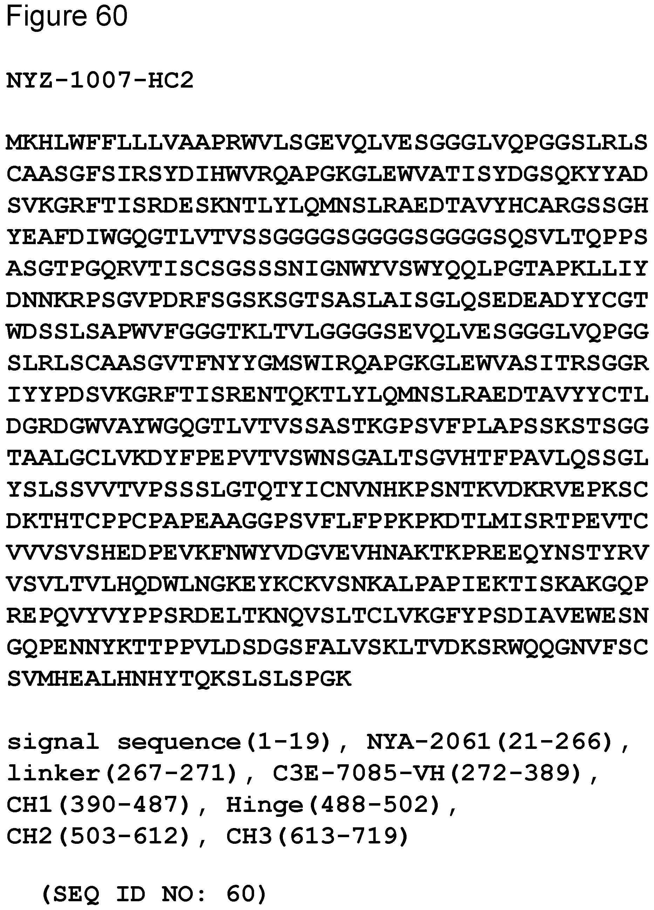

[Figure 60] Figure 60 shows the full-length amino acid sequence (SEQ ID NO: 60) of NYZ-1007-HC2. This includes asignal sequence (1-19), NYA-2061 (21-266), linker (267-271), C3E-7085-VH (272-389), CH1 (390-487), Hinge (488-502), CH2 (503-612), and CH3 (613-719).

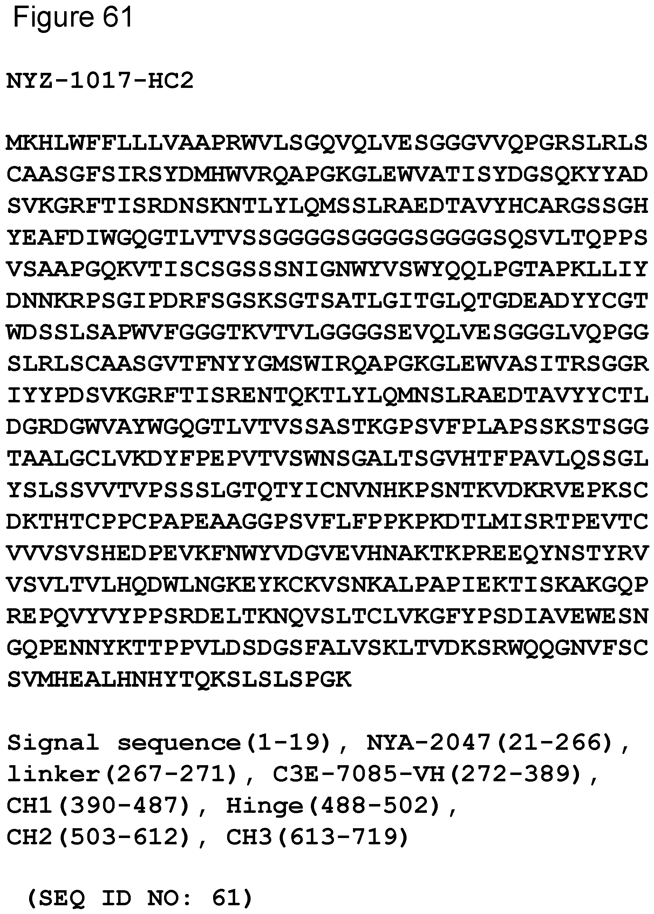

[Figure 61] Figure 61 shows the full-length amino acid sequence (SEQ ID NO: 61) of NYZ-1017-HC2. This includes a signal sequence (1-19), NYA-2047 (21-266), linker (267-271), C3E-7085-VH (272-389), CH1 (390-487), Hinge (488-502), CH2 (503-612), and CH3 (613-719).

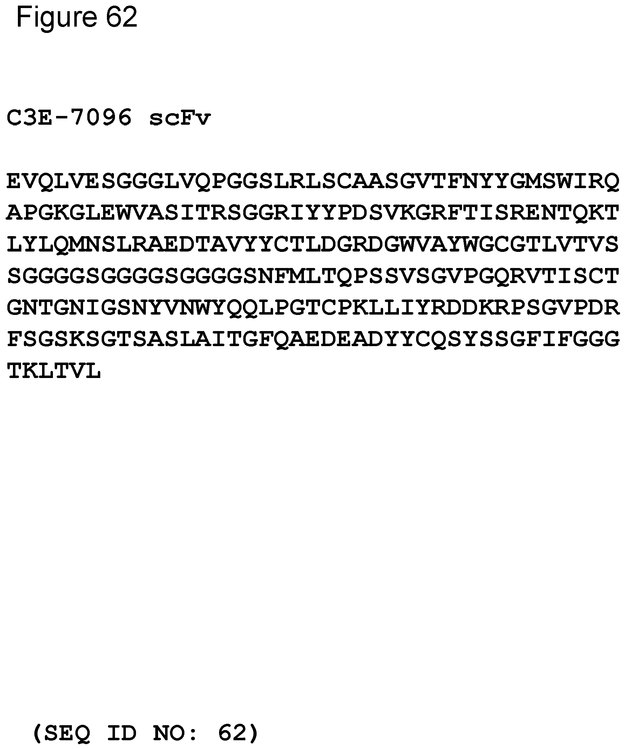

[Figure 62] Figure 62 shows the full-length amino acid sequence (SEQ ID NO: 62) of C3E-7096.

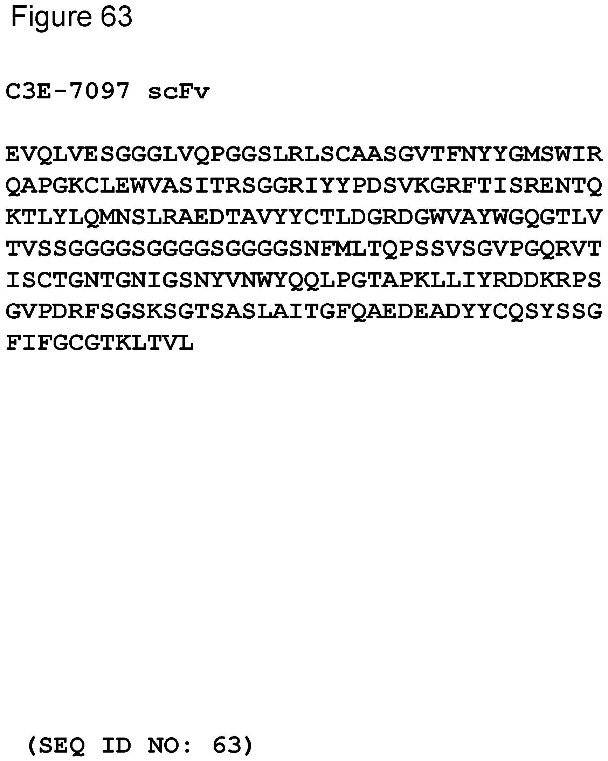

[Figure 63] Figure 63 shows the full-length amino acid sequence (SEQ ID NO: 63) of C3E-7097.

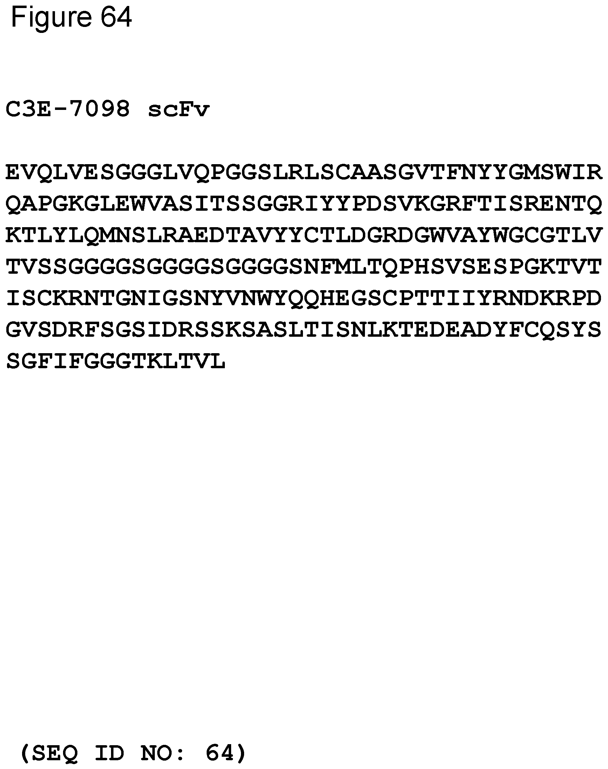

[Figure 64] Figure 64 shows the full-length amino acid sequence (SEQ ID NO: 64) of C3E-7098.

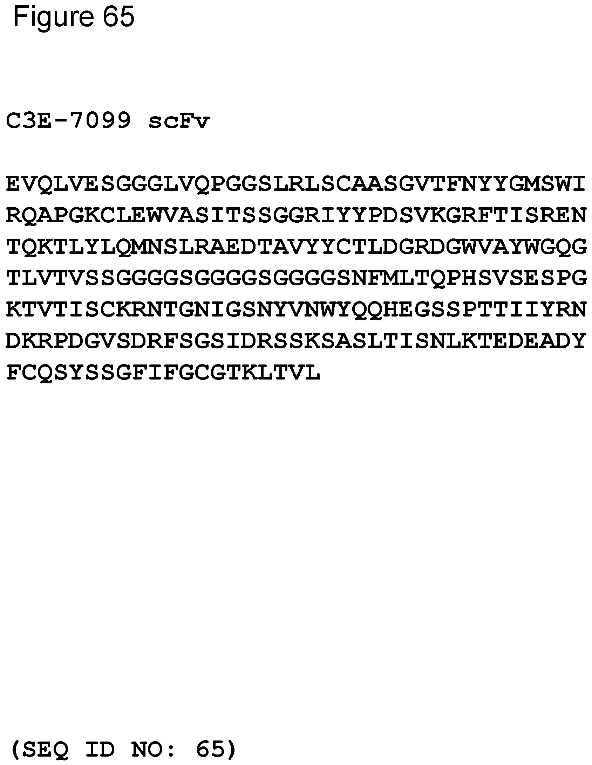

[Figure 65] Figure 65 shows the full-length amino acid sequence (SEQ ID NO: 65) of C3E-7099.

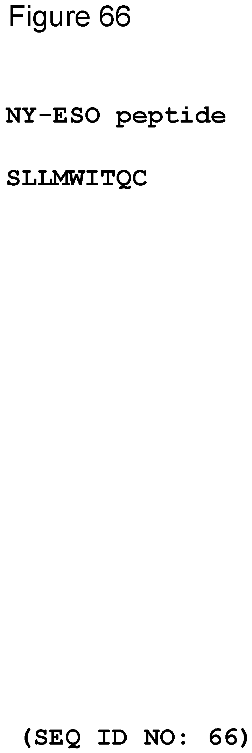

[Figure 66] Figure 66 shows the amino acid sequence (SEQ ID NO: 66) of NY-ESO.

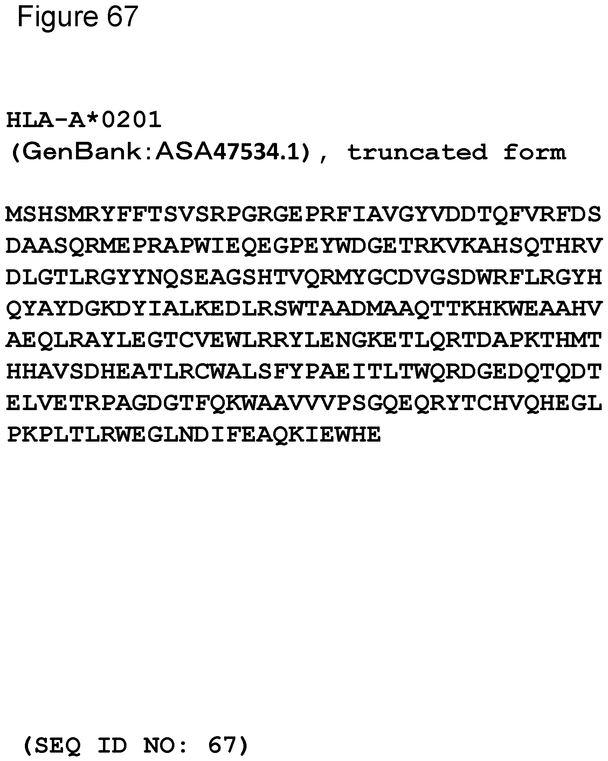

[Figure 67] Figure 67 shows the amino acid sequence (SEQ ID NO: 67) of a truncated form of HLA-A*0201 (GenBank: ASA47534.1) .

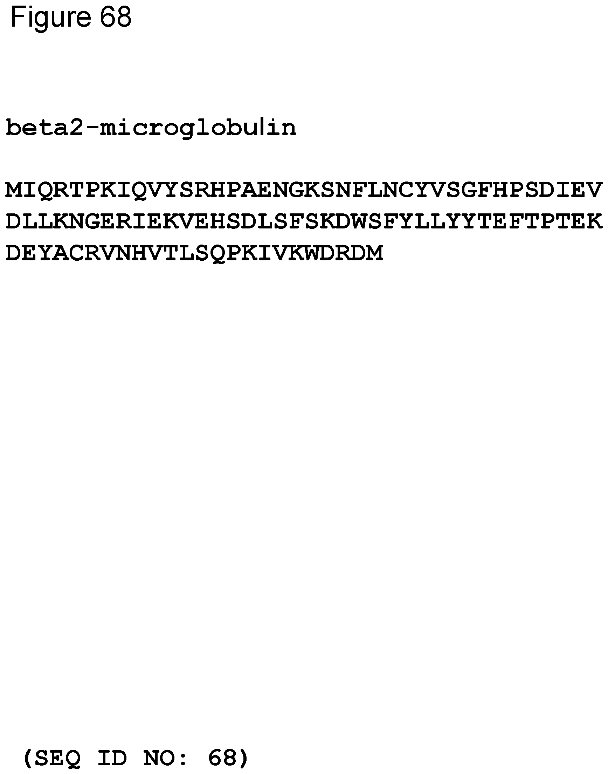

[Figure 68] Figure 68 shows the amino acid sequence (SEQ **ID NO:** 68) of β2-microglobulin.

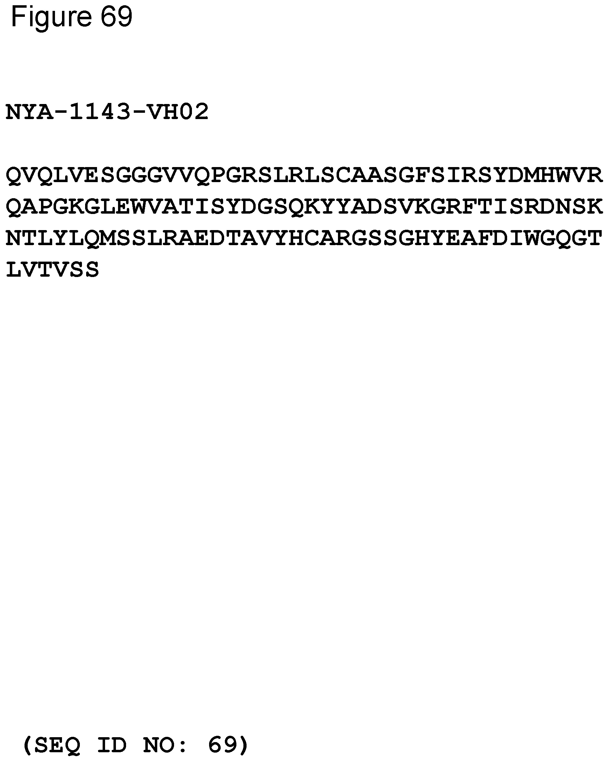

[Figure 69] Figure 69 shows the amino acid sequence (SEQ **ID NO:** 69) of NYA-1143-VH02.

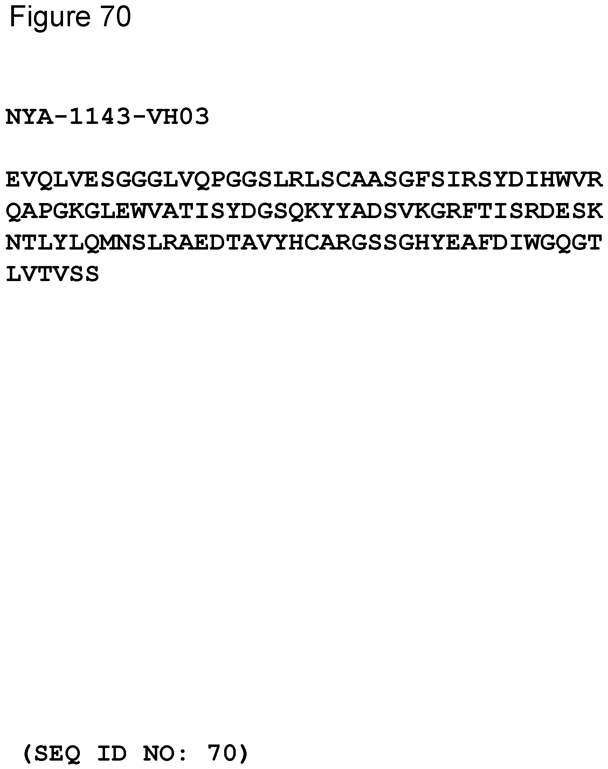

[Figure 70] Figure 70 shows the amino acid sequence (SEQ **ID NO:** 70) of NYA-1143-VH03.

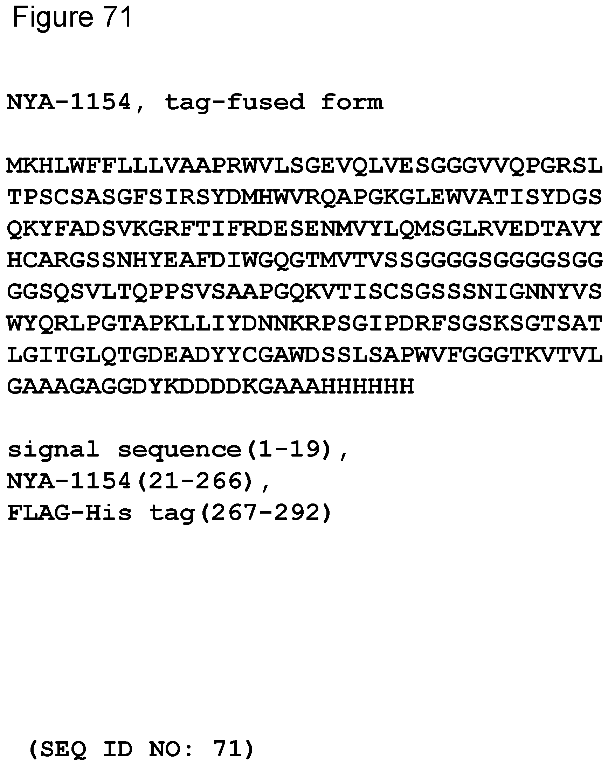

[Figure 71] Figure 71 shows the full-length amino acid sequence (SEQ ID **NO:** 71) of NYA-1154. This includes a signal sequence (1-19), NYA-1154 (21-266), and a FLAG-His tag (267-292).

[Figure 72(1)] Figure 72(1) is a diagram showing anti-tumor activity achieved by single administration of an Fc-conjugated type anti-HLA-A2/NY-ESO-anti-CD3 bispecific molecule NYZ-1010, combined administration of lenvatinib and pembrolizumab, and combined administration of NYZ-1010, lenvatinib, and pembrolizumab, to human T cell transfer models. The error bar in the diagram represents standard deviation (n = 5).

[Figure 72(2)] Figure 72(2) is a diagram showing the percentage change in tumor volume on Day 52 in each mouse using a tumor volume on Day 28 as a baseline following single administration of an Fc-conjugated type anti-HLA-A2/NY-ESO-anti-CD3 bispecific molecule NYZ-1010, combined administration of lenvatinib and pembrolizumab, and combined administration of NYZ-1010, lenvatinib, and pembrolizumab to human T cell transfer models.

[Figure 73(1)] Figure 73(1) is a diagram showing antitumor activity achieved by single administration of an Fc-conjugated type anti-HLA-A2/NY-ESO-anti-CD3 bispecific molecule NYZ-1010, single administration of an anti-mouse PD-1 antibody, and combined administration of NYZ-1010 and an anti-mouse PD-1 antibody to human CD3ε knock-in mouse models of BALB/C strain. The error bar in the diagram represents standard deviation (n = 5 to 10).

[Figure 73(2)] Figure 73(2) is a diagram showing an estimated tumor volume in each mouse following single administration of an Fc-conjugated type anti-HLA-A2/NY-ESO-anti-CD3 bispecific molecule NYZ-1010, single administration of an anti-mouse PD-1 antibody, and combined administration of NYZ-1010 and an anti-mouse PD-1 antibody to human CD3ε knock-in mouse models of BALB/C strain, and an estimated tumor volume after CT26 NY-SCT re-challenge in mice with complete regression and naive mice.

[Figure 74(1)] Figure 74(1) is a diagram showing anti-tumor activity achieved by single administration of an Fc-conjugated type anti-HLA-A2/NY-ESO-anti-CD3 bispecific molecule NYZ-1010, single administration of durvalumab, and combined administration of NYZ-1010 and durvalumab to human T cell transfer models. The error bar in the diagram represents standard deviation (n = 5).

[Figure 74(2)] Figure 74(2) is a diagram showing the percentage change in tumor volume on Day 49 in each mouse using a tumor volume on Day 24 as a baseline following single administration of an Fc-conjugated type anti-HLA-A2/NY-ESO-anti-CD3 bispecific molecule NYZ-1010, single administration of durvalumab, and combined administration of NYZ-1010 and durvalumab to human T cell transfer models.

[Figure 75(1)] Figure 75(1) is a diagram showing anti-tumor activity achieved by single administration of an Fc-conjugated type anti-HLA-A2/NY-ESO-anti-CD3 bispecific molecule NYZ-1010, single administration of an anti-mouse PD-L1 antibody, and combined administration of NYZ-1010 and an anti-mouse PD-L1 antibody to human CD3ε knock-in mouse models of BALB/C strain. The error bar in the diagram represents standard deviation (n = 5 to 10).

[Figure 75(2)] Figure 75(2) is a diagram showing an estimated tumor volume in each mouse following single administration of an Fc-conjugated type anti-HLA-A2/NY-ESO-anti-CD3 bispecific molecule NYZ-1010, single administration of an anti-mouse PD-L1 antibody, and combined administration of NYZ-1010 and an anti-mouse PD-L1 antibody to human CD3ε knock-in BALB/C mice, and an estimated tumor volume after CT26 Mock re-challenge in mice with complete regression and naive mice.

[Figure 76(1)] Figure 76(1) is a diagram showing the antitumor activity achieved by single administration of an Fc-conjugated type anti-HLA-A2/NY-ESO-anti-CD3 bispecific molecule NYZ-1010, single administration of an anti-mouse CTLA-4 antibody, and combined administration of NYZ-1010 and an anti-mouse CTLA-4 antibody to human CD3ε knock-in mouse models of BALB/C strain. The error bar in the diagram represents standard deviation (n = 5 to 10).

[Figure 76(2)] Figure 76(2) is a diagram showing an estimated tumor volume in each mouse following single administration of an Fc-conjugated type anti-HLA-A2/NY-ESO-anti-CD3 bispecific molecule NYZ-1010, single administration of an anti-mouse CTLA-4 antibody, and combined administration of NYZ-1010 and an anti-mouse CTLA-4 antibody to human CD3ε knock-in mouse models of BALB/C strain, and an estimated tumor volume after CT26 NY-SCT re-challenge in mice with complete regression, and naive mice.

[Figure 77(1)] Figure 77(1) is a diagram showing the anti-tumor activity achieved by single administration of an Fc-conjugated type anti-HLA-A2/NY-ESO-anti-CD3 bispecific molecule NYZ-1010, single administration of an anti-mouse TIGIT antibody, and combined administration of NYZ-1010 and an anti-mouse TIGIT antibody to human CD3ε knock-in BALB/C mice. The error bar in the diagram represents standard deviation (n = 5 to 10).

[Figure 77(2)] Figure 77(2) is a diagram showing an estimated tumor volume in each mouse following single administration of an Fc-conjugated type anti-HLA-A2/NY-ESO-anti-CD3 bispecific molecule NYZ-1010, single administration of an anti-mouse TIGIT antibody, and combined administration of NYZ-1010 and an anti-mouse TIGIT antibody to human CD3ε knock-in mouse models of BALB/C strain, and an estimated tumor volume after CT26 NY-SCT re-challenge in mice with complete regression and naive mice.

[Figure 78] Figure 78 is a diagram showing the antitumor activity achieved by single administration of an Fc-conjugated type anti-HLA-A2/NY-ESO-anti-CD3 bispecific molecule NYZ-1010, single administration of carboplatin, and combined administration of NYZ-1010 and carboplatin to human T cell transfer models. The error bar in the diagram represents standard deviation (n = 5).

[Figure 79(1)] Figure 79(1) is a diagram showing the antitumor activity achieved by single administration of an Fc-conjugated type anti-HLA-A2/NY-ESO-anti-CD3 bispecific molecule NYZ-1010, single administration of sacituzumab govitecan-hziy, and combined administration of NYZ-1010 and sacituzumab govitecan-hziy to human T cell transfer models. The error bar in the diagram represents standard deviation (n = 5).

[Figure 79(2)] Figure 79(2) is a diagram showing the percentage change in tumor volume on Day 21 of each mouse using a tumor volume on Day 6 as a baseline following single administration of an Fc-conjugated type anti-HLA-A2/NY-ESO-anti-CD3 bispecific molecule NYZ-1010, single administration of sacituzumab govitecan-hziy, and combined administration of NYZ-1010 and sacituzumab govitecan-hziy to human T cell transfer models.

[Figure 80(1)] Figure 80(1) is a diagram showing antitumor activity achieved by single administration of an Fc-conjugated type anti-HLA-A2/NY-ESO-anti-CD3 bispecific molecule NYZ-1010, single administration of doxorubicin, and combined administration of NYZ-1010 and doxorubicin to human T cell transfer models. The error bar in the diagram represents standard deviation (n = 5).

[Figure 80(2)] Figure 80(2) is a diagram showing the percentage change in tumor volume on Day 55 in each mouse using a tumor volume on Day 40 as a baseline following single administration of an Fc-conjugated type anti-HLA-A2/NY-ESO-anti-CD3 bispecific molecule NYZ-1010, single administration of doxorubicin, and combined administration of NYZ-1010 and doxorubicin to human T cell transfer models.

[Figure 81(1)] Figure 81(1) is a diagram showing antitumor activity achieved by single administration of an Fc-conjugated type anti-HLA-A2/NY-ESO-anti-CD3 bispecific molecule NYZ-1010, single administration of decitabine, and combined administration of NYZ-1010 and decitabine to human T cell transfer models. The error bar in the diagram represents standard deviation (n = 5).

[Figure 81(2)] Figure 81(2) is a diagram showing the percentage change in tumor volume on Day 32 in each mouse using a tumor volume on Day 7 as a baseline following single administration of an Fc-conjugated type anti-HLA-A2/NY-ESO-anti-CD3 bispecific molecule NYZ-1010, single administration of decitabine, and combined administration of NYZ-1010 and decitabine to human T cell transfer models.

[Figure 82] Figure 82 shows the sequence listing of Japanese Patent Application No. 2022-041253 filed on March 16, 2022.

### Description of Embodiments

Hereinafter, the present invention will be described in detail.

### 1. Definition

In the present invention, the term "gene" means a nucleotide strand comprising a nucleotide sequence encoding the amino acids of a protein, or its complementary strand. The "gene" is meant to include, for example, a polynucleotide, an oligonucleotide, DNA, mRNA, cDNA, and cRNA as the nucleotide strand comprising a nucleotide sequence encoding the amino acids of a protein, or its complementary strand. Such a gene is a single-stranded, double-stranded, or triple or more stranded nucleotide. The "gene" is also meant to include an association of DNA and RNA strands, a mixture of ribonucleotides (RNAs) and deoxyribonucleotides (DNAs) on one nucleotide strand, and a double-stranded or triple or more stranded nucleotide comprising such a nucleotide strand. In the present invention, a base sequence and a nucleotide sequence have the same meaning.

In the present invention, the terms "polynucleotide", a "nucleotide strand", a "nucleic acid", and a "nucleic acid molecule" have the same meaning and are also meant to include, for example, DNA, RNA, a probe, an oligonucleotide, and a primer. Such a polynucleotide is a single-stranded, double-stranded, or triple or more stranded polynucleotide. The "polynucleotide" is also meant to include an association of DNA and RNA strands, a mixture of ribonucleotides (RNAs) and deoxyribonucleotides (DNAs) on one polynucleotide strand, and an association of two strands or three or more strands comprising such a polynucleotide strand.

In the present invention, the terms "polypeptide", "peptide", and "protein" have the same meaning.

In the present invention, the term "antigen" has the same meaning as "immunogen".

In the present invention, the term "cell" includes, for example, various cells derived from individual animals, subcultured cells, primary cultured cells, cell lines, recombinant cells, and microbial cells.

In the present invention, the term "antibody" has the same meaning as an immunoglobulin. However, the "antibody" used for the anti-HLA/NY-ESO antibody of the present invention means an immunoglobulin having constant and variable regions. The antibody is not particularly limited and may be a natural immunoglobulin or may be an immunoglobulin produced by partial or complete synthesis. The anti-HLA/NY-ESO antibody of the present invention is included in the "molecule" mentioned below.

In the present invention, the term "NY-ESO peptide" means a peptide consisting of 9 amino acids from positions 157 to 165 of NY-ESO-1 or LAGE-1 (SLLMWITQC: SEQ ID NO: 1).

In the present invention, the term "HLA-A2/NY-ESO" means a complex of the NY-ESO peptide and histocompatibility leukocyte antigen-A2 (HLA-A2) and is also referred to as "HLA/NY-ESO".

In the present invention, the term "anti-HLA-A2/NY-ESO antibody" means an antibody that binds to HLA-A2/NY-ESO and in other words, an antibody that recognizes HLA-A2/NY-ESO. Likewise, the term "anti-HLA-A2/NY-ESO scFv" means a scFv that binds to HLA/NY-ESO and in other words, a scFv that recognizes HLA-A2/NY-ESO. The terms "anti-HLA-A2/NY-ESO antibody" and "anti-HLA-A2/NY-ESO scFv" are also referred to as an "anti-HLA/NY-ESO antibody" and "anti-HLA/NY-ESO scFv", respectively.

The basic structure of a quaternary antibody includes two identical light chains (L chains) and two identical heavy chains (H chains). Each light chain is linked to the heavy chain by one covalent disulfide bond. The two heavy chains are linked to each other by one or more disulfide bonds according to the isotypes of the heavy chains. Each of the light and heavy chains has regularly spaced intrachain disulfide bonds. Each of the heavy and light chains contains a constant region which exhibits a very high degree of amino acid sequence similarity and a variable region which exhibits a low degree of amino acid sequence similarity. The light chain has a variable region (VL) at the amino terminus followed by a constant region (CL). The heavy chain has a variable region (VH) at the amino terminus followed by three constant regions (CH1, CH2, and CH3). VL and VH are paired with each other, and CL is aligned with the first constant region (CH1) of the heavy chain. The pair of VL and VH forms a single antigen-binding site.

The constant regions of the antibody of the present invention are not particularly limited. Preferably, constant regions derived from a human antibody are used in the antibody of the present invention for the treatment or prevention of a disease in a human. Examples of the heavy chain constant region of the human antibody can include Cγ1, Cγ2, Cγ3, Cγ4, Cµ, Cδ, Cα1, Cα2, and Cε. Examples of the light chain constant region of the human antibody can include CK and Cλ.

Fab is composed of heavy chain VH followed by CH1 and light chain VL followed by CL. VH and VL each contain complementarity determining regions (CDRs).

Fc (also referred to as an Fc region) is the carboxyl-terminal region of the heavy chain constant regions and is a dimer comprising CH2 and CH3. The Fc of the present invention may be Fc having a natural sequence or may be a mutated form of Fc (referred to as "mutant Fc") containing a mutation in the natural sequence. In the multispecific molecule and the bispecific molecule of the present invention, the Fc region is preferably mutant Fc, and more preferably, a pair of Fc regions can form a heterodimer. Examples of the Fc pair can include a combination of Fc (i) contained in a first polypeptide and Fc (ii) contained in a second polypeptide as mentioned below. The pair of Fc regions is not limited thereto as long as the Fc molecules can associate with each other (heterodimerization).

Examples of the mutant Fc include, but are not limited to, a modified Fc region (including a heterodimeric Fc region) contained in a heteromultimer having improved stability as disclosed in WO2013/063702, Fc comprising an IgG antibody-derived immunoglobulin CH3 region with "knobs" and "holes" contained in a heteromultimer as disclosed in WO1996/27011, Fc comprising a CH3 domain contained in an electrostatically advantageous heterodimer by substituting one or more amino acid residues by charged amino acids, as disclosed in WO2009/089004, a heterodimeric Fc region contained in a heterodimer using a conformational mutation and/or a pI (isoelectric point) mutation as disclosed in WO2014/110601, and heterodimeric Fc comprising a CH3 domain having a modification to delete or reduce binding to protein A as disclosed in WO2010/151792.

The variable region is composed of regions, called hypervariable regions (HVRs), having extreme variability, and relatively invariable regions, called framework regions (FRs), interrupted by the hypervariable regions. The natural heavy chain and light chain variable regions each contain four FRs connected by three hypervariable regions. The hypervariable regions of each chain are kept in close proximity together with the hypervariable regions of another chain by FRs and contribute to the formation of an antigen-binding site in the antibody.

The heavy and light chains of an antibody molecule are known to each have three complementarity determining regions (CDRs). The complementarity determining regions are also called hypervariable regions. These regions are located in the variable regions of the antibody heavy and light chains. These sites have a particularly highly variable primary structure and are usually separated at three positions on the respective primary structures of heavy and light chain polypeptide strands. In the present invention, the complementarity determining regions of the heavy chain sequence of the antibody are referred to as CDRH1, CDRH2, and CDRH3, respectively, from the amino terminus to the carboxyl terminus of the heavy chain amino acid sequence, and the complementarity determining regions of the light chain sequence of the antibody are referred to as CDRL1, CDRL2, and CDRL3, respectively, from the amino terminus to- the carboxyl terminus of the light chain amino acid sequence. These sites are proximal to each other on the three-dimensional structure and determine specificity for the antigen which is bound.

In the present invention, the positions and lengths of CDRs were determined in accordance with the definition of IMGT (Developmental and Comparative Immunology 27 (2003) 55-77).

The FRs are variable regions which lie outside of the CDR residues. Each variable region generally has four FRs (FR1, FR2, FR3, and FR4).

The CDRs and the FRs contained in heavy and light chains are arranged in the order of FRH1-CDRH1-FRH2-CDRH2-FRH3-CDRH3-FRH4 and FRL1-CDRL1-FRL2-CDRL2-FRL3-CDRL3-FRL4, respectively, from the amino terminus towards the carboxy terminus.

The positions of CDRs and FRs can also be determined in accordance with various schemes which are well known in the art, for example, based on IMGT as well as Kabat, Chothia, AbM, contact, etc.

In the present invention, the term "antigen-binding fragment of the antibody" means a part of an antibody that comprises heavy chain and light chain variable regions and has binding activity towards the antigen. Examples of the "antigen-binding fragment of the antibody" can include, but are not limited to, antigen-binding fragments such as Fab, F(ab')₂, scFv, Fab', Fv, and single-domain antibody (sdAb). Such an antigen-binding fragment of the antibody may be obtained by treating a full-length molecule of the antibody protein with an enzyme such as papain or pepsin or may be a recombinant protein produced in an appropriate host cell using a recombinant gene. In the present invention, the term "binding fragment of the antibody" has the same meaning as the "antigen-binding fragment of the antibody".

In the present invention, the "site" to which an antibody binds, i.e., the "site" recognized by an antibody, means a part of a peptide antigen or a part of a higher-order structure of an antigen which is bound or recognized by the antibody. In the present invention, such a site is also referred to as an epitope or an antibody binding site. In the present invention, the term "mutant antibody" means a polypeptide that has an amino acid sequence derived from the amino acid sequence of the original antibody by the substitution, deletion, and/or addition (the addition includes insertion) (hereinafter, collectively referred to as a "mutation") of amino acid(s) and binds to the HLA/NY-ESO of the present invention. The number of mutated amino acids in such a mutant antibody is 1, 2, 3, 4, 5, 6, 7, 8, 9, 10, 12, 15, 20, 25, 30, 40, or 50. Such a mutant antibody is also encompassed by the "antibody" of the present invention.

In the present invention, the term "several" in "one or several" refers to 2 to 10.

In the present invention, the term "multispecific molecule" is not particularly limited as long as the molecule has two or more moieties capable of binding to a plurality of epitopes which are different from each other, and/or, capable of binding to epitopes on two or more molecules which are different from each other. The multispecific molecule is preferably a "multispecific antibody" in which each of two or more of such moieties is an antibody or an antigen-binding fragment thereof, or a molecule comprising the "multispecific antibody". The multispecific antibody can comprise one or two or more heavy chain variable (VH) and/or light chain variable (VL) regions and may be a full-length antibody having two or more types of heavy chains and light chains, i.e., an IgG-type multispecific antibody, or may be an antibody-related molecule consisting of two or more types of antigen-binding fragments having VLs and VHs, for example, a molecule consisting of or derived from a combination of Fab, Fab', Fv, scFv, sdAb, etc. (i.e., tandem scFv, diabody, single chain diabody, and triabody), though the multispecific antibody is not limited thereto. The multispecific antibody may contain a moiety which does not have an immunoglobulin framework.

Examples of activities or properties exerted by the anti-HLA/NY-ESO antibody of the present invention or the antigen-binding fragment of the antibody, or the molecule of the present invention can include biological activities and physicochemical properties (also referred to as physical properties) and can specifically include various biological activities such as cytotoxic activity, ADCC activity, and anti-tumor activity (all of which are mentioned below), binding activity against an antigen or an epitope, and physical properties such as stability during production or preservation and thermal stability.

In the present invention, the phrase "hybridize under stringent conditions" means hybridization under conditions involving hybridization at 65°C in a solution containing 5 × SSC, followed by washing at 65°C for 20 minutes in an aqueous solution containing 2 × SSC-0.1% SDS, at 65°C for 20 minutes in an aqueous solution containing 0.5 × SSC-0.1% SDS, and at 65°C for 20 minutes in an aqueous solution containing 0.2 × SSC-0.1% SDS, or hybridization under conditions equivalent thereto. SSC means an aqueous solution of 150 mM NaCl-15 mM sodium citrate, and n × SSC means SSC with an n-fold concentration.

In the present invention, the term "cytotoxicity" refers to some pathological change brought about to cells in one way or another and means not only direct trauma but any structural or functional damage to cells, including DNA cleavage, formation of base dimers, chromosomal break, damage on mitotic apparatus, and reduction in the activities of various enzymes. In the present invention, the term "cytotoxic activity" means activity that causes the cytotoxicity mentioned above.

In the present invention, the term "antibody dependent cellular cytotoxic activity", also called "ADCC activity", means the effect or activity of damaging target cells such as tumor cells by NK cells via antibodies.

In the present invention, the term "cytotoxic activity by the T cell redirection" means that the cytotoxicity is caused by a multispecific molecule comprising an antitumor antigen antibody and the anti-HLA/NY-ESO antibody. Specifically, the term means that the anti-tumor antigen antibody binds to target tumor cells while the anti-HLA/NY-ESO antibody binds to a T cell so that the target tumor cell and the T cell come close to each other to induce T cell activation-mediated cytotoxicity. The molecule can be contained in a pharmaceutical composition.

### 2. Antigen

### 2-1. HLA/NY-ESO antigen

In the present invention, the term "HLA/NY-ESO" has the same meaning as HLA/NY-ESO protein.

HLA/NY-ESO is a ternary complex of HLA-A2, β2-microglobulin, and NY-ESO peptide. HLA-A2 is an allele of HLA and is known as the most frequent allele in Caucasians. HLA forms a ternary complex with β2-microglobulin and a peptide fragment of a self-protein in the endoplasmic reticulum of cells, and extracellularly presents this complex, which in turn receives recognition by TCR (T cell receptor) of T cells. The NY-ESO peptide (SLLMWITQC: SEQ ID NO: 66) is a peptide consisting of 9 amino acids from positions 157 to 165 of NY-ESO-1 or LAGE-1 and is reportedly presented by HLA-A2.

### 2-2. CD3 antigen

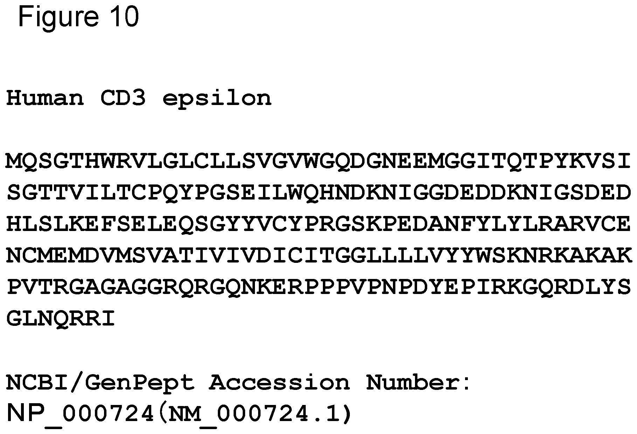

In the present invention, the term "CD3" has the same meaning as CD3 protein. CD3 is expressed, as a portion of a multimolecular T cell receptor complex, on T cells and is a complex of 5 types of polypeptides (γ, δ, ε, ζ, and η chains; molecular weights: 25000 to 28000, 21000, 20000, 16000, and 22000, respectively). γ, δ, ε, ζ, and η chains are also called subunits. An anti-CD3 antibody binds to a T cell and thereby induces T cell activation-mediated cytotoxicity. Many anti-CD3 antibodies bind to CD3ε. The nucleotide sequence of cDNA encoding human CD3ε is registered with NCBI/GenBank under accession No: NM_000733 (NM_000733.3), and the amino acid sequence of human CD3ε is registered with NCBI/GenPept under accession No: NP_000724 (NM_000724.1). The nucleotide sequence of cDNA encoding cynomolgus monkey CD3 is registered with GenBank under accession No: NM_001283615.1. The amino acid sequence of human CD3ε is described in Figure 10.

### 2-3. Preparation of antigen

Each aforementioned antigenic protein (HLA/NY-ESO or CD3) (hereinafter, HLA/NY-ESO and CD3 are also collectively referred to as the antigenic proteins) used in the present invention can be prepared by purification or isolation from animal tissues (including body fluids), cells derived from the tissues, or cultures of the cells, gene recombination, *in vitro* translation, chemical synthesis, etc. The cDNA of the antigenic protein can be obtained by, for example, a so-called PCR method which performs polymerase chain reaction (hereinafter, referred to as "PCR") (Saiki, R.K., et al., Science (1988) 239, 487-489) using a cDNA library derived from organs expressing the mRNA of the antigenic protein as a template and using primers capable of specifically amplifying the cDNA of the antigenic protein. The cDNA of the antigenic protein encompasses a polynucleotide that hybridizes under stringent conditions to a polynucleotide consisting of a nucleotide sequence complementary to the nucleotide sequence encoding the antigenic protein expressed in a human or a rat, and encoding a protein having biological activity equivalent to that of the antigenic protein. In addition, the cDNA of the antigenic protein encompasses a splicing variant transcribed from the gene locus of the antigenic protein expressed in a human or a rat, or a polynucleotide that hybridize thereto under stringent conditions, and encoding a protein having biological activity equivalent to that of the antigenic protein A nucleotide sequence encoding a protein that consists of an amino acid sequence derived from the amino acid sequence of the human or rat antigenic protein by the substitution, deletion, or addition of one to several amino acids, or from which the signal sequence has been deleted, and has biological activity equivalent to that of the antigenic protein is also included in the nucleotide sequence of the gene encoding the antigenic protein. A protein that consists of an amino acid sequence encoded by a splicing variant transcribed from the gene loci of the human or rat antigenic protein or an amino acid sequence derived from the amino acid sequence by the substitution, deletion, or addition of one to several amino acids and has biological activity equivalent to that of the antigenic protein is also included in the antigenic protein.

### 2-4 Binding specificity for antigenic protein

The anti-HLA/NY-ESO antibody of the present invention or the antigen-binding fragment thereof or the like recognizes HLA/NY-ESO. In other words, the anti-HLA/NY-ESO antibody or the antigen-binding fragment thereof binds to the HLA/NY-ESO antigen. The presence of HLA/NY-ESO is not known in non-human animals such as mice, rats, and cynomolgus monkeys. The anti-CD3 antibody or the antigen-binding fragment thereof, etc. contained in the multispecific antibody of the present invention recognizes, that is to say, binds to a CD3 antigen. Such an anti-CD3 antibody, etc. preferably binds to human CD3 and monkey CD3, and more preferably binds to human CD3 and cynomolgus monkey CD3. In contrast, such a preferable anti-CD3 antibody does not bind to either rat or mouse CD3.

In the present invention, the term "recognition", i.e., "binding", means binding which is not non-specific adsorption. Whether or not the antibody recognizes, that is to say, binds to a target antigen, can be evaluated on the basis of, for example, the dissociation constant

(hereinafter, referred to as "KD"). The KD value of the antibody, etc. of the present invention for HLA/NY-ESO or CD3 is preferably 1 × 10⁻⁵ M or less, 5 × 10⁻⁶ M or less, 2 × 10⁻⁶ M or less, or 1 × 10⁻⁶ M or less. The KD value for HLA/NY-ESO is preferably 5 × 10⁻⁷ M or less, 2 × 10⁻⁷ M or less, 1 × 10⁻⁷ M or less, 5 × 10⁻⁸ M or less, 2 × 10⁻⁸ M or less, 1 × 10⁻⁸ M or less, 5 × 10⁻⁹ M or less, or 2 × 10⁻⁹ M or less, more preferably 1 × 10⁻⁹ M or less. Examples of the anti-HLA/NY-ESO scFv of the present invention having excellent antigen-binding activity include NYA-1143, NYA-2023, NYA-2143, NYA-2044, NYA-2045, NYA-2060, NYA-2061, and NYA-3061. NYA-1143, NYA-2044, NYA-2045, NYA-2143, and NYA-1154,and have a KD value of 1 × 10⁻⁹ M or less with respect to HLA/NY-ESO. In the present invention, the binding of the antibody to the antigen can be assayed or determined by a system of biomolecular interaction analysis, such as SPR or BLI, ELISA,or RIA, or the like (see WO2021/200857). Examples of an anti-HLA/NY-ESO antibody, etc. of the present invention having excellent antigen-binding specificity include NYA-0001, NYA-1143, NYA-1163, NYA-2023, NYA-2027, NYA-2035, NYA-2044, NYA-2045, NYA-2047, NYA-2048, NYA-2060, NYA-2061, NYA-2143, and NYA-3061, each of which is an anti-HLA/NY-ESO scFv.

### 3. Antibody that binds specifically to HLA/NY-ESO or binding fragment thereof

### 3-1 anti-HLA/NY-ESO antibody or binding fragment thereof

The present invention provides an antibody that recognizes and binds to HLA/NY-ESO, or a binding fragment thereof. As mentioned above, HLA/NY-ESO is a complex comprising HLA-A2 and 9-mer NY-ESO peptide (SLLMWITQC: SEQ ID NO: 66). The NY-ESO peptide is a peptide derived from a cancer-testis antigen NY-ESO-1 or LAGE-1 which is an intracellular protein. HLA/NY-ESO is expressed on a cancer cell surface. The anti-HLA/NY-ESO antibody of the present invention and the antigen-binding fragment of the antibody (hereinafter, also referred to as the antibody, etc. of the present invention) may be either monoclonal or polyclonal antibodies. In the present invention, the isotype of a monoclonal antibody is not particularly limited, and examples include IgG such as IgG1, IgG2, IgG3, and IgG4, IgM, IgA such as IgA1 and IgA2, IgD, and IgE. The isotype and subclass of the monoclonal antibody can be determined by, for example, the Ouchterlony method, ELISA, or RIA. Examples of the monoclonal antibody of the present invention can include an antibody derived from a non-human animal (non-human animal antibodies), a human antibody, a chimeric antibody, and a humanized antibody. Preferably, a human antibody can be used to derive the antibody of the invention. A mutant of the antibody ("mutant antibody" as mentioned below) is also encompassed by the antibody of the present invention. For example, a human mutant antibody is also encompassed by the human antibody. Examples of non-human animal antibodies include antibodies derived from vertebrates such as mammals and birds. Examples of the mammalian-derived antibodies include antibodies derived from rodents such as mouse antibodies and rat antibodies. An example of a bird-derived antibody is a chicken antibody. Examples of chimeric antibodies include, but are not limited to, antibodies comprising a variable region derived from a non-human animal antibody bound to a constant region derived from a human antibody (human immunoglobulin). Examples of a humanized antibody include, but are not limited to, a humanized antibody prepared by transplanting CDRs in a variable region of a non-human animal antibody into a human antibody (a variable region of human immunoglobulin), a humanized antibody prepared by transplanting, in addition to CDRs, a part of a sequence of a framework region of a non-human animal antibody into a human antibody, and a humanized antibody prepared by substitution of 1 or more amino acids derived from a non-human animal antibody with amino acids derived from a human antibody.

The antibody can be prepared by various methods known in the art. The antibody can be prepared by a method known in the art involving the use of hybridomas, cell-mediated immunity or genetic recombination. A method for obtaining a phage display-derived human antibody selected from a human antibody library is also known. In the phage display method, for example, a human antibody variable region may be expressed as a scFv on phage surface, and a phage that binds to the antigen may then be selected. The phage which has been selected on the basis of its binding to the antigen can be subjected to genetic analysis to determine the DNA sequence encoding the variable regions of the human antibody that binds to the antigen. If the DNA sequence of scFv that binds to the antigen is determined, an expression vector comprising such a sequence can be prepared and introduced into an appropriate host cell that is allowed to express the human antibody. Thus, a human antibody can be obtained (WO92/01047, WO92/20791, WO93/06213, WO93/11236, WO93/19172, WO95/01438, WO95/15388, Annu.Rev.Immunol

(1994) 12, 433-455). The obtained antibody having high activity may be used as a lead antibody, and a gene encoding the lead antibody can be mutated to prepare a mutant having higher activity ("mutant antibody" as mentioned below).

A preferred combination of CDRH1 to CDRH3 contained in the heavy chain of the anti-HLA/NY-ESO antibody of the present invention or the antigen-binding fragment thereof is CDRH1 consisting of the amino acid sequence represented by SEQ ID NO: 1, CDRH2 consisting of the amino acid sequence represented by SEQ ID NO: **2,** and CDRH3 consisting of the amino acid sequence represented by SEQ ID NO: **3,** or the amino acid sequence represented by SEQ ID NO: 3 in which the amino acid at position 6 is N (Asn). A more preferred combination of CDRH1 to CDRH3 is contained in the NYA-0001 heavy chain variable region consisting of the amino acid sequence represented by SEQ ID NO: 13**,** the NYA-0082 heavy chain variable region consisting of the amino acid sequence represented by SEQ ID NO: 15, the NYA-2023 heavy chain variable region consisting of amino acids 21 to 140 of the amino acid sequence represented by SEQ ID NO: 18, the NYA-2027 heavy chain variable region consisting of amino acids 21 to 140 of the amino acid sequence represented by SEQ ID NO: 19**,** the NYA-1143 heavy chain variable region consisting of amino acids21 to 140 of the amino acid sequence represented by SEQ ID NO: 20**,** the NYA-1163 heavy chain variable region consisting of amino acids 21 to 140 of the amino acid sequence represented by SEQ ID NO: 17, the NYA-2023 heavy chain variable region consisting of amino acids 21 to 140 of the amino acid sequence represented by SEQ ID NO: 18, the NYA-2027 heavy chain variable region consisting of amino acids 21 to 140 of the amino acid sequence represented by SEQ ID NO: 19**,** the NYA-2035 heavy chain variable region consisting of amino acids21 to 140 of the amino acid sequence represented by SEQ ID NO: 22**,** the NYA-2044 heavy chain variable region consisting of amino acids 21 to 140 of the amino acid sequence represented by SEQ ID NO: 24, the NYA-2045 heavy chain variable region consisting of amino acids 21 to 140 of the amino acid sequence represented by SEQ ID NO: 25**,** the NYA-2047 heavy chain variable region consisting of amino acids 21 to 140 of the amino acid sequence represented by SEQ ID NO: 26**,** the NYA-2048 heavy chain variable region consisting of amino acids 21 to 140 of the amino acid sequence represented by SEQ ID NO: 27, the NYA-2060 heavy chain variable region consisting of amino acids 21 to 140 of the amino acid sequence represented by SEQ ID NO: 28, the NYA-2061 heavy chain variable region consisting of amino acids 21 to 140 of the amino acid sequence represented by SEQ ID NO: 53, or the NYA-2143 heavy chain variable region consisting of amino acids 21 to 140 of the amino acid sequence represented by SEQ ID **NO:** 21. Further examples of a combination of CDRH1 to CDRH3 are contained in the NYA-3061 heavy chain variable region consisting of amino acids 21 to 140 of the amino acid sequence represented by SEQ ID **NO:** 55, and the NYA-1154 heavy chain variable region consisting of amino acids 21 to 140 of the amino acid sequence represented by SEQ ID **NO:** 71.

A preferred combination of CDRL1 to CDRL3 contained in the light chain of the anti-HLA/NY-ESO antibody of the present invention or the antigen-binding fragment thereof is CDRL1 consisting of the amino acid sequence represented by SEQ ID NO: 4, or an amino acid sequence derived from the amino acid sequence represented by SEQ ID NO: 4 in which the amino acid at position 7 is W (Trp) and/or the amino acid at position 8 is K (Lys), CDRL2 consisting of the amino acid sequence represented by SEQ ID NO: 5, and CDRL3 consisting of the amino acid sequence represented by SEQ ID NO: 6, or an amino acid sequence derived from the amino acid sequence represented by SEQ ID NO: 6 in which the amino acid at position 2 is A (Ala) or S (Ser).

A more preferred combination of CDRL1 to CDRL3 is contained in the NYA-0001 light chain variable region consisting of the amino acid sequence represented by SEQ ID NO: 14, the NYA-0082 light chain variable region consisting of the amino acid sequence represented by SEQ ID NO: 16, the NYA-1143 light chain variable region consisting of amino acids 156 to 266 of the amino acid sequence represented by SEQ ID NO: 20, the NYA-1163 light chain variable region consisting of amino acids 156 to 266 of the amino acid sequence represented by SEQ ID NO: 26, the NYA-2023 light chain variable region consisting of amino acids 156 to 266 of the amino acid sequence represented by SEQ ID NO: 18, the NYA-2027 light chain variable region consisting of amino acids 156 to 266 of the amino acid sequence represented by SEQ ID NO: 19, the NYA-2035 light chain variable region consisting of amino acids 156 to 266 of the amino acid sequence represented by SEQ ID NO: 22, the NYA-2044 light chain variable region consisting of amino acids 156 to 266 of the amino acid sequence represented by SEQ ID NO: 24, the NYA-2045 light chain variable region consisting of amino acids 156 to 266 of the amino acid sequence represented by SEQ ID NO: 25, the NYA-2047 light chain variable region consisting of amino acids 156 to 266 of the amino acid sequence represented by SEQ ID NO: 26, the NYA-2048 light chain variable region consisting of amino acids 156 to 266 of the amino acid sequence represented by SEQ ID NO: 27, the NYA-2060 light chain variable region consisting of amino acids 156 to 266 of the amino acid sequence represented by SEQ ID NO: 28, the NYA-2061 light chain variable region consisting of amino acids 156 to 266 of the amino acid sequence represented by SEQ ID NO: 29, or the NYA-2143 light chain variable region consisting of amino acids 156 to 266 of the amino acid sequence represented by SEQ ID NO: 21. A more preferred combination of CDRL1 to CDRL3 is contained in the NYA-3061 light chain variable region consisting of amino acids 161 to 271 of the amino acid sequence represented by SEQ ID NO: 55, and the NYA-1154 light chain variable region consisting of amino acids 156 to 266 of the amino acid sequence represented by SEQ ID NO: 71.

¥A preferred combination of CDRH1 to CDRH3 contained in the heavy chain and CDRL1 to CDRL3 contained in the light chain of the anti-HLA/NY-ESO antibody of the present invention or the antigen-binding fragment thereof is a combination of CDRH1 consisting of the amino acid sequence represented by SEQ ID NO: 1, CDRH2 consisting of the amino acid sequence represented by SEQ ID NO: 2, and CDRH3 consisting of the amino acid sequence represented by SEQ ID NO: 3, or an amino acid sequence derived from the amino acid sequence represented by SEQ ID NO: 3 in which the amino acid at position 6 is N (Asn), CDRL1 consisting of the amino acid sequence represented by SEQ ID NO: 4, or an amino acid sequence derived from the amino acid sequence represented by SEQ ID NO: 4 in which the amino acid at position 7 is W (Trp) and/or the amino acid at position 8 is K (Lys), CDRL2 consisting of the amino acid sequence represented by DNN (the 5th sequence from the top in Figure 11: SEQ ID NO: 5 in Figure 82), and CDRL3 consisting of the amino acid sequence represented by SEQ ID NO: 6, or an amino acid sequence derived from the amino acid sequence represented by SEQ ID NO: 6 in which the amino acid at position 2 is A (Ala) or S (Ser). A more preferred combination of CDRH1 to CDRH3 and CDRL1 to CDRL3 is contained in the NYA-0001 heavy chain and light chain variable regions consisting of the amino acid sequences represented by SEQ ID NOs: 13 and 14, respectively, the NYA-1143 heavy chain and light chain variable regions consisting of amino acids 21 to 140 and 156 to 266, respectively, of the amino acid sequence represented by SEQ ID NO: 20, the NYA-1163 heavy chain and light chain variable regions consisting of amino acids 21 to 140 and 156 to 266, respectively, of the amino acid sequence represented by SEQ ID NO: 17,the NYA-2023 heavy chain and light chain variable regions consisting of amino acids 21 to 140 and 156 to 266, respectively, of the amino acid sequence represented by SEQ ID NO: 18, the NYA-2027 heavy chain and light chain variable regions consisting of amino acids 21 to 140 and 156 to 266, respectively, of the amino acid sequence represented by SEQ ID NO: 19, the NYA-2035 heavy chain and light chain variable regions consisting of amino acids 21 to 140 and 156 to 266, respectively, of the amino acid sequence represented by SEQ ID NO: 22, the NYA-2044 heavy chain and light chain variable regions consisting of amino acids21 to 140 and 156 to 266, respectively, of the amino acid sequence represented by SEQ ID NO: 24, the NYA-2045 heavy chain and light chain variable regions consisting of amino acids 21 to 140 and 156 to 266, respectively, of the amino acid sequence represented by SEQ ID NO: 25, the NYA-2047 heavy chain and light chain variable regions consisting of amino acids 21 to 140 and 156 to 266, respectively, of the amino acid sequence represented by SEQ ID NO: 26, the NYA-2048 heavy chain and light chain variable regions consisting of amino acids 21 to 140 and 156 to 266, respectively, of the amino acid sequence represented by SEQ ID NO: 27, the NYA-2060 heavy chain and light chain variable regions consisting of amino acids21 to 140 and 156 to 266, respectively, of the amino acid sequence represented by SEQ ID NO: 28, the NYA-2061 heavy chain and light chain variable regions consisting of amino acids 21 to 140 and 156 to 266, respectively, of the amino acid sequence represented by SEQ ID NO: 29, or the NYA-2143 heavy chain and light chain variable regions consisting of amino acids 21 to 140 and 156 to 266, respectively, of the amino acid sequence represented by SEQ ID NO: 21. Other preferred combinations of CDRH1 to CDRH3 and CDRL1 to CDRL3 are contained in the NYA-3061 heavy chain and light chain variable regions consisting of amino acids 21 to 140 and 161 to 271, respectively, of the amino acid sequence represented by SEQ ID NO: 55, and the NYA-1154 heavy chain and light chain variable regions consisting of amino acids 21 to 140 and 156 to 266, respectively, of the amino acid sequence represented by SEQ ID NO: 71.

Preferred examples of a heavy chain variable region of the anti-HLA/NY-ESO antibody of the present invention or the antigen-binding fragment thereof include the heavy chain CDRs described above and variable regions comprising such heavy chain CDRs in combination. More preferred examples include the NYA-0001 heavy chain variable region, the NYA-0082 heavy chain variable region, theNYA-1143 heavy chain variable region, theNYA-1163 heavy chain variable region, theNYA-2023 heavy chain variable region, theNYA-2027 heavy chain variable region, theNYA-2035 heavy chain variable region, theNYA-2044 heavy chain variable region, theNYA-2045 heavy chain variable region, theNYA-2047 heavy chain variable region, theNYA-2048 heavy chain variable region, theNYA-2060 heavy chain variable region, theNYA-2061 heavy chain variable region, theNYA-2143 heavy chain variable region, theNYA-3061 heavy chain variable region, and theNYA-1154 heavy chain variable region. The respective amino acid sequences of the heavy chain variable regions are as described above.

Preferred examples of the light chain variable region of the anti-HLA/NY-ESO antibody of the present invention or the antigen-binding fragment thereof include the light chain CDRs described above and variable regions including such light chain CDRs in combination. More preferable examples include the NYA-0001 light chain variable region, the NYA-0082 light chain variable region, the NYA-1143 light chain variable region, the NYA-1163 light chain variable region, the NYA-2023 light chain variable region, the NYA-2027 light chain variable region, the NYA-2035 light chain variable region, the NYA-2044 light chain variable region, the NYA-2045 light chain variable region, the NYA-2047 light chain variable region, the NYA-2048 light chain variable region, the NYA-2060 light chain variable region, the NYA-2061 light chain variable region, the NYA-2143 light chain variable region, the NYA-3061 light chain variable region, and the NYA-1154 light chain variable region. The respective amino acid sequences of the light chain variable regions are as described above.

Preferred examples of heavy chain and light chain variable regions of the anti-HLA/NY-ESO antibody of the present invention or the antigen-binding fragment thereof include the heavy chain and light chain CDRs described above or variable regions including such CDRs in combination.More preferred examples include the NYA-0001 heavy chain and light chain variable regions, the NYA-0082 heavy chain and light chain variable regions, the NYA-1143 heavy chain and light chain variable regions, the NYA-1163 heavy chain and light chain variable regions, the NYA-2023 heavy chain and light chain variable regions, the NYA-2027 heavy chain and light chain variable regions, the NYA-2035 heavy chain and light chain variable regions, the the NYA-2044 heavy chain and light chain variable regions, the NYA-2045 heavy chain and light chain variable regions, the NYA-2047 heavy chain and light chain variable regions, the NYA-2048 heavy chain and light chain variable regions, the NYA-2060 heavy chain and light chain variable regions, the NYA-2061 heavy chain and light chain variable regions, a combination of NYA-2143 heavy chain and light chain variable regions, a combination of NYA-3061 heavy chain and light chain variable regions, and a combination of NYA-1154 heavy chain and light chain variable regions.

Preferable examples of a heavy chain of the anti-HLA/NY-ESO antibody of the present invention or the antigen-binding fragment thereof include a heavy chain comprising a heavy chain variable region listed above. More preferred examples include the NYA-0001 heavy chain, the NYA-0082 heavy chain, theNYA-1143 heavy chain, theNYA-1163 heavy chain, theNYA-2023 heavy chain, theNYA-2027 heavy chain, theNYA-2035 heavy chain, theNYA-2044 heavy chain, theNYA-2045 heavy chain, theNYA-2047 heavy chain, theNYA-2048 heavy chain, theNYA-2060 heavy chain, theNYA-2061 heavy chain, theNYA-2143 heavy chain, theNYA-3061 heavy chain, and the NYA-1154 heavy chain.

Preferred examples of a light chain of the anti-HLA/NY-ESO antibody of the present invention or the antigen-binding fragment thereof include a light chain comprising a light chain variable region listed above. More preferred examples include the NYA-0001 light chain, theNYA-0082 light chain, theNYA-1143 light chain, theNYA-1163 light chain, theNYA-2023 light chain, theNYA-2027 light chain, theNYA-2035 light chain, theNYA-2044 light chain, theNYA-2045 light chain, theNYA-2047 light chain, theNYA-2048 light chain, theNYA-2060 light chain, theNYA-2061 light chain, theNYA-2143 light chain, theNYA-3061 light chain, and the NYA-1154 light chain.

Preferred examples of a heavy chain and a light chain of the anti-HLA/NY-ESO antibody of the present invention or the antigen-binding fragment thereof include heavy and light chains including the preferred or more preferred heavy chain and light chain variable regions, respectively, described above. More preferred examples include the heavy and light chains of NYA-0001, NYA-1143, NYA-1163, NYA-2023, NYA-2027, NYA-2035, NYA-2044, NYA-2045, NYA-2047, NYA-2048, NYA-2060, NYA-2061, NYA-2143, NYA-3061, and NYA-1154.

The antigen-binding fragment of the antibody means a fragment that maintains at least the antigen-binding activity of the functions of the antibody, or a modified form thereof. Examples of the functions of the antibody include, in general, antigen- binding activity, the activity of regulating antigen activity, antibody dependent cellular cytotoxic activity, and complement dependent cytotoxic activity. Examples of the functions of the antibody, etc. of the present invention and the multispecific molecule comprising the antibody, etc. of the present invention include the T cell redirection, the activation of T cells, and cytotoxic activity against cancer cells by the activation of T cells.

The antigen-binding fragment of the antibody is not particularly limited as long as the fragment of the antibody maintains at least antigen-binding activity of the functions of the antibody. Examples thereof include, but are not limited to, Fab, Fab', F(ab')₂, Fv, single chain Fv (scFv) comprising heavy and light chain Fv molecules linked via an appropriate linker, and single domain antibody (sdAb). A molecule comprising a region other than the antigen- binding fragment of the antibody of the present invention, as in the case of a scFv comprising a linker portion is encompassed by the antigen-binding fragment of the antibody of the present invention.

A molecule that is derived from the antibody of the invention by the deletion of one or several or more amino acids from the amino terminus and/or carboxyl terminus, and that retains at least some of the functions of the antibody is also encompassed by the antigen-binding fragment of the antibody of the invention. A modified form of the antigen-binding fragment of the antibody is also encompassed by the antibody of the present invention or antigen-binding fragment thereof, or a modified antibody or fragment (described below).

According to one aspect, the antibody of the present invention or the antigen-binding fragment thereof is ascFv. The scFv is obtained by linking the heavy chain and light chain variable regions of the antibody via a polypeptide linker (Pluckthun A., The Pharmacology of Monoclonal Antibodies 113, Rosenburg and Moore, ed., Springer Verlag, New York, 269-315 (1994); and Nature Biotechnology (2005), 23, 1126-1136). Also, a tandem scFv comprising two scFv molecules linked via a polypeptide linker can be used as a bispecific molecule. Alternatively, a triabody or the like comprising three or more scFv molecules may be used as a multispecific molecule.

Preferred examples of a scFv specific for HLA/NY-ESO (also referred to as "anti-HLA/NY-ESO scFv") include scFv comprising CDRH1 to CDRH3 and CDRL1 to CDRL3 described above. More preferred examples of a scFv include a scFV comprising the heavy chain and light chain variable regions described above. Further preferred examples of a scFV include NYA-0001 (amino acids 21 to 266 of the amino acid sequence represented by SEQ ID NO: 30)), NYA-0082 (which comprises the amino acid sequence represented by SEQ ID NO: 15 and the amino acid sequence represented by SEQ ID NO: 16), NYA-1143 (amino acids 21 to 266 of the amino acid sequence represented by SEQ ID NO: 20), NYA-1163 (amino acids 21 to 266 of the amino acid sequence represented by SEQ ID NO: 17), NYA-2023 (amino acids 21 to 266 of the amino acid sequence represented by SEQ ID NO: 18), NYA-2027 (amino acids 21 to 266 of the amino acid sequence represented by SEQ ID NO: 19), NYA-2035 (amino acids 21 to 266 of the amino acid sequence represented by SEQ ID NO: 22), NYA-2044 (amino acids 21 to 266 of the amino acid sequence represented by SEQ ID NO: 24), NYA-2045 (amino acids 21 to 266 of the amino acid sequence represented by SEQ ID NO: 25), NYA-2047 (amino acids 21 to 266 of the amino acid sequence represented by SEQ ID NO: 26), NYA-2048 (amino acids 21 to 266 of the amino acid sequence represented by SEQ ID NO: 27), NYA-2060 (amino acids 21 to 266 of the amino acid sequence represented by SEQ ID NO: 28), NYA-2061 (amino acids 21 to 266 of the amino acid sequence represented by SEQ ID NO: 29), and NYA-2143 (amino acids 21 to 266 of the amino acid sequence represented by SEQ ID NO: 21). Further examples include NYA-3061 (amino acids 21 to 271 of the amino acid sequence represented by SEQ ID NO: 55), and NYA-1154 (amino acids 21 to 266 of the amino acid sequence represented by SEQ ID NO: 71).

According to a preferred aspect, the anti-HLA/NY-ESO scFv is a scFV comprising a FLAG-His tag fused to the carboxy-terminus (simply referred to as a "tagged form"). Preferable examples of the tagged form include an NYA-0001 tagged form (amino acids 20 to 292 of SEQ ID NO: 30), an NYA-1143 tagged form (amino acids 20 to 292 of SEQ ID NO: 20), an NYA-1163 tagged form (amino acids 20 to 292 of SEQ ID NO: 17), an NYA-2023 tagged form (amino acids 20 to 292 of SEQ ID NO: 18), an NYA-2027 tagged form (amino acids 20 to 292 of SEQ ID NO: 19), an NYA-2035 tagged form (amino acids 20 to 292 of SEQ ID NO: 22), an NYA-2044 tagged form (amino acids 20 to 292 of SEQ ID NO: 24), an NYA-2045 tagged form (amino acids 20 to 292 of SEQ ID NO: 25), an NYA-2047 tagged form (amino acids 20 to 292 of SEQ ID NO: 26), an NYA-2048 tagged form (amino acids 20 to 292 of SEQ ID NO: 27), an NYA-2060 tagged form (amino acids 20 to 292 of SEQ ID NO: 28), an NYA-2061 tagged form (amino acids 20 to 292 of SEQ ID NO: 29), an NYA-2143 tagged form (amino acids 20 to 292 of SEQ ID NO: 21), an NYA-3061 tagged form (amino acid sequence prepared by adding amino acids 267 to 292 of SEQ ID NO: 22 to the carboxy-terminus of amino acids 21 to 271 of SEQ ID NO: 56), and an NYA-1154 tagged form (amino acids 21 to 292 of SEQ ID NO: 71).

Of these, NYA-2023 and its tagged form, NYA-2047 and its tagged form, NYA-2048 and its tagged form, NYA-2060 and its tagged form, and NYA-2061 and its tagged form have excellent biological activity, physical properties, and the like in an Fc-conjugated type bispecific molecule and are thus more preferred.

When anti-HLA/NY-ESO scFv and its tagged form are to be expressed in a host cell, , a signal peptide can be added to its amino terminus. Examples of the amino acid sequence of the signal peptide-conjugated anti-HLA/NY-ESO scFv tagged form can include amino acid sequences prepared by adding a FLAG-His tag (amino acids 267 to 292 of SEQ ID NO: 22) to the carboxy-termini of SEQ ID NOs: 30, 20, 17 to 19, 22, 24, 25, 26 to 29, and 21, and amino acids21 to 271 of SEQ ID NO: 56.

The scFv can be obtained by a phage display method (Nature Biotechnology (2005), 23, (9), p. 1105-1116) which involves allowing the variable regions of an antibody to be expressed as single chain antibody (scFv) on a phage surface and selecting a phage that binds to the antigen. The gene of the phage selected upon its binding to the antigen can be analysed, so that a DNA sequence encoding a human antigen variable region binding to the antigen can be determined. If the DNA sequence of the antigen-binding scFv is determined, an expression vector comprising such sequence may be prepared, introduced into an appropriate host cell, and expressed therein. Thus, a human antibody can be obtained(WO92/01047, WO92/20791, WO93/06213, WO93/11236, WO93/19172, WO95/01438, WO95/15388, Annu. Rev. Immunol (1994) 12, p.433-455, Nature Biotechnology (2005) 23 (9), p.1105-1116).

The antibody of the present invention may comprise a single heavy chain variable region and may not comprise a light chain sequence. Such an antibody, called a single domain antibody (sdAb) or a nanobody, has been reported to maintain the ability to bind to an antigen (Muyldemans S. et al., Protein Eng., (1994), 7 (9), 1129-35; and Hamers-Casterman C. et al., Nature (1993), 363 (6428), 446-448). Such antibodies are encompassed by the antigen-binding fragment of the antibody according to the present invention.

The present invention also includes a single chain immunoglobulin comprising full-length heavy and light chain sequences of the antibody linked via an appropriate linker (Lee, H-S, et al., Molecular Immunology (1999) 36, 61-71; and Shirrmann, T. et al., mAbs (2010), 2 (1), 1-4). Such a single chain immunoglobulin can be dimerized in order to retain the structure and activity similar to those of an antibody that is inherently atetramer. The anti-HLA/NY-ESO antibody of the present invention may a single chain immunoglobulin.

In the scFv of the present invention, the heavy chain and light chain variable regions may be connected to each other by a disulfide bond.

The anti-HLA/NY-ESO antibody of the present invention may be an antibody comprised of portions derived from a plurality of different antibodies, provided that the antibody binds to HLA/NY-ESO. Examples of such an antibody include an antibody arising from replacement of heavy and/or light chains , an antibody arising from replacement of full-length heavy and/or light chains, an antibody arising from replacement of variable or constant regions, and an antibody arising from replacement of all or some CDRs, from a plurality of different antibodies. The heavy chain and light chain variable regions of the chimeric antibody may be derived from different anti-HLA/NY-ESO antibodies of the present invention. In the heavy chain and light chain variable regions of a humanized antibody, heavy chain CDR1 to CDR3 and light chain CDR1 to CDR3 may be derived from two or more types of anti-HLA/NY-ESO antibodies of the present invention. In the heavy chain and light chain variable regions of a human antibody, a combination of heavy chain CDR1 to CDR3 and light chain CDR1 to CDR3 may be a derived from two or more types of the anti-HLA/NY-ESO antibodies of the present invention.

The anti-HLA/NY-ESO antibody of the present invention also encompasses an antibody that comprises an amino acid sequence encoded by a nucleotide sequence contained in a polynucleotide that hybridizes under stringent conditions to a complementary strand of a polynucleotide comprising a nucleotide sequence encoding an amino acid sequence contained in the anti-HLA/NY-ESO antibody of the present invention, and which binds to HLA/NY-ESO.

The anti-HLA/NY-ESO antibody of the present invention or the antigen-binding fragment thereof may be an antibody or an antigen-binding fragment thereof exhibiting 90%, 91%, 92%, 93%, 94%, 95%, 96%, 97%, 98%, or 99% or more identity to an amino acid sequence contained in the heavy chain variable region of the anti-HLA/NY-ESO antibody according to the present invention (preferably, an amino acid sequence corresponding to amino acid positions 21 to 140 of the amino acid sequence represented by SEQ ID NO: 18, the amino acid sequence represented by SEQ ID NO: 69 or 70, the amino acid sequence represented by SEQ ID NO: 39, or an amino acid sequence corresponding to amino acid positions 21 to 140 of the amino acid sequence represented by SEQ ID NO: 57, 60, or 61) and/or an amino acid sequence contained in the light chain variable region of the anti-HLA/NY-ESO antibody according to the present invention which is preferably an amino acid sequence corresponding to positions 156 to 266 of the amino acid sequence represented by SEQ ID NO: 18, an amino acid sequence corresponding to amino acid positions 156 to 266 of the amino acid sequence represented by SEQ ID NO: 28, the amino acid sequence represented by SEQ ID NO: 23, an amino acid sequence corresponding to positions 161 to 271 of the amino acid sequence represented by SEQ ID NO: 57, or an amino acid sequence corresponding to positions 156 to 266 of the amino acid sequence represented by SEQ ID NO: 60 or 61)or an antigen-binding fragment thereof.

When the position and length of a light chain variable region is determined in accordance with a scheme other than IMGT (e.g., Kabat, Chothia, AbM, and contact), one,two or more amino acids, for example, arginine or glycine, may further be included in the carboxyl terminus of the amino acid sequence of the light chain variable region determined in accordance with the scheme of IMGT. An antibody or a binding fragment thereof having such a light chain variable region is also encompassed by the antibody of the present invention or the binding fragment thereof.

The ability of the antibody, etc. of the present invention to bind to HLA/NY-ESO, particularly, human and/or cynomolgus monkey HLA/NY-ESO, may be optimized by introducing a mutation into a binding fragment of the anti-HLA/NY-ESO antibody of the present invention. Specific examples of methods for introducing a mutation include random mutagenesis using error-prone PCR, site-directed amino acid mutagenesis using an NNK library, site-directed mutagenesis using structure information, and any combination thereof.

### 3-2. Mutant of anti-HLA/NY-ESO antibody (mutant antibody)

A mutant antibody of the anti-HLA/NY-ESO antibody of the present invention preferably exhibits, for example, reduced sensitivity to protein degradation or oxidation, maintained or improved biological activity or function, suppressed reduction or change in such biological activity or function, improved or regulated antigen-binding ability, physicochemical properties or functional properties. Proteins are known to vary in their functions or activities upon alteration of a particular amino acid side chain present on its surface. Examples of such alterations include the deamidation of an asparagine side chain and the isomerization of an aspartic acid side chain. An antibody resulting from substitution of a particular amino acid with another amino acid in order to prevent such a change in amino acid side chain is also within the scope of the mutant antibody of the present invention.

An exampleof a mutant antibody of the present invention includes an antibody comprising an amino acid sequence derived from the amino acid sequence of the original antibody by conservative amino acid substitution. A conservative amino acid substitution occurs within an amino acid group in which amino acids are related by their side chains.

Preferred amino acid groups are as follows: an acidic group including aspartic acid and glutamic acid; a basic group including lysine, arginine, and histidine; a nonpolar group including alanine, valine, leucine, isoleucine, proline, phenylalanine, methionine, and tryptophan; and an uncharged polar family including glycine, asparagine, glutamine, cysteine, serine, threonine, and tyrosine. Other preferred amino acid groups are as follows: an aliphatic hydroxy group including serine and threonine; an amide-containing group including asparagine and glutamine; an aliphatic group including alanine, valine, leucine, and isoleucine; and an aromatic group including phenylalanine, tryptophan, and tyrosine. Such amino acid substitution in the mutant antibody is preferably performed without reducing the antigen-binding activity of the original antibody.

The anti-HLA/NY-ESO antibody of the present invention, the antigen-binding fragment thereof, the mutant (mutant antibody or antigen-binding fragment thereof) thereof, or the molecule of the present invention encompasses: a mutant antibody comprising an amino acid sequence derived from the amino acid sequence of the antibody of the present invention such as NYA-2023 by conservative amino acid substitution and/or any other mutation, and binds to HLA/NY-ESO, an antigen-binding fragment thereof, and a molecule comprising the same; a chimeric antibody, a humanized antibody, or a human antibody that comprises a CDR having an amino acid sequence which is derived from the amino acid sequence of any of CDRH1 to CDRH3 and CDRL1 to CDRL3 derived from the antibody of the present invention such as NYA-2023 by conservative amino acid substitution and/or other mutation, and binds to HLA/NY-ESO, an antigen-binding fragment thereof, or a molecule comprising the same.

### 3-3. Binding fragment of anti-HLA/NY-ESO antibody

According to one aspect, the present invention provides an antigen-binding fragment (hereinafter, simply referred to as a "binding fragment") of the anti-HLA/NY-ESO antibody of the present invention. The binding fragment of the anti-HLA/NY-ESO antibody of the present invention encompasses binding fragments of chimeric antibodies, humanized antibodies, or human antibodies. The binding fragment of the antibody means a fragment that retains at least antigen-binding activity of the functions of the antibody, or a modified form thereof. Examples of such functions of the antibody include, in general, antigen- binding activity, the activity of regulating antigen activity (e.g., agonistic activity), the activity of internalizing an antigen into a cell, and the activity of inhibiting or promoting the interaction between an antigen and a substance interacting therewith.

The binding fragment of the antibody is not particularly limited provided that it retains at least antigen-binding activity of the activity of the antibody. Examples of such a binding fragment of the antibody include, but are not limited to, Fab, Fab', F(ab')₂, single chain Fab (scFab) comprising the carboxyl terminus of a Fab light chain and the amino terminus of a Fab heavy chain linked via an appropriate linker, Fv, single chain Fv (scFv) comprising heavy and light chain Fv molecules linked via an appropriate linker, and single domain antibody (sdab; also called nanobody) having a single heavy chain variable region and lacking a light chain sequence (Muyldemans S. et. al., Protein Eng., (1994) 7 (9), 1129-35, Hamers-Casterman C. et. al., Nature (1993) 363 (6428), 446-448). A molecule comprising regions other than the binding fragment of the antibody of the present invention, such as scFab and scFv comprising a linker moiety, is within the scope of the binding fragment of the present invention.

### 3-4. Modified anti-HLA/NY-ESO antibody, binding fragment thereof, and conjugate thereof

The present invention provides a modified antibody or binding fragment thereof. The modified antibody of the present invention or binding fragment thereof has been subjected to chemical or biological modification. Examples of chemical modification include, for example, conjugating a chemical moiety to an amino acid backbone, and chemically modifying an N-linked or O-linked carbohydrate chain. Examples of biological modification include post-translational modification (e.g., N-linked or O-linked glycosylation, processing of an amino-terminal or carboxyl-terminal region, deamidation, isomerization of aspartic acid, or oxidation of methionine), and adding a methionine residue to the amino terminus via expression in a prokaryotic host cell. A modified antibody of the invention or binding fragment thereof also includes a labeled antibody or binding fragment thereof. The label permits detection or isolation of the antibody or the antigen-binding fragment. Examples of labels which may be used include, an enzyme label, a fluorescent labeled form, or an affinity-label . A modification of the antibody of the present invention or binding fragment thereof as described above is useful for improving the stability or blood retention of the original antibody of the present invention or the original binding fragment thereof, reducing antigenicity, or detection or isolation of the antibody or the antigen, and other purposes.

Examples of the chemical moiety contained in a chemical modification include water-soluble polymers such as polyethylene glycol(PEG), ethylene glycol/propylene glycol copolymers, carboxymethylcellulose, dextran, and polyvinyl alcohol.

Examples of biological modification include a modification by enzyme treatment, cell treatment, or the like; conjugation with another peptide such as a tag, via genetic recombination, and a product prepared by using host cells expressing an endogenous or exogenous sugar chain-modifying enzyme.

Such a modification may be made at an arbitrary position or a desired position in the antibody or the binding fragment thereof. Alternatively, the same or two or more different modifications may be made at one or two or more positions therein.

However, the deletion from heavy chain sequences or the modification in the heavy or light chain sequences, rarely affect the ability of the antibody to bind to the antigen and its effector functions (complement activation, antibody dependent cytotoxic effects, etc.).

Thus, the present invention also encompasses an antibody that has been subjected to such deletion or modification. Examples include a deletion variant derived by the deletion of 1 or 2 amino acids from the heavy chain carboxyl terminus (Journal of Chromatography A; 705; 129-134 (1995)), an amidated form of the deletion variant having a heavy chain that lacks two amino acid residues (glycine and lysine) at the carboxyl terminus and further, comprises an amidated proline residue at the carboxyl terminus (Analytical Biochemistry, 360: 75-83

(2007)), and a modified antibody having a pyroglutamylated amino-terminal glutamine or glutamic acid residue in the heavy or light chain (International Publication No. WO 2013/147153) (they are collectively referred to as "deletion mutants"). However, as long as the antigen-binding ability and effector functions are retained, the deletion mutants comprising deletion of amino acid residues at the carboxyl terminus of the antibody heavy or light chain according to the present invention are not limited to the types described above. When the antibody according to the present invention comprises two or more chains (e.g., heavy chains), the two or more chains (e.g., heavy chains) may be heavy chains of any one type selected from the group consisting of the full-length heavy chain and the deletion mutants described above, or may be a combination of heavy chains of any two types selected therefrom. The quantitative ratio of each deletion mutant or the ratio of the number of molecules of each deletion mutant may be influenced by the type and culture conditions of cultured mammalian cells producing the antibody according to the present invention. In some examples, deletion of one carboxyl-terminal amino acid residue in each of the two heavy chains as main components may be a principal feature of the antibody according to the present invention.

The antibody of the present invention or the antigen-binding fragment thereof (e.g., contained in the multispecific antibody of the present invention), when comprising one to several amino acids which have been added to the amino terminus and/or the carboxyl terminus which are derived from, for example, an expression vector and/or a signal sequence, (and is partially or entirely modified as described above), is included in the scope of the modified antibody of the present invention or the modified antigen-binding fragment thereof as long as the desired antigen- binding activity is maintained. A molecule comprising such a modified antibody or antigen-binding fragment is also within the scope of the multispecific antibody of the present invention.

In the present invention, the "antibody or antigen-binding fragment thereof" also encompasses the "modified antibody or antigen-binding fragment thereof". The "antibody or antigen-binding fragment thereof" contained in the multispecific antibody of the present invention encompasses the "modified e antibody or antigen-binding fragment thereof".

The antibody dependent cellular cytotoxic activity of the antibody of the present invention may be enhanced by regulating the modification (glycosylation, defucosylation, etc.) of a sugar chain bound to the antibody. For example, International Publication Nos. WO 99/54342, WO 00/61739, and WO 02/31140 disclose techniques for regulating sugar chain modification of antibodies, although the techniques are not limited thereto.

The present invention also encompasses conjugates formed by linking the aforementioned antibodies to other molecules via linkers (immunoconjugates). As an example, an antibody which is conjugated to a radioactive material or a compound (drug) having pharmacological activity is an ADC (antibody-drug conjugate) (Methods Mol Biol. (2013) 1045: 1-27; Nature Biotechnology (2005) 23, p. 1137-1146).

In addition, the present invention also encompasses conjugates comprising antibodies connected to other functional polypeptides. An examples of such an antibody-peptide complex includes a complex of the antibody and an albumin binding polypeptide (Protein Eng Des Sel. (2012) (2): 81-8).

A modified antibody, an antibody that has been subjected to regulated sugar chain modification, and conjugates as described above are encompassed by the antibody of the present invention. A binding fragment of the modified antibody, an antibody that has been subjected to regulated sugar chain modification, and conjugates as described above are encompassed by the binding fragment of the antibody of the present invention.

### 4. Method for producing antibody

The antibody of the present invention can be produced in a cell as a recombinant antibody, for example, by inserting DNA encoding a heavy chain variable region or DNA encoding a light chain variable region into an expression vector, transforming a host cell for expression with the vectors, and culturing the host cell.

With regard to the DNA encoding the antibody, DNA encoding a heavy chain is obtained by ligating DNA encoding a heavy chain variable region to DNA encoding a heavy chain constant region, and DNA encoding a light chain is obtained by ligating DNA encoding a light chain variable region to DNA encoding a light chain constant region.

The anti-HLA/NY-ESO antibody of the present invention can be produced by inserting the DNA encoding a heavy chain and the DNA encoding a light chain into an expression vector, transfecting a host cell with the vector, and culturing the host cell. In this case, the DNA encoding the heavy chain and the DNA encoding the light chain may be introduced into the same expression vector, and the host cell can be transfected with the vector. Alternatively, the DNA encoding the heavy chain and the DNA encoding the light chain may be inserted into separate vectors, and a host cell can be transfected with both vectors. In this respect, DNA encoding a heavy chain variable region and DNA encoding a light chain variable region may be inserted into a vector into which DNA encoding a heavy chain constant region and DNA encoding a light chain constant region have been introduced beforehand. The vector may contain DNA encoding a signal peptide which promotes secretion of the antibody from the host cell. In this case, the DNA encoding a signal peptide is ligated in-frame to the DNA encoding the antibody. After production of the antibody, the signal peptide can be removed to obtain the antibody as a mature protein.

The DNA encoding a heavy chain variable region, the DNA encoding a light chain variable region, DNA obtained by ligating the DNA encoding a heavy chain variable region to DNA encoding a heavy chain constant region, or DNA obtained by ligating the DNA encoding a light chain variable region to DNA encoding a light chain constant region may be operably ligated to an element such as a promoter, an enhancer, and a polyadenylation signal. In this context, the term "operably ligated" means that the element is connected to the DNA so as to exert its functions.

The expression vector is not particularly limited as long as it is capable of replicating in a host such as an animal cell, a bacterium, or a yeast cell. Examples of vectors include plasmids and phages that are known in the art. Examples of a vector used to construct the expression vector include pcDNA(TM) (Thermo Fisher Scientific Inc.), Flexi(R) vector (Promega Corp.), pUC19, pUEX2 (manufactured by Amersham plc), pGEX-4T, pKK233-2 (manufactured by Pharmacia), and pMAM-neo (manufactured by Clontech Laboratories, Inc.). Both prokaryotic cells (e.g., *E. coli* and *Bacillus subtilis*) and eukaryotic cells (e.g., yeasts and animal cells) can be used as the host cells, and eukaryotic cells are preferably used. For example, the human embryonic kidney cell line HEK293 cells or Chinese hamster ovary (CHO) cells can be used as the animal cells. The expression vector may be introduced into a host cell by a method known in the art. Examples of such a method include electroporation, a calcium phosphate precipitation method, and DEAE-dextran transfection. The antibody, once produced, can be purified by using standard protein separation and purification methods. For example, affinity chromatography, other chromatography techniques, filtration, ultrafiltration, salting-out, and dialysis can be appropriately selected and combined.

### 5. Multispecific antibody that binds to HLA/NY-ESO

The multispecific antibody of the present invention comprises the anti-HLA/NY-ESO antibody of the present invention or the antigen-binding fragment thereof. The multispecific antibody of the present invention is preferably a multispecific antibody having two or more antigen-binding sites. Specifically, the multispecific antibody is capable of binding to two or more different epitopes on one molecule, or different epitopes on two or more different molecules, and comprises a plurality of different antigen-binding fragments different. Such a multispecific antibody includes, but is not limited to, IgG type multispecific antibodies, multispecific antibodies having two or more types of variable regions, for example, tandem scFv (taFv), single chain diabody, antibody fragments such as diabody and triabody, and antibody fragments connected by a covalent or noncovalent bond. The multispecific antibody may comprise Fc.

The multispecific antibody of the present invention can comprise, in addition to the anti-HLA/NY-ESO antibody of the present invention or the antigen-binding fragment thereof, one,two or more additional antibodies or antigen-binding fragments of the antibodies. Examples of the antigen-binding fragments of the additional antibodies include Fab, F(ab)', Fv, scFv, and sdAb.

Preferably, the multispecific antibody of the present invention further comprises an anti-CD3 antibody or an antigen-binding fragment thereof and also specifically binds to CD3.

The anti-CD3 antibody or the antigen-binding fragment thereof contained in the multispecific antibody of the present invention is not particularly limited as long as it is an antibody that binds to human CD3, or a binding fragment thereof. Preferably, the anti-CD3 antibody or the antigen-binding fragment thereof also binds to CD3 of a non-human primate such as a cynomolgus monkey. A more preferred example of an anti-CD3 antibody or an antigen-binding fragment thereof comprises a heavy chain variable region CDRH1 consisting of the amino acid sequence represented by SEQ ID NO: 7, heavy chain variable region CDRH2 consisting of the amino acid sequence represented by SEQ ID NO: 8, heavy chain variable region CDRH3 consisting of the amino acid sequence represented by SEQ ID NO: 9, alight chain variable region CDRL1 consisting of the amino acid sequence represented by SEQ ID NO: 10, a light chain variable region CDRL2 consisting of the amino acid sequence represented by RDD (the 5th sequence from the top in Figure 12; SEQ ID NO: 11 in Figure 82), and a light chain variable region CDRL3 consisting of the amino acid sequence represented by SEQ ID NO: 12.

More preferred examples of the antibody or the antigen-binding fragment thereof comprising CDRH1 to CDRH3 and CDRL1 to CDRL3 described above include an antibody or an antigen-binding fragment thereof comprising a C3E-7034 heavy chain variable region consisting of an amino acid sequence corresponding to amino acid positions 2 to 119 of the amino acid sequence represented by SEQ ID NO: 46, a C3E-7036 heavy chain variable region consisting of an amino acid sequence corresponding to amino acid positions 2 to 119 of the amino acid sequence represented by SEQ ID NO: 47, a C3E-7085 heavy chain variable region consisting of an amino acid sequence corresponding to amino acid positions 2 to 119 of the amino acid sequence represented by SEQ ID NO: 48, a C3E-7088 heavy chain variable region consisting of an amino acid sequence corresponding to amino acid positions 2 to 119 of the amino acid sequence represented by SEQ ID NO: 49, a C3E-7093 heavy chain variable region consisting of an amino acid sequence corresponding to amino acid positions 2 to 119 of the amino acid sequence represented by SEQ ID NO: 140, a C3E-7096 heavy chain variable region consisting of an amino acid sequence corresponding to amino acid positions 272 to 389 of the amino acid sequence represented by SEQ ID NO: 54, a C3E-7096 heavy chain variable region consisting of an amino acid sequence corresponding to amino acid positions 277 to 394 of the amino acid sequence represented by SEQ ID NO: 55, or a C3E-7097 heavy chain variable region consisting of an amino acid sequence corresponding to amino acid positions 277 to 394 of the amino acid sequence represented by SEQ ID NO: 56.

Further preferred examples of the antibody or the antigen-binding fragment thereof comprising CDRH1 to CDRH3 and CDRL1 to CDRL3 described above include an antibody or an antigen-binding fragment thereof comprising a C3E-7034 light chain variable region consisting of an amino acid sequence corresponding to amino acid positions 135 to 243 of the amino acid sequence represented by SEQ ID NO: 46, a C3E-7036 light chain variable region consisting of an amino acid sequence corresponding to amino acid positions 135 to 241 of the amino acid sequence represented by SEQ ID NO: 47, a C3E-7085 light chain variable region consisting of an amino acid sequence corresponding to amino acid positions 135 to 241 of the amino acid sequence represented by SEQ ID NO: 48, a C3E-7088 light chain variable region consisting of an amino acid sequence corresponding to amino acid positions 135 to 243 of the amino acid sequence represented by SEQ ID NO: 49, a C3E-7093 light chain variable region consisting of an amino acid sequence corresponding to amino acid positions 135 to 243 of the amino acid sequence represented by SEQ ID NO: 50, a C3E-7096 light chain variable region consisting of an amino acid sequence corresponding to amino acid positions 405 to 511 of the amino acid sequence represented by SEQ ID NO: 54, a C3E-7096 light chain variable region consisting of an amino acid sequence corresponding to amino acid positions 410 to 516 of the amino acid sequence represented by SEQ ID NO: 55, or a C3E-7097 light chain variable region consisting of an amino acid sequence corresponding to amino acid positions 410 to 516 of the amino acid sequence represented by SEQ ID NO: 56.

Furhter preferred examples of the antibody or the antigen-binding fragment thereof comprising CDRH1 to CDRH3 and CDRL1 to CDRL3 described above can include an antibody or an antigen-binding fragment thereof comprising a combination of C3E-7034 heavy chain and light chain variable regions consisting of amino acids 2 to 119 and 135 to 243 of SEQ ID NO: 46, a combination of C3E-7036 heavy chain and light chain variable regions consisting of amino acids2 to 119 and 135 to 241 of SEQ ID NO: 47, a combination of C3E-7038 heavy chain and light chain variable regions consisting of amino acids 2 to 119 and 135 to 243 of SEQ ID NO: 51, a combination of C3E-7085 heavy chain and light chain variable regions consisting of amino acids 2 to 119 and 135 to 241 of SEQ ID NO: 48, a combination of C3E-7088 heavy chain and light chain variable regions consisting of amino acids 2 to 119 and 135 to 243 of SEQ ID NO: 49, a combination of C3E-7093 heavy chain and light chain variable regions consisting of amino acids 2 to 119 and 135 to 243 of SEQ ID NO: 50, a combination of C3E-7096 heavy chain and light chain variable regions consisting of amino acids 272 to 389 and 405 to 511 of SEQ ID NO: 54, a combination of C3E-7096 heavy chain and light chain variable regions consisting of amino acids 277 to 394 and 410 to 516 of SEQ ID NO: 55, or a combination of C3E-7097 heavy chain and light chain variable regions consisting of amino acids 277 to 394 and 410 to 516 of SEQ ID NO: 56.

Further preferred examples of the antibody or the antigen-binding fragment thereof comprising CDRH1 to CDRH3 and CDRL1 to CDRL3 described above include C3E-7034 scFv consisting of an amino acid sequence corresponding to amino acid positions 2 to 243 of the amino acid sequence represented by SEQ ID NO: 46, C3E-7036 scFv consisting of an amino acid sequence corresponding to amino acid positions 2 to 241 of the amino acid sequence represented by SEQ ID NO: 47, C3E-7078 scFv consisting of an amino acid sequence corresponding to amino acid positions 2 to 243 of the amino acid sequence represented by SEQ ID NO: 51, C3E-7085 scFv consisting of an amino acid sequence corresponding to amino acid positions 2 to 241 of the amino acid sequence represented by SEQ ID NO: 48, C3E-7088 scFv consisting of an amino acid sequence corresponding to amino acid positions 2 to 243 of the amino acid sequence represented by SEQ ID NO: 49, C3E-7093 scFv consisting of an amino acid sequence corresponding to amino acid positions 2 to 243 of the amino acid sequence represented by SEQ ID NO: 50, and an antibody comprising any one of these scFv molecules, and a binding fragment of the antibody. In a preferred aspect, scFv specific for CD3 (also referred to as "anti-CD3 scFv") includes a FLAG-His tag provided at the carboxyl-terminus (also referred to as a "tagged form"). Preferred examples of the tagged form include C3E-7034 (SEQ ID NO: 46), C3E-7036 (SEQ ID NO: 47), C3E-7085 (SEQ ID NO: 48), C3E-7088 (SEQ ID NO: 49) and C3E-7093 (SEQ ID NO: 50), and more preferably, C3E-7085.

Preferred examples of the multispecific molecule of the present invention include bispecific molecules. The term "bispecific" means being capable of binding to two different epitopes on a single molecule, or different epitopes on two or more molecules. A bispecific antibody or an antigen-binding fragment having is encompassed by the present invention. The bispecific molecule of the present invention binds to HLA/NY-ESO and further binds to CD3.

Examples of the bispecific molecule of the present invention include those having the following structures (formats).

In a dual scFv type bispecific molecule, two scFv molecules that bind to different respective epitopes are each linked to a chain of dimeric Fc, either via a linker or directly without a linker. Alternatively, two types of scFv molecules that bind to different respective epitopes are linked to CH and CL, respectively, via a linker, and are each further linked to a chain of dimeric Fc, via a linker. In this bispecific molecule, each Fc linked to the scFv molecules is mutated to enable hetero-association with the other Fc and formation of a heterodimer. The dual scFv type bispecific molecule is referred to as a dual type bispecific molecule or simply dual type (Figure 7(7b)).

In the present invention, for example, a dual type bispecific molecule consisting of anti-HLA-A2/NY-ESO scFv and anti-CD3 scFv can be used.

Alternatively, the bispecific molecule of the present invention may be a bispecific molecule formed by Fab and scFv that bind to different respective epitopes such that the Fab of a first antibody and the scFv of a second antibody are each linked to a chain of dimeric Fc, respectively, via a linker. In this bispecific molecule, eachFc as linked to Fab or scFv is mutated to enable hetero-association with the other Fc and formation of a heterodimer. Such a bispecific molecule is referred to as a hybrid type bispecific molecule or hybrid type (Figure 7 (7a)). In the present invention, for example, hybrid type consisting of anti-HLA-A2/NY-ESO Fab and anti-CD3 scFv can be used.

In the bispecific molecule, the Fab of the first antibody and the scFv of the second antibody may be linked to one chain of dimeric Fc via a linker. In this embodiment, the Fab may be connected to the Fc, and the scFv may be connected to the Fab. Alternatively, the scFv may be connected to the Fc, and the Fab may be connected to the scFv. Preferably, the Fab is connected to the Fc, and the scFv is connected to the Fab. The association between the Fab and the scFv can be achieved via a linker which enables the scFv to be connected to a variable region of the Fab. In this bispecific molecule, each chain of dimeric Fc is mutated to form a heterodimer which is connected to scFv and Fab by means of a linker. Such a bispecific molecule is referred to as an scFv-Fab-heterodimer Fc type bispecific molecule or scFv-Fab-heterodimer Fc type (Figure 7(7c)).

In the present invention, for example, scFv-Fab-heterodimer Fc type consisting of anti-CD3 scFv and anti-HLA-A2/NY-ESO Fab can be used.

taFv (Figure 5(5c)) comprising two types of scFv molecules of first and second antibodies, respectively, which are connected via a linker may also be connected to one chain of dimeric Fc either via a linker or directly without any linker. Such a bispecific molecule is referred to as a taFv-heterodimer Fc type bispecific molecule or taFv-heterodimer Fc type (Figure 5(5d)). In this bispecific molecule, each Fc is mutated to enable hetero-association and formation of a Fc heterodimer.

The order in which the first and second antibodies are connected in the taFv is not limited. When the order of connection of the first and second antibodies in the taFv is reversed with respect to a set order of first and second antibodies (referred to as taFv-heterodimer Fc type), the bispecific molecule is referred to as taFv (inverted)-heterodimer Fc type (or referred to as taFv (inverted)-Fc type).

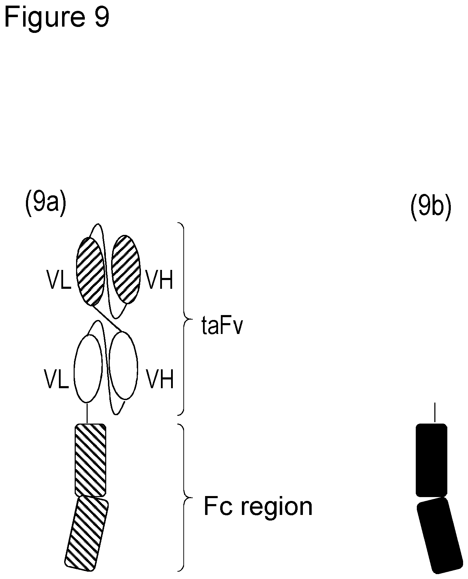

Figure 7(7a) shows the structure of a hybrid type bispecific molecule, Figure 7(7b) shows the structure of a dual type bispecific antibody, and Figure 7(7c) shows the structure of a scFv-Fab-heterodimer Fc type bispecific antibody. Figure 5(5a) shows the structure of scFv, Figure 5(5b) shows the structure of Fab, Figure 5(5c) shows the structure of taFv, Figure 5(5d) shows the structure of a taFv-heterodimer Fc type bispecific antibody, and Figure 5(5e) shows the structure of a taFv-Fab-heterodimer Fc type bispecific antibody. Figure 8(8a) shows the structure of a taFv-heterodimer Fc type bispecific antibody (which is the same as in Figure 5(5d)), Figure 8(8b) shows the structure of a taFv (inverted)-heterodimer Fc type bispecific antibody, Figure 9(9a) shows the structure of the first polypeptide contained in the taFv (inverted)-heterodimer Fc type bispecific antibody, and Figure 9(9b) shows the structure of the second polypeptide contained in the taFv (inverted)-heterodimer Fc type bispecific antibody.

In the bispecific antibody of the present invention, a plurality of polypeptides are associated with each other.

In the present invention, for example, anti-HLA-A2/NY-ESO scFv and anti-CD3 scFv can be used as a taFv. Preferably, the taFv-heterodimer Fc type bispecific antibody comprises: (a) a first polypeptide comprising scFv that specifically binds to HLA/NY-ESO, scFv that specifically binds to CD3, and an immunoglobulin Fc region (i) in that order from the N-terminus towards the C-terminus; and a second polypeptide comprising an immunoglobulin hinge region and Fc region (ii). More preferably, (b) the first polypeptide and the second polypeptide are associated with each other via the Fc region (i) and the Fc region (ii). The Fc regions of the first polypeptide and the second polypeptide may comprise a mutation for forming a heterodimer. An example of a taFv-heterodimer Fc type bispecific antibody is shown in Figure 5(5d). As shown in Figure 5(5d), the Fc region (i) of the first polypeptide is connected to the Fc region (ii) of the second polypeptide (solid), and thus,the first polypeptide is associated with the second polypeptide. Figure 6(6a) shows the first polypeptide, and Figure 6(6b) shows the second polypeptide. For example, in Figure 5(5d), the first scFv (unfilled) is anti-HLA-A2/NY-ESO scFv, and the second scFv (positively hatched) is anti-CD3 scFv.

The first polypeptide contained in a more preferable taFv-heterodimer Fc type bispecific antibody of the present invention comprises an amino acid sequence corresponding to positions 21 to 511 of the amino acid sequence represented by SEQ ID NO: 32, an amino acid sequence corresponding to positions 21 to 511 of the amino acid sequence represented by SEQ ID NO: 34, an amino acid sequence corresponding to positions 21 to 511 of the amino acid sequence represented by SEQ ID NO: 35, an amino acid sequence corresponding to positions 21 to 511 of the amino acid sequence represented by SEQ ID NO: 36, an amino acid sequence corresponding to positions 21 to 511 of the amino acid sequence represented by SEQ ID NO: 37, an amino acid sequence corresponding to positions 21 to 511 of the amino acid sequence represented by SEQ ID NO: 38, an amino acid sequence corresponding to positions 21 to 511 of the amino acid sequence represented by SEQ ID NO: 39, an amino acid sequence corresponding to positions 21 to 511 of the amino acid sequence represented by SEQ ID NO: 40, an amino acid sequence corresponding to positions 21 to 511 of the amino acid sequence represented by SEQ ID NO: 41, an amino acid sequence corresponding to positions 21 to 511 of the amino acid sequence represented by SEQ ID NO: 42, an amino acid sequence corresponding to positions 21 to 511 of the amino acid sequence represented by SEQ ID NO: 43, an amino acid sequence corresponding to positions 21 to 511 of the amino acid sequence represented by SEQ ID NO: 33, an amino acid sequence corresponding to positions 21 to 511 of the amino acid sequence represented by SEQ ID NO: 52, an amino acid sequence corresponding to positions 21 to 511 of the amino acid sequence represented by SEQ ID NO: 53, an amino acid sequence corresponding to positions 20 to 511 of the amino acid sequence represented by SEQ ID NO: 54, an amino acid sequence corresponding to positions 20 to 516 of the amino acid sequence represented by SEQ ID NO: 55, or an amino acid sequence corresponding to positions 20 to 516 of the amino acid sequence represented by SEQ ID NO: 56. The first polypeptide contained in further preferred taFv-heterodimer Fc type bispecific molecule comprises an amino acid sequence corresponding to positions 529 to 745 of the amino acid sequence represented by SEQ ID NO: 32, 34, 35, 36, 37, 38, 39, 40, 41, 42, 43, 32, 52, 53, or 54, or an amino acid sequence corresponding to positions 534 to 750 of the amino acid sequence represented by SEQ ID NO: 55 or 56. The first polypeptide contained in further preferred taFv-heterodimer Fc type bispecific molecule consists of an amino acid sequence corresponding to positions 21 to 745 of the amino acid sequence represented by SEQ ID NO: 32, an amino acid sequence corresponding to positions 21 to 745 of the amino acid sequence represented by SEQ ID NO: 34, an amino acid sequence corresponding to positions 21 to 745 of the amino acid sequence represented by SEQ ID NO: 35, an amino acid sequence corresponding to positions 21 to 745 of the amino acid sequence represented by SEQ ID NO: 36, an amino acid sequence corresponding to positions 21 to 745 of the amino acid sequence represented by SEQ ID NO: 37, an amino acid sequence corresponding to positions 21 to 745 of the amino acid sequence represented by SEQ ID NO: 38, an amino acid sequence corresponding to positions 21 to 745 of the amino acid sequence represented by SEQ ID NO: 39, an amino acid sequence corresponding to positions 21 to 745 of the amino acid sequence represented by SEQ ID NO: 40, an amino acid sequence corresponding to positions 21 to 745 of the amino acid sequence represented by SEQ ID NO: 41, an amino acid sequence corresponding to positions 21 to 745 of the amino acid sequence represented by SEQ ID NO: 42, an amino acid sequence corresponding to positions 21 to 745 of the amino acid sequence represented by SEQ ID NO: 43, an amino acid sequence corresponding to positions 21 to 745 of the amino acid sequence represented by SEQ ID NO: 33, an amino acid sequence corresponding to positions 21 to 745 of the amino acid sequence represented by SEQ ID NO: 52, an amino acid sequence corresponding to positions 21 to 745 of the amino acid sequence represented by SEQ ID NO: 53, or an amino acid sequence corresponding to positions 20 to 745 of the amino acid sequence represented by SEQ ID NO: 54. Alternatively, the first polypeptide consists of an amino acid sequence corresponding to positions 20 to 750 of the amino acid sequence represented by SEQ ID NO: 55 or an amino acid sequence corresponding to positions 20 to 750 of the amino acid sequence represented by SEQ ID NO: 56.

The second polypeptide contained in a preferred taFv-heterodimer Fc type bispecific molecule of the present invention comprises a human antibody-derived hinge region and mutant Fc. The second polypeptide contained in further preferred taFv-heterodimer Fc type bispecific molecule comprises an amino acid sequence corresponding to positions 20 to 246 of the amino acid sequence represented by SEQ ID NO: 31.

Of these, preferred examples of the taFv-heterodimer Fc type bispecific antibody of the present invention can include NYF-0016 in which the first polypeptide consisting of an amino acid sequence corresponding to positions 21 to 745 of the amino acid sequence represented by SEQ ID NO: 32 and the second polypeptide consisting of an amino acid sequence corresponding to positions 21 to 246 of the amino acid sequence represented by SEQ ID NO: 31 are associated with each other, NYF-0022 in which the first polypeptide consisting of an amino acid sequence corresponding to positions 21 to 745 of the amino acid sequence represented by SEQ ID NO: 84 and the second polypeptide consisting of an amino acid sequence corresponding to positions 21 to 246 of the amino acid sequence represented by SEQ ID NO: 31 are associated with each other, NYF-0023 in which the first polypeptide consisting of an amino acid sequence corresponding to positions 21 to 745 of the amino acid sequence represented by SEQ ID NO: 35 and the second polypeptide consisting of an amino acid sequence corresponding to positions 21 to 246 of the amino acid sequence represented by SEQ ID NO: 31 are associated with each other, NYF-0027 in which the first polypeptide consisting of an amino acid sequence corresponding t positions 21 to 745 of the amino acid sequence represented by SEQ ID NO: 36 and the second polypeptide consisting of an amino acid sequence corresponding to positions 21 to 246 of the amino acid sequence represented by SEQ ID NO: 31 are associated with each other, NYF-0035 in which the first polypeptide consisting of an amino acid sequence corresponding to positions 21 to 745 of the amino acid sequence represented by SEQ ID NO: 37 and the second polypeptide consisting of an amino acid sequence corresponding to positions 21 to 246 of the amino acid sequence represented by SEQ ID NO: 31 are associated with each other, NYF-0044 in which the first polypeptide consisting of an amino acid sequence corresponding to positions 21 to 745 of the amino acid sequence represented by SEQ ID NO: 38 and the second polypeptide consisting of an amino acid sequence corresponding to positions 21 to 246 of the amino acid sequence represented by SEQ ID NO: 31 are associated with each other, NYF-0045 in which the first polypeptide consisting of an amino acid sequence corresponding to positions 21 to 745 of the amino acid sequence represented by SEQ ID NO: 39 and the second polypeptide consisting of an amino acid sequence corresponding to positions 21 to 246 of the amino acid sequence represented by SEQ ID NO: 31 are associated with each other, NYF-0047 in which the first polypeptide consisting of an amino acid sequence corresponding to positions 21 to 745 of the amino acid sequence represented by SEQ ID NO: 40 and the second polypeptide consisting of an amino acid sequence corresponding to positions 21 to 246 of the amino acid sequence represented by SEQ ID NO: 31 are associated with each other, NYF-0048 in which the first polypeptide consisting of an amino acid sequence corresponding to positions 21 to 745 of the amino acid sequence represented by SEQ ID NO: 41 and the second polypeptide consisting of an amino acid sequence corresponding to positions 21 to 246 of the amino acid sequence represented by SEQ ID NO: 31 are associated with each other, NYF-0060 in which the first polypeptide consisting of an amino acid sequence corresponding to positions 21 to 745 of the amino acid sequence represented by SEQ ID NO: 42 and the second polypeptide consisting of an amino acid sequence corresponding to positions 21 to 246 of the amino acid sequence represented by SEQ ID NO: 31 are associated with each other, NYF-0061 in which the first polypeptide consisting of an amino acid sequence corresponding to positions 21 to 745 of the amino acid sequence represented by SEQ ID NO: 43 and the second polypeptide consisting of an amino acid sequence corresponding to positions 21 to 246 of the amino acid sequence represented by SEQ ID NO: 31 are associated with each other, NYF-0019 in which the first polypeptide consisting of an amino acid sequence corresponding to positions 21 to 745 of the amino acid sequence represented by SEQ ID NO: 33 and the second polypeptide consisting of an amino acid sequence corresponding to positions 21 to 246 of the amino acid sequence represented by SEQ ID NO: 31 are associated with each other, NYF-0014 in which the first polypeptide consisting of an amino acid sequence corresponding to positions 21 to 745 of the amino acid sequence represented by SEQ ID NO: 52 and the second polypeptide consisting of an amino acid sequence corresponding to positions 21 to 246 of the amino acid sequence represented by SEQ ID NO: 31 are associated with each other, and NYF-0082 in which the first polypeptide consisting of an amino acid sequence corresponding to positions 21 to 745 of the amino acid sequence represented by SEQ ID **NO:** 53 and the second polypeptide consisting of an amino acid sequence corresponding to positions 21 to 246 of the amino acid sequence represented by SEQ ID **NO:** 31 are associated with each other. Preferred examples of the taFv-heterodimer Fc type bispecific antibody of the present invention further include NYZ-0038 in which the first polypeptide consisting of an amino acid sequence corresponding to positions 20 to 745 of the amino acid sequence represented by SEQ ID NO: 54 and the second polypeptide consisting of an amino acid sequence corresponding to positions 20 to 246 of the amino acid sequence represented by SEQ ID NO: 31 are associated with each other, NYZ-0082 in which the first polypeptide consisting of an amino acid sequence corresponding to positions 20 to 750 of the amino acid sequence represented by SEQ ID NO: 55 and the second polypeptide consisting of an amino acid sequence corresponding to positions 20 to 246 of the amino acid sequence represented by SEQ ID NO: 31 are associated with each other, and NYZ-0083 in which the first polypeptide consisting of an amino acid sequence corresponding to positions 20 to 750 of the amino acid sequence represented by SEQ ID NO: 56 and the second polypeptide consisting of an amino acid sequence corresponding to positions 20 to 246 of the amino acid sequence represented by SEQ ID NO: 31 are associated with each other.

Of these, NYF-0023, NYF-0047, NYF-0048, NYF-0060, NYF-0061, NYZ-0038, NYZ-0082, and NYZ-0083, in particular, have excellent biological activities, physical properties, and the like and are thus preferred.

taFv of a first antibody may be connected to one chain of a dimeric Fc either via a linker or directly without a linker, and Fab of the first antibody or the second antibody may be connected to the other chain of the Fc dimer either via a linker or directly without a linker. In this bispecific molecule, and referring to Figure 5 (5e), Fab is provided N-terminal to the Fc region (ii) (solid) of the second polypeptide in the taFv-heterodimer Fc type. Such a bispecific molecule is referred to as a taFv-Fab-heterodimer Fc type bispecific molecule or taFv-Fab-heterodimer Fc type (Figure 5(5e)).

In the present invention, for example, taFv comprising anti-HLA-A2/NY-ESO scFv and anti-CD3 scFv, and HLA/NY-ESO Fab, can be included in the taFv-Fab-heterodimer Fc type bispecific molecule.

The taFv-Fab-heterodimer Fc type bispecific antibody preferably comprises (a): a first polypeptide comprising scFv that specifically binds to human HLA/NY-ESO, scFv that specifically binds to CD3, and an immunoglobulin Fc region (i) in that order from the N-terminus towards the C-terminus; a second polypeptide consisting of an immunoglobulin heavy chain comprising an Fc region (ii); and a third polypeptide consisting of an immunoglobulin light chain. More preferably, (b) the second polypeptide and the third polypeptide are associated with each other, and (c) the first polypeptide and the second polypeptide are associated with each other via the Fc region (i) and the Fc region (ii). An example of the taFv-Fab-heterodimer Fc type bispecific antibody is shown in Figure 5(5e). Figure 6(6a) shows the first polypeptide, Figure 6(6c) shows the second polypeptide, and Figure 6(6d) shows the third polypeptide. As shown in Figure 5(5e), the Fc region (ii) (solid) of the second polypeptide consisting of an immunoglobulin heavy chain comprising the Fc region (ii) is associated with the Fc region (i) of the first polypeptide, and an immunoglobulin light chain is further connected to the second polypeptide. In this embodiment, the taFv-Fab-heterodimer Fc type bispecific molecule comprises taFv and the immunoglobulin Fc regions, and Fab connected thereto.. Examples of the amino acid sequence of the first polypeptide contained in a preferred taFv-Fab-heterodimer Fc type bispecific molecule include an amino acid sequence corresponding to positions 21 to 511 of the amino acid sequence represented by SEQ ID NO: 32, an amino acid sequence corresponding to positions 21 to 511 of the amino acid sequence represented by SEQ ID NO: 34, an amino acid sequence corresponding to positions 21 to 511 of the amino acid sequence represented by SEQ ID NO: 35, an amino acid sequence corresponding to positions 21 to 511 of the amino acid sequence represented by SEQ ID NO: 36, an amino acid sequence corresponding to positions 21 to 511 of the amino acid sequence represented by SEQ ID NO: 37, an amino acid sequence corresponding to positions 21 to 511 of the amino acid sequence represented by SEQ ID NO: 38, an amino acid sequence corresponding to positions 21 to 511 of the amino acid sequence represented by SEQ ID NO: 39, an amino acid sequence corresponding to positions 21 to 511 of the amino acid sequence represented by SEQ ID NO: 40, an amino acid sequence corresponding to positions 21 to 511 of the amino acid sequence represented by SEQ ID NO: 41, an amino acid sequence corresponding to positions 21 to 511 of the amino acid sequence represented by SEQ ID NO: 42, an amino acid sequence corresponding to positions 21 to 511 of the amino acid sequence represented by SEQ ID NO: 43, an amino acid sequence corresponding to positions 21 to 511 of the amino acid sequence represented by SEQ ID NO: 33, an amino acid sequence corresponding to positions 21 to 511 of the amino acid sequence represented by SEQ ID NO: 52, an amino acid sequence corresponding to positions 21 to 511 of the amino acid sequence represented by SEQ ID NO: 53, an amino acid sequence corresponding to positions 21 to 511 of the amino acid sequence represented by SEQ ID NO: 54, **an** amino acid sequence corresponding to positions 21 to 516 of the amino acid sequence represented by SEQ ID NO: 55, and an amino acid sequence corresponding to positions 21 to 516 of the amino acid sequence represented by SEQ ID NO: 56. The first polypeptide included in a further preferred taFv-Fab-heterodimer Fc type bispecific molecule consists of an amino acid sequence corresponding to positions 21 to 745 of the amino acid sequence represented by SEQ ID NO: 32, an amino acid sequence corresponding to positions 21 to 745 of the amino acid sequence represented by SEQ ID NO: 34, an amino acid sequence corresponding to positions 21 to 745 of the amino acid sequence represented by SEQ ID NO: 35, an amino acid sequence corresponding to positions 21 to 745 of the amino acid sequence represented by SEQ ID NO: 36, an amino acid sequence corresponding to positions 21 to 745 of the amino acid sequence represented by SEQ ID NO: 37, an amino acid sequence corresponding to positions 21 to 745 of the amino acid sequence represented by SEQ ID NO: 38, an amino acid sequence corresponding to positions 21 to 745 of the amino acid sequence represented by SEQ ID NO: 39, an amino acid sequence corresponding to positions 21 to 745 of the amino acid sequence represented by SEQ ID NO: 40, an amino acid sequence corresponding to positions 21 to 745 of the amino acid sequence represented by SEQ ID NO: 41, an amino acid sequence corresponding to positions 21 to 745 of the amino acid sequence represented by SEQ ID NO: 42, an amino acid sequence corresponding to positions 21 to 745 of the amino acid sequence represented by SEQ ID NO: 43, an amino acid sequence corresponding to positions 21 to 745 of the amino acid sequence represented by SEQ ID NO: 33, an amino acid sequence corresponding to positions 21 to 745 of the amino acid sequence represented by SEQ ID NO: 52, an amino acid sequence corresponding to positions 21 to 745 of the amino acid sequence represented by SEQ ID NO: 53, an amino acid sequence corresponding to positions 21 to 745 of the amino acid sequence represented by SEQ ID NO: 54, an amino acid sequence corresponding to positions 21 to 750 of the amino acid sequence represented by SEQ ID NO: 55, or an amino acid sequence corresponding to positions 21 to 750 of the amino acid sequence represented by SEQ ID NO: 56.

The second polypeptide contained in a preferred taFv-Fab-heterodimer Fc type bispecific antibody of the present invention comprises a human antibody or humanized antibody heavy chain variable region, CH1 region, and hinge region, and mutant Fc. The second polypeptide of a further preferred taFv-Fab-heterodimer Fc type bispecific antibody comprises an amino acid sequence corresponding to positions 20 to 242 of the amino acid sequence represented by SEQ ID NO: 44.

The third polypeptide contained in a preferred taFv-Fab-heterodimer Fc type bispecific antibody of the present invention comprises a human antibody or humanized antibody light chain variable region and constant region. The third polypeptide of a further preferred taFv-Fab-heterodimer Fc type bispecific antibody comprises an amino acid sequence corresponding to positions 21 to 131 of the amino acid sequence represented by SEQ ID NO: 45.

The variable region and the CH1 region of the second polypeptide, and the third polypeptide in the preferred taFv-Fab-heterodimer Fc type bispecific molecule described above, constitute Fab. The Fab is preferably Fab of an anti-HLA/NY-ESO antibody, for example, Fab of NYA-0001.

In the present invention, for example, anti-HLA-A2/NY-ESO scFv or anti-CD3 scFv, and anti-HLA/NY-ESO Fab or anti-CD3 Fab, may be included in the scFv-Fab-heterodimer Fc-type bispecific molecule..

The scFv-Fab-heterodimer Fc type bispecific molecule preferably comprises (a): a first polypeptide comprising scFv that specifically binds to human HLA/NY-ESO, a heavy chain variable region and constant region CH1 of an antibody that specifically binds to CD3, and an immunoglobulin Fc region (i) in that order from the **N-**terminus towards the C-terminus; a second polypeptide comprising an immunoglobulin hinge region and Fc region (ii); and a third polypeptide comprising an antibody light chain consisting of a variable region and a constant region. More preferably, (b) the first polypeptide and the second polypeptide are associated with each other via the Fc region (i) and the Fc region (ii), and the first polypeptide is associated with (the antibody light chain of) the third polypeptide at the antibody heavy chain variable region and constant region CH1. The Fc regions of the first polypeptide and the second polypeptide may be a wild-type or may comprise a mutation enabling heterodimer formation. An example of the scFv-Fab-heterodimer Fc type bispecific molecule is shown in Figure 7 (7c). The right half of Figure 7(7c) corresponds to the first polypeptide and the third polypeptide, and the left half corresponds to the second polypeptide. As shown in Figure 7 (7c), the Fc region (i) of the first polypeptide and the Fc region (ii) of the second polypeptide (solid) are associated with each other, and the first polypeptide and the third polypeptide are associated with each other. For example, in Figure 7(7c), the scFv (positively hatched) is anti-HLA-A2/NY-ESO scFv, and the Fab (open, checked, and horizontal lines) is anti-CD3 Fab.

Examples of the amino acid sequence contained in the first polypeptide contained in a preferable scFv-Fab-heterodimer Fc type bispecific antibody can include an amino acid sequence from positions 21 to 394 of the amino acid sequence represented by SEQ ID NO: 57, further preferably an amino acid sequence from positions 20 to 724 thereof.

An example of the amino acid sequence contained in the first polypeptide of a preferred scFv-Fab-heterodimer Fc type bispecific antibody is an amino acid sequence corresponding to positions 21 to 389 of the amino acid sequence represented by, SEQ ID NO: 60. More preferably, the amino acid sequence corresponds to positions 20 to 719 of SEQ ID NO: 60.

Another example of an amino acid sequence contained in the first polypeptide of a preferred scFv-Fab-heterodimer Fc type bispecific antibody include an amino acid sequence corresponding to positions 21 to 389 of the amino acid sequence represented by SEQ ID NO: 61, and preferably, an amino acid sequence corresponding to positions 20 to 719 of SEQ ID NO: 61.

The second polypeptide contained in a preferred scFv-Fab-heterodimer Fc type bispecific antibody of the present invention comprises a human antibody-derived hinge region and mutant Fc. The second polypeptide included in a further preferred scFv-Fab-heterodimer Fc type bispecific molecule comprises an amino acid sequence corresponding to positions 20 to 246 of the amino acid sequence represented by SEQ ID NO: 31.

The third polypeptide contained in a preferred scFv-Fab-heterodimer Fc type bispecific antibody of the present invention comprises a human antibody-derived light chain. Preferably, the third polypeptide comprises an amino acid sequence corresponding to positions 21 to 127 of the amino acid sequence represented by SEQ ID NO: 58, and more preferably, an amino acid sequence corresponding to positions 21 to 233 of SEQ ID NO: 58.

Of these, preferred examples of the scFv-Fab-heterodimer Fc type bispecific molecule of the present invention include NYZ-1010 in which the first polypeptide consisting of an amino acid sequence corresponding to positions 20 to 724 of the amino acid sequence represented by SEQ ID NO: 57, the second polypeptide consisting of an amino acid sequence corresponding to positions 20 to 246 of the amino acid sequence represented by SEQ ID NO: 31, and the third polypeptide consisting of an amino acid sequence corresponding to positions 21 to 233 of the amino acid sequence represented by SEQ ID NO: 58, are associated with each other.

Preferable examples of the scFv-Fab-heterodimer Fc type bispecific molecule of the present invention include NYZ-1007 in which the first polypeptide consisting of an amino acid sequence corresponding to positions 20 to 719 of the amino acid sequence represented by SEQ ID NO: 60, the second polypeptide consisting of an amino acid sequence corresponding to positions 20 to 246 of the amino acid sequence represented by SEQ ID NO: 31, and the third polypeptide consisting of an amino acid sequence corresponding to positions 21 to 233 of the amino acid sequence represented by SEQ ID NO: 58, are associated with each other.

Preferred examples of the scFv-Fab-heterodimer Fc type bispecific molecule of the present invention further include NYZ-1017 in which the first polypeptide consisting of an amino acid sequence corresponding to positions 20 to 719 of the amino acid sequence represented by SEQ ID NO: 61, the second polypeptide consisting of an amino acid sequence corresponding to positions 20 to 246 of the amino acid sequence represented by SEQ ID NO: 31, and the third polypeptide consisting of an amino acid sequence corresponding to positions 21 to 233 of the amino acid sequence represented by SEQ ID NO: 58 are associated with each other.

One, two, or three or more peptides contained in the bispecific molecule of the present invention can be a "deletion mutant" mentioned above. Specifically, the bispecific molecule may comprise a mutation (including deletion) of one or two (or more) amino acids at the carboxyl terminus, in particular, at the carboxyl terminus derived from an antibody heavy chain. For example, the amino acid residue at the carboxyl terminus of the amino acid sequence of the first polypeptide included in NYZ-1010, which is a preferred scFv-Fab-heterodimer Fc type bispecific molecule of the present invention, as represented by SEQ ID NO: 57, may be any of Lys724 or Gly723 resulting from the deletion of one amino acid, or a mixture of first polypeptides including Lys and Gly at the carboxyl terminus. Similarly, the amino acid residue at the carboxyl terminus of the amino acid sequence of the second polypeptide contained in NYZ-1010, a preferred scFv-Fab-heterodimer Fc type bispecific molecule of the present invention, as represented by SEQ ID NO: 84, may be any of Lys246 or Gly245 resulting from the deletion of one amino acid, or a mixture of second polypeptides including Lys and Gly at the carboxyl terminus.

The scFv and the Fab contained in the bispecific molecule of the present invention are preferably derived from a humanized antibody or human antibody, and the Fc included in the bispecific molecule is preferably derived from a human antibody.

In the bispecific molecule of the present invention, a heavy chain variable region and a light chain variable region may be connected in that order from the amino terminus of the antibody. Alternatively,a light chain variable region and a heavy chain variable region may be connected in that order, from the amino terminus of the antibody. A linker may optionally be present between both the variable regions. The variable region on the amino-terminal side may optionally comprise a glycine residue at the amino terminus. In a tandem scFv type bispecific molecule, a linker, a FLAG tag, and/or a His tag may optionally be provided at carboxyl terminus of the variable region on the carboxyl-terminal side. In a preferred aspect, for example, a heavy chain variable region, a first linker, a light chain variable region, a second linker, a FLAG tag, and a His tag are connected in this order from the amino terminus.

Examples of the linker include a single chain polypeptide or a single chain oligopeptide, or synthetic products such as PEG, nucleotides, sugar chains, and compounds. In addition, any linker known in the art may be used without particular limitation as long as the linker is capable of connecting two polypeptides.

A peptide linker may be, for example, 5 to 30 amino acids in length.. When the bispecific molecule contains a plurality of linkers, each peptide linkers may be of the same length or of different lengths.

An example of a peptide linker is a repeated (Gly·Gly·Gly·Gly·Ser) peptide. One to several amino acid residues other than Gly and Ser may be added thereto.

Of the structures (formats) that may be adopted by the multispecific antibody, particularly, the bispecific antibody, of the present invention, taFv-heterodimer Fc type, taFv-Fab-heterodimer Fc type, and scFv-Fab-heterodimer Fc type are preferred, and taFv-heterodimer Fc type is further preferred. An arrangement in which anti-HLA/NY-ESO scFv and anti-CD3 scFv are positioned in that order from the N-terminus towards the C-terminus (taFv-heterodimer Fc type) is more preferred than an arrangement in which the order is reversed (taFv (inversed)-heterodimer Fc type). In addition, scFv-Fab-heterodimer Fc type is also further preferred.

The present invention also encompasses a molecule that comprises an amino acid sequence encoded by a nucleotide sequence contained in a polynucleotide that hybridizes under stringent conditions to a complementary strand of a polynucleotide comprising a nucleotide sequence encoding an amino acid sequence contained in the molecule of the present invention, and binds to HLA/NY-ESO, and preferably, further binds to CD3.

The present invention also includes a molecule that comprises an amino acid sequence having 90%, 91%, 92%, 93%, 94%, 95%, 96%, 97%, 98%, or 99% or more identity to an amino acid sequence contained in the molecule of the present invention, and binds to HLA/NY-ESO, and preferably further binds to CD3.

The antibody of the present invention, the binding fragment thereof, and the multispecific antibody comprising the antibody or the binding fragment have excellent biological activity, physicochemical properties (hereinafter, referred to as "physical properties"), safety, and *in vivo* kinetics. (a) Examples of the biological activity or an index thereof include antigen-binding activity, *in vitro* cytotoxic activity, and *in vivo* anti-tumor activity. For example, a dissociation constant (KD value) for HLA/NY-ESO is 100 nM or less or 50 nM or less, preferably 20 nM or less or 10 nM or less, more preferably 5 nM or less. For example, the EC₅₀ value of cytotoxic activity exerted against an endogenous human NY-ESO-expressing cell line U266B1 and/or NCI-H1703 using human peripheral blood mononuclear cells as effector cells is 20 nM or less, preferably 10 nM or less, more preferably 5 nM or less (examples of the measurement and calculation of the *in vitro* cytotoxic activity can include, but are not limited to, a method described in Example 8 of WO2021/200857). For example, 0.1 mL of a suspension of human squamous cell lung cancer cell line NCI-H1703 at 6 × 10⁷ cells/mL is subcutaneously transplanted to each NOG mouse. Four days later, 0.2 mL of a suspension of human peripheral blood mononuclear cells at 3.75 × 10⁷ cells/mL is transplanted into the tail vein. The antibody administration is initiated after 14 days, and the antibody is administered once a week for three weeks, after which tumor volume is measured. Tumor growth inhibitory activity relative to a control group which has been administered a vehicle is 50% or higher, preferably 75% or higher, more preferably 90% or higher *(in vivo* anti-tumor activity can be assayed and determined by the method described in Example 9). (b) Impurities contained in biopharmaceuticals may affect drug safety. Therefore, it is necessary to establish appropriate procedures to set the specification for and to control the amount of impurities during production and storage. In particular, HMWS (aggregates), are a major impurity, and are associated with a risk of immunogenicity, reduced drug efficacy, and the like. Thus, strict control of HMWS is required. For the purpose of controlling the level of impurities, evaluation of the level of impurities should be assessed over time (whether or not the amount of impurities increases or decreases) during and after production. Long-term stability tests may be used to select antibodies with a longer shelf life. Other examples of desirablephysicochemical properties used as indicators for selecting an antibody intended for pharmaceutical use include acid resistance (the suppression of HMWS formation, etc.) and solution stability (the suppression of HMWS formation, etc.). Other examples of indicators include a high yield of product obtained by culturing recombinant cells which are obtained by introducing a gene encoding an amino acid sequence contained in the antibody of the present invention or the binding fragment thereof, or the molecule comprising the antibody or the binding fragment into a host cell suitable for the production of the antibody, for example, a Expi293F cell. A preferred antibody of the present invention, antigen-binding fragment thereof, and multispecific antibody comprising the antibody or the binding fragment, having the physicochemical properties described above are advantageous for the following reasons: a solution containing the antibody, binding fragment thereof or multispecific antibody may be exposed to acidic conditions, and consequently, the processes for producing the antibody, binding fragment thereof or multispecific antibody, for example, by protein A or ion exchange chromatography or virus inactivation, is facilitated or easy to perform; the formation of HMWS is maintained at a low level in solution, and consequently, the production, formulation, distribution or storage of medicaments comprising the antibody, binding fragment thereof or multispecific antibody is ffacilitated or easy to perform. Accordingly, pharmaceutical products comprising the antibody, binding fragment thereof or multispecific antibody may be produced efficiently. With regard to acid resistance, for example, the content of HMWS resulting from incubation of the antibody, binding fragment thereof or multispecific antibody at pH 3.5 at room temperature for 1 hour, as determined by size exclusion chromatography is 5% or less, preferably 2% or less, more preferably 1% or less (the HMWS content for evaluating acid resistance can be determined using a method described in Example 19-1 of WO2021/200857). With regard to solution stability, for example, the content of HMWS resulting from dissolving the antibody, binding fragment thereof or multispecific antibody at a concentration of 25 mg/ml in 25 mM histidine and 5% sorbitol (pH 6.0), and storing at 25°C for 6 days, as determined by size exclusion chromatography is 20% or less, preferably 10% or less, (the HMWS content for evaluating solution stability can be determined using a method described in Example 19-2 of WO2021/200857). Production efficiency or yield can be measured and determined in accordance with the , methods described in Examples 20 and 21 of WO2021/200857, but the method is not limited thereto (c) Examples of safety or indicators of safety include antigen recognition properties and observations at the time of administration. For example, the antibody, etc. of the present invention recognizes a plurality of amino acids on wild-type NY-ESO peptide but does not bind to a homologous peptide having an amino acid sequence which is similar to but not the same as the wild-type NY-ESO peptide, and has a low risk of adverse reactions caused by off-target effects. The antibody, etc. of the present invention has low immunogenicity in ISPRI web-based immunogenicity screening (EpiVax, Inc) and is thus expected to have a low risk of adverse reactions such as cytokine production arising from anti-drug_antibodies. For example, when NYF-0023, NYF-0045, NYF-0047, NYF-0048, NYF-0060, NYF-0061, NYZ-0082, or NYZ-1010 as contained in the bispecific antibody of the present invention, was administered to Balb/c mice, no significant problems were observed in relation to half-life in blood, nor were there any significant problems relating to weight loss or other toxicity effects. Additionally, when a single dose of NYZ-0082 or NYZ-1010 was administered to cynomolgus monkeys, no significant problems were observed in relation to half-life in blood, nor where there any changes in general state, body weight, feed intake, body temperature measurement, and plasma levels of cytokines, caused by the administration of the antibodies. (d) Examples of the kinetics or indicators thereof include half-life in blood. For example, when several bispecific antibodies in accordance with the present invention wereadministered to Balb/c mice or cynomolgus monkeys, no significant problems were observed in relation to half-life in blood. The antibody, binding fragment thereof, and molecule of the invention have excellent biological activity, physicochemical properties, safety, and *in vivo* kinetics, and therefore, can be incorporated into pharmaceutical compositions. Preferable examples of the antibody of the present invention or the antigen-binding fragment thereof having the antigen-binding activity described in (a) and the properties described in (b) include, but are not limited to, NYA-1143, NYA-1163, NYA-2023, NYA-2027, NYA-2035, NYA-2044,NYA-2045, NYA-2047, NYA-2048, NYA-2060, NYA-2031, NYA-2047, NYA-2061, NYA-2143, and NYA-3061, more preferably NYA-2047, NYA-2061, NYA-2143, and NYA-3061. Preferable examples of the multispecific antibody of the present invention having the properties described in(a) to (d) include, but are not limited to, NYF-0016, NYF-0019, NYF-0022, NYF-0023, NYF-0027, NYF-0035, NYF-0044, NYF-0045, NYF-0047, NYF-0048, NYF-0058, NYF-0060, NYF-0061, NYZ-0038, NYZ-0082, NYZ-0088, and NYZ-1010, more preferably NYF-0061, NYZ-0038, NYZ-0082, NYZ-0088, NYZ-1007, NYZ-1010, and NYZ-1017.

In the present invention, the "site" to which an antibody binds, i.e., the "site" recognized by an antibody, means a part of a peptide antigen or a part of a higher-order structure of an antigen which is bound or recognized by the antibody. In the present invention, such a site is also referred to as an epitope or an antibody binding site. Examples of the site on HLA/NY-ESO bound or recognized by the anti-HLA/NY-ESO antibody of the present invention include a plurality of amino acids and parts of higher-order structures in the HLA/NY-ESO peptide.

The present invention also encompasses an "antibody or binding fragment thereof that binds to the same site" as the antibody of the present invention or the binding fragment thereof. The "antibody that binds to the same site" is another antibody that binds to the site on the antigen molecule recognized by a given antibody. If a second antibody binds to part of a peptide antigen or part of a three-dimensional structure on an antigen molecule bound by a first antibody, the first and second antibodies can be determined as binding to the same site. When the first antibody of the present invention has the antigen-binding activity described above in (a), the second antibody that binds to the same site on HLA/NY-ESO as the first antibody is very likely to have a similar activity to the first antibody. Such a second antibody is also encompassed by the present invention. An antibody that competes with the first antibody of the present invention for binding to HLA/NY-ESO is also encompassed by the present invention as long as the antibody has the antigen-binding activity described in (a). An antibody that binds to a site on HLA/NY-ESO recognized by the monoclonal antibody of the present invention, an antibody that binds to HLA/NY-ESO competitively with the monoclonal antibody of the invention, and binding fragments thereof preferably have one,two or more, preferably three or more, and more preferably, each of *in vitro* cytotoxic activity and *in vivo* anti-tumor activity described in (a) and the properties described in (b) to (d).

The antibody binding site can be determined by a method well known to those skilled in the art, such as an immunoassay. For example, a series of peptides are prepared by appropriately cleaving the amino acid sequence of the antigen from its C-terminus or **N-**terminus, and the reactivity of the antibody to the peptide fragments is studied to roughly determine a recognition site. Then, shorter peptides are synthesized, and the reactivity of the antibody to these peptides can be studied in order to determine the binding site. Alternatively, for example, a particular site or region in the amino acid sequence of the antigen or antigen fragment peptides or the like. is deleted, or substituted by another amino acid sequence, or a mutation is introduced into the amino acid sequence, and the reactivity of the antibody to the resulting peptide can be studied in order to determine the binding site. The antigen fragment peptides can be prepared, for example, by genetic recombination or peptide synthesis.

When the antibody binds to or recognizes a part of a higher-order structure of the antigen, the binding site for the antibody can be determined by identifying amino acid residues on the antigen adjacent to the antibody using X-ray structural analysis. For example, the antibody or its fragment and the antigen or its fragment can be bound to each other, crystallized, and structurally analyzed in order to identify an amino acid residue on the antigen which is at a suitable distance for interacting with the antibody ("interaction distance"). The interaction distance is 8 angstroms or less, preferably 6 angstroms or less, more preferably 4 angstroms or less. One or more amino acid residues being at such an interaction distance with respect to the antibody may constitute the antigen-binding site (epitope) of the antibody. When there are two or more such amino acid residues, these amino acids may not be adjacent to each other in the primary sequence.

The anti-HLA/NY-ESO antibody of the present invention or the binding fragment thereof specifically recognizes a plurality of amino acids in the amino acid sequence of HLA/NY-ESO. An antibody or a binding fragment thereof that recognizes the plurality of amino acids, competes with the antibody of the present invention or the binding fragment thereof for binding to HLA/NY-ESO, or which has an interaction distance with the plurality of amino acids is also encompassed by the present invention. Furthermore, a multispecific antibody comprising such an antibody or a binding fragment thereof is also encompassed by the present invention.

The multispecific antibody of the present invention can be used in combination with an immune checkpoint inhibitor.

### [Immune checkpoint inhibitor]

In the present invention, "immune checkpoint inhibitor" refers to a drug that inhibits an immunosuppressive system and activates antitumor immunity, and has the same meaning as a "compound that inhibits an immune checkpoint molecule".

Preferred examples of the immune checkpoint inhibitor used in the present invention include, but are not particularly limited to, anti-PD-1 antibodies, anti-PD-L1 antibodies, anti-CTLA-4 antibodies, and anti-TIGIT antibodies and can more preferably include anti-PD-1 antibodies and anti-PD-L1 antibodies. In the present invention, "anti-PD-1 antibody" refers to an antibody that specifically binds to PD-1 (programmed cell death-1; CD279; PDCD1), and preferably refers to an antibody having an effect of reducing, inhibiting, and/or interfering with signal transduction resulting from the interaction between PD-1 and its binding partner PD-L1 or PD-L2. The anti-PD-1 antibody used in the present invention is not particularly limited as long as its efficacy and safety have been clinically confirmed. Preferred examples thereof include nivolumab (International Publication No. WO 2006/121168, etc.) and pembrolizumab (International Publication No. WO 2008/156712, etc.). For example, a commercially available anti-PD-1 antibody for research **(e.g.,** clone RMP1-14) may be used to confirm a combined effect of the ant-PD1 antibody and the multispecific antibody comprising the anti-HLA-A2/NY-ESO antibody of the present invention in preclinical research. In the present invention, "anti-PD-L1 antibody" refers to an antibody that specifically binds to PD-L1 (programmed cell death ligand 1; CD274; B7-H1), and preferably refers to an antibody having an effect of reducing, inhibiting, and/or interfering with signal transduction resulting from the interaction between PD-L1 and its binding partner PD-1 or B7.1 (CD80). The anti-PD-L1 antibody used in the present invention is not particularly limited as long as its efficacy and safety have been clinically confirmed. Preferred examples of the anti-PD-L1 antibody include atezolizumab (International Publication No. WO 2010/077634, etc.), durvalumab (International Publication No. WO 2011/066389, etc.), and avelumab (International Publication No. WO 2013/079174, etc.). For example, a commercially available anti-PD-L1 antibody for research (e.g., clone 10F.9G2) may be used to confirm a combined effect of the anti-PD-L1 antibody and the multispecific antibody comprising the anti-HLA-A2/NY-ESO antibody of the present invention in preclinical research. In the present invention, the "anti-CTLA-4 antibody" refers to antibody that specifically binds to CTLA-4 (cytotoxic T-lymphocyte-associated protein 4; CD152), and preferably refers to an antibody having an effect of reducing, inhibiting, and/or interfering with signal transduction resulting from the interaction between CTLA-4 and its binding partner B7.1 (CD80) or B7.2 (CD86). The anti-CTLA-4 antibody used in the present invention is not particularly limited as long as its efficacy and safety have been clinically confirmed. Preferred examples of an anti-CTLA-4 antibody include ipilimumab (International Publication No. WO 2001/014424, etc.), tremelimumab (International Publication No. WO 2000/037504, etc.), spartalizumab (International Publication No. WO 2015/112900, etc.), and cemiplimab (International Publication No. WO 2015/196051, etc.). For example, a commercially available anti-CTLA-4 antibody for research **(e.g.,** clone 9H10) may be used to confirm a combined effect of the anti-CTLA-4 antibody and the multispecific antibody comprising the anti-HLA-A2/NY-ESO antibody used in the present invention in preclinical research. In the present invention, "anti-TIGIT antibody" refers to antibody that specifically binds to TIGIT (T cell immunoreceptor with Ig and ITIM domains), and preferably refers to an antibody having an effect of reducing, inhibiting, and/or interfering with signal transduction resulting from the interaction between TIGIT and its binding partner CD155. The anti-TIGIT antibody used in the present invention is not particularly limited as long as its efficacy and safety have been clinically confirmed. Preferred examples of the anti-TIGIT antibody include tiragolumab (International Publication No. WO 2017/053748) and vibostolimab (International Publication No**.** WO 2016/028656). For example, a commercially available anti-TIGIT antibody for research **(e.g.,** clone 1B4) may be used to confirm a combined effect of the anti-TIGIT antibody and the multispecific antibody comprising the anti-HLA-A2/NY-ESO antibody of the present invention in preclinical research.

In the present invention, when the "immune checkpoint inhibitor" is an antibody, an antigen-binding fragment of the antibody is also within the scope of the "antibody". In the present invention, the immune checkpoint inhibitor that is used in combination with the multispecific antibody may be an antigen-binding fragment of any of the antibodies described above, and the antigen-binding fragment may comprise other moieties.

### [Chemotherapeutic]

In the present invention, the term "chemotherapeutic" means a chemically synthesized drug among compounds having anticancer or antitumor activity.

The chemotherapeutic used in the present invention is not particularly limited and it is preferably a topoisomerase inhibitor, a microtubule inhibitor, a platinum-containing drug, a DNA demethylating agent, an anticancer antibiotic, or an alkylating agent. Preferable examples of the topoisomerase inhibitor include irinotecan, topotecan, etoposide, and a drug conjugate sacituzumab govitecan having bound SN-38, an active metabolite of irinotecan. Preferable examples of the microtubule inhibitor include paclitaxel, docetaxel, vincristine, vinblastine, vindesine, eribulin, vinorelbine, and albumin-bound paclitaxel (Nab paclitaxel). Preferable examples of the platinum-containing drug can include oxaliplatin, carboplatin, cisplatin, and nedaplatin. Preferable examples of the DNA demethylating agent include azacytidine and decitabine. Preferable examples of the anticancer antibiotic include doxorubicin, bleomycin, and liposomal doxorubicin. Preferable examples of the alkylating agent include ifosfamide, cyclophosphamide, and dacarbazine. However, the chemotherapeutic that is used in combination with the multispecific antibody of the present invention is not limited thereto.

### [Medicament]

The present invention provides a pharmaceutical composition comprising the multispecific molecule in combination with an immune checkpoint inhibitor or a chemotherapeutic. The present invention also provides a method of treatment comprising administering the multispecific molecule in combination with an immune checkpoint inhibitor or a chemotherapeutic.

In the pharmaceutical composition and the method of treatment of the present invention, the multispecific molecule and the immune checkpoint inhibitor or the chemotherapeutic may be contained as active ingredients in separate preparations and administered at simultaneously or at different times. The multispecific molecule and the immune checkpoint inhibitor or the chemotherapeutic may be contained as active ingredients in a single preparation and administered simultaneously. Alternatively, the multispecific molecule according to the present invention may be contained as an active ingredient in a single preparation and administered for the treatment of a disease that is improved by activating anti-tumor immunity.

The pharmaceutical composition and the method of the present invention can be used for the treatment of a cancer and, preferably, can be used for the treatment of at least one disease selected from the group consisting of: lung cancer (including non-small cell lung cancer), urothelial cancer, large intestine cancer (also called colorectal cancer; including colon cancer and rectal cancer), prostate cancer, ovary cancer, pancreatic cancer, breast cancer, urinary bladder cancer, stomach cancer (also called gastric adenocarcinoma), gastroesophageal junction adenocarcinoma, gastrointestinal stromal tumor, uterine cervical cancer, esophageal cancer, squamous cell cancer, peritoneal cancer, liver cancer, hepatocellular cancer, endometrial cancer, uterine cancer, salivary gland cancer, kidney cancer, vulval cancer, thyroid cancer, penis cancer, leukemia, malignant lymphoma, plasmacytoma, myeloma, neuroepithelial tissue tumor, nerve sheath tumor, head- and-neck cancer, skin cancer, pharyngeal cancer, gallbladder cancer, bile duct cancer, mesothelioma, Paget's disease, and sarcoma.

The pharmaceutical composition of the present invention can be selected and used as as a drug for the primary treatment for cancers. The method of treatment of the present invention may also be a primary treatment for cancers. As a result, the pharmaceutical composition or method can delay the growth of cancer cells, inhibit their proliferation, and further destroy the cancer cells. These effects can allow cancer patients to be free from symptoms caused by cancers or achieve improvement in QOL of cancer patients and attain a therapeutic effect by sustaining the lives of the cancer patients. Even if the pharmaceutical composition and the method of treatment do not enable the destruction of all cancer cells, they may allow cancer patients to have an improved QOL while achieving longer-term survival, by inhibiting or controlling the proliferation of cancer cells. The pharmaceutical composition of the present invention can be used as a sole drug in the medication, or the method of treatment of the invention may be used as the sole method of treatment. Alternatively, the pharmaceutical composition or method of treatment can be used in combination with an additional therapy as an adjuvant therapy, and can be combined with surgical operation, radiotherapy, hormone therapy, or the like. Furthermore, the pharmaceutical composition may be used as a drug for use in neoadjuvant therapy, or the method of treatment may be used as a neoadjuvant therapy. In addition to the therapeutic use as described above, the pharmaceutical composition and the method of treatment of the present invention can also be expected to have a prophylactic effect such as the inhibition of proliferation of small metastatic cancer cells and their destruction. For example, an effect of inhibiting and destroying cancer cells in a body fluid in the course of metastasis or an effect of, for example, inhibiting and destroying small cancer cells immediately after implantation in any tissue can be expected. Thus, the inhibition of cancer metastasis or a prophylactic effect can be expected, particularly, after surgical removal of a cancer. The pharmaceutical composition and the method of treatment of the present invention can be applied as a systemic therapy to patients or applied locally to cancer tissues. Preferably, the pharmaceutical composition of the present invention is administered to a mammal and more preferably, to a human. Preferably, in the method of treatment of the present invention, the subject to be treated is a mammal and more preferably, a human. The pharmaceutical composition of the present invention can be administered as a pharmaceutical composition containing one or more pharmaceutically compatible ingredients. An appropriate substance for use in the pharmaceutical composition of the present invention can be selected from pharmaceutical additives and other pharmaceutical components known in the art, and applied or administered at a given dose or concentration. The pharmaceutical composition typically contains, for example, one or more pharmaceutical carriers (e.g., a sterilized liquid). In this context, the liquid includes, for example, water and an oil (petroleum or an oil of animal origin, plant origin, or synthetic origin). The oil may be, for example, peanut oil, soybean oil, mineral oil, or sesame oil. Water is a more typical carrier when the pharmaceutical composition is intravenously administered. An aqueous salt solution, an aqueous dextrose solution and an aqueous glycerol solution may also be used as liquid carriers, particularly, for injectable solutions. A suitable pharmaceutical excipient can be selected from those known in the art. The composition may also contain, if desired, a small amount of a wetting agent or an emulsifier, or a pH buffering agent. Examples of suitable pharmaceutical carriers are described in "Remington's Pharmaceutical Sciences" by E.W**.** Martin. The prescription thereof corresponds to the form of administration.

Various delivery systems are known in the art and can be used for administering the pharmaceutical composition of the present invention. Examples of the administration route can include, but are not limited to, intracutaneous, intramuscular, intraperitoneal, intravenous, and subcutaneous routes. The administration may be based on, for example, infusion or bolus injection. According to a particularly preferred embodiment, the multispecific molecule and the immune checkpoint inhibitor or the chemotherapeutic used in the present invention are administered by infusion. Parenteral administration is a preferred administration route. In a typical embodiment, the pharmaceutical composition is provided as a pharmaceutical composition suitable for intravenous administration to humans according to conventional procedures. The composition for intravenous administration is typically a solution in a sterile and isotonic aqueous buffer solution. If necessary, the pharmaceutical composition may also contain a solubilizing agent and a local anesthetic for alleviating pain at an injection site **(e.g.,** lignocaine). In general, these ingredients are provided, for example, either separately or together as a mixture in a single dosage form, as a dried or freeze-dried powder or an anhydrous concentrate in a container sealed by hermetical sealing in an ampoule or a sachet that indicates the amount of the active agent. When the pharmaceutical composition is administered by infusion, it can be administered, for example, from an infusion bottle containing water or saline of sterile pharmaceutical grade. When the pharmaceutical composition is administered by injection, an ampoule of injectable sterile water or saline can be provided such that, for example, the ingredients are mixed before administration.

The pharmaceutical composition may contain a therapeutic agent for a cancer other than the multispecific molecule according to the present invention and the immune checkpoint inhibitor or the chemotherapeutic. The pharmaceutical composition of the inventionmay be administered in combination with an additional therapeutic agent for treating a cancer and can thereby potentiate an anti-tumor effect. The method of treatment of the present invention may comprise administering a therapeutic agent for treating a cancer in addition to the multispecific molecule according to the present invention and the immune checkpoint inhibitor or the chemotherapeutic. The additional therapeutic agent for treating a cancer may be administered to an individual concurrently with, separately from, or continuously with the pharmaceutical composition of the present invention, or their administration may be staggered at intervals. Examples of such a therapeutic agent for treating a cancer can include pemetrexed, sorafenib, everolimus, tanespimycin, and bevacizumab. The therapeutic agent is not limited thereto as long as it has anti-tumor activity.

The molecular targeted drug used in the present invention is not particularly limited and it is preferably a VEGF inhibitor, an FGFR inhibitor, or a multikinase inhibitor. Preferable examples of the VEGF inhibitor include bevacizumab, ramucirumab, Zaltrap, and axitinib. Preferable examples of the FGFR inhibitor include pemigatinib and futibatinib. Preferredexamples of the multikinase inhibitor include sorafenib, sunitinib, pazopanib, regorafenib, and lenvatinib. However, the chemotherapeutic that is used in combination with the multispecific antibody of the present invention is not limited thereto.

Such a pharmaceutical composition can be formulated as a freeze-dried preparation or a liquid preparation which has a given composition and necessary purity. A freeze-dried preparation may contain asuitable pharmaceutical additive that is used in the art. Likewise, a liquid preparation may contain a liquid agent and further contain various pharmaceutical additives that are used in the art.

The composition and concentration of the pharmaceutical composition may vary depending on the method of administration. The multispecific molecule contained in the pharmaceutical composition of the present invention and the immune checkpoint inhibitor or the chemotherapeutic, in terms of affinity for the antigen, i.e., a dissociation constant (Kd value) for the antigen, can exert drug efficacy even at a smaller dose when the affinity is higher (the Kd value is lower). Thus, the doses of the multispecific molecule and the immune checkpoint inhibitor or the chemotherapeutic may be set based on the magnitude of the affinity for their antigens. Each of the multispecific molecule of the present invention and the immune checkpoint inhibitor or the chemotherapeutic may be administered to a human, for example, at a dose of approximately 0.0001 to 100 mg. A predetermined dose can be administered once every 1 to 180 days or may be administered at two, three, four or more times per day at appropriate intervals.

A method for administering the multispecific molecule of the present invention may comprise, for example, administering the multispecific molecule in an amount of 0.001 mg/kg to 8 mg/kg once every three weeks. The administration can be performed once every three weeks (q3w), once a week (q1w), once every two weeks (q2w), or once every four weeks (q4w).

The present invention will be specifically described with reference to the examples given below. However, the present invention is not limited to these examples. These examples are by no means to be interpreted in a limited way.

### Examples

Hereinafter, the present invention will be described in more detail with reference to Examples. However, the present invention is not limited to the Examples.

Procedures related to gene manipulation in the Examples described below were performed according to the methods described in experimental manuals used by those skilled in the art, or using commercially available reagents or kits in accordance with the instruction manuals, unless otherwise specified.

Example 1. *In vivo* combinatorial effect of Fc-conjugated type anti-HLA-A2/NY-ESO-anti-CD3 bispecific molecule (NYZ-1010) and paclitaxel on a human squamous cell lung cancer cell line

A human squamous cell lung cancer cell line NCI-H1703 (ATCC) was adjusted to a concentration of 6 × 10⁷ cells/mL with PBS containing 50% Matrigel (Corning Inc.) and subcutaneously transplanted in an amount of 0.1 mL into NSG mice (female, 4 to 6 weeks old) (Day 0). After approximately 1 week (Days 6 to 7), the major axis (mm) and minor axis (mm) of each tumor were measured over time using electronic digital calipers, and an estimated tumor volume was calculated according to the following equation.

Estimated tumor volume (mm³) = Average estimated tumor volume of each individual: Estimated tumor volume (mm3) of each individual = 1/2 × [Tumor major axis] × [Tumor minor axis] × [Tumor minor axis]

On Day 12, grouping based on the tumor volume was carried out at n = 5 per group. T cells proliferated by the stimulation of human PBMC with CD3/CD28 Dynabeads (Thermo Fisher Scientific Inc.) were adjusted to a concentration of 5 × 10⁷ cells/mL with PBS and transplanted in an amount of 0.2 mL into the tail vein of each mouse. After three to four hours, an anti-HLA-A2/NY-ESO-anti-CD3 bispecific molecule (NYZ-1010, 0.02 mg/kg; prepared by the method described in WO2021/200857) was administered into the tail vein of each mouse. Paclitaxel (20 mg/kg) was administered intraperitoneally. The administration was carried out on Days 13 and 20. Tumor growth inhibition (%) was calculated according to the following equation: Tumor growth inhibition (%) = 100 - (Estimated tumor volume of each group/Estimated tumor volume of a vehicle control × 100)

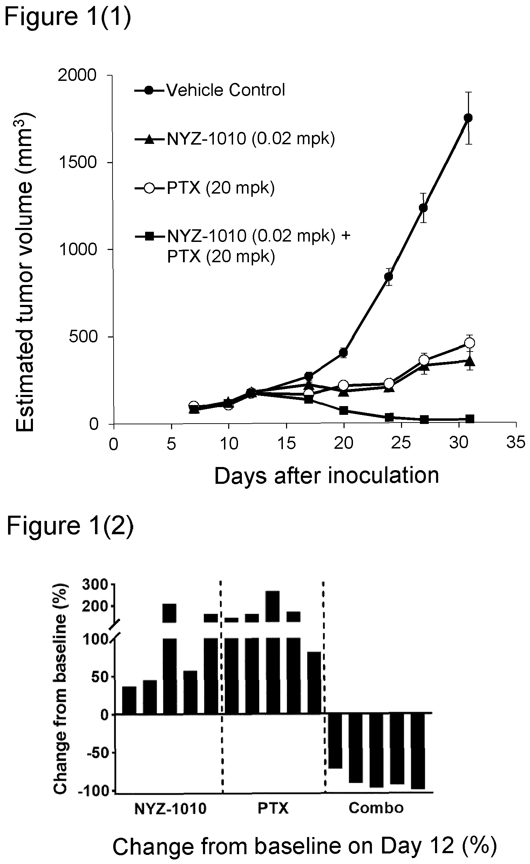

Figure 1(1) shows the results of single administration of NYZ-1010, single administration of paclitaxel, and combined administration of NYZ-1010 and paclitaxel. 31 days after tumor inoculation, TGI was 79.8% in the single administration group receiving NYZ-1010 and 74.1% in the single administration group receiving paclitaxel, whereas it was 99.1% in the combination group receiving NYZ-1010 and paclitaxel, indicating that this combination enhances an antitumor effect. The percentage change in tumor volume on Day 31 in each individual mouse using the tumor volume on Day 12 as a baseline (change from baseline (%)) was calculated according to the equation given below, and is shown in Figure 1(2). The average change from baseline (%) of each group is also described in Table 1. A combinatorial effect of tumor regression by NYZ-1010 and paclitaxel was observed.

Change from baseline (%) = (Estimated tumor volume at Day 31 - Estimated tumor volume at Day 12) of each individual / Estimated tumor volume at Day 12 × 100

**[Table 1]**

| Compound | Change from baseline (%) |
|---|---|
| NYZ-1010 | 101.7 (n=5) |
| Paclitaxel | 164.0 (n=5) |
| NYZ-1010+Paclitaxel | -91.1 (n=5) |

Example 2. *In vivo* combinatorial effect of Fc-conjugated type anti-HLA-A2/NY-ESO-anti-CD3 bispecific molecule (NYZ-1010) and Nab paclitaxel on a human squamous cell lung cancer cell line

A human squamous cell lung cancer cell line NCI-H1703 (ATCC) was adjusted to a concentration of 6 × 10⁷ cells/mL with PBS containing 50% Matrigel (Corning Inc.) and subcutaneously transplanted in an amount of 0.1 mL into NSG mice (female, 4 to 6 weeks old) (Day 0). After approximately 1 week, (Days 6 to 7), the major axis (mm) and minor axis (mm) of each tumor were measured over time using electronic digital calipers, and an estimated tumor volume was calculated according to the following equation.

Estimated tumor volume (mm³) = Average estimated tumor volume of each individual: Estimated tumor volume (mm3) of each individual = 1/2 × [Tumor major axis] × [Tumor minor axis] × [Tumor minor axis]

On Day 13, grouping based on the tumor volume was carried out at n = 5 per group. T cells proliferated by the stimulation of human PBMC with CD3/CD28 Dynabeads (Thermo Fisher Scientific Inc.) were adjusted to a concentration of 5 × 10⁷ cells/mL with PBS and transplanted in an amount of 0.2 mL into the tail vein of each mouse. After three to four hours, an anti-HLA-A2/NY-ESO-anti-CD3 bispecific molecule (NYZ-1010, 0.02 mg/kg) was administered into the tail vein of each mouse. Nab paclitaxel (20 mg/kg) was administered intraperitoneally. The administration was carried out on Days 13 and 20. Tumor growth inhibition (%) was calculated according to the following equation. Tumor growth inhibition (%) = 100 - (Estimated tumor volume of each group/Estimated tumor volume of a vehicle control × 100)

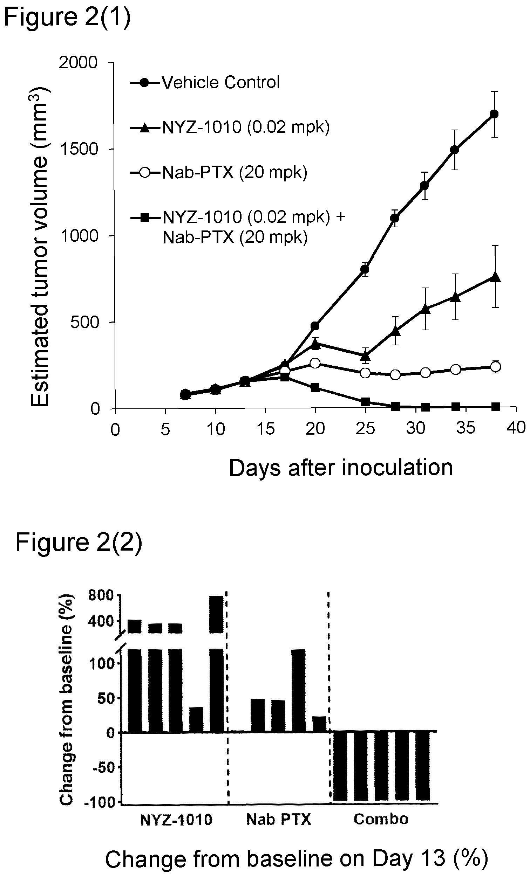

Figure 2(1) shows results of single administration of NYZ-1010, single administration of Nab paclitaxel, and combined administration of NYZ-1010 and Nab paclitaxel. 38 days after tumor inoculation, TGI was 55.4% in the single administration group receiving NYZ-1010 and 86.3% in the single administration group receiving Nab paclitaxel, whereas it was 100% in the combination group receiving NYZ-1010 and Nab paclitaxel, indicating that this combination enhances an antitumor effect. In particular, complete disappearance of tumor was observed in each of the five mice in the combination group receiving NYZ-1010 and Nab paclitaxel on Day 38. The percentage change in tumor volume on Day 38 in each individual mouse using the tumor volume on Day 13 as a baseline (change from baseline (%)) was calculated according to the equation given below, and is shown in Figure 2(2). The average change from baseline (%) in each group is also described in Table 2. A combinatorial effect of tumor regression by NYZ-1010 and Nab paclitaxel was observed. Change from baseline (%) = (Estimated tumor volume at Day 38 - Estimated tumor volume at Day 13) of each individual/Estimated tumor volume at Day 13 × 100

**[Table 2]**

| Compound | Change from baseline (%) |
|---|---|
| NYZ-1010 | 389.1 (n=5) |
| Nab Paclitaxel | 47.8 (n=5) |
| NYZ-1010+Nab Paclitax el | -100.0 (n=5) |

Example 3. *In vivo* combinatorial effect of Fc-conjugated type anti-HLA-A2/NY-ESO-anti-CD3 bispecific molecule (NYZ-1010) and anti-PD-1 antibody pembrolizumab on a human lung adenocarcinoma cell line

A human lung adenocarcinoma cell line NCI-H522 (ATCC) was adjusted to a concentration of 5 × 10⁷ cells/mL with PBS containing 50% Matrigel (Corning Inc.) and subcutaneously transplanted in an amount of 0.1 mL into NSG mice (female, 4 to 6 weeks old) (Day 0). After approximately 2 weeks, the major axis (mm) and minor axis (mm) of each tumor were measured over time using electronic digital calipers, and an estimated tumor volume was calculated according to the following equation.

Estimated tumor volume (mm³) = Average estimated tumor volume of each individual: Estimated tumor volume (mm3) of each individual = 1/2 × [Tumor major axis] × [Tumor minor axis] × [Tumor minor axis]

On Day 25, grouping based on the tumor volume was carried out at n = 5 per group. T cells proliferated by the stimulation of human PBMC with CD3/CD28 Dynabeads (Thermo Fisher Scientific Inc.) were adjusted to a concentration of 5 × 10⁷ cells/mL with PBS and transplanted in an amount of 0.2 mL into the tail vein of each mouse. After three to four hours, an anti-HLA-A2/NY-ESO-anti-CD3 bispecific molecule (NYZ-1010, 0.01 mg/kg) was administered into the tail vein of each mouse. Pembrolizumab (Merck KGaA, 10 mg/kg) was administered into the tail vein of each mouse. The administration was carried out on Days 25, 32, and 39 (NYZ-1010) or on Days 28, 32, 35, 39, and 42 (pembrolizumab). Tumor growth inhibition (%) was calculated according to the following equation. Tumor growth inhibition (%) = 100 - (Estimated tumor volume of each group/Estimated tumor volume of a vehicle control × 100)

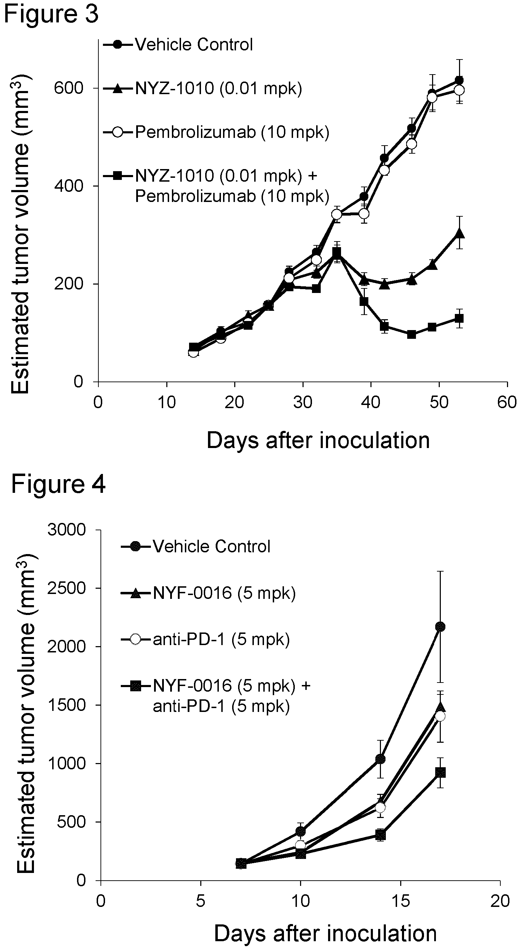

Figure 3 shows results of single administration of NYZ-1010, single administration of pembrolizumab, and combined administration of NYZ-1010 and pembrolizumab. 53 days after tumor inoculation, TGI was 50.5% in the single administration group receiving NYZ-1010 and 3.1% in the single administration group receiving pembrolizumab, whereas it was 79.0% in the combination group receiving NYZ-1010 and pembrolizumab, indicating that this combination provides an enhanced antitumor effect.

### Example 4. Preparation of human CD3ε knock-in mouse

The anti-HLA-A2/NY-ESO-anti-CD3 bispecific molecule binds to human CD3ε but does not cross-react with rodent CD3ε. Accordingly, in order to enable the anti-HLA-A2/NY-ESO-anti-CD3 bispecific molecule to be evaluated *in vivo,* human CD3ε knock-in mice were prepared. A vector was prepared by knocking-in CRISPR-Cas9 and a human CD3ε sequence to be bound by the anti-HLA-A2/NY-ESO-anti-CD3 bispecific molecule to mouse CD3ε, and introduced to fertilized eggs of C57BL/6J mice by microinjection. After microinjection, the fertilized eggs were transplanted to the fallopian tubes or uteri of female mice, and the tail tissues of the resulting offspring were biopsied, followed by PCR and sequence analysis. Individual mice having the mutation of interest were selected and used in the subsequent experiments.

Example 5. *In vivo* combinatorial effect of Fc-conjugated type anti-HLA-A2/NY-ESO-anti-CD3 bispecific molecule (NYF-0016) and anti-mouse PD-1 antibody using a human CD3ε knock-in mouse

Genes encoding HLA-A2, NY-ESO, and β2M single chain trimer (abbreviated to NY-SCT) were introduced into a mouse large intestine cancer cell line MC-38 provided by the National Cancer Institute, using a retroviral vector. The cells express HLA-A2/NY-ESO on the cell membrane. The resulting MC-38 NY-SCT cells were adjusted to a concentration of 8 × 10⁶ cells/mL with PBS (FUJIFILM Wako Pure Chemical Corp.) and subcutaneously transplanted in an amount of 0.1 mL into each human CD3ε knock-in mouse (female, 6 to 7 weeks old) as prepared in Example 4 (Day 0). From Day 7, the major axis (mm) and minor axis (mm) of tumor were measured over time using electronic digital calipers, and an estimated tumor volume was calculated according to the following equation.

Estimated tumor volume (mm³) = Average estimated tumor volume of each individual: Estimated tumor volume (mm3) of each individual = 1/2 × [Tumor major axis] × [Tumor minor axis] × [Tumor minor axis]

On Day 7, grouping based on the tumor volume was carried out at n = 5 per group. An anti-HLA-A2/NY-ESO-anti-CD3 bispecific molecule (NYF-0016, 5 mg/kg; prepared by the method described in WO2021/200857) was administered into the tail vein of each mouse. An anti-mouse PD-1 antibody (clone RMP1-14, Bio X Cell, 5 mg/kg) was administered into the tail vein of each mouse. The administration was carried out on days 7 and 10. Tumor growth inhibition (%) was calculated according to the following equation. Tumor growth inhibition (%) = 100 - (Estimated tumor volume of each group/Estimated tumor volume of a vehicle control × 100)

Figure 4 shows results of single administration of NYF-0016, single administration of the anti-mouse PD-1 antibody, and combined administration of NYF-0016 and the anti-mouse PD-1 antibody. 17 days after tumor inoculation, TGI was 31.5% in the single administration group receiving NYF-0016 and 35.3% in the single administration group receving the anti-mouse PD-1 antibody, whereas it was 57.5% in the combination group receiving NYF-0016 and the anti-mouse PD-1 antibody, indicating that this combination provides an enhanced antitumor effect.

Example 6. *In vivo* combinatorial effect of Fc-conjugated type anti-HLA-A2/NY-ESO-anti-CD3 bispecific molecule (NYZ-1010), anti-PD-1 antibody pembrolizumab, and multikinase inhibitor lenvatinib on a human lung adenocarcinoma cell line

A human lung adenocarcinoma cell line NCI-H522 (ATCC) was adjusted to a concentration of 5 × 10⁷ cells/mL with PBS containing 50% Matrigel (Corning Inc.) and subcutaneously transplanted in an amount of 0.1 mL into NSG mice (female, 4 to 6 weeks old) (Day 0). After approximately 3 weeks, the major axis (mm) and minor axis (mm) of each tumor were measured over time using electronic digital calipers, and an estimated tumor volume was calculated according to the following equation.

Estimated tumor volume (mm³) = Average estimated tumor volume of each individual: Estimated tumor volume (mm3) of each individual = 1/2 × [Tumor major axis] × [Tumor minor axis] × [Tumor minor axis]

On Day 28, grouping based on the tumor volume was carried out at n = 5 per group. T cells proliferated by the stimulation of human PBMC with CD3/CD28 Dynabeads (Thermo Fisher Scientific Inc.) were adjusted to a concentration of 5 × 10⁷ cells/mL with PBS and transplanted in an amount of 0.2 mL into the tail vein of each mouse. After three to four hours, an anti-HLA-A2/NY-ESO-anti-CD3 bispecific molecule (NYZ-1010, 0.01 mg/kg) was administered into the tail vein of each ouse. Pembrolizumab (Merck KGaA, 10 mg/kg) was administered intraperitoneally, and lenvatinib (30 mpk) was orally administered. The administration was carried out on Days 28, 35, and 42 (NYZ-1010), on days 32, 39, and 46 (pembrolizumab), or once a day on days 28 to 32, once a day on Days 35 to 39, and once a day on Days 42 to 36 (lenvatinib). Tumor growth inhibition (%) was calculated according to the following quation.Tumor growth inhibition (%) = 100 - (Estimated tumor volume of each group/Estimated tumor volume of a vehicle control × 100)

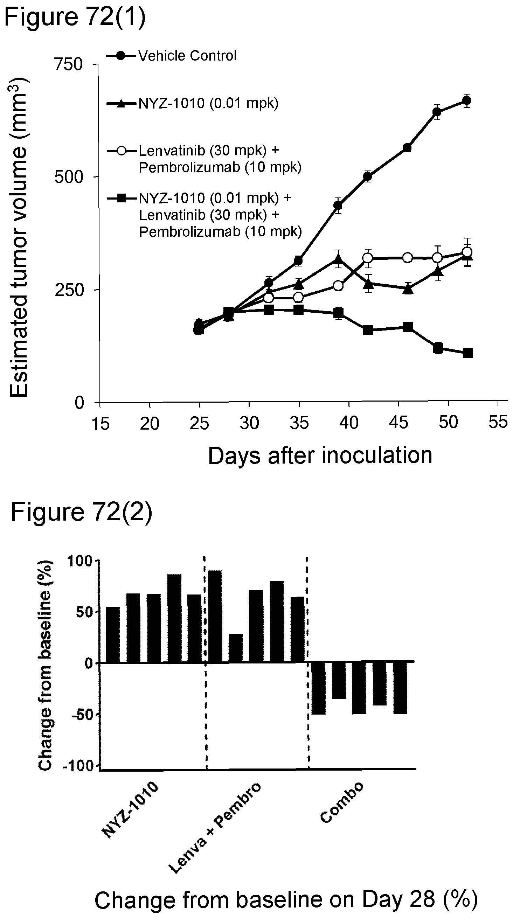

Figure 72(1) shows the results of single administration of NYZ-1010, combined administration of lenvatinib and pembrolizumab, and combined administration of NYZ-1010, lenvatinib, and pembrolizumab. 52 days after inoculation, TGI was 51.4% in the single administration group receiving NYZ-1010 and 50.6% in the combined administration group receiving lenvatinib and pembrolizumab, whereas it was 84.1% in the combination group receiving NYZ-1010, lenvatinib, and pembrolizumab, indicating that the combination of three agents provides enhanced antitumor effect. The percentage change in tumor volume on Day 52 in each individual mouse using the tumor volume on Day 28 as a baseline (change from baseline (%)) was calculated according to the equation given below, and is shown in Figure 72(2). The average change from baseline (%) of each group is also described in Table 3. A combinatorial effect of NYZ-1010, lenvatinib, and pembrolizumab on tumour regression was observed. Change from baseline (%) = (Estimated tumor volume at Day 52 - Estimated tumor volume at Day 28) of each individual/Estimated tumor volume at Day 28 × 100

**[Table 3]**

| Compound | Change from baseline (%) |
|---|---|
| NYZ-1010 | 68.4 (n=5) |
| lenvatinib+Pembroliz umab | 66.3 (n=5) |
| NYZ-1010+lenvatinib+ Pembrolizumab | -46.8 (n=5) |

### Example 7. Preparation of human CD3ε knock-in BALB/C mice

The human CD3ε knock-in mice of C57BL/6J mouse strain prepared in Example 4 were backcrossed with mice of BALB/C strain to prepare human CD3ε knock-in mice of BALB/C strain. The tail tissues of the resulting offspring were subjected to PCR and sequence analysis. Individual mice having a high rate of homozygous substitution were selected, bred, and used in the subsequent experiments.

Example 8. *In vivo* combinatorial effect of Fc-conjugated type anti-HLA-A2/NY-ESO-anti-CD3 bispecific molecule (NYZ-1010) and anti-mouse PD-1 antibody using human CD3ε knock-in mouse of BALB/C strain

Genes encoding HLA-A2, NY-ESO, and β2M single chain trimer (abbreviated to NY-SCT) were introduced into a mouse large intestine cancer cell line CT26.WT provided by ATCC using a retroviral vector. The resulting CT26 NY-SCT cells express HLA-A2/NY-ESO on the cell membrane. CT26 NY-SCT cells were adjusted to a concentration of 1 × 10⁷ cells/mL with PBS (FUJIFILM Wako Pure Chemical Corp.) and subcutaneously transplanted in an amount of 0.1 mL into each human CD3ε knock-in mouse of BALB/C strain (female, 7 to 9 weeks old) prepared in Example 7 (Day 0). From Day 8, the major axis (mm) and minor axis (mm) of each tumor were measured over time using electronic digital calipers, and an estimated tumor volume was calculated according to the following equation.

Estimated tumor volume (mm³) = Average estimated tumor volume of each individual: Estimated tumor volume (mm3) of each individual = 1/2 × [Tumor major axis] × [Tumor minor axis] × [Tumor minor axis]

On Day 8, grouping based on the tumor volume was carried out at n = 5 to 10 per group. An anti-HLA-A2/NY-ESO-anti-CD3 bispecific molecule (NYZ-1010, 5 mg/kg) was administered into the tail vein of each mouse. An anti-mouse PD-1 antibody (clone RMP1-14, Bio X Cell, 5 mg/kg) was administered intraperitoneally. The administration was carried out on Day 8**.** Tumor growth inhibition (%) was calculated according to the following equation.Tumor growth inhibition (%) = 100 - (Estimated tumor volume of each group/Estimated tumor volume of a vehicle control × 100)

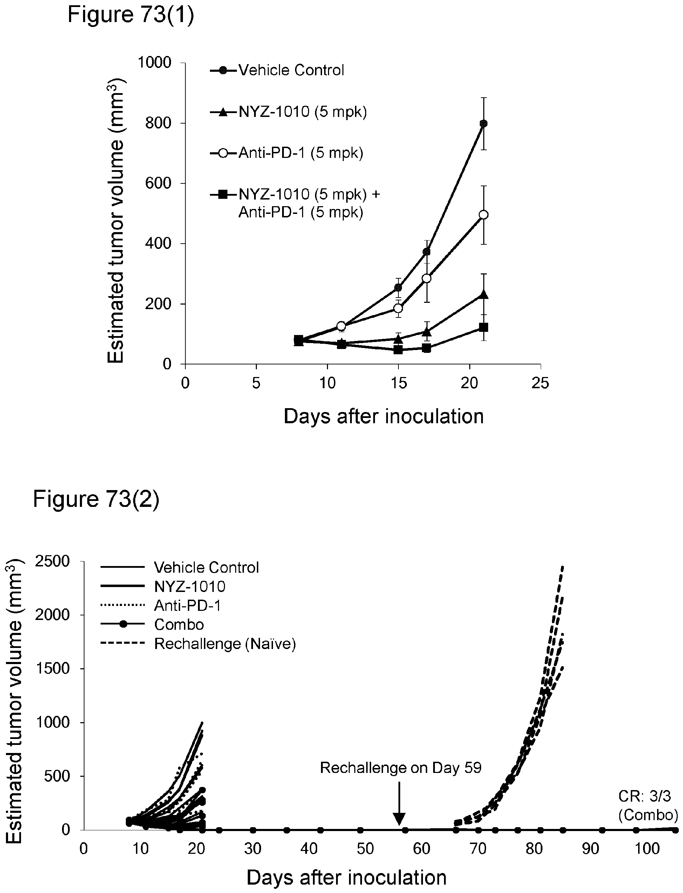

Figure 73(1) shows results of single administration of NYZ-1010, single administration of the anti-mouse PD-1 antibody, and combined administration of NYZ-1010 and the anti-mouse PD-1 antibody. 21 days after tumor inoculation, TGI was 70.8% in the single administration group receiving NYZ-1010 and 38.0% in the single administration group receiving the anti-mouse PD-1 antibody, whereas it was 84.8% in the combination group receiving NYZ-1010 and the anti-mouse PD-1 antibody, indicating that this combination provides an enhanced antitumor effect.

As seen from Figure 73(2), in each single administration group, the tumor remained, and drug efficacy was limited (gray solid line and fine dashed line). In contrast, in the combination group receiving NYZ-1010 and the anti-mouse PD-1 antibody, three out of 10 mice, an estimated tumor volume of 0 mm³ was observed. These mice maintained complete regression (solid line with filled circle). The mice with complete regression were rechallenged with CT26 NY-SCT, or these cells were transplanted to human CD3ε knock-in mice of BALB/C strain which had not been subjected to prior tumor transplantation (hereinafter, referred to as naive mice, female, 7 to 9 weeks old). CT26 NY-SCT cells were adjusted to a concentration of 1 × 10⁷ cells/mL with PBS (FUJIFILM Wako Pure Chemical Corp.) and subcutaneously transplanted in an amount of 0.1 mL (Day 59). In the naive mice, the tumor engrafted and grew (coarse dashed line). In mice with complete regression brought about by the combination of NYZ-1010 and the anti-mouse PD-1 antibody, the tumor did not engraft, and complete regression was maintained (solid line with filled circle), indicating that immune memory against the tumor cells had been established. Example 9. *In vivo* combinatorial effect of Fc-conjugated type anti-HLA-A2/NY-ESO-anti-CD3 bispecific molecule (NYZ-1010) and anti-PD-L1 antibody durvalumab on a human lung adenocarcinoma cell line

A human lung adenocarcinoma cell line NCI-H522 (ATCC) was adjusted to 5 × 10⁷ cells/mL with PBS containing 50% Matrigel (Corning Inc.) and subcutaneously transplanted in an amount of 0.1 mL into NSG mice (female, 4 to 6 weeks old) (Day 0). After approximately 1 week, (Days 6 to 7), the major axis (mm) and minor axis (mm) of each tumor were measured over time using electronic digital calipers, and an estimated tumor volume was calculated according to the following equation.

Estimated tumor volume (mm³) = Average estimated tumor volume of each individual: Estimated tumor volume (mm3) of each individual = 1/2 × [Tumor major axis] × [Tumor minor axis] × [Tumor minor axis]

On Day 24, grouping based on the tumor volume was carried out at n = 5 per group. On Day 25, T cells proliferated by the stimulation of human PBMC with CD3/CD28 Dynabeads (Thermo Fisher Scientific Inc.) were adjusted to a concentration of 5 × 10⁷ cells/mL with PBS and transplanted in an amount of 0.2 mL into the tail vein of each mouse. After three to four hours, an anti-HLA-A2/NY-ESO-anti-CD3 bispecific molecule (NYZ-1010, 0.02 mg/kg) was administered into the tail vein of each mouse. Durvalumab (AstraZeneca K.K., 10 mg/kg) was administered intraperitoneally. The administration was carried out on Days 25 and 32 (NYZ-1010) or on Days 28, 32, 35, and 39 (durvalumab). Tumor growth inhibition (%) and change from baseline (%) were calculated according to the equation given below. Tumor growth inhibition (%) = 100 - (Estimated tumor volume of each group/Estimated tumor volume of a vehicle control × 100)

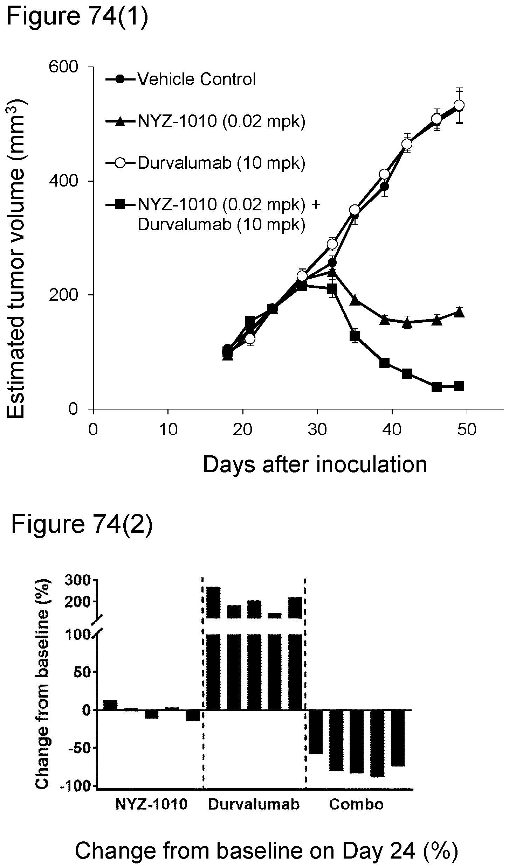

Figure 74(1) shows the results of single administration of NYZ-1010, single administration of durvalumab, and combined administration of NYZ-1010 and durvalumab. 49 days after inoculation, TGI was 67.7% in the single administration group receiving NYZ-1010 and - 0.8% in the single administration group receiving durvalumab, whereas it was 92.5% in the combination group receiving NYZ-1010 and durvalumab, indicating that this combination provides an enhanced an antitumor effect. The percentage change in tumor volume on Day 49 of each individual mouse using the tumor volume on Day 24 as a baseline (change from baseline (%)) was calculated according to the equation given below, and is shown in Figure 74(2). The average change from baseline (%) in each group is also described in Table 4. A strong combinatorial effect of tumor regression was observed with NYZ-1010 and durvalumab Change from baseline (%) = (Estimated tumor volume on Day 49 - Estimated tumor volume on Day 24) of each individual/Estimated tumor volume on Day 24 × 100

**[Table 4]**

| Compound | Change from baseline (%) |
|---|---|
| NYZ-1010 | -3.1 (n=5) |
| Durvalumab | 204.7 (n=5) |
| NYZ-1010+Durvalumab | -76.9 (n=5) |

Example 10. *In vivo* combinatorial effect of Fc-conjugated type anti-HLA-A2/NY-ESO-anti-CD3 bispecific molecule (NYZ-1010) and anti-mouse PD-L1 antibody using human CD3ε knock-in mouse of BALB/C strain

CT26 NY-SCT cells prepared in Example 8 were adjusted to a concentration of 1 × 10⁷ cells/mL with PBS (FUJIFILM Wako Pure Chemical Corp.) and subcutaneously transplanted in an amount of 0.1 mL into human CD3ε knock-in mouse of BALB/C strain (female, 6 or more_weeks old) as prepared in Example 7 (Day 0). From Day 8, the major axis (mm) and minor axis (mm) of tumor were measured over time using electronic digital calipers, and an estimated tumor volume was calculated according to the following expression.

Estimated tumor volume (mm³) = Average estimated tumor volume of each individual: Estimated tumor volume (mm3) of each individual = 1/2 × [Tumor major axis] × [Tumor minor axis] × [Tumor minor axis]

On Day 8, grouping based on the tumor volume was carried out at n = 5 to 10 per group. An anti-HLA-A2/NY-ESO-anti-CD3 bispecific molecule (NYZ-1010, 5 mg/kg) was administered into the tail vein of each mouse. An anti-mouse PD-L1 antibody (clone 10F.9G2, Bio X Cell, 5 mg/kg) was administered intraperitoneally. The administration was carried out on Day 8. Tumor growth inhibition (%) was calculated according to the following equation. Tumor growth inhibition (%) = 100 - (Estimated tumor volume of each group/Estimated tumor volume of a vehicle control × 100)

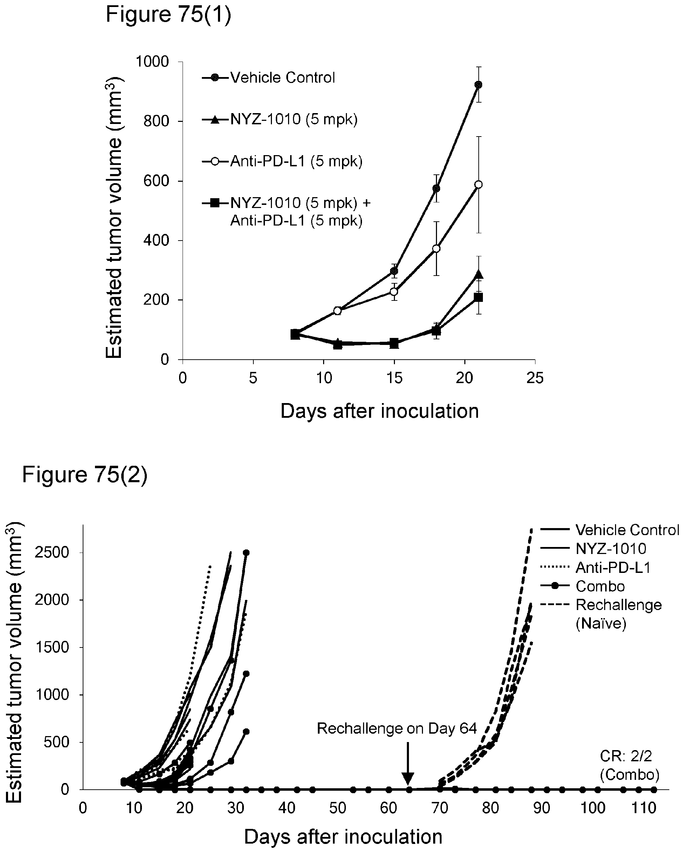

Figure 75(1) shows results of single administration of NYZ-1010, single administration of the anti-mouse PD-L1 antibody, and combined administration of NYZ-1010 and the anti-mouse PD-L1 antibody. 21 days after inoculation, TGI was 68.7% in the single administration group receiving NYZ-1010 and 36.4% in the single administration group receiving the anti-mouse PD-L1 antibody, whereas it was 77.4% in the combination group receiving NYZ-1010 and the anti-mouse PD-L1 antibody, indicating that this combination provides an enhanced an antitumor effect.

As seen from Figure 75(2), in each single administration group, the tumor remained, and drug efficacy was limited (gray solid line and fine dashed line). In contrast, in the combination group receiving NYZ-1010 and the anti-mouse PD-L1 antibody, an estimated tumor volume of 0 mm³ was observed in 2 out of 10 mice. These mice maintained complete regression (solid line with filled circle). The mice with complete regression were rechallenged with CT26.WT cells harboring only a retroviral vector (hereinafter, referred to as CT26 Mock), or these cells were transplanted into naive mice (female, 15 to 17 weeks old). CT26 Mock cells were adjusted to a concentration of 1 × 10⁷ cells/mL with PBS (FUJIFILM Wako Pure Chemical Corp.) and subcutaneously transplanted in an amount of 0.1 mL (Day 64). In the naive mice, the tumor engrafted and grew (coarse dashed line). In the mice with complete regression brought about by NYZ-1010 and the anti-mouse PD-L1 antibody, the tumor did not engraft, and complete regression was maintained (solid line with filled circle), indicating that immune memory against the tumor cells had been established.

Example 11. *In vivo* combinatorial effect of Fc-conjugated type anti-HLA-A2/NY-ESO-anti-CD3 bispecific molecule (NYZ-1010) and anti-mouse CTLA-4 antibody using human CD3ε knock-in mouse of BALB/C strain

CT26 NY-SCT cells prepared in Example 8 were adjusted to a concentration 1 × 10⁷ cells/mL with PBS (FUJIFILM Wako Pure Chemical Corp.) and subcutaneously transplanted in an amount of 0.1 mL into human CD3ε knock-in mouse of BALB/C strain (female, 7 to 9 weeks old) prepared in Example 7 (Day 0). From Day 8, the major axis (mm) and minor axis (mm) of each tumor were measured over time using electronic digital calipers, and an estimated tumor volume was calculated according to the following equation.

Estimated tumor volume (mm³) = Average estimated tumor volume of each individual: Estimated tumor volume (mm3) of each individual = 1/2 × [Tumor major axis] × [Tumor minor axis] × [Tumor minor axis]

On Day 8, grouping based on the tumor volume was carried out at n = 5 to 10 per group. An anti-HLA-A2/NY-ESO-anti-CD3 bispecific molecule (NYZ-1010, 5 mg/kg) was administered into the tail vein of each ouse. An anti-mouse CTLA-4 antibody (clone 9D9, Bio X Cell, 5 mg/kg) was administered intraperitoneally. The administration was carried out on Day 8. Tumor growth inhibition (%) was calculated according to the following equation. Tumor growth inhibition (%) = 100 - (Estimated tumor volume of each group/Estimated tumor volume of a vehicle control × 100)

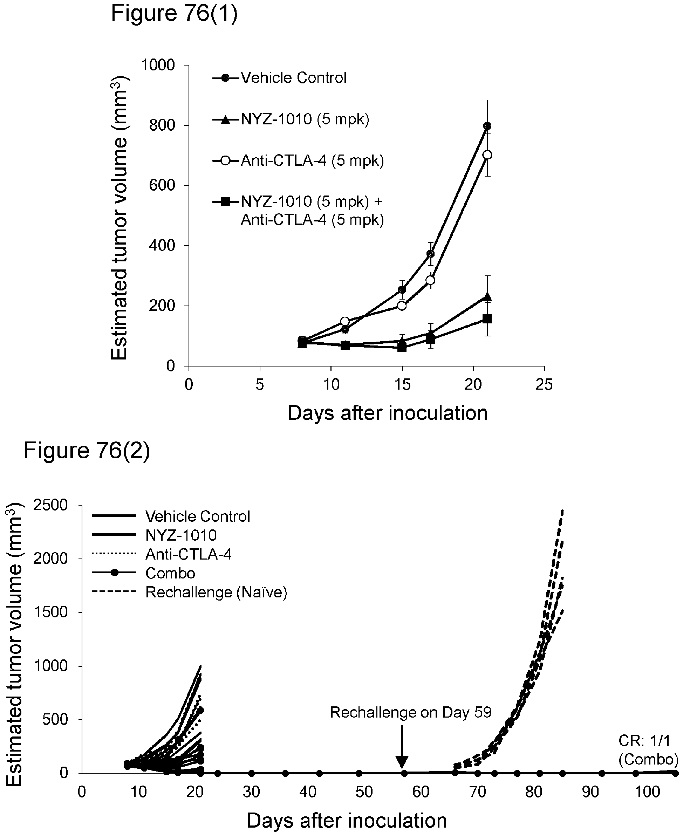

Figure 76(1) shows the results of single administration of NYZ-1010, single administration of the anti-mouse CTLA-4 antibody, and combined administration of NYZ-1010 and the anti-mouse CTLA-4 antibody. 21 days after inoculation, TGI was 70.8% in the single administration group receiving NYZ-1010 and 12.1% in the single administration group receiving the anti-mouse CTLA-4 antibody, whereas it was 80.4% in the combination group receiving NYZ-1010 and the anti-mouse CTLA-4 antibody, indicating that this combination provides an enhanced an antitumor effect.

As seen from Figure 76(2), in each single administration group, the tumor remained, and drug efficacy was limited (gray solid line and fine dashed line). In contrast, in the combination group receiving NYZ-1010 and the anti-mouse CTLA-4 antibody, an estimated tumor volume of 0 mm³ was observed in 1 out of 10 mice. This mouse maintained complete regression (solid line with filled circle). The mouse with complete regression was rechallenged with CT26 NY-SCT cells, or these cells were transplanted into naive mice (female, 7 to 9 weeks old). CT26 NY-SCT cells were adjusted to a concentration of 1 × 10⁷ cells/mL with PBS (FUJIFILM Wako Pure Chemical Corp.) and subcutaneously transplanted in an amount of 0.1 mL (Day 59). In the naive mice, the tumor engrafted and grew (coarse dashed line). In the mice with complete regression brought about by NYZ-1010 and the anti-mouse CTLA-4 antibody, the tumor did not engraft, and complete regression was maintained (solid line with filled circle), indicating that immune memory against the tumor cells had been established.

Example 12. *In vivo* combinatorial effect of Fc-conjugated type anti-HLA-A2/NY-ESO-anti-CD3 bispecific molecule (NYZ-1010) and anti-mouse TIGIT antibody using human CD3ε knock-in mouse

CT26 NY-SCT cells prepared in Example 8 were adjusted to a concentration of 1 × 10⁷ cells/mL with PBS (FUJIFILM Wako Pure Chemical Corp.) and subcutaneously transplanted in an amount of 0.1 mL into human CD3ε knock-in mouse of BALB/C strain (female, 7 to 9 weeks old) prepared in Example 7 (Day 0). From Day 8, the major axis (mm) and minor axis (mm) of each tumor were measured over time using electronic digital calipers, and an estimated tumor volume was calculated according to the following equation.

Estimated tumor volume (mm³) = Average estimated tumor volume of each individual: Estimated tumor volume (mm3) of each individual = 1/2 × [Tumor major axis] × [Tumor minor axis] × [Tumor minor axis]

On Day 8, grouping based on the tumor volume was carried out at n = 5 to 10 per group. An anti-HLA-A2/NY-ESO-anti-CD3 bispecific molecule (NYZ-1010, 5 mg/kg) was administered into the tail vein of each mouse. An anti-mouse TIGIT antibody (clone 1B4, Absolute Antibody, 5 mg/kg) was administered intraperitoneally. The administration was carried out on Day 8. Tumor growth inhibition (%) was calculated according to the following equation. Tumor growth inhibition (%) = 100 - (Estimated tumor volume of each group/Estimated tumor volume of a vehicle control × 100)

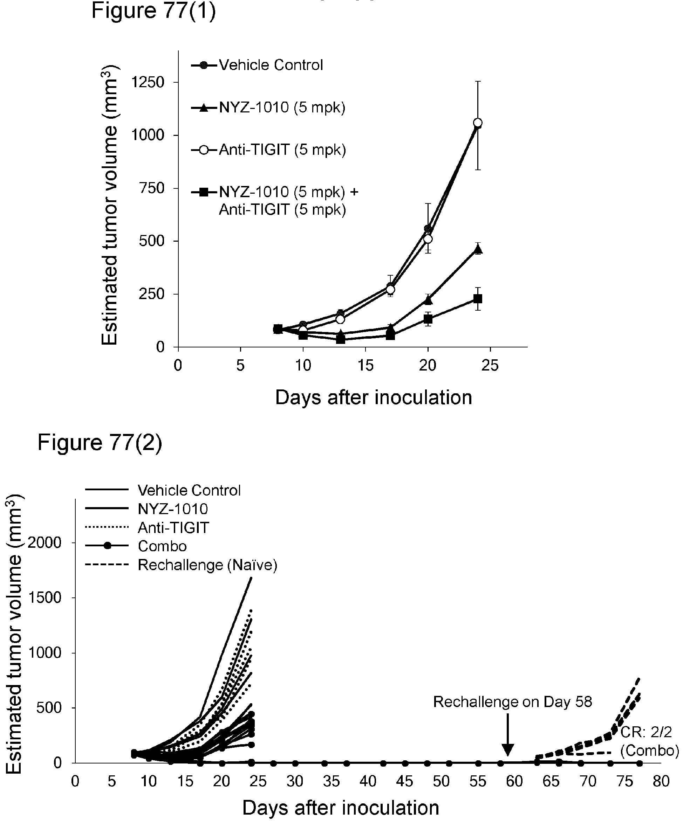

Figure 77(1) shows the results od single administration of NYZ-1010, single administration of the anti-mouse TIGIT antibody, and combined administration of NYZ-1010 and the anti-mouse TIGIT antibody. 24 days after tumor inoculation, TGI was 55.5% in the single administration group receiving NYZ-1010 and -1.2% in the single administration group receiving the anti-mouse TIGIT antibody, whereas it was 78.3% in the combination group receiving NYZ-1010 and the anti-mouse TIGIT antibody, indicating that this combination provides an enhanced an antitumor effect.

As seen from Figure 77(2), in each single administration group, the tumor remained, and drug efficacy was limited (gray solid line and fine dashed line). In contrast, in the combination group, an estimated tumor volume of 0 mm³ was observed in two out of ten mice. These mice maintained complete regression (solid line with filled circle). The mice with complete regression were rechallenged with CT26 NY-SCT cells, or these cells were transplanted into naive mice (female, 15 to 17 weeks old). CT26 NY-SCT cells were adjusted to a concentration of 1 × 10⁷ cells/mL with PBS (FUJIFILM Wako Pure Chemical Corp.) and subcutaneously transplanted in an amount of 0.1 mL (Day 58). In the naive mice, the tumor engrafted and grew (coarse dashed line). In the mice with complete regression brought about by NYZ-1010 and the anti-mouse TIGIT antibody, the tumor did not engraft, and complete regression was maintained (solid line with filled circle), indicating that immune memory against the tumor cells had been established.

Example 13. *In vivo* combinatorial effect of Fc-conjugated type anti-HLA-A2/NY-ESO-anti-CD3 bispecific molecule (NYZ-1010) and carboplatin on a human squamous cell lung cancer cell line

A human squamous cell lung cancer cell line NCI-H1703 (ATCC) was adjusted to a concentration of 6 × 10⁷ cells/mL with PBS containing 50% Matrigel (Corning Inc.) and subcutaneously transplanted in an amount of 0.1 mL into NSG mice (female, 4 to 6 weeks old) (Day 0). After approximately 1 week, (Days 6 to 7), the major axis (mm) and minor axis (mm) of each tumor were measured over time using electronic digital calipers, and an estimated tumor volume was calculated according to the following equation.

Estimated tumor volume (mm³) = Average estimated tumor volume of each individual: Estimated tumor volume (mm3) of each individual = 1/2 × [Tumor major axis] × [Tumor minor axis] × [Tumor minor axis]

On Day 14, grouping based on the tumor volume was carried out at n = 5 per group. T cells proliferated by the stimulation of human PBMC with CD3/CD28 Dynabeads (Thermo Fisher Scientific Inc.) were adjusted to a concentration of 5 × 10⁷ cells/mL with PBS and transplanted in an amount of 0.2 mL into the tail vein of each mouse. After three to four hours, an anti-HLA-A2/NY-ESO-anti-CD3 bispecific molecule (NYZ-1010, 0.02 mg/kg) was administered into the tail vein of each mouse. Carboplatin (Tokyo Chemical Industry Co., Ltd., 50 mg/kg) was administered intraperitoneally. The administration was carried out on Days 14 and 21. Tumor growth inhibition (%)as calculated according to the equation given below. Tumor growth inhibition (%) = 100 - (Estimated tumor volume of each group/Estimated tumor volume of a vehicle control × 100)

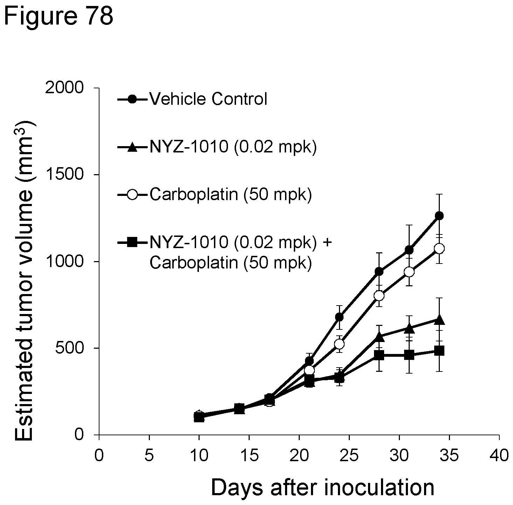

Figure 78 shows the results of single administration of NYZ-1010, single administration of carboplatin, and combined administration of NYZ-1010 and carboplatin. 34 days after inoculation, TGI was 47.2% in the single administration group receiving NYZ-1010 and 15.0% in the single administration group receiving carboplatin, whereas it was 61.6% in the combination group receiving NYZ-1010 and carboplatin, indicating that this combination provides an enhanced an antitumor effect.

Example 14. *In vivo* combinatorial effect of Fc-conjugated type anti-HLA-A2/NY-ESO-anti-CD3 bispecific molecule (NYZ-1010) and sacituzumab govitecan-hziy on a human esophageal cancer cell line

A human esophageal cancer cell line OE-19 (DSMZ) was adjusted to a concentration of 5 × 10⁷ cells/mL with PBS containing 50% Matrigel (Corning Inc.) and subcutaneously transplanted in an amount of 0.1 mL into NSG mice (female, 4 to 6 weeks old) (Day 0). After approximately 1 week, (Days 6 to 7), the major axis (mm) and minor axis (mm) of each tumor were measured over time using electronic digital calipers, and an estimated tumor volume was calculated according to the following equation.

Estimated tumor volume (mm³) = Average estimated tumor volume of each individual: Estimated tumor volume (mm3) of each individual = 1/2 × [Tumor major axis] × [Tumor minor axis] × [Tumor minor axis]

On Day 6, grouping based on the tumor volume was carried out at n = 5 per group. Sacituzumab govitecan-hziy (25 mg/kg) was administered into the tail vein of each mouse. On Day 7, T cells proliferated by the stimulation of human PBMC with CD3/CD28 Dynabeads (Thermo Fisher Scientific Inc.) were adjusted to a concentration of 5 × 10⁷ cells/mL with PBS and transplanted in an amount of 0.2 mL into the tail vein of each mouse. After three to four hours, an anti-HLA-A2/NY-ESO-anti-CD3 bispecific molecule (NYZ-1010, 0.01 mg/kg) was administered into the tail vein of each mouse. The administration was carried out on Day 7 (NYZ-1010) or on days 6 and 13 (sacituzumab govitecan-hziy). Tumor growth inhibition (%) and change from baseline(%) were calculated according to the expressions given below. Tumor growth inhibition (%) = 100 - (Estimated tumor volume of each group/Estimated tumor volume of a vehicle control × 100)

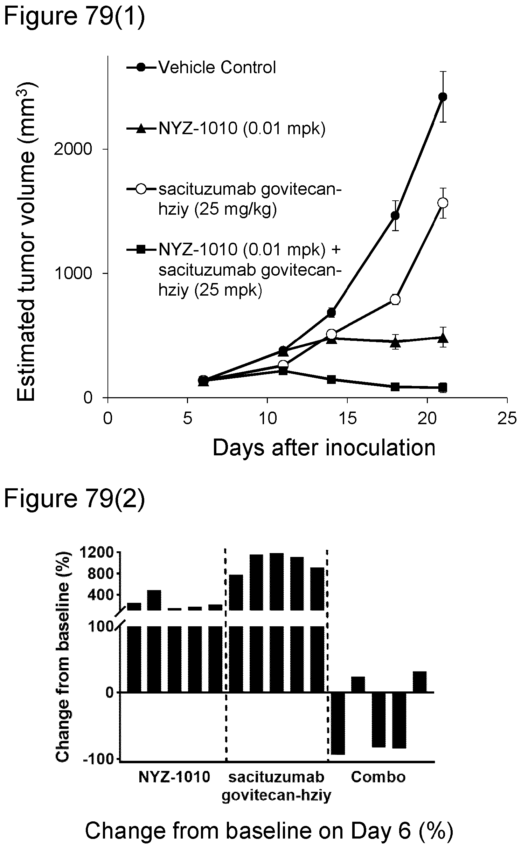

Figure 79(1) shows the results of single administration of NYZ-1010, single administration of sacituzumab govitecan-hziy, and combined administration of NYZ-1010 and sacituzumab govitecan-hziy. 21 days after inoculation, TGI was 79.9% in the single administration group receiving NYZ-1010 and 35.4% in the single administration group receiving sacituzumab govitecan-hziy, whereas it was 96.7% in the combination group receiving NYZ-1010 and sacituzumab govitecan-hziy, indicating that this combination provides an enhanced an antitumor effect. The percentage change in tumor volume on Day 21 in each individual mouse using the tumor volume on Day 6 as a baseline (change from baseline (%)) was calculated according to the equation given below, and is shown in Figure 79(2). The average change from baseline (%) in each group is also described in Table 5. A combinatorial effect of tumor regression by NYZ-1010 and sacituzumab govitecan-hziy was observed. Change from baseline (%) = (Estimated tumor volume at Day 21 - Estimated tumor volume at Day 6) / Estimated tumor volume at Day 6 × 100

**[Table 5]**

| Compound | Change from baseline (%) |
|---|---|
| NYZ-1010 | 252.7 (n=5) |
| sacituzumab goviteca n-hziy | 1027.8 (n=5) |
| NYZ-1010+sacituzumab govitecan-hziy | -40.9 (n=5) |

Example 15. *In vivo* combinatorial effect of Fc-conjugated type anti-HLA-A2/NY-ESO-anti-CD3 bispecific molecule (NYZ-1010) and doxorubicin on a human synovial sarcoma cell line A human synovial sarcoma cell line SW982 (ATCC) was adjusted to a concentration of 5 × 10⁷ cells/mL with PBS containing 50% Matrigel (Corning Inc.) and subcutaneously transplanted in an amount of 0.1 mL into NSG mice (female, 4 to 6 weeks old) (Day 0). After approximately 1 week (Days 6 to 7), the major axis (mm) and minor axis (mm) of tumor were measured over time using electronic digital calipers, and an estimated tumor volume was calculated according to the following equation.

Estimated tumor volume (mm³) = Average estimated tumor volume of each individual: Estimated tumor volume (mm3) of each individual = 1/2 × [Tumor major axis] × [Tumor minor axis] × [Tumor minor axis]

On Day 40, grouping based on the tumor volume was carried out at n = 5 per group. Doxorubicin (Sandoz K.K., 2.5 mg/kg) was administered into the tail vein of each mouse. On Day 41, T cells proliferated by the stimulation of human PBMC with CD3/CD28 Dynabeads (Thermo Fisher Scientific Inc.) were adjusted to a concentration of 5 × 10⁷ cells/mL with PBS and transplanted in an amount of 0.2 mL into the tail vein of each mouse. After three to four hours, an anti-HLA-A2/NY-ESO-anti-CD3 bispecific molecule (NYZ-1010, 0.01 mg/kg) was administered into the tail vein of each mouse. The administration was carried out on Day 41 (NYZ-1010) or on Days 40 and 47 (doxorubicin). Tumor growth inhibition (%) and change from baseline(%) were calculated according to the expressions given below. Tumor growth inhibition (%) = 100 - (Estimated tumor volume of each group/Estimated tumor volume of a vehicle control × 100)

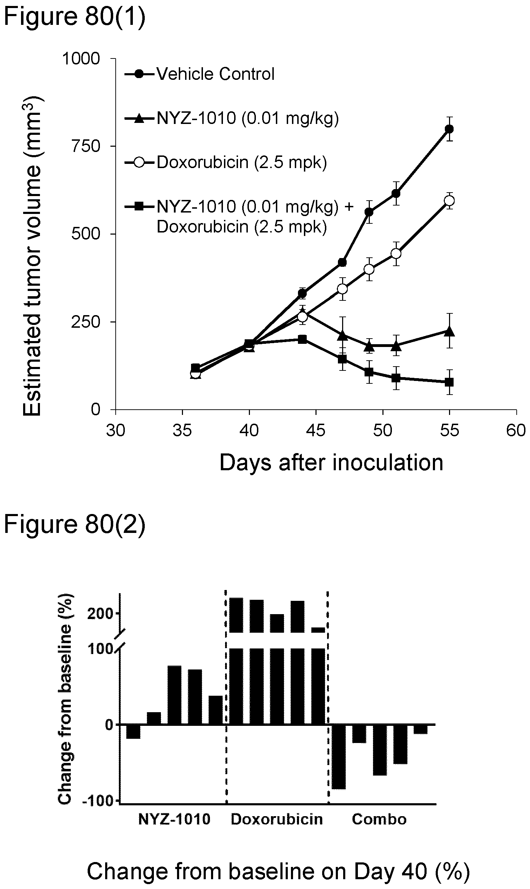

Figure 80(1) shows the results of single administration of NYZ-1010, single administration of doxorubicin, and combined administration of NYZ-1010 and doxorubicin. 55 days after inoculation, TGI was 71.8% in the single administration group receiving NYZ-1010 and 25.6% in the single administration group receiving doxorubicin, whereas it was 90.2% in the combination group receiving NYZ-1010 and doxorubicin, indicating that this combination provides an enhanced an antitumor effect. The percentage change in tumor volume on Day 55 in each individual mouse using the tumor volume on Day 40 as a baseline (change from baseline (%)) was calculated according to the equation given below, and is shown in Figure 80(2). The average change from baseline (%) in each group is also described in Table 6. A combinatorial effect of tumor regression by NYZ-1010 and doxorubicin was observed. Change from baseline (%) = (Estimated tumor volume at Day 55 - Estimated tumor volume at Day 40)/Estimated tumor volume at Day 40 × 100

**[Table 6]**

| Compound | Change from baseline (%) |
|---|---|
| NYZ-1010 | 37.1 (n=5) |
| Doxorubicin | 214.1 (n=5) |
| NYZ-1010+Doxorubicin | -48.2 (n=5) |

Example 16. *In vivo* combinatorial effect of Fc-conjugated type anti-HLA-A2/NY-ESO-anti-CD3 bispecific molecule (NYZ-1010) and decitabine on a human squamous cell lung cancer cell line

A human squamous cell lung cancer cell line NCI-H1703 (ATCC) was adjusted to a concentration of 6 × 10⁷ cells/mL with PBS containing 50% Matrigel (Corning Inc.) and subcutaneously transplanted in an amount of 0.1 mL into NSG mice (female, 4 to 6 weeks old) (Day 0). After approximately 1 week (Days 6 to 7), the major axis (mm) and minor axis (mm) of each tumor were measured over time using electronic digital calipers, and an estimated tumor volume was calculated according to the following equation.

Estimated tumor volume (mm³) = Average estimated tumor volume of each individual: Estimated tumor volume (mm3) of each individual = 1/2 × [Tumor major axis] × [Tumor minor axis] × [Tumor minor axis]

On Day 7, grouping based on the tumor volume was carried out at n = 5 per group. Decitabine (Tokyo Chemical Industry Co., Ltd., 3 mg/kg) was administered into the tail vein of each mouse. On Day 14, T cells proliferated by the stimulation of human PBMC with CD3/CD28 Dynabeads (Thermo Fisher Scientific Inc.) were adjusted to a concentration of 5 × 10⁷ cells/mL with PBS and transplanted in an amount of 0.2 mL into the tail vein of each mouse. After three to four hours, an anti-HLA-A2/NY-ESO-anti-CD3 bispecific molecule (NYZ-1010, 0.02 mg/kg) was administered into the tail vein of each mouse. The administration was carried out on Days 14 and 21 (NYZ-1010) or on Days 7, 9, and 11 (decitabine). Tumor growth inhibition (%) and change from baseline(%) were calculated according to the equations given below. Tumor growth inhibition (%) = 100 - (Estimated tumor volume of each group/Estimated tumor volume of a vehicle control × 100) Change from baseline (%) = (Estimated tumor volume at Day 32 - Estimated tumor volume at Day 7)/Estimated tumor volume at Day 7 × 100

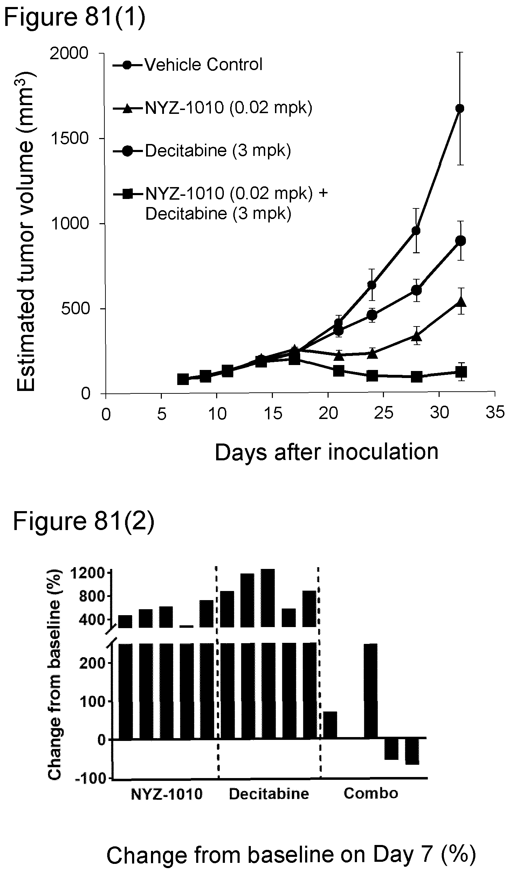

Figure 81(1) shows the results of single administration of NYZ-1010, single administration of decitabine, and combined administration of NYZ-1010 and decitabine. 32 days after inoculation, TGI was 68.0% in the single administration group receiving NYZ-1010 and 46.7% in the single administration group receiving decitabine, whereas it was 93.0% in the combination group receiving NYZ-1010 and decitabine, indicating that this combination provides an enhanced an antitumor effect. The percentage change in tumor volume on Day 32 of each individual mouse using the tumor volume on Day 7 as a baseline (change from baseline (%)) was calculated according to the equation given above, and is shown in Figure 81(2). The average change from baseline (%) in each group is also described in Table 7.

**[Table 7]**

| Compound | Change from baseline (%) |
|---|---|
| NYZ-1010 | 531.3 (n=5) |
| Decitabine | 947.6 (n=5) |
| NYZ-1010+Decitabine | 38.0 (n=5) |

### Industrial Applicability

The combination of an antibody and another agent provided by the present invention enables various cancers to be treated or prevented.

### Free Text of Sequence Listing

SEQ ID NO: 1: Amino acid sequence of CDRH1 contained in NYA-0001
SEQ ID NO: 2: Amino acid sequence of CDRH2 contained in NYA-0001
SEQ ID NO: 3: Missing (SEQ ID NO: 3 in Japanese Patent Application No. 2022-041253 filed in March 16, 2022 represented the amino acid sequence of CDRH3 contained in NYA-0001)
SEQ ID NO: 4: Amino acid sequence of CDRL1 contained in NYA-0001
SEQ ID NO: 5: Amino acid sequence of CDRL2 contained in NYA-0001
SEQ ID NO: 6: Amino acid sequence of CDRL3 contained in NYA-0001
SEQ ID NO: 7: Amino acid sequence of the heavy chain CDRH1 of C3E-7085
SEQ ID NO: 8: Amino acid sequence of the heavy chain CDRH2 of C3E-7085
SEQ ID NO: 9: Amino acid sequence of the heavy chain CDRH3 of C3E-7085
SEQ ID NO: 10: Amino acid sequence of the light chain CDRL1 of C3E-7085
SEQ ID NO: 11: Missing (SEQ ID NO: 11 in the priority application filed in March 16, 2022 represented the amino acid sequence of the light chain CDRL2 of C3E-7085)
SEQ ID NO: 12: Amino acid sequence of the light chain CDRL3 of C3E-7085
SEQ ID NO: 13: Amino acid sequence of a heavy chain variable region of NYA-0001
SEQ ID NO: 14: Amino acid sequence of a light chain variable region of NYA-0001
SEQ ID NO: 15: Amino acid sequence of a heavy chain variable region of NYA-0082
SEQ ID NO: 16: Amino acid sequence of a light chain variable region of NYA-0082
SEQ ID NO: 17: Full length amino acid sequence of NYA-1163
SEQ ID NO: 18: Full length amino acid sequence of NYA-2023
SEQ ID NO: 19: Full length amino acid sequence of NYA-2027
SEQ ID NO: 20: Full length amino acid sequence of NYA-1143
SEQ ID NO: 21: Full length amino acid sequence of NYA-2143
SEQ ID NO: 22: Full length amino acid sequence of NYA-2035
SEQ ID NO: 23: Amino acid sequence of NYA-1143-VL01 SEQ ID NO: 24: Full length amino acid sequence of NYA-2044
SEQ ID NO: 25: Full length amino acid sequence of NYA-2045
SEQ ID NO: 26: Full length amino acid sequence of NYA-2047
SEQ ID NO: 27: Full length amino acid sequence of NYA-2048
SEQ ID NO: 28: Full length amino acid sequence of NYA-2060
SEQ ID NO: 29: Full length amino acid sequence of NYA-2061
SEQ ID NO: 30: Full length amino acid sequence of NYA-0001
SEQ ID NO: 31: Full length amino acid sequence of HC1 SEQ ID NO: 32: Full length amino acid sequence of NYF-0016-HC2
SEQ ID NO: 33: Full length amino acid sequence of NYF-0019-HC2
SEQ ID NO: 34: Full length amino acid sequence of NYF-0022-HC2
SEQ ID NO: 35: Full length amino acid sequence of NYF-0023-HC2
SEQ ID NO: 36: Full length amino acid sequence of NYF-0027-HC2
SEQ ID NO: 37: Full length amino acid sequence of NYF-0035-HC2
SEQ ID NO: 38: Full length amino acid sequence of NYF-0044-HC2
SEQ ID NO: 39: Full length amino acid sequence of NYF-0045-HC2
SEQ ID NO: 40: Full length amino acid sequence of NYF-0047-HC2
SEQ ID NO: 41: Full length amino acid sequence of NYF-0048-HC2
SEQ ID NO: 42: Full length amino acid sequence of NYF-0060-HC2
SEQ ID NO: 43: Full length amino acid sequence of NYF-0061-HC2
SEQ ID NO: 44: Full length amino acid sequence of NYA-0001-Fab-HC1-κ delete
SEQ ID NO: 45: Full length amino acid sequence of NYA-0001-LC
SEQ ID NO: 46: Amino acid sequence of C3E-7034
SEQ ID NO: 47: Amino acid sequence of C3E-7036
SEQ ID NO: 48: Amino acid sequence of C3E-7085
SEQ ID NO: 49: Amino acid sequence of C3E-7088
SEQ ID NO: 50: Amino acid sequence of C3E-7093
SEQ ID NO: 51: Amino acid sequence of C3E-7078
SEQ ID NO: 52: Full length amino acid sequence of NYF-0014-HC2
SEQ ID NO: 53: Full length amino acid sequence of NYF-0082-HC2
SEQ ID NO: 54: Full length amino acid sequence of NYF-0083-HC2
SEQ ID NO: 55: Full length amino acid sequence of NYZ-0082-HC2
SEQ ID NO: 56: Full length amino acid sequence of NYZ-0083-HC2
SEQ ID NO: 57: Full length amino acid sequence of NYZ-1010-HC2
SEQ ID NO: 58: Full length amino acid sequence of C3E-7085-LC
SEQ ID NO: 59: Full-length amino acid sequence of C3E-7085 scFv
SEQ ID NO: 60: Full length amino acid sequence of NYZ-1007-HC2
SEQ ID NO: 61: Full length amino acid sequence of NYZ-1017-HC2
SEQ ID NO: 62: Full-length amino acid sequence of C3E-7096
SEQ ID NO: 63: Full-length amino acid sequence of C3E-7097
SEQ ID NO: 64: Full-length amino acid sequence of C3E-7098
SEQ ID NO: 65: Full-length amino acid sequence of C3E-7099
SEQ ID NO: 66: Amino acid sequence of NY-ESO
SEQ ID NO: 67: Amino acid sequence of a truncated form of HLA-A*0201 (GenBank: ASA47534.1)
SEQ ID NO: 68: Amino acid sequence of β2-microglobulin SEQ ID NO: 69: Amino acid sequence of NYA-1143-VH02
SEQ ID NO: 70: Amino acid sequence of NYA-1143-VH03
SEQ ID NO: 71: Full length amino acid sequence of NYA-1154

## Claims

1. A pharmaceutical composition for the treatment and/or prevention of a cancer, comprising the following multispecific antibody [i], the pharmaceutical composition being used in combination with the following compound [ii]:
[i]
a multispecific antibody comprising
an antibody that binds to HLA/NY-ESO or an antigen-binding fragment thereof, comprising
a heavy chain CDRH1 consisting of the amino acid sequence represented by SEQ ID NO: 1,
a heavy chain CDRH2 consisting of the amino acid sequence represented by SEQ ID NO: 2,
a heavy chain CDRH3 consisting of the amino acid sequence represented by SEQ ID NO: 3, or a heavy chain CDRH3 consisting of the amino acid sequence represented by SEQ ID NO: 3, in which an amino acid at position 6 is N,
a light chain CDRL1 consisting of the amino acid sequence represented by SEQ ID NO: 4, or a light chain CDRL1 consisting of the amino acid sequence represented by SEQ ID NO: 4 in which the amino acid at position 7 is W and/or the amino acid at position 8 is K,
a light chain CDRL2 consisting of the amino acid sequence represented by Asp Asn Asn (the 5th sequence from the top in Figure 11; SEQ ID NO: 5 in Figure 82), and
a light chain CDRL3 consisting of the amino acid sequence represented by SEQ ID NO: 6, or a light chain CDRL3 consisting of the amino acid sequence represented by SEQ ID NO: 6 in which the amino acid at position 2 is A or S, and
an antibody that binds to CD3 or an antigen-binding fragment thereof, comprising
a heavy chain CDRH1 consisting of the amino acid sequence represented by SEQ ID NO: 7,
a heavy chain CDRH2 consisting of the amino acid sequence represented by SEQ ID NO: 8, or heavy chain CDRH2 consisting of the amino acid sequence represented by SEQ ID NO: 8 in which the amino acid at position 3 is N or S,
a heavy chain CDRH3 consisting of the amino acid sequence represented by SEQ ID NO: 9,
a light chain CDRL1 consisting of the amino acid sequence represented by SEQ ID NO: 10,
a light chain CDRL2 consisting of the amino acid sequence represented by Arg Asp Asp (the 5th sequence from the top in Figure 12: SEQ ID NO: 11 in Figure 82), or a light chain CDRL2 consisting of the amino acid sequence represented by Arg Asp Asp (the 5th sequence from the top in Figure 12: SEQ ID NO: 11 in Figure 82) in which the amino acid at position 2 is G, Q, N, S, or A, and
a light chain CDRL3 consisting of the amino acid sequence represented by SEQ ID NO: 12;
[ii]
a compound that inhibits an immune checkpoint molecule or a chemotherapeutic.

2. The pharmaceutical composition according to claim 1, wherein the multispecific antibody is a bispecific antibody.

3. The pharmaceutical composition according to claim 1 or 2, wherein the antibody that binds to HLA/NY-ESO or the antigen-binding fragment thereof comprises one or two or more sets of CDRH1 to CDRH3 and CDRL1 to CDRL3 selected from the group consisting of the following sets (i) to (v):
(i)
a heavy chain CDRH1 consisting of the amino acid sequence represented by SEQ ID NO: 1,
a heavy chain CDRH2 consisting of the amino acid sequence represented by SEQ ID NO: 2,
a heavy chain CDRH3 consisting of the amino acid sequence represented by SEQ ID NO: 3,
a light chain CDRL1 consisting of the amino acid sequence represented by SEQ ID NO: 4,
a light chain CDRL2 consisting of the amino acid sequence represented by Asp Asn Asn (the 5th sequence from the top in Figure 11; SEQ ID NO: 5 in Figure 82), and
a light chain CDRL3 consisting of the amino acid sequence represented by SEQ ID NO: 6,
(ii)
a heavy chain CDRH1 consisting of the amino acid sequence represented by SEQ ID NO: 1,
a heavy chain CDRH2 consisting of the amino acid sequence represented by SEQ ID NO: 2,
a heavy chain CDRH3 consisting of the amino acid sequence represented by SEQ ID NO: 3,
a light chain CDRL1 consisting of the amino acid sequence represented by SEQ ID NO: 4 in which the amino acid at position 7 is W,
a light chain CDRL2 consisting of the amino acid sequence represented by Asp Asn Asn (the 5th sequence from the top in Figure 11; SEQ ID NO: 5 in Figure 82), and
a light chain CDRL3 consisting of the amino acid sequence represented by SEQ ID NO: 6,
(iii)
a heavy chain CDRH1 consisting of the amino acid sequence represented by SEQ ID NO: 1,
a heavy chain CDRH2 consisting of the amino acid sequence represented by SEQ ID NO: 2,
a heavy chain CDRH3 consisting of the amino acid sequence represented by SEQ ID NO: 3 in which the amino acid at position 6 is N,
a light chain CDRL1 consisting of the amino acid sequence represented by SEQ ID NO: 4,
a light chain CDRL2 consisting of the amino acid sequence represented by Asp Asn Asn (the 5th sequence from the top in Figure 11; SEQ ID NO: 5 in Figure 82), and
a light chain CDRL3 consisting of the amino acid sequence represented by SEQ ID NO: 6 in which the amino acid at position 2 is A,
(iv)
a heavy chain CDRH1 consisting of the amino acid sequence represented by SEQ ID NO: 1,
a heavy chain CDRH2 consisting of the amino acid sequence represented by SEQ ID NO: 2,
a heavy chain CDRH3 consisting of the amino acid sequence represented by SEQ ID NO: 3,
a light chain CDRL1 consisting of the amino acid sequence represented by SEQ ID NO: 4,
a light chain CDRL2 consisting of the amino acid sequence represented by Asp Asn Asn (the 5th sequence from the top in Figure 11; SEQ ID NO: 5 in Figure 82), and
a light chain CDRL3 consisting of the amino acid sequence represented by SEQ ID NO: 6 in which the amino acid at position 2 is S, and
(v)
a heavy chain CDRH1 consisting of the amino acid sequence represented by SEQ ID NO: 1,
a heavy chain CDRH2 consisting of the amino acid sequence represented by SEQ ID NO: 2,
a heavy chain CDRH3 consisting of the amino acid sequence represented by SEQ ID NO: 3,
a light chain CDRL1 consisting of the amino acid sequence represented by SEQ ID NO: 4 in which the amino acid at position 7 is W and the amino acid at position 8 is K,
a light chain CDRL2 consisting of the amino acid sequence represented by Asp Asn Asn (the 5th sequence from the top in Figure 11; SEQ ID NO: 5 in Figure 82), and
a light chain CDRL3 consisting of the amino acid sequence represented by SEQ ID NO: 6 in which the amino acid at position 2 is A.

4. The pharmaceutical composition according to any one of claims 1 to 3, wherein the antibody that binds to HLA/NY-ESO or the antigen-binding fragment thereof comprises a heavy chain variable region consisting of an amino acid sequence having 95% or higher sequence identity to an amino acid sequence corresponding to amino acid positions 21 to 140 of the amino acid sequence represented by SEQ ID NO: 18, or the amino acid sequence represented by SEQ ID NO: 69 or SEQ ID NO: 70, and a light chain variable region consisting of an amino acid sequence having 95% or higher sequence identity to an amino acid sequence corresponding to amino acid positions 156 to 266 of the amino acid sequence represented by SEQ ID NO: 18 or SEQ ID NO: 28, or the amino acid sequence represented by SEQ ID NO: 23.

5. The pharmaceutical composition according to any one of claims 1 to 4, wherein the antibody that binds to HLA/NY-ESO or the antigen-binding fragment thereof comprises
a heavy chain variable region consisting of the amino acid sequence represented by SEQ ID NO: 13,
a heavy chain variable region consisting of the amino acid sequence represented by SEQ ID NO: 15,
a heavy chain variable region consisting of an amino acid sequence corresponding to positions 21 to 140 of the amino acid sequence represented by SEQ ID NO: 20,
a heavy chain variable region consisting of an amino acid sequence corresponding to positions 21 to 140 of the amino acid sequence represented by SEQ ID NO: 17,
a heavy chain variable region consisting of an amino acid sequence corresponding to positions 21 to 140 of the amino acid sequence represented by SEQ ID NO: 18,
a heavy chain variable region consisting of an amino acid sequence corresponding to positions 21 to 140 of the amino acid sequence represented by SEQ ID NO: 19,
a heavy chain variable region consisting of an amino acid sequence corresponding to positions 21 to 140 of the amino acid sequence represented by SEQ ID NO: 22,
a heavy chain variable region consisting of an amino acid sequence corresponding to positions 21 to 140 of the amino acid sequence represented by SEQ ID NO: 24,
a heavy chain variable region consisting of an amino acid sequence corresponding to positions 21 to 140 of the amino acid sequence represented by SEQ ID NO: 25,
a heavy chain variable region consisting of an amino acid sequence corresponding to positions 21 to 140 of the amino acid sequence represented by SEQ ID NO: 26,
a heavy chain variable region consisting of an amino acid sequence corresponding to positions 21 to 140 of the amino acid sequence represented by SEQ ID NO: 27,
a heavy chain variable region consisting of an amino acid sequence corresponding to positions 21 to 140 of the amino acid sequence represented by SEQ ID NO: 28,
a heavy chain variable region consisting of an amino acid sequence corresponding to positions 21 to 140 of the amino acid sequence represented by SEQ ID NO: 29,
a heavy chain variable region consisting of an amino acid sequence corresponding to positions 21 to 140 of the amino acid sequence represented by SEQ ID NO: 21,
a heavy chain variable region consisting of an amino acid sequence corresponding to positions 21 to 140 of the amino acid sequence represented by SEQ ID NO: 55, or
a heavy chain variable region consisting of an amino acid sequence corresponding to positions 21 to 140 of the amino acid sequence represented by SEQ ID NO: 71, and
a light chain variable region consisting of the amino acid sequence represented by SEQ ID NO: 14,
a light chain variable region consisting of the amino acid sequence represented by SEQ ID NO: 16,
a light chain variable region consisting of an amino acid sequence corresponding to positions 156 to 266 of the amino acid sequence represented by SEQ ID NO: 20,
a light chain variable region consisting of an amino acid sequence corresponding to positions 156 to 266 of the amino acid sequence represented by SEQ ID NO: 17,
a light chain variable region consisting of an amino acid sequence corresponding to positions 156 to 266 of the amino acid sequence represented by SEQ ID NO: 18,
a light chain variable region consisting of an amino acid sequence corresponding to positions 156 to 266 of the amino acid sequence represented by SEQ ID NO: 19,
a light chain variable region consisting of an amino acid sequence corresponding to positions 156 to 266 of the amino acid sequence represented by SEQ ID NO: 22,
a light chain variable region consisting of an amino acid sequence corresponding to positions 156 to 266 of the amino acid sequence represented by SEQ ID NO: 24,
a light chain variable region consisting of an amino acid sequence corresponding to positions 156 to 266 of the amino acid sequence represented by SEQ ID NO: 25,
a light chain variable region consisting of an amino acid sequence corresponding to positions 156 to 266 of the amino acid sequence represented by SEQ ID NO: 26,
a light chain variable region consisting of an amino acid sequence corresponding to positions 156 to 266 of the amino acid sequence represented by SEQ ID NO: 27,
a light chain variable region consisting of an amino acid sequence corresponding to positions 156 to 266 of the amino acid sequence represented by SEQ ID NO: 28,
a light chain variable region consisting of an amino acid sequence corresponding to positions 156 to 266 of the amino acid sequence represented by SEQ ID NO: 29,
a light chain variable region consisting of an amino acid sequence corresponding to positions 156 to 266 of the amino acid sequence represented by SEQ ID NO: 21,
a light chain variable region consisting of an amino acid sequence corresponding to positions 161 to 271 of the amino acid sequence represented by SEQ ID NO: 55, or
a light chain variable region consisting of an amino acid sequence corresponding to positions 156 to 266 of the amino acid sequence represented by SEQ ID NO: 71.

6. The pharmaceutical composition according to any one of claims 1 to 5, wherein the antibody that binds to HLA/NY-ESO or the antigen-binding fragment thereof comprises
a heavy chain variable region consisting of the amino acid sequence represented by SEQ ID NO: 13 and a light chain variable region consisting of the amino acid sequence represented by SEQ ID NO: 14,
a heavy chain variable region consisting of the amino acid sequence represented by SEQ ID NO: 15 and a light chain variable region consisting of the amino acid sequence represented by SEQ ID NO: 16,
a heavy chain variable region consisting of an amino acid sequence from positions 21 to 140 of the amino acid sequence represented by SEQ ID NO: 20 and a light chain variable region consisting of an amino acid sequence corresponding to positions 156 to 266 of the amino acid sequence represented by SEQ ID NO: 20,
a heavy chain variable region consisting of an amino acid sequence corresponding to positions 21 to 140 of the amino acid sequence represented by SEQ ID NO: 17 and a light chain variable region consisting of an amino acid sequence corresponding to positions 156 to 266 of the amino acid sequence represented by SEQ ID NO: 17,
a heavy chain variable region consisting of an amino acid sequence corresponding to positions 21 to 140 of the amino acid sequence represented by SEQ ID NO: 18 and a light chain variable region consisting of an amino acid sequence corresponding to positions 156 to 266 of the amino acid sequence represented by SEQ ID NO: 18,
a heavy chain variable region consisting of an amino acid sequence corresponding to positions 21 to 140 of the amino acid sequence represented by SEQ ID NO: 19 and a light chain variable region consisting of an amino acid sequence corresponding to positions 156 to 266 of the amino acid sequence represented by SEQ ID NO: 19,
a heavy chain variable region consisting of an amino acid sequence corresponding to positions 21 to 140 of the amino acid sequence represented by SEQ ID NO: 22 and a light chain variable region consisting of an amino acid sequence corresponding to positions 156 to 266 of the amino acid sequence represented by SEQ ID NO: 22,
a heavy chain variable region consisting of an amino acid sequence corresponding to positions 21 to 140 of the amino acid sequence represented by SEQ ID NO: 24 and a light chain variable region consisting of an amino acid sequence corresponding to positions 156 to 266 of the amino acid sequence represented by SEQ ID NO: 24,
a heavy chain variable region consisting of an amino acid sequence corresponding to positions 21 to 140 of the amino acid sequence represented by SEQ ID NO: 25 and a light chain variable region consisting of an amino acid sequence corresponding to positions 156 to 266 of the amino acid sequence represented by SEQ ID NO: 25,
a heavy chain variable region consisting of an amino acid sequence corresponding to positions 21 to 140 of the amino acid sequence represented by SEQ ID NO: 26 and a light chain variable region consisting of an amino acid sequence corresponding to positions 156 to 266 of the amino acid sequence represented by SEQ ID NO: 26,
a heavy chain variable region consisting of an amino acid sequence corresponding to positions 21 to 140 of the amino acid sequence represented by SEQ ID NO: 27 and a light chain variable region consisting of an amino acid sequence corresponding to positions 156 to 266 of the amino acid sequence represented by SEQ ID NO: 27,
a heavy chain variable region consisting of an amino acid sequence corresponding to positions 21 to 140 of the amino acid sequence represented by SEQ ID NO: 28 and a light chain variable region consisting of an amino acid sequence corresponding to positions 156 to 266 of the amino acid sequence represented by SEQ ID NO: 28,
a heavy chain variable region consisting of an amino acid sequence corresponding to positions 21 to 140 of the amino acid sequence represented by SEQ ID NO: 29 and a light chain variable region consisting of an amino acid sequence corresponding to positions 156 to 266 of the amino acid sequence represented by SEQ ID NO: 29,
a heavy chain variable region consisting of an amino acid sequence corresponding to positions 21 to 140 of the amino acid sequence represented by SEQ ID NO: 21 and a light chain variable region consisting of an amino acid sequence corresponding to positions 156 to 266 of the amino acid sequence represented by SEQ ID NO: 21,
a heavy chain variable region consisting of an amino acid sequence corresponding to positions 21 to 140 of the amino acid sequence represented by SEQ ID NO: 55 and a light chain variable region consisting of an amino acid sequence corresponding to positions 161 to 271 of the amino acid sequence represented by SEQ ID NO: 55, or
a heavy chain variable region consisting of an amino acid sequence corresponding to positions 21 to 140 of the amino acid sequence represented by SEQ ID NO: 71 and a light chain variable region consisting of an amino acid sequence corresponding to positions 156 to 266 of the amino acid sequence represented by SEQ ID NO: 71.

7. The pharmaceutical composition according to any one of claims 1 to 6, wherein the antibody that binds to HLA/NY-ESO or the antigen-binding fragment thereof is a scFv.

8. The pharmaceutical composition according to any one of claims 1 to 7, wherein the antibody that binds to HLA/NY-ESO or the antigen-binding fragment thereof is a scFv consisting of an amino acid sequence corresponding to positions 21 to 266 of the amino acid sequence represented by SEQ ID NO: 30,
a scFv comprising a heavy chain variable region consisting of the amino acid sequence represented by SEQ ID NO: 15 and a light chain variable region consisting of the amino acid sequence represented by SEQ ID NO: 16,
a scFv consisting of an amino acid sequence corresponding to positions 21 to 266 of the amino acid sequence represented by SEQ ID NO: 20,
a scFv consisting of an amino acid sequence corresponding to positions 21 to 266 of the amino acid sequence represented by SEQ ID NO: 17,
a scFv consisting of an amino acid sequence corresponding to positions 21 to 266 of the amino acid sequence represented by SEQ ID NO: 18,
a scFv consisting of an amino acid sequence corresponding to positions 21 to 266 of the amino acid sequence represented by SEQ ID NO: 19,
a scFv consisting of an amino acid sequence corresponding to positions 21 to 266 of the amino acid sequence represented by SEQ ID NO: 22,
a scFv consisting of an amino acid sequence corresponding to positions 21 to 266 of the amino acid sequence represented by SEQ ID NO: 24,
a scFv consisting of an amino acid sequence corresponding to positions 21 to 266 of the amino acid sequence represented by SEQ ID NO: 25,
a scFv consisting of an amino acid sequence corresponding to positions 21 to 266 of the amino acid sequence represented by SEQ ID NO: 26,
a scFv consisting of an amino acid sequence corresponding to positions 21 to 266 of the amino acid sequence represented by SEQ ID NO: 27,
a scFv consisting of an amino acid sequence corresponding to positions 21 to 266 of the amino acid sequence represented by SEQ ID NO: 28,
a scFv consisting of an amino acid sequence corresponding to positions 21 to 266 of the amino acid sequence represented by SEQ ID NO: 29,
a scFv consisting of an amino acid sequence corresponding to positions 21 to 266 of the amino acid sequence represented by SEQ ID NO: 21,
a scFv consisting of an amino acid sequence corresponding to positions 21 to 271 of the amino acid sequence represented by SEQ ID NO: 55, or
a scFv consisting of an amino acid sequence corresponding to positions 21 to 266 of the amino acid sequence represented by SEQ ID NO: 71.

9. The pharmaceutical composition according to any one of claims 1 to 8, wherein the antibody that binds to CD3 or the antigen-binding fragment thereof comprises one or two or more sets of CDRH1 to CDRH3 and CDRL1 to CDRL3 selected from the group consisting of the following sets (i) to (x):
(i)
a heavy chain CDRH1 consisting of the amino acid sequence represented by SEQ ID NO: 7,
a heavy chain CDRH2 consisting of the amino acid sequence represented by SEQ ID NO: 8,
a heavy chain CDRH3 consisting of the amino acid sequence represented by SEQ ID NO: 9,
a light chain CDRL1 consisting of the amino acid sequence represented by SEQ ID NO: 10,
a light chain CDRL2 consisting of the amino acid sequence represented by Arg Asp Asp (the 5th sequence from the top in Figure 12; SEQ ID NO: 11 in Figure 82), and
a light chain CDRL3 consisting of the amino acid sequence represented by SEQ ID NO: 12,
(ii)
a heavy chain CDRH1 consisting of the amino acid sequence represented by SEQ ID NO: 7,
a heavy chain CDRH2 consisting of the amino acid sequence represented by SEQ ID NO: 8 in which the amino acid at position 3 is N,
a heavy chain CDRH3 consisting of the amino acid sequence represented by SEQ ID NO: 9,
a light chain CDRL1 consisting of the amino acid sequence represented by SEQ ID NO: 10,
a light chain CDRL2 consisting of the amino acid sequence represented by Arg Asp Asp (the 5th sequence from the top in Figure 12; SEQ ID NO: 11 in Figure 82), and
a light chain CDRL3 consisting of the amino acid sequence represented by SEQ ID NO: 12,
(iii)
a heavy chain CDRH1 consisting of the amino acid sequence represented by SEQ ID NO: 7,
a heavy chain CDRH2 consisting of the amino acid sequence represented by SEQ ID NO: 8 in which the amino acid at position 3 is S,
a heavy chain CDRH3 consisting of the amino acid sequence represented by SEQ ID NO: 9,
a light chain CDRL1 consisting of the amino acid sequence represented by SEQ ID NO: 10,
a light chain CDRL2 consisting of the amino acid sequence represented by Arg Asp Asp (the 5th sequence from the top in Figure 12; SEQ ID NO: 11 in Figure 82), and
a light chain CDRL3 consisting of the amino acid sequence represented by SEQ ID NO: 12,
(iv)
a heavy chain CDRH1 consisting of the amino acid sequence represented by SEQ ID NO: 7,
a heavy chain CDRH2 consisting of the amino acid sequence represented by SEQ ID NO: 8,
a heavy chain CDRH3 consisting of the amino acid sequence represented by SEQ ID NO: 9,
a light chain CDRL1 consisting of the amino acid sequence represented by SEQ ID NO: 10,
a light chain CDRL2 consisting of the amino acid sequence represented by Arg Asp Asp (the 5th sequence from the top in Figure 12; SEQ ID NO: 11 in Figure 82) in which the amino acid at position 2 is G, and
a light chain CDRL3 consisting of the amino acid sequence represented by SEQ ID NO: 12,
(v)
a heavy chain CDRH1 consisting of the amino acid sequence represented by SEQ ID NO: 7,
a heavy chain CDRH2 consisting of the amino acid sequence represented by SEQ ID NO: 8,
a heavy chain CDRH3 consisting of the amino acid sequence represented by SEQ ID NO: 9,
a light chain CDRL1 consisting of the amino acid sequence represented by SEQ ID NO: 10,
a light chain CDRL2 consisting of the amino acid sequence represented by Arg Asp Asp (the 5th sequence from the top in Figure 12; SEQ ID NO: 11 in Figure 82) in which the amino acid at position 2 is Q, and
a light chain CDRL3 consisting of the amino acid sequence represented by SEQ ID NO: 12,
(vi)
a heavy chain CDRH1 consisting of the amino acid sequence represented by SEQ ID NO: 7,
a heavy chain CDRH2 consisting of the amino acid sequence represented by SEQ ID NO: 8,
a heavy chain CDRH3 consisting of the amino acid sequence represented by SEQ ID NO: 9,
a light chain CDRL1 consisting of the amino acid sequence represented by SEQ ID NO: 10,
a light chain CDRL2 consisting of the amino acid sequence represented by Arg Asp Asp (the 5th sequence from the top in Figure 12; SEQ ID NO: 11 in Figure 82) in which the amino acid at position 2 is N, and
a light chain CDRL3 consisting of the amino acid sequence represented by SEQ ID NO: 12,
(vii)
a heavy chain CDRH1 consisting of the amino acid sequence represented by SEQ ID NO: 7,
a heavy chain CDRH2 consisting of the amino acid sequence represented by SEQ ID NO: 8,
a heavy chain CDRH3 consisting of the amino acid sequence represented by SEQ ID NO: 9,
a light chain CDRL1 consisting of the amino acid sequence represented by SEQ ID NO: 10,
a light chain CDRL2 consisting of the amino acid sequence represented by Arg Asp Asp (the 5th sequence from the top in Figure 12; SEQ ID NO: 11 in Figure 82) in which the amino acid at position 2 is S, and
a light chain CDRL3 consisting of the amino acid sequence represented by SEQ ID NO: 12,
(viii)
a heavy chain CDRH1 consisting of the amino acid sequence represented by SEQ ID NO: 7,
a heavy chain CDRH2 consisting of the amino acid sequence represented by SEQ ID NO: 8,
a heavy chain CDRH3 consisting of the amino acid sequence represented by SEQ ID NO: 9,
a light chain CDRL1 consisting of the amino acid sequence represented by SEQ ID NO: 10,
a light chain CDRL2 consisting of the amino acid sequence represented by Arg Asp Asp (the 5th sequence from the top in Figure 12; SEQ ID NO: 11 in Figure 82) in which the amino acid at position 2 is A, and
a light chain CDRL3 consisting of the amino acid sequence represented by SEQ ID NO: 12,
(ix)
a heavy chain CDRH1 consisting of the amino acid sequence represented by SEQ ID NO: 7,
a heavy chain CDRH2 consisting of the amino acid sequence represented by SEQ ID NO: 8 in which the amino acid at position 3 is S,
a heavy chain CDRH3 consisting of the amino acid sequence represented by SEQ ID NO: 9,
a light chain CDRL1 consisting of the amino acid sequence represented by SEQ ID NO: 10,
a light chain CDRL2 consisting of the amino acid sequence represented by Arg Asp Asp (the 5th sequence from the top in Figure 12; SEQ ID NO: 11 in Figure 82) in which the amino acid at position 2 is N, and
a light chain CDRL3 consisting of the amino acid sequence represented by SEQ ID NO: 12 and
(x)
a heavy chain CDRH1 consisting of the amino acid sequence represented by SEQ ID NO: 7,
a heavy chain CDRH2 consisting of the amino acid sequence represented by SEQ ID NO: 8 in which the amino acid at position 3 is S,
a heavy chain CDRH3 consisting of the amino acid sequence represented by SEQ ID NO: 9,
a light chain CDRL1 consisting of the amino acid sequence represented by SEQ ID NO: 10,
a light chain CDRL2 consisting of the amino acid sequence represented by Arg Asp Asp (the 5th sequence from the top in Figure 12: SEQ ID NO: 11 in Figure 82) in which the amino acid at position 2 is S, and
a light chain CDRL3 consisting of the amino acid sequence represented by SEQ ID NO: 12.

10. The pharmaceutical composition according to claim 9, wherein
the antibody that binds to CD3 or the antigen-binding fragment thereof comprises
a heavy chain variable region consisting of an amino acid sequence corresponding to positions 2 to 119 of the amino acid sequence represented by SEQ ID NO: 46,
a heavy chain variable region consisting of an amino acid sequence corresponding to positions 2 to 119 of the amino acid sequence represented by SEQ ID NO: 47,
a heavy chain variable region consisting of an amino acid sequence corresponding to positions 2 to 119 of the amino acid sequence represented by SEQ ID NO: 51,
a heavy chain variable region consisting of an amino acid sequence corresponding to positions 2 to 119 of the amino acid sequence represented by SEQ ID NO: 48,
a heavy chain variable region consisting of an amino acid sequence corresponding to positions 2 to 119 of the amino acid sequence represented by SEQ ID NO: 49,
a heavy chain variable region consisting of an amino acid sequence corresponding to positions 2 to 119 of the amino acid sequence represented by SEQ ID NO: 50,
a heavy chain variable region consisting of an amino acid sequence corresponding to positions 272 to 389 of the amino acid sequence represented by SEQ ID NO: 54,
a heavy chain variable region consisting of an amino acid sequence corresponding to positions 277 to 394 of the amino acid sequence represented by SEQ ID NO: 55, or
a heavy chain variable region consisting of an amino acid sequence corresponding to positions 277 to 394 of the amino acid sequence represented by SEQ ID NO: 56, and
a light chain variable region consisting of an amino acid sequence corresponding to positions 135 to 243 of the amino acid sequence represented by SEQ ID NO: 46,
a light chain variable region consisting of an amino acid sequence corresponding to positions 135 to 241 of the amino acid sequence represented by SEQ ID NO: 47,
a light chain variable region consisting of an amino acid sequence corresponding to positions 135 to 243 of the amino acid sequence represented by SEQ ID NO: 51,
a light chain variable region consisting of an amino acid sequence corresponding to positions 135 to 241 of the amino acid sequence represented by SEQ ID NO: 48,
a light chain variable region consisting of an amino acid sequence corresponding to positions 135 to 243 of the amino acid sequence represented by SEQ ID NO: 49,
a light chain variable region consisting of an amino acid sequence corresponding to positions 135 to 243 of the amino acid sequence represented by SEQ ID NO: 50,
a light chain variable region consisting of an amino acid sequence corresponding to positions 405 to 511 of the amino acid sequence represented by SEQ ID NO: 54,
a light chain variable region consisting of an amino acid sequence corresponding to positions 410 to 516 of the amino acid sequence represented by SEQ ID NO: 55, or
a light chain variable region consisting of an amino acid sequence corresponding to positions 410 to 516 of the amino acid sequence represented by SEQ ID NO: 56.

11. The pharmaceutical composition according to claim 8 or 9, wherein
the antibody that binds to CD3 or the antigen-binding fragment thereof comprises
a heavy chain variable region consisting of an amino acid sequence corresponding to positions 2 to 119 of the amino acid sequence represented by SEQ ID NO: 46 and a light chain variable region consisting of an amino acid sequence corresponding to positions 135 to 243 of the amino acid sequence represented by SEQ ID NO: 46,
a heavy chain variable region consisting of an amino acid sequence corresponding to positions 2 to 119 of the amino acid sequence represented by SEQ ID NO: 47 and a light chain variable region consisting of an amino acid sequence corresponding to positions 135 to 241 of the amino acid sequence represented by SEQ ID NO: 47,
a heavy chain variable region consisting of an amino acid sequence corresponding to positions 2 to 119 of the amino acid sequence represented by SEQ ID NO: 51 and a light chain variable region consisting of an amino acid sequence corresponding to positions 135 to 243 of the amino acid sequence represented by SEQ ID NO: 51,
a heavy chain variable region consisting of an amino acid sequence corresponding to positions 2 to 119 of the amino acid sequence represented by SEQ ID NO: 48 and a light chain variable region consisting of an amino acid sequence corresponding to positions 135 to 241 of the amino acid sequence represented by SEQ ID NO: 48,
a heavy chain variable region consisting of an amino acid sequence corresponding to positions 2 to 119 of the amino acid sequence represented by SEQ ID NO: 49 and a light chain variable region consisting of an amino acid sequence corresponding to positions 135 to 243 of the amino acid sequence represented by SEQ ID NO: 49,
a heavy chain variable region consisting of an amino acid sequence corresponding to positions 2 to 119 of the amino acid sequence represented by SEQ ID NO: 50 and a light chain variable region consisting of an amino acid sequence corresponding to positions 135 to 243 of the amino acid sequence represented by SEQ ID NO: 50,
a heavy chain variable region consisting of an amino acid sequence corresponding to positions 272 to 389 of the amino acid sequence represented by SEQ ID NO: 54 and a light chain variable region consisting of an amino acid sequence corresponding to positions 405 to 511 of the amino acid sequence represented by SEQ ID NO: 54,
a heavy chain variable region consisting of an amino acid sequence corresponding to positions 277 to 394 of the amino acid sequence represented by SEQ ID NO: 55 and a light chain variable region consisting of an amino acid sequence corresponding to positions 410 to 516 of the amino acid sequence represented by SEQ ID NO: 55, or
a heavy chain variable region consisting of an amino acid sequence corresponding to positions 277 to 394 of the amino acid sequence represented by SEQ ID NO: 56 and a light chain variable region consisting of an amino acid sequence corresponding to positions 410 to 516 of the amino acid sequence represented by SEQ ID NO: 56.

12. The pharmaceutical composition according to any one of claims 1 to 11, wherein the antibody that binds to CD3 or the antigen-binding fragment thereof is scFv.

13. The pharmaceutical composition according to any one of claims 1 to 12, wherein the antibody that binds to CD3 or the antigen-binding fragment thereof is a scFv consisting of an amino acid sequence corresponding to positions 2 to 243 of the amino acid sequence represented by SEQ ID NO: 46,
a scFv consisting of an amino acid sequence corresponding to positions 2 to 241 of the amino acid sequence represented by SEQ ID NO: 47,
a scFv consisting of an amino acid sequence corresponding to positions 2 to 243 of the amino acid sequence represented by SEQ ID NO: 51,
a scFv consisting of an amino acid sequence corresponding to positions 2 to 241 of the amino acid sequence represented by SEQ ID NO: 48,
a scFv consisting of an amino acid sequence corresponding to positions 2 to 243 of the amino acid sequence represented by SEQ ID NO: 49,
a scFv consisting of an amino acid sequence corresponding to positions 2 to 243 of the amino acid sequence represented by SEQ ID NO: 50,
a scFv consisting of an amino acid sequence corresponding to positions 272 to 511 of the amino acid sequence represented by SEQ ID NO: 54,
a scFv consisting of an amino acid sequence corresponding to positions 277 to 516 of the amino acid sequence represented by SEQ ID NO: 55, or
a scFv consisting of an amino acid sequence corresponding to positions 277 to 516 of the amino acid sequence represented by SEQ ID NO: 56.

14. The pharmaceutical composition according to any one of claims 1 to 13, wherein the pharmaceutical composition comprises: a first polypeptide comprising the antibody that binds to HLA/NY-ESO or the antigen-binding fragment thereof which is scFv, the antibody that binds to CD3 or the antigen-binding fragment thereof which is scFv, and an Fc region (i) in the presented order from the N terminus toward the C terminus; and a second polypeptide comprising an Fc region (ii), wherein preferably, the first polypeptide and the second polypeptide are connected to each other via the Fc region (i) and the Fc region (ii).

15. The pharmaceutical composition according to claim 14, wherein the first polypeptide comprises an amino acid sequence corresponding to positions 21 to 511 of the amino acid sequence represented by SEQ ID NO: 32, an amino acid sequence corresponding to positions 21 to 511 of the amino acid sequence represented by SEQ ID NO: 34, an amino acid sequence corresponding to positions 21 to 511 of the amino acid sequence represented by SEQ ID NO: 35, an amino acid sequence corresponding to positions 21 to 511 of the amino acid sequence represented by SEQ ID NO: 36, an amino acid sequence corresponding to positions 21 to 511 of the amino acid sequence represented by SEQ ID NO: 37, an amino acid sequence corresponding to positions 21 to 511 of the amino acid sequence represented by SEQ ID NO: 38, an amino acid sequence corresponding to positions 21 to 511 of the amino acid sequence represented by SEQ ID NO: 39, an amino acid sequence corresponding to positions 21 to 511 of the amino acid sequence represented by SEQ ID NO: 40, an amino acid sequence corresponding to positions 21 to 511 of the amino acid sequence represented by SEQ ID NO: 41, an amino acid sequence corresponding to positions 21 to 511 of the amino acid sequence represented by SEQ ID NO: 42, an amino acid sequence corresponding to positions 21 to 511 of the amino acid sequence represented by SEQ ID NO: 43, an amino acid sequence corresponding to positions 21 to 511 of the amino acid sequence represented by SEQ ID NO: 33, an amino acid sequence corresponding to positions 21 to 511 of the amino acid sequence represented by SEQ ID NO: 52, an amino acid sequence corresponding to positions 21 to 511 of the amino acid sequence represented by SEQ ID NO: 53, an amino acid sequence corresponding to positions 21 to 511 of the amino acid sequence represented by SEQ ID NO: 54, an amino acid sequence corresponding to positions 21 to 516 of the amino acid sequence represented by SEQ ID NO: 55, or an amino acid sequence corresponding to positions 21 to 516 of the amino acid sequence represented by SEQ ID NO: 56.

16. The pharmaceutical composition according to claim 14 or 15, wherein the first polypeptide comprises an amino acid sequence corresponding to positions 529 to 745 of the amino acid sequence represented by SEQ ID NO: 32, 34, 35, 36, 37, 38, 39, 40, 41, 42, 43, 33, 52, 53, or 54, or an amino acid sequence corresponding to positions 534 to 750 of the amino acid sequence represented by SEQ ID NO: 55 or 56.

17. The pharmaceutical composition according to any one of claims 14 to 16, wherein the first polypeptide consists of an amino acid sequence corresponding to positions 20 to 745 of the amino acid sequence represented by SEQ ID NO: 32, an amino acid sequence corresponding to positions 20 to 745 of the amino acid sequence represented by SEQ ID NO: 34, an amino acid sequence corresponding to positions 20 to 745 of the amino acid sequence represented by SEQ ID NO: 35, an amino acid sequence corresponding to positions 20 to 745 of the amino acid sequence represented by SEQ ID NO: 36, an amino acid sequence corresponding to positions 20 to 745 of the amino acid sequence represented by SEQ ID NO: 37, an amino acid sequence corresponding to positions 20 to 745 of the amino acid sequence represented by SEQ ID NO: 38, an amino acid sequence corresponding to positions 20 to 745 of the amino acid sequence represented by SEQ ID NO: 39, an amino acid sequence corresponding to positions 20 to 745 of the amino acid sequence represented by SEQ ID NO: 40, an amino acid sequence corresponding to positions 20 to 745 of the amino acid sequence represented by SEQ ID NO: 41, an amino acid sequence corresponding to positions 20 to 745 of the amino acid sequence represented by SEQ ID NO: 42, an amino acid sequence corresponding to positions 20 to 745 of the amino acid sequence represented by SEQ ID NO: 43, an amino acid sequence corresponding to positions 20 to 745 of the amino acid sequence represented by SEQ ID NO: 33, an amino acid sequence corresponding to positions 20 to 745 of the amino acid sequence represented by SEQ ID NO: 52, an amino acid sequence corresponding to positions 20 to 745 of the amino acid sequence represented by SEQ ID NO: 53, an amino acid sequence corresponding to positions 20 to 745 of the amino acid sequence represented by SEQ ID NO: 54, an amino acid sequence corresponding to positions 20 to 750 of the amino acid sequence represented by SEQ ID NO: 55, or an amino acid sequence corresponding to positions 20 to 750 of the amino acid sequence represented by SEQ ID NO: 56.

18. The pharmaceutical composition according to any one of claims 14 to 17, wherein the second polypeptide comprises an amino acid sequence corresponding to positions 20 to 246 of the amino acid sequence represented by SEQ ID NO: 31.

19. The pharmaceutical composition according to any one of claims 1 to 13, wherein the pharmaceutical composition comprises a first polypeptide, a second polypeptide, and a third polypeptide,
the first polypeptide comprising the antibody that binds to HLA/NY-ESO or the antigen-binding fragment thereof which is a scFv, an antibody that specifically binds to CD3 or an antigen-binding fragment thereof which is a scFv, and an Fc region (i) in that order from the N-terminus towards the C-terminus,
the second polypeptide consisting of an immunoglobulin heavy chain comprising an Fc region (ii), and
the third polypeptide consisting of an immunoglobulin light chain, wherein
preferably, the second polypeptide and the third polypeptide are associated with each other, and
the first polypeptide and the second peptide are associated with each other via their respective Fc regions.

20. The pharmaceutical composition according to claim 19, wherein the second polypeptide comprises an amino acid sequence corresponding to amino acid positions 20 to 242 of the amino acid sequence represented by SEQ ID NO: 44.

21. The pharmaceutical composition according to claim 19 or 20, wherein the third polypeptide comprises the amino acid sequence represented by SEQ ID NO: 45.

22. The pharmaceutical composition according to any one of claims 19 to 21, wherein the first polypeptide comprises an amino acid sequence corresponding to positions 21 to 511 of the amino acid sequence represented by SEQ ID NO: 32, an amino acid sequence corresponding to positions 21 to 511 of the amino acid sequence represented by SEQ ID NO: 34, an amino acid sequence corresponding to positions 21 to 511 of the amino acid sequence represented by SEQ ID NO: 35, an amino acid sequence corresponding to positions 21 to 511 of the amino acid sequence represented by SEQ ID NO: 36, an amino acid sequence corresponding to positions 21 to 511 of the amino acid sequence represented by SEQ ID NO: 37, an amino acid sequence corresponding to positions 21 to 511 of the amino acid sequence represented by SEQ ID NO: 38, an amino acid sequence corresponding to positions 21 to 511 of the amino acid sequence represented by SEQ ID NO: 39, an amino acid sequence corresponding to positions 21 to 511 of the amino acid sequence represented by SEQ ID NO: 40, an amino acid sequence corresponding to positions 21 to 511 of the amino acid sequence represented by SEQ ID NO: 41, an amino acid sequence corresponding to positions 21 to 511 of the amino acid sequence represented by SEQ ID NO: 42, an amino acid sequence corresponding to positions 21 to 511 of the amino acid sequence represented by SEQ ID NO: 43, an amino acid sequence corresponding to positions 21 to 511 of the amino acid sequence represented by SEQ ID NO: 33, an amino acid sequence corresponding to positions 21 to 511 of the amino acid sequence represented by SEQ ID NO: 52, an amino acid sequence corresponding to positions 21 to 511 of the amino acid sequence represented by SEQ ID NO: 53, an amino acid sequence corresponding to positions 21 to 511 of the amino acid sequence represented by SEQ ID NO: 54, an amino acid sequence corresponding to positions 21 to 516 of the amino acid sequence represented by SEQ ID NO: 55, or an amino acid sequence corresponding to positions 21 to 516 of the amino acid sequence represented by SEQ ID NO: 56.

23. The pharmaceutical composition according to any one of claims 19 to 22, wherein the first polypeptide consists of an amino acid sequence corresponding to positions 20 to 745 of the amino acid sequence represented by SEQ ID NO: 32, an amino acid sequence corresponding to positions 20 to 745 of the amino acid sequence represented by SEQ ID NO: 34, an amino acid sequence corresponding to positions 20 to 745 of the amino acid sequence represented by SEQ ID NO: 35, an amino acid sequence corresponding to positions 20 to 745 of the amino acid sequence represented by SEQ ID NO: 36, an amino acid sequence corresponding to positions 20 to 745 of the amino acid sequence represented by SEQ ID NO: 37, an amino acid sequence corresponding to positions 20 to 745 of the amino acid sequence represented by SEQ ID NO: 38, an amino acid sequence corresponding to positions 20 to 745 of the amino acid sequence represented by SEQ ID NO: 39, an amino acid sequence corresponding to positions 20 to 745 of the amino acid sequence represented by SEQ ID NO: 40, an amino acid sequence corresponding to positions 20 to 745 of the amino acid sequence represented by SEQ ID NO: 41, an amino acid sequence corresponding to positions 20 to 745 of the amino acid sequence represented by SEQ ID NO: 42, an amino acid sequence corresponding to positions 20 to 745 of the amino acid sequence represented by SEQ ID NO: 43, an amino acid sequence corresponding to positions 20 to 745 of the amino acid sequence represented by SEQ ID NO: 33, an amino acid sequence corresponding to positions 20 to 745 of the amino acid sequence represented by SEQ ID NO: 52, an amino acid sequence corresponding to positions 20 to 745 of the amino acid sequence represented by SEQ ID NO: 53, an amino acid sequence corresponding to positions 20 to 745 of the amino acid sequence represented by SEQ ID NO: 54, an amino acid sequence corresponding to positions 20 to 750 of the amino acid sequence represented by SEQ ID NO: 55, or an amino acid sequence corresponding to positions 20 to 750 of the amino acid sequence represented by SEQ ID NO: 56.

24. The pharmaceutical composition according to any one of claims 9 to 11, wherein the pharmaceutical composition comprises:
a first polypeptide comprising an antibody that specifically binds to HLA/NY-ESO or an antigen-binding fragment thereof which is a scFv, a heavy chain variable region and constant region CH1 of the antibody that binds to CD3, and an immunoglobulin Fc region (i) in thatorder from the N-terminus toward the C- terminus;
a second polypeptide comprising an immunoglobulin hinge region and Fc region (ii); and
a third polypeptide comprising a light chain of the antibody that binds to CD3, the light chain consisting of a variable region and a constant region, wherein
preferably, the first polypeptide and the second polypeptide are associated with each other via the Fc region (i) and the Fc region (ii), and the first polypeptide is associated with the third polypeptide at the antibody heavy chain variable region and constant region CH1.

25. The pharmaceutical composition according to any one of claims 9 to 11 and 24, wherein the first polypeptide comprises an amino acid sequence corresponding to positions 20 to 724 of the amino acid sequence represented by SEQ ID NO: 57, an amino acid sequence corresponding to positions 20 to 719 of the amino acid sequence represented by SEQ ID NO: 60, or an amino acid sequence corresponding to positions 20 to 719 of the amino acid sequence represented by SEQ ID NO: 61.

26. The pharmaceutical composition according to any one of claims 9 to 11, 24 and 25, wherein the second polypeptide comprises an amino acid sequence corresponding to positions 20 to 246 of the amino acid sequence represented by SEQ ID NO: 31.

27. The pharmaceutical composition according to any one of claims 9 to 11 and 24 to 26, wherein the third polypeptide comprises an amino acid sequence corresponding to positions 21 to 127 of the amino acid sequence represented by SEQ ID NO: 58.

28. The pharmaceutical composition according to any one of claims 9 to 11 and 24 to 27, wherein the third polypeptide comprises an amino acid sequence corresponding to positions 21 to 233 of the amino acid sequence represented by SEQ ID NO: 58.

29. The pharmaceutical composition according to any one of claims 1 to 28, wherein the amino acid sequences of one or two or more polypeptides contained in the pharmaceutical composition lack one or two carboxy-terminal amino acids.

30. The pharmaceutical composition according to any one of claims 1 to 29, wherein the cancer is one,two or more members selected from the group consisting of kidney cancer, melanoma, squamous cell cancer, basal cell cancer, conjunctival cancer, oral cancer, larynx cancer, throat cancer, thyroid cancer, lung cancer (non-small cell lung cancer (adenocarcinoma, squamous cell cancer, and large-cell cancer) and small-cell lung cancer), breast cancer, esophageal cancer, stomach cancer, duodenum cancer, small intestine cancer, large intestine cancer, rectal cancer, appendix cancer, anus cancer, liver cancer, gallbladder cancer, bile duct cancer, pancreatic cancer, adrenal cancer, urinary bladder cancer, prostate cancer, uterine cancer, vaginal cancer, liposarcoma, angiosarcoma, chondrosarcoma, rhabdomyosarcoma, Ewing's sarcoma, osteosarcoma, undifferentiated pleomorphic sarcoma, myxofibrosarcoma, malignant peripheral nerve sheath tumor, retroperitoneal sarcoma, synovial sarcoma, uterine sarcoma, gastrointestinal stromal tumor, leiomyosarcoma, epithelioid sarcoma, B cell lymphoma, T/NK cell lymphoma, Hodgkin's lymphoma, myeloid leukemia, lymphoid leukemia, myeloproliferative disease, myelodysplastic syndrome, multiple myeloma, testicle cancer, and ovary cancer.

31. The pharmaceutical composition according to any one of claims 1 to 30, wherein the pharmaceutical composition is administered after the compound that inhibits an immune checkpoint molecule or the chemotherapeutic.

32. The pharmaceutical composition according to any one of claims 1 to 30, wherein the pharmaceutical composition is administered before the compound that inhibits an immune checkpoint molecule or the chemotherapeutic.

33. The pharmaceutical composition according to any one of claims 1 to 30, wherein the pharmaceutical composition is administered concurrently with the compound that inhibits an immune checkpoint molecule or the chemotherapeutic.

34. The pharmaceutical composition according to any one of claims 1 to 30 and 33, wherein the pharmaceutical composition comprises the compound that inhibits an immune checkpoint molecule or the chemotherapeutic.

35. The pharmaceutical composition according to any one of claims 1 to 34, wherein the pharmaceutical composition is used in combination with one,two or more additional medicaments and/or therapies.

36. The pharmaceutical composition according to any one of claims 1 to 35, wherein compound [ii] is the compound that inhibits an immune checkpoint molecule.

37. The pharmaceutical composition according to any one of claims 1 to 36, wherein the immune checkpoint molecule is selected from the group consisting of PD-1, PD-L1, PD-L2, CTLA-4, and TIGIT.

38. The pharmaceutical composition according to any one of claims 1 to 37, wherein the immune checkpoint molecule is PD-1.

39. The pharmaceutical composition according to claim 38, wherein the compound that inhibits the immune checkpoint molecule is nivolumab or pembrolizumab, an antigen-binding fragment thereof, or a compound comprising the antigen-binding fragment thereof.

40. The pharmaceutical composition according to any one of claims 1 to 37, wherein the immune checkpoint molecule is PD-L1.

41. The pharmaceutical composition according to claim 40, wherein the compound that inhibits the immune checkpoint molecule is atezolizumab, durvalumab, or avelumab, an antigen-binding fragment thereof, or a compound comprising the antigen-binding fragment thereof.

42. The pharmaceutical composition according to any one of claims 1 to 37, wherein the immune checkpoint molecule is CTLA-4.

43. The pharmaceutical composition according to claim 42, wherein the compound that inhibits the immune checkpoint molecule is ipilimumab, tremelimumab, spartalizumab, or cemiplimab, an antigen-binding fragment thereof, or a compound comprising the antigen- binding fragment thereof.

44. The pharmaceutical composition according to any one of claims 1 to 37, wherein the immune checkpoint molecule is TIGIT.

45. The pharmaceutical composition according to claim 44, wherein the compound that inhibits the immune checkpoint molecule is tiragolumab or vibostolimab, an antigen-binding fragment thereof, or a compound comprising the antigen-binding fragment thereof.

46. The pharmaceutical composition according to any one of claims 1 to 36, wherein the compound that inhibits an immune checkpoint molecule is pembrolizumab, nivolumab, spartalizumab, cemiplimab, avelumab, atezolizumab, durvalumab, ipilimumab, or tremelimumab.

47. The pharmaceutical composition according to any one of claims 1 to 39, wherein the compound that inhibits an immune checkpoint molecule is pembrolizumab, an antigen-binding fragment of pembrolizumab, or a compound comprising the antigen-binding fragment thereof.

48. The pharmaceutical composition according to any one of claims 1 to 35, wherein the compound [ii] is the chemotherapeutic.

49. The pharmaceutical composition according to any one of claims 1 to 35 and 48, wherein the chemotherapeutic is a microtubule inhibitor.

50. The pharmaceutical composition according to any one of claims 1 to 35, 48 and 49, wherein the chemotherapeutic is a taxane-based microtubule inhibitor.

51. The pharmaceutical composition according to any one of claims 1 to 35 and 48 to 50, wherein the chemotherapeutic is selected from the group consisting of paclitaxel, docetaxel, vincristine, vinblastine, vindesine, eribulin, vinorelbine, albumin-bound paclitaxel, oxaliplatin, carboplatin, cisplatin, nedaplatin, azacytidine, decitabine, doxorubicin, bleomycin, liposomal doxorubicin, ifosfamide, cyclophosphamide, and dacarbazine.

52. The pharmaceutical composition according to any one of claims 1 to 35 and 48 to 51, wherein the chemotherapeutic is paclitaxel or albumin-bound paclitaxel.

53. The pharmaceutical composition according to any one of claims 1 to 52, wherein the pharmaceutical composition is further used in combination with a multikinase inhibitor.

54. The pharmaceutical composition according to claim 53, wherein the multikinase inhibitor is one,two or more members selected from the group consisting of sorafenib, sunitinib, pazopanib, regorafenib, and lenvatinib.

55. The pharmaceutical composition according to claim 53 or 54, wherein the multikinase inhibitor is lenvatinib.

56. The pharmaceutical composition according to any one of claims 53 to 55, wherein the compound [ii] is the compound that inhibits an immune checkpoint molecule, preferably pembrolizumab, an antigen-binding fragment of pembrolizumab, or a compound comprising the antigen-binding fragment thereof.

57. The pharmaceutical composition according to any one of claims 53 to 56, wherein the compound that inhibits an immune checkpoint molecule is pembrolizumab, an antigen--binding fragment of pembrolizumab, or a compound comprising the antigen-binding fragment thereof, and the multikinase inhibitor is lenvatinib.

58. The pharmaceutical composition according to any one of claims 1 to 35 and 48 to 52, wherein the chemotherapeutic is a taxane-based microtubule inhibitor selected from the group consisting of paclitaxel, docetaxel, vincristine, vinblastine, vindesine, eribulin, vinorelbine, and albumin-bound paclitaxel.

59. [i] A multispecific antibody and [ii] a compound that inhibits an immune checkpoint molecule or a chemotherapeutic according to any one of claims 1 to 47 for the treatment and/or prevention of a cancer.

60. [i] A multispecific antibody and [ii] a compound that inhibits an immune checkpoint molecule or a chemotherapeutic, and a multikinase inhibitor according to any one of claims 53 to 57 for the treatment and/or prevention of a cancer.

61. [i] A multispecific antibody and [ii] a compound that inhibits an immune checkpoint molecule or a chemotherapeutic according to any one of claims 1 to 47 for the treatment and/or prevention of a cancer.

62. Use of [i] a multispecific antibody and [ii] a compound that inhibits an immune checkpoint molecule or a chemotherapeutic, and a multikinase inhibitor according to any one of claims 53 to 57 for preparing a pharmaceutical composition for the treatment and/or prevention of a cancer.

63. A method for treating and/or preventing a cancer, comprising administering [i] a multispecific antibody and [ii] a compound that inhibits an immune checkpoint molecule or a chemotherapeutic according to any one of claims 1 to 47.

64. A method for treating and/or preventing a cancer, comprising administering [i] a multispecific antibody and [ii] a compound that inhibits an immune checkpoint molecule or a chemotherapeutic, and a multikinase inhibitor according to any one of claims 53 to 57.
